(19) Europäisches Patentamt
European Patent Office
Office européen des brevets

(11) EP 0 664 802 B1

(12) **EUROPEAN PATENT SPECIFICATION**

(45) Date of publication and mention of the grant of the patent:
**15.03.2000 Bulletin 2000/11**

(21) Application number: **93914465.5**

(22) Date of filing: **16.06.1993**

(51) Int. Cl.$^7$: **C07D 401/06**, A61K 31/44, C07D 401/10, C07D 401/14

(86) International application number:
**PCT/US93/05603**

(87) International publication number:
**WO 94/08990 (28.04.1994 Gazette 1994/10)**

# (54) N-ARYLHETEROARYLALKYL 1-PHENYL-IMIDAZOL-2-ONE COMPOUNDS FOR TREATMENT OF CIRCULATORY DISORDERS

N-ARYLHETEROARYLALKYL-1-PHENYL-IMIDAZOL-2-ON VERBINDUNGEN BEI DER BEHANDLUNG VON ZIRKULATORISCHEN STÖRUNGEN

COMPOSES N-ARYLHETEROARYLALKYLE 1-PHENYLE-IMIDAZOLE-2-ONES POUR LE TRAITEMENT DES MALADIES CIRCULATOIRES

(84) Designated Contracting States:
**AT BE CH DE DK ES FR GB GR IE IT LI LU NL SE**

(30) Priority: **13.10.1992 US 960156**

(43) Date of publication of application:
**02.08.1995 Bulletin 1995/31**

(73) Proprietor: **G.D. SEARLE & CO.**
**Chicago IL 60680-5110 (US)**

(72) Inventors:
- **REITZ, David, B.**
  **Chesterfield, MO 63017 (US)**
- **MANNING, Robert, E.**
  **St. Louis, MO 63131 (US)**

(74) Representative:
**Beil, Hans Chr., Dr. et al**
**BEIL, WOLFF & BEIL,**
**Rechtsanwälte,**
**Postfach 80 01 40**
**65901 Frankfurt (DE)**

(56) References cited:
**EP-A- 0 412 494**
**EP-A- 0 475 898**
**EP-A- 0 480 204**
**EP-A- 0 508 393**
**WO-A-92/07834**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 664 802 B1

## Description

### Related Application

This is a continuation-in-part of U.S. Application Ser. No. PCT/US92/02439 filed 1 April 1992 which is a continuation-in-part of U.S. Application Ser. No. 07/681,011 filed 5 April 1991.

### Field of the Invention

Non-peptidic N-arylheteroarylalkyl imidazol-2-one compounds are described for use in treatment of circulatory disorders such as hypertension and congestive heart failure. Of particular interest are angiotensin II antagonist compounds provided by a 1-phenyl-imidazol-2-one having an arylheteroarylmethyl moiety attached at the 3-nitrogen atom of the 1-phenyl-imidazol-2-one.

### Background of the Invention

The renin-angiotensin system is one of the hormonal mechanisms involved in regulation of pressure/volume homeostasis and in expression of hypertension. Activation of the renin-angiotensin cascade begins with renin secretion from the juxtaglomerular apparatus of the kidney and culminates in the formation of angiotensin II, the primary active species of this system. This octapeptide, angiotensin II, is a potent vasoconstrictor agent and also produces other physiological effects such as promoting aldosterone secretion, promoting sodium and fluid retention, inhibiting renin secretion, increasing sympathetic nervous system activity, increasing vasopressin secretion, causing positive cardiac inotropic effect and modulating other hormonal systems.

Previous studies have shown that antagonizing angiotensin II at its receptors is a viable approach to inhibit the renin-angiotensin system, given the pivotal role of this octapeptide which mediates the actions of the renin-angiotensin system through interaction with various tissue receptors. There are several known angiotensin II antagonists, most of which are peptidic in nature. Such peptidic compounds are of limited use due to their lack of oral bioavailability or their short duration of action. Also, commercially-available peptidic angiotensin II antagonists (e.g., Saralasin) have a significant residual agonist activity which further limit their therapeutic application.

Non-peptidic compounds with angiotensin II antagonist properties are known. For example, the sodium salt of 2-n-butyl-4-chloro-1-(2-chlorobenzyl)imidazole-5-acetic acid has specific competitive angiotensin II antagonist activity as shown in a series of binding experiments, functional assays and in vivo tests [P. C. Wong et al, J. Pharmacol. Exp. Ther., 247(1), 1-7 (1988)]. Also, the sodium salt of 2-butyl-4-chloro-1-(2-nitrobenzyl)imidazole-5-acetic acid has specific competitive angiotensin II antagonist activity as shown in a series of binding experiments, functional assays and in vivo tests [A. T. Chiu et al, European J. Pharmacol., 157, 13-21 (1988)]. A family of 1-benzylimidazole-5-acetate derivatives has been shown to have competitive angiotensin II antagonist properties [A. T. Chiu et al, J. Pharmacol. Exp. Ther., 250(3), 867-874 (1989)]. U.S. Patent No. 4,816,463 to Blankey et al describes a family of 4,5,6,7-tetrahydro-1H-imidazo(4,5-c)-tetrahydro-pyridine derivatives useful as antihypertensives, some of which are reported to antagonize the binding of labelled angiotensin II to rat adrenal receptor preparation and thus cause a significant decrease in mean arterial blood pressure in conscious hypertensive rats. EP No. 253,310, published 20 January 1988, describes a series of aralkyl imidazole compounds, including in particular a family of biphenylmethyl substituted imidazoles, as antagonists to the angiotensin II receptor. EP No. 323,841 published 12 July 1989 describes four classes of angiotensin II antagonists, namely, biphenylmethylpyrroles, biphenylmethylpyrazoles, biphenylmethyl-1,2,3-triazoles and biphenylmethyl 4-substituted-4H-1,2,4-triazoles, including the compound 3,5-dibutyl-4-[(2'-carboxybiphenyl-4-yl)methyl]-4H-1,2,4-triazole. U.S. Patent No. 4,880,804 to Carini et al describes a family of biphenylmethylbenzimidazole compounds as angiotensin II receptor blockers for use in treatment of hypertension and congestive heart failure.

There are several families of known compounds having one or two oxo substituents on a triazole ring. For example, East German Patent No. 160,447 published 3 August 1983 describes a family of 1,2,4-triazolin-5-one compounds, specifically 2,4-dihydro-4,5-bis(phenylmethyl)-3H-1,2,4-triazol-3-one, for use as herbicides. Belgian Patent No. 806,146 published 16 October 1972 describes a family of triazolinone compounds, including the compound (3-(4-m-chlorophenyl-1-piperazinyl)-propyl)-3,4-diethyl-1,2,4-triazolin-5-one, having tranquilizer, hypotensive and analgesic activities. Belgian Patent No. 631,842 published 28 February 1963 describes a family of 1,2,4-triazolones having hypnotic, tranquilizer, narcotic, sedative and analgetic activities, which includes a class of 4-N-aralkyl-1,2,4-triazol-5-one compounds. EP #7,180 published 15 June 1978 describes a family of 1,2-disubstituted-4-alkyl-1,2,4-triazolidine-3,5-dione compounds having a wide variety of activities, such as antiulcer, bronchodilator, antifertility and cardiovascular-related activities which include antihypertensive, antiarrhythmic, platelet aggregation inhibition and smooth muscle activities. EP #283,310 published 18 March 1987 describes a family of $N^1$-diarylmethyl-$N^2$-aminoalkyl-diaza-heterocyclic derivatives for treating cerebral vascular and ischemic diseases and for protecting against anoxia.

EP-A 0 508 393 (only designating PT) has been filed on April 6, 1992, claiming the priority of April 1, 1992 and April 5, 1991, published on October 14, 1992, discloses N-arylheteroarylalkylimidazol-2-one compounds for treatments of circulatory disorders.

WO-A 92/17469 has been filed on April 1, 1992, claiming also the priority of April 5, 1991 and published on October 15, 1992, is also concerned with N-arylheteroarylalkylimidazol-2-one compounds for treatment of circulatory disorders.

WO-A 92/07834 discloses N-substituted biphenylalkyl compounds having angiotensin-II-antagonistic activity.

EP-A 0 480 204 describes tetrazolyl-phenyl/pyridyl-substituted imidazoles which are useful as angiotensin-II-receptor antagonists.

EP-A 0 475 898 is concerned with $N^1$-biphenyl-diaza-2-one-heterocyclic derivatives having antihypertensive activity.

EP-A 0 412 594 discloses substituted triazolinones, triazolinethiones and triazolinimines as angiotensin-II-antagonists.

DESCRIPTION OF THE INVENTION

A class of N-substituted arylheteroarylalkyl 1-phenyl-imidazol-2-one compounds useful in treating circulatory disorders, particularly cardiovascular disorders, is defined by Formula I:

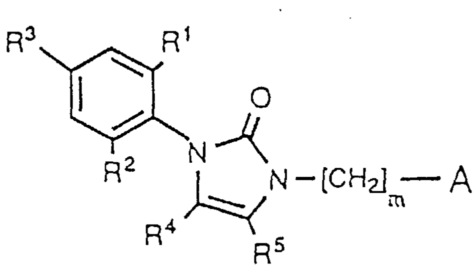

wherein A is selected from

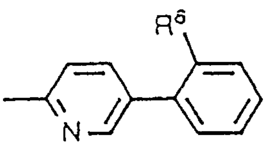
,
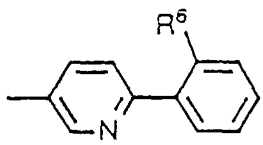
,
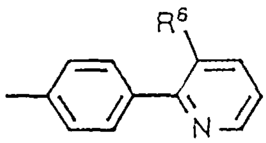

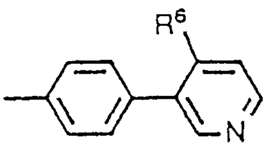
,
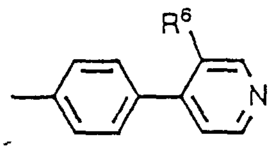
and
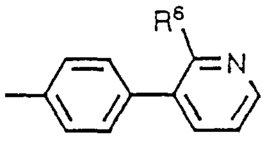
;

wherein m is a number selected from one to four, inclusive;

wherein each of $R^1$, $R^2$ and $R^3$ is independently selected from alkyl, alkoxy, cyano, halo, hydroxy, nitro, amino, carboxyl, alkoxycarbonyl, formyl, alkylcarbonyl and haloalkylcarbonyl; wherein further $R^3$ may be hydrogen wherein further when $R^1$ is ethyl or tertbutyl, then $R^2$ can be hydrogen with the proviso that $R^1$ and $R^2$ cannot be fluoro simultaneously; and with the further proviso that $R^1$ and $R^2$ cannot be chloro simultaneously; wherein $R^4$ is selected from hydrogen alkyl, halo, haloalkyl, formyl, carboxyl and alkoxyalkyl; wherein $R^5$ is selected from alkylphenyl, phenylalkyl, cycloalkyl and cycloalkylalkyl; wherein $R^6$ is an acidic group selected from COOH and

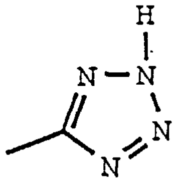

;

or a stereoisomer or a tautomer thereof or a pharmaceutically-acceptable salt thereof.

Regioisomers of compounds of Formula I are also embraced as part of the invention, particularly those regioisomers formed by various substitutions on nitrogen atoms of the imidazole ring relative to substitutions on the carbon atoms of the imidazode ring. For purposes of nomenclature, a numbering system for the imidazole ring is shown below for a preferred set of compounds of the invention within Formula I:

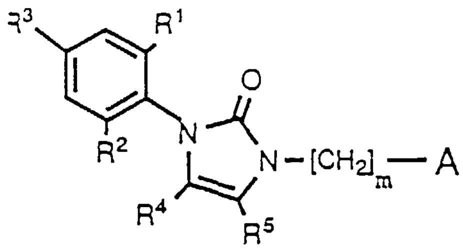

Compounds of Formula I would be useful in treating a variety of circulatory disorders and circulatory-related disorders, including cardiovascular disorders, such as hypertension, congestive heart failure and arteriosclerosis, and to treat other disorders such as glaucoma. These compounds would also be useful as adjunctive therapies. For example, compounds of Formula I may be used in combination with other drugs, such as a diuretic, to treat hypertension. Also, compounds of Formula I could be used in conjunction with certain surgical procedures. For example, these compounds could be used to prevent post-angioplasty re-stenosis. Compounds of Formula I are therapeutically effective in treatment of cardiovascular disorders by acting as antagonists to, or blockers of, the angiotensin II (AII) receptor. Compounds of Formula I would be therapeutically effective in treatment of the above-mentioned circulatory and cardiovascular disorders or would be precursors to, or prodrugs of, therapeutically-effective compounds.

Within Formula I there is a first group of compounds of more interest as represented by Formula II:

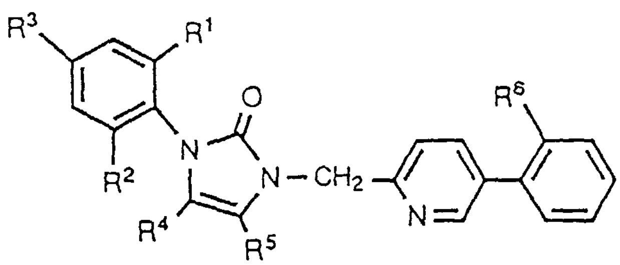

(II)

wherein each of $R^1$, $R^2$ and $R^3$ is independently selected from methyl, ethyl, n-propyl, isopropyl, tert-butyl, methoxy, ethoxy, propoxy, isopropoxy, tert-butoxy, cyano, fluoro, chloro, iodo, bromo, hydroxy, nitro, amino, N-methylamino, N,N-dimethylamino, N-ethylamino, N,N-diethylamino, carboxyl, methoxycarbonyl, ethoxycarbonyl, formyl, methylcarbonyl, ethylcarbonyl and trifluoromethylcarbonyl; wherein further $R^3$ may be hydrogen wherein further when $R^1$ is ethyl or tert-butyl, then $R^2$ can be hydrogen with the proviso that $R^1$ and $R^2$ cannot be fluoro simultaneously; and with the further proviso that $R^1$ and $R^2$ cannot be chloro simultaneously; wherein $R^4$ is selected from hydrogen methyl, fluoro, chloro, monofluoromethyl, difluoromethyl, trifluoromethyl, formyl, carboxyl and dimethoxymethyl; wherein $R^5$ is selected from methyl, ethyl, n-propyl, isopropyl, n-butyl, sec-butyl, isobutyl, tert-butyl, n-pentyl, isopentyl, neopentyl, phenyl, benzyl,

phenethyl, cyclopropyl, cyclobutyl, cyclopentyl, cyclohexyl, cyclopropylmethyl, cyclopropylethyl, cyclobutylmethyl, cyclobutylethyl, cyclopentylmethyl, cyclopentylethyl, cyclohexylmethyl and cyclohexylethyl; wherein $R^6$ is an acidic group selected from COOH and

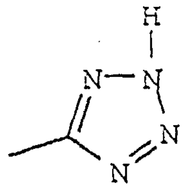

;

or a stereoisomer or a tautomer thereof or a pharmaceutically-acceptable salt thereof.

Within this first group of compounds of Formula II there is a first class of higher-interest compounds wherein $R^1$ is selected from methyl, ethyl, n-propyl, isopropyl, tert-butyl, methoxy, fluoro, chloro, iodo, bromo, carboxyl, formyl, methylcarbonyl and trifluoromethylcarbonyl; wherein each of $R^2$, $R^3$ and $R^4$ is hydrogen wherein $R^5$ is selected from methyl, ethyl, n-propyl, isopropyl, n-butyl, sec-butyl, isobutyl, tert-butyl, n-pentyl, isopentyl and neopentyl; wherein $R^6$ is an acidic group selected from COOH and

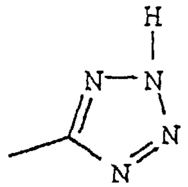

;

or a stereoisomer or a tautomer thereof or a pharmaceutically-acceptable salt thereof.

A family of specific compounds of particular interest within this first class of higher-interest compounds of the first group of compounds of Formula II consists of compounds and their stereoisomers and tautomers and the pharmaceutically-acceptable salts thereof, said compounds consisting of

1-(2-ethylphenyl)-4-propyl-1,3-dihydro-3-[[5-[2-(1H-tetrazol-5-yl)phenyl]-2-pyridinyl]methyl]-2H-imidazol-2-one;
1-(2-tertbutylphenyl)-4-propyl-1,3-dihydro-3-[[5-[2-(1H-tetrazol-5-yl)phenyl]-2-pyridinyl]methyl]-2H-imidazol-2-one;
1-(2-ethylphenyl)-4-butyl-1,3-dihydro-3-[[5-[2-(1H-tetrazol-5-yl)phenyl]-2-pyridinyl]methyl]-2H-imidazol-2-one;
1-(2-tertbutylphenyl)-4-butyl-1,3-dihydro-3-[[5-[2-(1H-tetrazol-5-yl)phenyl]-2-pyridinyl]methyl]-2H-imidazol-2-one;

Within this first group of compounds of Formula II there is a second class of higher-interest compounds wherein each of $R^1$ and $R^2$ is independently selected from methyl, ethyl, n-propyl, isopropyl, tert-butyl, methoxy, fluoro, chloro, iodo, bromo, carboxyl, formyl, methylcarbonyl and trifluoromethylcarbonyl; wherein each of $R^3$ and $R^4$ is hydrido; wherein $R^5$ is selected from methyl, ethyl, n-propyl, isopropyl, n-butyl, sec-butyl, isobutyl, tert-butyl, n-pentyl, isopentyl and neopentyl; wherein $R^6$ is an acidic group selected from COOH and

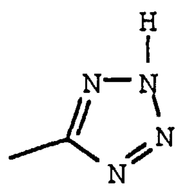

;

or a stereoisomer or a tautomer thereof or a pharmaceutically-acceptable salt thereof.

A family of specific compounds of particular interest within this second class of higher-interest compounds of the first group of compounds of Formula II consists of compounds and their stereoisomers and tautomers and the pharmaceutically-acceptable salts thereof, said compounds consisting of

1-(2,6-dimethylphenyl)-4-propyl-1,3-dihydro-3-[[5-[2-(1H-tetrazol-5-yl)phenyl]-2-pyridinyl]methyl]-2H-imidazol-2-one;

1-(2-methyl-6-ethylphenyl)-4-propyl-1,3-dihydro-3-[[5-[2-(1H-tetrazol-5-yl)phenyl]-2-pyridinyl]methyl]-2H-imidazol-2-one;

1-(2-methyl-6-isopropylphenyl)-4-propyl-1,3-dihydro-3-[[5-[2-(1H-tetrazol-5-yl)phenyl]-2-pyridinyl]methyl]-2H-imidazol-2-one;

1-(2-methyl-6-tertbutylphenyl)-4-propyl-1,3-dihydro-3-[[5-[2-(1H-tetrazol-5-yl)phenyl]-2-pyridinyl]methyl]-2H-imidazol-2-one;

1-(2-methyl-6-fluorophenyl)-4-propyl-1,3-dihydro-3-[[5-[2-(1H-tetrazol-5-yl)phenyl]-2-pyridinyl]methyl]-2H-imidazol-2-one;

1-(2-methyl-6-chlorophenyl)-4-propyl-1,3-dihydro-3-[[5-[2-(1H-tetrazol-5-yl)phenyl]-2-pyridinyl]methyl]-2H-imidazol-2-one;

1-(2-methyl-6-methoxyphenyl)-4-propyl-1,3-dihydro-3-[[5-[2-(1H-tetrazol-5-yl)phenyl]-2-pyridinyl]methyl]-2H-imidazol-2-one;

1-(2-ethyl-6-methylphenyl)-4-propyl-1,3-dihydro-3-[[5-[2-(1H-tetrazol-5-yl)phenyl]-2-pyridinyl]methyl]-2H-imidazol-2-one;

1-(2,6-diethylphenyl)-4-propyl-1,3-dihydro-3-[[5-[2-(1H-tetrazol-5-yl)phenyl]-2-pyridinyl]methyl]-2H-imidazol-2-one;

1-(2-ethyl-6-isopropylphenyl)-4-propyl-1,3-dihydro-3-[[5-[2-(1H-tetrazol-5-yl)phenyl]-2-pyridinyl]methyl]-2H-imidazol-2-one;

1-(2-ethyl-6-tertbutylphenyl)-4-propyl-1,3-dihydro-3-[[5-[2-(1H-tetrazol-5-yl)phenyl]-2-pyridinyl]methyl]-2H-imidazol-2-one;

1-(2-ethyl-6-fluorophenyl)-4-propyl-1,3-dihydro-3-[[5-[2-(1H-tetrazol-5-yl)phenyl]-2-pyridinyl]methyl]-2H-imidazol-2-one;

1-(2-ethyl-6-chlorophenyl)-4-propyl-1,3-dihydro-3-[[5-[2-(1H-tetrazol-5-yl)phenyl]-2-pyridinyl]methyl]-2H-imidazol-2-one;

1-(2-ethyl-6-methoxyphenyl)-4-propyl-1,3-dihydro-3-[[5-[2-(1H-tetrazol-5-yl)phenyl]-2-pyridinyl]methyl]-2H-imidazol-2-one;

1-(2-propyl-6-methylphenyl)-4-propyl-1,3-dihydro-3-[[5-[2-(1H-tetrazol-5-yl)phenyl]-2-pyridinyl]methyl]-2H-imidazol-2-one;

1-(2-propyl-6-ethylphenyl)-4-propyl-1,3-dihydro-3-[[5-[2-(1H-tetrazol-5-yl)phenyl]-2-pyridinyl]methyl]-2H-imidazol-2-one;

1-(2-propyl-6-isopropylphenyl)-4-propyl-1,3-dihydro-3-[[5-[2-(1H-tetrazol-5-yl)phenyl]-2-pyridinyl]methyl]-2H-imidazol-2-one;

1-(2-propyl-6-tertbutylphenyl)-4-propyl-1,3-dihydro-3-[[5-[2-(1H-tetrazol-5-yl)phenyl]-2-pyridinyl]methyl]-2H-imidazol-2-one;

1-(2-propyl-6-fluorophenyl)-4-propyl-1,3-dihydro-3-[[5-[2-(1H-tetrazol-5-yl)phenyl]-2-pyridinyl]methyl]-2H-imidazol-2-one;

1-(2-propyl-6-chlorophenyl)-4-propyl-1,3-dihydro-3-[[5-[2-(1H-tetrazol-5-yl)phenyl]-2-pyridinyl]methyl]-2H-imidazol-2-one;

1-(2-propyl-6-methoxyphenyl)-4-propyl-1,3-dihydro-3-[[5-[2-(1H-tetrazol-5-yl)phenyl]-2-pyridinyl]methyl]-2H-imidazol-2-one;

1-(2-isopropyl-6-methylphenyl)-4-propyl-1,3-dihydro-3-[[5-[2-(1H-tetrazol-5-yl)phenyl]-2-pyridinyl]methyl]-2H-imidazol-2-one;

1-(2-isopropyl-6-ethylphenyl)-4-propyl-1,3-dihydro-3-[[5-[2-(1H-tetrazol-5-yl)phenyl]-2-pyridinyl]methyl]-2H-imidazol-2-one;

1-(2,6-diisopropylphenyl)-4-propyl-1,3-dihydro-3-[[5-[2-(1H-tetrazol-5-yl)phenyl]-2-pyridinyl]methyl]-2H-imidazol-2-one;

1-(2-isopropyl-6-tertbutylphenyl)-4-propyl-1,3-dihydro-3-[[5-[2-(1H-tetrazol-5-yl)phenyl]-2-pyridinyl]methyl]-2H-imidazol-2-one;

1-(2-isopropyl-6-fluorophenyl)-4-propyl-1,3-dihydro-3-[[5-[2-(1H-tetrazol-5-yl)phenyl]-2-pyridinyl]methyl]-2H-imidazol-2-one;

1-(2-isopropyl-6-chlorophenyl)-4-propyl-1,3-dihydro-3-[[5-[2-(1H-tetrazol-5-yl)phenyl]-2-pyridinyl]methyl]-2H-imidazol-2-one;

1-(2-isopropyl-6-methoxyphenyl)-4-propyl-1,3-dihydro-3-[[5-[2-(1H-tetrazol-5-yl)phenyl]-2-pyridinyl]methyl]-2H-imidazol-2-one;

1-(2-tertbutyl-6-methylphenyl)-4-propyl-1,3-dihydro-3-[[5-[2-(1H-tetrazol-5-yl)phenyl]-2-pyridinyl]methyl]-2H-imidazol-2-one;

1-(2-tertbutyl-6-ethylphenyl)-4-propyl-1,3-dihydro-3-[[5-[2-(1H-tetrazol-5-yl)phenyl]-2-pyridinyl]methyl]-2H-imidazol-2-one;

1-(2-tertbutyl-6-isopropylphenyl)-4-propyl-1,3-dihydro-3-[[5-[2-(1H-tetrazol-5-yl)phenyl]-2-pyridinyl]methyl]-2H-imidazol-2-one;

1-(2,6-ditertbutylphenyl)-4-propyl-1,3-dihydro-3-[[5-[2-(1H-tetrazol-5-yl)phenyl]-2-pyridinyl]methyl]-2H-imidazol-2-one;

1-(2-tertbutyl-6-fluorophenyl)-4-propyl-1,3-dihydro-3-[[5-[2-(1H-tetrazol-5-yl)phenyl]-2-pyridinyl]methyl]-2H-imidazol-2-one;

1-(2-tertbutyl-6-chlorophenyl)-4-propyl-1,3-dihydro-3-[[5-[2-(1H-tetrazol-5-yl)phenyl]-2-pyridinyl]methyl]-2H-imidazol-2-one;

1-(2-tertbutyl-6-methoxyphenyl)-4-propyl-1,3-dihydro-3-[[5-[2-(1H-tetrazol-5-yl)phenyl]-2-pyridinyl]methyl]-2H-imidazol-2-one;

1-(2-fluoro-6-methylphenyl)-4-propyl-1,3-dihydro-3-[[5-[2-(1H-tetrazol-5-yl)phenyl]-2-pyridinyl]methyl]-2H-imidazol-2-one;

1-(2-fluoro-6-ethylphenyl)-4-propyl-1,3-dihydro-3-[[5-[2-(1H-tetrazol-5-yl)phenyl]-2-pyridinyl]methyl]-2H-imidazol-2-one;

1-(2-fluoro-6-isopropylphenyl)-4-propyl-1,3-dihydro-3-[[5-[2-(1H-tetrazol-5-yl)phenyl]-2-pyridinyl]methyl]-2H-imidazol-2-one;

1-(2-fluoro-6-tertbutylphenyl)-4-propyl-1,3-dihydro-3-[[5-[2-(1H-tetrazol-5-yl)phenyl]-2-pyridinyl]methyl]-2H-imidazol-2-one;

1-(2,6-difluorophenyl)-4-propyl-1,3-dihydro-3-[[5-[2-(1H-tetrazol-5-yl)phenyl]-2-pyridinyl]methyl]-2H-imidazol-2-one;

1-(2-fluoro-6-chlorophenyl)-4-propyl-1,3-dihydro-3-[[5-[2-(1H-tetrazol-5-yl)phenyl]-2-pyridinyl]methyl]-2H-imidazol-2-one;

1-(2-fluoro-6-methoxyphenyl)-4-propyl-1,3-dihydro-3-[[5-[2-(1H-tetrazol-5-yl)phenyl]-2-pyridinyl]methyl]-2H-imidazol-2-one;

1-(2-chloro-6-methylphenyl)-4-propyl-1,3-dihydro-3-[[5-[2-(1H-tetrazol-5-yl)phenyl]-2-pyridinyl]methyl]-2H-imidazol-2-one;

1-(2-chloro-6-ethylphenyl)-4-propyl-1,3-dihydro-3-[[5-[2-(1H-tetrazol-5-yl)phenyl]-2-pyridinyl]methyl]-2H-imidazol-2-one;

1-(2-chloro-6-isopropylphenyl)-4-propyl-1,3-dihydro-3-[[5-[2-(1H-tetrazol-5-yl)phenyl]-2-pyridinyl]methyl]-2H-imidazol-2-one;

1-(2-chloro-6-tertbutylphenyl)-4-propyl-1,3-dihydro-3-[[5-[2-(1H-tetrazol-5-yl)phenyl]-2-pyridinyl]methyl]-2H-imidazol-2-one;

1-(2-chloro-6-fluorophenyl)-4-propyl-1,3-dihydro-3-[[5-[2-(1H-tetrazol-5-yl)phenyl]-2-pyridinyl]methyl]-2H-imidazol-2-one;

1-(2-chloro-6-methoxyphenyl)-4-propyl-1,3-dihydro-3-[[5-[2-(1H-tetrazol-5-yl)phenyl]-2-pyridinyl]methyl]-2H-imidazol-2-one;

1-(2-bromo-6-methylphenyl)-4-propyl-1,3-dihydro-3-[[5-[2-(1H-tetrazol-5-yl)phenyl]-2-pyridinyl]methyl]-2H-imidazol-2-one;

1-(2-bromo-6-ethylphenyl)-4-propyl-1,3-dihydro-3-[[5-[2-(1H-tetrazol-5-yl)phenyl]-2-pyridinyl]methyl]-2H-imidazol-2-one;

1-(2-bromo-6-isopropylphenyl)-4-propyl-1,3-dihydro-3-[[5-[2-(1H-tetrazol-5-yl)phenyl]-2-pyridinyl]methyl]-2H-imidazol-2-one;

1-(2-bromo-6-tertbutylphenyl)-4-propyl-1,3-dihydro-3-[[5-[2-(1H-tetrazol-5-yl)phenyl]-2-pyridinyl]methyl]-2H-imidazol-2-one;

1-(2-bromo-6-fluorophenyl)-4-propyl-1,3-dihydro-3-[[5-[2-(1H-tetrazol-5-yl)phenyl]-2-pyridinyl]methyl]-2H-imidazol-2-one;

1-(2-bromo-6-chlorophenyl)-4-propyl-1,3-dihydro-3-[[5-[2-(1H-tetrazol-5-yl)phenyl]-2-pyridinyl]methyl]-2H-imidazol-2-one;

1-(2-bromo-6-methoxyphenyl)-4-propyl-1,3-dihydro-3-[[5-[2-(1H-tetrazol-5-yl)phenyl]-2-pyridinyl]methyl]-2H-imidazol-2-one;

1-(2-methoxy-6-methylphenyl)-4-propyl-1,3-dihydro-3-[[5-[2-(1H-tetrazol-5-yl)phenyl]-2-pyridinyl]methyl]-2H-imidazol-2-one;

1-(2-methoxy-6-ethylphenyl)-4-propyl-1,3-dihydro-3-[[5-[2-(1H-tetrazol-5-yl)phenyl]-2-pyridinyl]methyl]-2H-imidazol-2-one;

1-(2-methoxy-6-isopropylphenyl)-4-propyl-1,3-dihydro-3-[[5-[2-(1H-tetrazol-5-yl)phenyl]-2-pyridinyl]methyl]-2H-imidazol-2-one;

1-(2-methoxy-6-tertbutylphenyl)-4-propyl-1,3-dihydro-3-[[5-[2-(1H-tetrazol-5-yl)phenyl]-2-pyridinyl]methyl]-2H-imidazol-2-one;

1-(2-methoxy-6-fluorophenyl)-4-propyl-1,3-dihydro-3-[[5-[2-(1H-tetrazol-5-yl)phenyl]-2-pyridinyl]methyl]-2H-imidazol-2-one;

1-(2-methoxy-6-chlorophenyl)-4-propyl-1,3-dihydro-3-[[5-[2-(1H-tetrazol-5-yl)phenyl]-2-pyridinyl]methyl]-2H-imidazol-2-one;

1-(2,6-dimethoxyphenyl)-4-propyl-1,3-dihydro-3-[[5-[2-(1H-tetrazol-5-yl)phenyl]-2-pyridinyl]methyl]-2H-imidazol-2-one;

1-(2-carboxy-6-methylphenyl)-4-propyl-1,3-dihydro-3-[[5-[2-(1H-tetrazol-5-yl)phenyl]-2-pyridinyl]methyl]-2H-imidazol-2-one;

1-(2-carboxy-6-ethylphenyl)-4-propyl-1,3-dihydro-3-[[5-[2-(1H-tetrazol-5-yl)phenyl]-2-pyridinyl]methyl]-2H-imidazol-2-one;

1-(2-carboxy-6-isopropylphenyl)-4-propyl-1,3-dihydro-3-[[5-[2-(1H-tetrazol-5-yl)phenyl]-2-pyridinyl]methyl]-2H-imidazol-2-one;

1-(2-carboxy-6-tertbutylphenyl)-4-propyl-1,3-dihydro-3-[[5-[2-(1H-tetrazol-5-yl)phenyl]-2-pyridinyl]methyl]-2H-imidazol-2-one;

1-(2-carboxy-6-fluorophenyl)-4-propyl-1,3-dihydro-3-[[5-[2-(1H-tetrazol-5-yl)phenyl]-2-pyridinyl]methyl]-2H-imidazol-2-one;

1-(2-carboxy-6-chlorophenyl)-4-propyl-1,3-dihydro-3-[[5-[2-(1H-tetrazol-5-yl)phenyl]-2-pyridinyl]methyl]-2H-imidazol-2-one;

1-(2-carboxy-6-methoxyphenyl)-4-propyl-1,3-dihydro-3-[[5-[2-(1H-tetrazol-5-yl)phenyl]-2-pyridinyl]methyl]-2H-imidazol-2-one;

1-(2-acetyl-6-methylphenyl)-4-propyl-1,3-dihydro-3-[[5-[2-(1H-tetrazol-5-yl)phenyl]-2-pyridinyl]methyl]-2H-imidazol-2-one;

1-(2-acetyl-6-ethylphenyl)-4-propyl-1,3-dihydro-3-[[5-[2-(1H-tetrazol-5-yl)phenyl]-2-pyridinyl]methyl]-2H-imidazol-2-one;

1-(2-acetyl-6-isopropylphenyl)-4-propyl-1,3-dihydro-3-[[5-[2-(1H-tetrazol-5-yl)phenyl]-2-pyridinyl]methyl]-2H-imidazol-2-one;

1-(2-acetyl-6-tertbutylphenyl)-4-propyl-1,3-dihydro-3-[[5-[2-(1H-tetrazol-5-yl)phenyl]-2-pyridinyl]methyl]-2H-imidazol-2-one;

1-(2-acetyl-6-fluorophenyl)-4-propyl-1,3-dihydro-3-[[5-[2-(1H-tetrazol-5-yl)phenyl]-2-pyridinyl]methyl]-2H-imidazol-2-one;

1-(2-acetyl-6-chlorophenyl)-4-propyl-1,3-dihydro-3-[[5-[2-(1H-tetrazol-5-yl)phenyl]-2-pyridinyl]methyl]-2H-imidazol-2-one;

1-(2-acetyl-6-methoxyphenyl)-4-propyl-1,3-dihydro-3-[[5-[2-(1H-tetrazol-5-yl)phenyl]-2-pyridinyl]methyl]-2H-imidazol-2-one;

1-(2,6-dimethylphenyl)-4-butyl-1,3-dihydro-3-[[5-[2-(1H-tetrazol-5-yl)phenyl]-2-pyridinyl]methyl]-2H-imidazol-2-one;

1-(2-methyl-6-ethylphenyl)-4-butyl-1,3-dihydro-3-[[5-[2-(1H-tetrazol-5-yl)phenyl]-2-pyridinyl]methyl]-2H-imidazol-2-one;

1-(2-methyl-6-isopropylphenyl)-4-butyl-1,3-dihydro-3-[[5-[2-(1H-tetrazol-5-yl)phenyl]-2-pyridinyl]methyl]-2H-imidazol-2-one;

1-(2-methyl-6-tertbutylphenyl)-4-butyl-1,3-dihydro-3-[[5-[2-(1H-tetrazol-5-yl)phenyl]-2-pyridinyl]methyl]-2H-imidazol-2-one;

1-(2-methyl-6-fluorophenyl)-4-butyl-1,3-dihydro-3-[[5-[2-(1H-tetrazol-5-yl)phenyl]-2-pyridinyl]methyl]-2H-imidazol-2-one;

1-(2-methyl-6-chlorophenyl)-4-butyl-1,3-dihydro-3-[[5-[2-(1H-tetrazol-5-yl)phenyl]-2-pyridinyl]methyl]-2H-imidazol-2-one;

1-(2-methyl-6-methoxyphenyl)-4-butyl-1,3-dihydro-3-[[5-[2-(1H-tetrazol-5-yl)phenyl]-2-pyridinyl]methyl]-2H-imidazol-2-one;

1-(2-ethyl-6-methylphenyl)-4-butyl-1,3-dihydro-3-[[5-[2-(1H-tetrazol-5-yl)phenyl]-2-pyridinyl]methyl]-2H-imidazol-2-one;

1-(2,6-diethylphenyl)-4-butyl-1,3-dihydro-3-[[5-[2-(1H-tetrazol-5-yl)phenyl]-2-pyridinyl]methyl]-2H-imnidazol-2-one;

1-(2-ethyl-6-isopropylphenyl)-4-butyl-1,3-dihydro-3-[[5-[2-(1H-tetrazol-5-yl)phenyl]-2-pyridinyl]methyl]-2H-imidazol-2-one;

1-(2-ethyl-6-tertbutylphenyl)-4-butyl-1,3-dihydro-3-[[5-[2-(1H-tetrazol-5-yl)phenyl]-2-pyridinyl]methyl]-2H-imidazol-2-one;

1-(2-ethyl-6-fluorophenyl)-4-butyl-1,3-dihydro-3-[[5-[2-(1H-tetrazol-5-yl)phenyl]-2-pyridinyl]methyl]-2H-imidazol-2-one;

1-(2-ethyl-6-chlorophenyl)-4-butyl-1,3-dihydro-3-[[5-[2-(1H-tetrazol-5-yl)phenyl]-2-pyridinyl]methyl]-2H-imidazol-2-one;
1-(2-ethyl-6-methoxyphenyl)-4-butyl-1,3-dihydro-3-[[5-[2-(1H-tetrazol-5-yl)phenyl]-2-pyridinyl]methyl]-2H-imidazol-2-one;
1-(2-propyl-6-methylphenyl)-4-butyl-1,3-dihydro-3-[[5-[2-(1H-tetrazol-5-yl)phenyl]-2-pyridinyl]methyl]-2H-imidazol-2-one;
1-(2-propyl-6-ethylphenyl)-4-butyl-1,3-dihydro-3-[[5-[2-(1H-tetrazol-5-yl)phenyl]-2-pyridinyl]methyl]-2H-imidazol-2-one;
1-(2-propyl-6-isopropylphenyl)-4-butyl-1,3-dihydro-3-[[5-[2-(1H-tetrazol-5-yl)phenyl]-2-pyridinyl]methyl]-2H-imidazol-2-one;
1-(2-propyl-6-tertbutylphenyl)-4-butyl-1,3-dihydro-3-[[5-[2-(1H-tetrazol-5-yl)phenyl]-2-pyridinyl]methyl]-2H-imidazol-2-one;
1-(2-propyl-6-fluorophenyl)-4-butyl-1,3-dihydro-3-[[5-[2-(1H-tetrazol-5-yl)phenyl]-2-pyridinyl]methyl]-2H-imidazol-2-one;
1-(2-propyl-6-chlorophenyl)-4-butyl-1,3-dihydro-3-[[5-[2-(1H-tetrazol-5-yl)phenyl]-2-pyridinyl]methyl]-2H-imidazol-2-one;
1-(2-propyl-6-methoxyphenyl)-4-butyl-1,3-dihydro-3-[[5-[2-(1H-tetrazol-5-yl)phenyl]-2-pyridinyl]methyl]-2H-imidazol-2-one;
1-(2-isopropyl-6-methylphenyl)-4-butyl-1,3-dihydro-3-[[5-[2-(1H-tetrazol-5-yl)phenyl]-2-pyridinyl]methyl]-2H-imidazol-2-one;
1-(2-isopropyl-6-ethylphenyl)-4-butyl-1,3-dihydro-3-[[5-[2-(1H-tetrazol-5-yl)phenyl]-2-pyridinyl]methyl]-2H-imidazol-2-one;
1-(2,6-diisopropylphenyl)-4-butyl-1,3-dihydro-3-[[5-[2-(1H-tetrazol-5-yl)phenyl]-2-pyridinyl]methyl]-2H-imidazol-2-one;
1-(2-isopropyl-6-tertbutylphenyl)-4-butyl-1,3-dihydro-3-[[5-[2-(1H-tetrazol-5-yl)phenyl]-2-pyridinyl]methyl]-2H-imidazol-2-one;
1-(2-isopropyl-6-fluorophenyl)-4-butyl-1,3-dihydro-3-[[5-[2-(1H-tetrazol-5-yl)phenyl]-2-pyridinyl]methyl]-2H-imidazol-2-one;
1-(2-isopropyl-6-chlorophenyl)-4-butyl-1,3-dihydro-3-[[5-[2-(1H-tetrazol-5-yl)phenyl]-2-pyridinyl]methyl]-2H-imidazol-2-one;
1-(2-isopropyl-6-methoxyphenyl)-4-butyl-1,3-dihydro-3-[[5-[2-(1H-tetrazol-5-yl)phenyl]-2-pyridinyl]methyl]-2H-imidazol-2-one;
1-(2-tertbutyl-6-methylphenyl)-4-butyl-1,3-dihydro-3-[[5-[2-(1H-tetrazol-5-yl)phenyl]-2-pyridinyl]methyl]-2H-imidazol-2-one;
1-(2-tertbutyl-6-ethylphenyl)-4-butyl-1,3-dihydro-3-[[5-[2-(1H-tetrazol-5-yl)phenyl]-2-pyridinyl]methyl]-2H-imidazol-2-one;
1-(2-tertbutyl-6-isopropylphenyl)-4-butyl-1,3-dihydro-3-[[5-[2-(1H-tetrazol-5-yl)phenyl]-2-pyridinyl]methyl]-2H-imidazol-2-one;
1-(2,6-ditertbutylphenyl)-4-butyl-1,3-dihydro-3-[[5-[2-(1H-tetrazol-5-yl)phenyl]-2-pyridinyl]methyl]-2H-imidazol-2-one;
1-(2-tertbutyl-6-fluorophenyl)-4-butyl-1,3-dihydro-3-[[5-[2-(1H-tetrazol-5-yl)phenyl]-2-pyridiny]methyl]-2H-imidazol-2-one;
1-(2-tertbutyl-6-chlorophenyl)-4-butyl-1,3-dihydro-3-[[5-[2-(1H-tetrazol-5-yl)phenyl]-2-pyridinyl]methyl]-2H-imidazol-2-one;
1-(2-tertbutyl-6-methoxyphenyl)-4-butyl-1,3-dihydro-3-[[5-[2-(1H-tetrazol-5-yl)phenyl]-2-pyridinyl]methyl]-2H-imidazol-2-one;
1-(2-fluoro-6-methylphenyl)-4-butyl-1,3-dihydro-3-[[5-[2-(1H-tetrazol-5-yl)phenyl]-2-pyridinyl]methyl]-2H-imidazol-2-one;
1-(2-fluoro-6-ethylphenyl)-4-butyl-1,3-dihydro-3-[[5-[2-(1H-tetrazol-5-yl)phenyl]-2-pyridinyl]methyl]-2H-imidazol-2-one;
1-(2-fluoro-6-isopropylphenyl)-4-butyl-1,3-dihydro-3-[[5-[2-(1H-tetrazol-5-yl)phenyl]-2-pyridinyl]methyl]-2H-imidazol-2-one;
1-(2-fluoro-6-tertbutylphenyl)-4-butyl-1,3-dihydro-3-[[5-[2-(1H-tetrazol-5-yl)phenyl]-2-pyridinyl]methyl]-2H-imidazol-2-one;
1-(2,6-difluorophenyl)-4-butyl-1,3-dihydro-3-[[5-[2-(1H-tetrazol-5-yl)phenyl]-2-pyridinyl]methyl]-2H-imidazol-2-one;
1-(2-fluoro-6-chlorophenyl)-4-butyl-1,3-dihydro-3-[[5-[2-(1H-tetrazol-5-yl)phenyl]-2-pyridinyl]methyl]-2H-imidazol-2-one;
1-(2-fluoro-6-methoxyphenyl)-4-butyl-1,3-dihydro-3-[[5-[2-(1H-tetrazol-5-yl)phenyl]-2-pyridinyl]methyl]-2H-imidazol-2-one;

1-(2-chloro-6-methylphenyl)-4-butyl-1,3-dihydro-3-[[5-[2-(1H-tetrazol-5-yl)phenyl]-2-pyridinyl]methyl]-2H-imidazol-2-one;

1-(2-chloro-6-ethylphenyl)-4-butyl-1,3-dihydro-3-[[5-[2-(1H-tetrazol-5-yl)phenyl]-2-pyridinyl]methyl]-2H-imidazol-2-one;

1-(2-chloro-6-isopropylphenyl)-4-butyl-1,3-dihydro-3-[[5-[2-(1H-tetrazol-5-yl)phenyl]-2-pyridinyl]methyl]-2H-imidazol-2-one;

1-(2-chloro-6-tertbutylphenyl)-4-butyl-1,3-dihydro-3-[[5-[2-(1H-tetrazol-5-yl)phenyl]-2-pyridinyl]methyl]-2H-imidazol-2-one;

1-(2-chloro-6-fluorophenyl)-4-butyl-1,3-dihydro-3-[[5-[2-(1H-tetrazol-5-yl)phenyl]-2-pyridinyl]methyl]-2H-imidazol-2-one;

1-(2-chloro-6-methoxyphenyl)-4-butyl-1,3-dihydro-3-[[5-[2-(1H-tetrazol-5-yl)phenyl]-2-pyridinyl]methyl]-2H-imidazol-2-one;

1-(2-bromo-6-methylphenyl)-4-butyl-1,3-dihydro-3-[[5-[2-(1H-tetrazol-5-yl)phenyl]-2-pyridinyl]methyl]-2H-imidazol-2-one;

1-(2-bromo-6-ethylphenyl)-4-butyl-1,3-dihydro-3-[[5-[2-(1H-tetrazol-5-yl)phenyl]-2-pyridinyl]methyl]-2H-imidazol-2-one;

1-(2-bromo-6-isopropylphenyl)-4-butyl-1,3-dihydro-3-[[5-[2-(1H-tetrazol-5-yl)phenyl]-2-pyridinyl]methyl]-2H-imidazol-2-one;

1-(2-bromo-6-tertbutylphenyl)-4-butyl-1,3-dihydro-3-[[5-[2-(1H-tetrazol-5-yl)phenyl]-2-pyridinyl]methyl]-2H-imidazol-2-one;

1-(2-bromo-6-fluorophenyl)-4-butyl-1,3-dihydro-3-[[5-[2-(1H-tetrazol-5-yl)phenyl]-2-pyridinyl]methyl]-2H-imidazol-2-one;

1-(2-bromo-6-chlorophenyl)-4-butyl-1,3-dihydro-3-[[5-[2-(1H-tetrazol-5-yl)phenyl]-2-pyridinyl]methyl]-2H-imidazol-2-one;

1-(2-bromo-6-methoxyphenyl)-4-butyl-1,3-dihydro-3-[[5-[2-(1H-tetrazol-5-yl)phenyl]-2-pyridinyl]methyl]-2H-imidazol-2-one;

1-(2-methoxy-6-methylphenyl)-4-butyl-1,3-dihydro-3-[[5-[2-(1H-tetrazol-5-yl)phenyl]-2-pyridinyl]methyl]-2H-imidazol-2-one;

1-(2-methoxy-6-ethylphenyl)-4-butyl-1,3-dihydro-3-[[5-[2-(1H-tetrazol-5-yl)phenyl]-2-pyridinyl]methyl]-2H-imidazol-2-one;

1-(2-methoxy-6-isopropylphenyl)-4-butyl-1,3-dihydro-3-[[5-[2-(1H-tetrazol-5-yl)phenyl]-2-pyridinyl]methyl]-2H-imidazol-2-one;

1-(2-methoxy-6-tertbutylphenyl)-4-butyl-1,3-dihydro-3-[[5-[2-(1H-tetrazol-5-yl)phenyl]-2-pyridinyl)methyl]-2H-imidazol-2-one;

1-(2-methoxy-6-fluorophenyl)-4-butyl-1,3-dihydro-3-[[5-[2-(1H-tetrazol-5-yl)phenyl]-2-pyridinyl]methyl]-2H-imidazol-2-one;

1-(2-methoxy-6-chlorophenyl)-4-butyl-1,3-dihydro-3-[[5-[2-(1H-tetrazol-5-yl)phenyl]-2-pyridinyl]methyl]-2H-imidazol-2-one;

1-(2,6-dimethoxyphenyl)-4-butyl-1,3-dihydro-3-[[5-[2-(1H-tetrazol-5-yl)phenyl]-2-pyridinyl]methyl]-2H-imidazol-2-one;

1-(2-carboxy-6-methylphenyl)-4-butyl-1,3-dihydro-3-[[5-[2-(1H-tetrazol-5-yl)phenyl]-2-pyridinyl]methyl]-2H-imidazol-2-one;

1-(2-carboxy-6-ethylphenyl)-4-butyl-1,3-dihydro-3-[[5-[2-(1H-tetrazol-5-yl)phenyl]-2-pyridinyl]methyl]-2H-imidazol-2-one;

1-(2-carboxy-6-isopropylphenyl)-4-butyl-1,3-dihydro-3-[[5-[2-(1H-tetrazol-5-yl)phenyl]-2-pyridinyl]methyl]-2H-imidazol-2-one;

1-(2-carboxy-6-tertbutylphenyl)-4-butyl-1,3-dihydro-3-[[5-[2-(1H-tetrazol-5-yl)phenyl]-2-pyridinyl]methyl]-2H-imidazol-2-one;

1-(2-carboxy-6-fluorophenyl)-4-butyl-1,3-dihydro-3-[[5-[2-(1H-tetrazol-5-yl)phenyl]-2-pyridinyl]methyl]-2H-imidazol-2-one;

1-(2-carboxy-6-chlorophenyl)-4-butyl-1,3-dihydro-3-[[5-[2-(1H-tetrazol-5-yl)phenyl]-2-pyridinyl]methyl]-2H-imidazol-2-one;

1-(2-carboxy-6-methoxyphenyl)-4-butyl-1,3-dihydro-3-[[5-[2-(1H-tetrazol-5-yl)phenyl]-2-pyridinyl]methyl]-2H-imidazol-2-one;

1-(2-acecyl-6-methylphenyl)-4-butyl-1,3-dihydro-3-[[5-[2-(1H-tetrazol-5-yl)phenyl]-2-pyridinyl]methyl]-2H-imidazol-2-one;

1-(2-acetyl-6-ethylphenyl)-4-butyl-1,3-dihydro-3-[[5-[2-(1H-tetrazol-5-yl)phenyl]-2-pyridinyl]methyl]-2H-imidazol-2-one;
1-(2-acetyl-6-isopropylphenyl)-4-butyl-1,3-dihydro-3-[[5-[2-(1H-tetrazol-5-yl)phenyl]-2-pyridinyl]methyl]-2H-imidazol-2-one;
1-(2-acetyl-6-tertbutylphenyl)-4-butyl-1,3-dihydro-3-[[5-[2-(1H-tetrazol-5-yl)phenyl]-2-pyridinyl]methyl]-2H-imidazol-2-one;
1-(2-acetyl-6-fluorophenyl)-4-butyl-1,3-dihydro-3-[[5-[2-(1H-tetrazol-5-yl)phenyl]-2-pyridinyl]methyl]-2H-imidazol-2-one;
1-(2-acetyl-6-chlorophenyl)-4-butyl-1,3-dihydro-3-[[5-[2-(1H-tetrazol-5-yl)phenyl]-2-pyridinyl]methyl]-2H-imidazol-2-one; and
1-(2-acetyl-6-methoxyphenyl)-4-butyl-1,3-dihydro-3-[[5-[2-(1H-tetrazol-5-yl)phenyl]-2-pyridinyl]methyl]-2H-imidazol-2-one.

Within Formula I there is a first group of compounds of more interest as represented by Formula III:

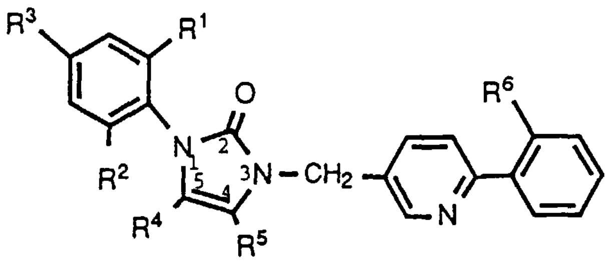

(III)

wherein each of $R^1$, $R^2$ and $R^3$ is independently selected from methyl, ethyl, n-propyl, isopropyl, tert-butyl, methoxy, ethoxy, propoxy, isopropoxy, tert-butoxy, cyano, fluoro, chloro, iodo, bromo, hydroxy, nitro, amino, N-methylamino, N,N-dimethylamino, N-ethylamino, N,N-diethylamino, carboxyl, methoxycarbonyl, ethoxycarbonyl, formyl, methylcarbonyl, ethylcarbonyl and trifluoromethylcarbonyl; wherein further $R^3$ may be hydrogen wherein further when $R^1$ is ethyl or tert.-butyl then $R^2$ can be hydrogen with the proviso that $R^1$, $R^2$ can not be simultaneously fluoro or chloro; wherein $R^4$ is selected from hydrogen methyl, fluoro, chloro, monofluoromethyl, difluoromethyl, trifluoromethyl, formyl, carboxyl and dimethoxymethyl; wherein $R^5$ is selected from methyl, ethyl, n-propyl, isopropyl, n-butyl, sec-butyl, isobutyl, tert-butyl, n-pentyl, isopentyl, neopentyl, phenyl, benzyl, phenethyl, cyclopropyl, cyclobutyl, cyclopentyl, cyclohexyl, cyclopropylmethyl, cyclopropylethyl, cyclobutylmethyl, cyclobutylethyl, cyclopentylmethyl, cyclopentylethyl, cyclohexylmethyl and cyclohexylethyl; wherein $R^6$ is an acidic group selected from COOH and

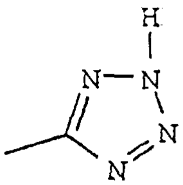

;

or a stereoisomer or a tautomer thereof or a pharmaceutically-acceptable salt thereof.

A family of specific compounds of particular interest within this first class of higher-interest compounds of the first group of compounds of Formula III consists of compounds and their stereoisomers and tautomers and the pharmaceutically-acceptable salts thereof, said compounds consisting of

1-(2-ethylphenyl)-4-propyl-1,3-dihydro-3-[[6-[2-(1H-tetrazol-5-yl)phenyl]-3-pyridinyl]methyl]-2H-imidazol-2-one;
1-(2-ethylphenyl)-4-butyl-1,3-dihydro-3-[[6-[2-(1H-tetrazol-5-yl)phenyl]-3-pyridinyl]methyl]-2H-imidazol-2-one;
1-(2-tertbutylphenyl)-4-butyl-1,3-dihydro-3-[[6-[2-(1H-tetrazol-5-yl)phenyl]-3-pyridinyl]methyl]-2H-imidazol-2-one;

A family of specific compounds of particular interest within this second class of higher-interest compounds of the first group of compounds of Formula III consists of compounds and their stereoisomers and tautomers and the pharmaceutically-acceptable salts thereof, said compounds consisting of 1-(2,6-dimethylphenyl)-4-propyl-1,3-dihydro-3-[[6-[2-(1H-tetrazol-5-yl)phenyl]-3-pyridinyl]methyl]-2H-imidazol-2-one;

1-(2-methyl-6-ethylphenyl)-4-propyl-1,3-dihydro-3-[[6-[2-(1H-tetrazol-5-yl)phenyl]-3-pyridinyl]methyl]-2H-imidazol-2-one;

1-(2-methyl-6-isopropylphenyl)-4-propyl-1,3-dihydro-3-[[6-[2-(1H-tetrazol-5-yl)phenyl]-3-pyridinyl]methyl]-2H-imidazol-2-one;

1-(2-methyl-6-tertbutylphenyl)-4-propyl-1,3-dihydro-3-[[6-[2-(1H-tetrazol-5-yl)phenyl]-3-pyridinyl]methyl]-2H-imidazol-2-one;

1-(2-methyl-6-fluorophenyl)-4-propyl-1,3-dihydro-3-[[6-[2-(1H-tetrazol-5-yl)phenyl]-3-pyridinyl]methyl]-2H-imidazol-2-one;

1-(2-methyl-6-chlorophenyl)-4-propyl-1,3-dihydro-3-[[6-[2-(1H-tetrazol-5-yl)phenyl]-3-pyridinyl]methyl]-2H-imidazol-2-one;

1-(2-methyl-6-methoxyphenyl)-4-propyl-1,3-dihydro-3-[[6-[2-(1H-tetrazol-5-yl)phenyl]-3-pyridinyl]methyl]-2H-imidazol-2-one;

1-(2-ethyl-6-methylphenyl)-4-propyl-1,3-dihydro-3-[[6-[2-(1H-tetrazol-5-yl)phenyl]-3-pyridinyl]methyl]-2H-imidazol-2-one;

1-(2,6-diethylphenyl)-4-propyl-1,3-dihydro-3-[[6-[2-(1H-tetrazol-5-yl)phenyl]-3-pyridinyl]methyl]-2H-imidazol-2-one;

1-(2-ethyl-6-isopropylphenyl)-4-propyl-1,3-dihydro-3-[[6-[2-(1H-tetrazol-5-yl)phenyl]-3-pyridinyl]methyl]-2H-imidazol-2-one;

1-(2-ethyl-6-tertbutylphenyl)-4-propyl-1,3-dihydro-3-[[6-[2-(1H-tetrazol-5-yl)phenyl]-3-pyridinyl]methyl]-2H-imidazol-2-one;

1-(2-ethyl-6-fluorophenyl)-4-propyl-1,3-dihydro-3-[[6-[2-(1H-tetrazol-5-yl)phenyl]-3-pyridinyl]methyl]-2H-imidazol-2-one;

1-(2-ethyl-6-chlorophenyl)-4-propyl-1,3-dihydro-3-[[6-[2-(1H-tetrazol-5-yl)phenyl]-3-pyridinyl]methyl]-2H-imidazol-2-one;

1-(2-ethyl-6-methoxyphenyl)-4-propyl-1,3-dihydro-3-[[6-[2-(1H-tetrazol-5-yl)phenyl]-3-pyridinyl]methyl]-2H-imidazol-2-one;

1-(2-propyl-6-methylphenyl)-4-propyl-1,3-dihydro-3-[[6-[2-(1H-tetrazol-5-yl)phenyl]-3-pyridinyl]methyl]-2H-imidazol-2-one;

1-(2-propyl-6-ethylphenyl)-4-propyl-1,3-dihydro-3-[[6-[2-(1H-tetrazol-5-yl)phenyl]-3-pyridinyl]methyl]-2H-imidazol-2-one;

1-(2-propyl-6-isopropylphenyl)-4-propyl-1,3-dihydro-3-[[6-[2-(1H-tetrazol-5-yl)phenyl]-3-pyridinyl]methyl]-2H-imidazol-2-one;

1-(2-propyl-6-tertbutylphenyl)-4-propyl-1,3-dihydro-3-[[6-[2-(1H-tetrazol-5-yl)phenyl]-3-pyridinyl]methyl]-2H-imidazol-2-one;

1-(2-propyl-6-fluorophenyl)-4-propyl-1,3-dihydro-3-[[6-[2-(1H-tetrazol-5-yl)phenyl]-3-pyridinyl]methyl]-2H-imidazol-2-one;

1-(2-propyl-6-chlorophenyl)-4-propyl-1,3-dihydro-3-[[6-[2-(1H-tetrazol-5-yl)phenyl]-3-pyridinyl]methyl]-2H-imidazol-2-one;

1-(2-propyl-6-methoxyphenyl)-4-propyl-1,3-dihydro-3-[[6-[2-(1H-tetrazol-5-yl)phenyl]-3-pyridinyl]methyl]-2H-imidazol-2-one;

1-(2-isopropyl-6-methylphenyl)-4-propyl-1,3-dihydro-3-[[6-[2-(1H-tetrazol-5-yl)phenyl]-3-pyridinyl]methyl]-2H-imidazol-2-one;

1-(2-isopropyl-6-ethylphenyl)-4-propyl-1,3-dihydro-3-[[6-[2-(1H-tetrazol-5-yl)phenyl]-3-pyridinyl]methyl]-2H-imidazol-2-one;

1-(2,6-diisopropylphenyl)-4-propyl-1,3-dihydro-3-[[6-[2-(1H-tetrazol-5-yl)phenyl]-3-pyridinyl]methyl]-2H-imidazol-2-one;

1-(2-isopropyl-6-tertbutylphenyl)-4-propyl-1,3-dihydro-3-[[6-[2-(1H-tetrazol-5-yl)phenyl]-3-pyridinyl]methyl]-2H-imidazol-2-one;

1-(2-isopropyl-6-fluorophenyl)-4-propyl-1,3-dihydro-3-[[6-[2-(1H-tetrazol-5-yl)phenyl]-3-pyridinyl]methyl]-2H-imidazol-2-one;

1-(2-isopropyl-6-chlorophenyl)-4-propyl-1,3-dihydro-3-[[6-[2-(1H-tetrazol-5-yl)phenyl]-3-pyridinyl]methyl]-2H-imidazol-2-one;

1-(2-isopropyl-6-methoxyphenyl)-4-propyl-1,3-dihydro-3-[[6-[2-(1H-tetrazol-5-yl)phenyl]-3-pyridinyl]methyl]-2H-imidazol-2-one;

1-(2-tertbutyl-6-methylphenyl)-4-propyl-1,3-dihydro-3-[[6-[2-(1H-tetrazol-5-yl)phenyl]-3-pyridinyl]methyl]-2H-imidazol-2-one;

1-(2-tertbutyl-6-ethylphenyl)-4-propyl-1,3-dihydro-3-[[6-[2-(1H-tetrazol-5-yl)phenyl]-3-pyridinyl]methyl]-2H-imidazol-2-one;

1-(2-tertbutyl-6-isopropylphenyl)-4-propyl-1,3-dihydro-3-[[6-[2-(1H-tetrazol-5-yl)phenyl]-3-pyridinyl]methyl]-2H-imidazol-2-one;

1-(2,6-ditertbutylphenyl)-4-propyl-1,3-dihydro-3-[[6-[2-(1H-tetrazol-5-yl)phenyl]-3-pyridinyl]methyl]-2H-imidazol-2-one;

1-(2-tertbutyl-6-fluorophenyl)-4-propyl-1,3-dihydro-3-[[6-[2-(1H-tetrazol-5-yl)phenyl]-3-pyridinyl]methyl]-2H-imidazol-2-one;

1-(2-tertbutyl-6-chlorophenyl)-4-propyl-1,3-dihydro-3-[[6-[2-(1H-tetrazol-5-yl)phenyl]-3-pyridinyl]methyl]-2H-imidazol-2-one;

1-(2-tertbutyl-6-methoxyphenyl)-4-propyl-1,3-dihydro-3-[[6-[2-(1H-tetrazol-5-yl)phenyl]-3-pyridinyl]methyl]-2H-imidazol-2-one;

1-(2-fluoro-6-methylphenyl)-4-propyl-1,3-dihydro-3-[[6-[2-(1H-tetrazol-5-yl)phenyl]-3-pyridinyl]methyl]-2H-imidazol-2-one;

1-(2-fluoro-6-ethylphenyl)-4-propyl-1,3-dihydro-3-[[6-[2-(1H-tetrazol-5-yl)phenyl]-3-pyridinyl]methyl]-2H-imidazol-2-one;

1-(2-fluoro-6-isopropylphenyl)-4-propyl-1,3-dihydro-3-[[6-[2-(1H-tetrazol-5-yl)phenyl]-3-pyridinyl]methyl]-2H-imidazol-2-one;

1-(2-fluoro-6-tertbutylphenyl)-4-propyl-1,3-dihydro-3-[[6-[2-(1H-tetrazol-5-yl)phenyl]-3-pyridinyl]methyl]-2H-imidazol-2-one;

1-(2-fluoro-6-chlorophenyl)-4-propyl-1,3-dihydro-3-[[6-[2-(1H-tetrazol-5-yl)phenyl]-3-pyridinyl]methyl]-2H-imidazol-2-one;

1-(2-fluoro-6-methoxyphenyl)-4-propyl-1,3-dihydro-3-[[6-[2-(1H-tetrazol-5-yl)phenyl]-3-pyridinyl]methyl]-2H-imidazol-2-one;

1-(2-chloro-6-methylphenyl)-4-propyl-1,3-dihydro-3-[[6-[2-(1H-tetrazol-5-yl)phenyl]-3-pyridinyl]methyl]-2H-imidazol-2-one;

1-(2-chloro-6-ethylphenyl)-4-propyl-1,3-dihydro-3-[[6-[2-(1H-tetrazol-5-yl)phenyl]-3-pyridinyl]methyl]-2H-imidazol-2-one;

1-(2-chloro-6-isopropylphenyl)-4-propyl-1,3-dihydro-3-[[6-[2-(1H-tetrazol-5-yl)phenyl]-3-pyridinyl]methyl]-2H-imidazol-2-one;

1-(2-chloro-6-tertbutylphenyl)-4-propyl-1,3-dihydro-3-[[6-[2-(1H-tetrazol-5-yl)phenyl]-3-pyridinyl]methyl]-2H-imidazol-2-one;

1-(2-chloro-6-fluorophenyl)-4-propyl-1,3-dihydro-3-[[6-[2-(1H-tetrazol-5-yl)phenyl]-3-pyridinyl]methyl]-2H-imidazol-2-one;

1-(2,6-dichlorophenyl)-4-propyl-1,3-dihydro-3-[[6-[2-(1H-tetrazol-5-yl)phenyl]-3-pyridinyl]methyl]-2H-imidazol-2-one;

1-(2-chloro-6-methoxyphenyl)-4-propyl-1,3-dihydro-3-[[6-[2-(1H-tetrazol-5-yl)phenyl]-3-pyridinyl]methyl]-2H-imidazol-2-one;

1-(2-bromo-6-methylphenyl)-4-propyl-1,3-dihydro-3-[[6-[2-(1H-tetrazol-5-yl)phenyl]-3-pyridinyl]methyl]-2H-imidazol-2-one;

1-(2-bromo-6-ethylphenyl)-4-propyl-1,3-dihydro-3-[[6-[2-(1H-tetrazol-5-yl)phenyl]-3-pyridinyl]methyl]-2H-imidazol-2-one;

1-(2-bromo-6-isopropylphenyl)-4-propyl-1,3-dihydro-3-[[6-[2-(1H-tetrazol-5-yl)phenyl]-3-pyridinyl]methyl]-2H-imidazol-2-one;

1-(2-bromo-6-tertbutylphenyl)-4-propyl-1,3-dihydro-3-[[6-[2-(1H-tetrazol-5-yl)phenyl]-3-pyridinyl]methyl]-2H-imidazol-2-one;

1-(2-bromo-6-fluorophenyl)-4-propyl-1,3-dihydro-3-[[6-[2-(1H-tetrazol-5-yl)phenyl]-3-pyridinyl]methyl]-2H-imidazol-2-one;

1-(2-bromo-6-chlorophenyl)-4-propyl-1,3-dihydro-3-[[6-[2-(1H-tetrazol-5-yl)phenyl]-3-pyridinyl]methyl]-2H-imidazol-2-one;

1-(2-bromo-6-methoxyphenyl)-4-propyl-1,3-dihydro-3-[[6-[2-(1H-tetrazol-5-yl)phenyl]-3-pyridinyl]methyl]-2H-imidazol-2-one;

1-(2-methoxy-6-methylphenyl)-4-propyl-1,3-dihydro-3-[[6-[2-(1H-tetrazol-5-yl)phenyl]-3-pyridinyl]methyl]-2H-imidazol-2-one;

1-(2-methoxy-6-ethylphenyl)-4-propyl-1,3-dihydro-3-[[6-[2-(1H-tetrazol-5-yl)phenyl]-3-pyridinyl]methyl]-2H-imidazol-2-one;

1-(2-methoxy-6-isopropylphenyl)-4-propyl-1,3-dihydro-3-[[6-[2-(1H-tetrazol-5-yl)phenyl]-3-pyridinyl]methyl]-2H-imidazol-2-one;

1-(2-methoxy-6-tertbutylphenyl)-4-propyl-1,3-dihydro-3-[[6-[2-(1H-tetrazol-5-yl)phenyl]-3-pyridinyl]methyl]-2H-imidazol-2-one;

1-(2-methoxy-6-fluorophenyl)-4-propyl-1,3-dihydro-3-[[6-[2-(1H-tetrazol-5-yl)phenyl]-3-pyridinyl]methyl]-2H-imidazol-2-one;

1-(2-methoxy-6-chlorophenyl)-4-propyl-1,3-dihydro-3-[[6-[2-(1H-tetrazol-5-yl)phenyl]-3-pyridinyl]methyl]-2H-imidazol-2-one;

1-(2,6-dimethoxyphenyl)-4-propyl-1,3-dihydro-3-[[6-[2-(1H-tetrazol-5-yl)phenyl]-3-pyridinyl]methyl]-2H-imidazol-2-one;

1-(2-carboxy-6-methylphenyl)-4-propyl-1,3-dihydro-3-[[6-[2-(1H-tetrazol-5-yl)phenyl]-3-pyridinyl]methyl]-2H-imidazol-2-one;

1-(2-carboxy-6-ethylphenyl)-4-propyl-1,3-dihydro-3-[[6-[2-(1H-tetrazol-5-yl)phenyl]-3-pyridinyl]methyl]-2H-imidazol-2-one;

1-(2-carboxy-6-isopropylphenyl)-4-propyl-1,3-dihydro-3-[[6-[2-(1H-tetrazol-5-yl)phenyl]-3-pyridinyl]methyl]-2H-imidazol-2-one;

1-(2-carboxy-6-tertbutylphenyl)-4-propyl-1,3-dihydro-3-[[6-[2-(1H-tetrazol-5-yl)phenyl]-3-pyridinyl]methyl]-2H-imidazol-2-one;

1-(2-carboxy-6-fluorophenyl)-4-propyl-1,3-dihydro-3-[[6-[2-(1H-tetrazol-5-yl)phenyl]-3-pyridinyl]methyl]-2H-imidazol-2-one;

1-(2-carboxy-6-chlorophenyl)-4-propyl-1,3-dihydro-3-[[6-[2-(1H-tetrazol-5-yl)phenyl]-3-pyridinyl]methyl]-2H-imidazol-2-one;

1-(2-carboxy-6-methoxyphenyl)-4-propyl-1,3-dihydro-3-[[6-[2-(1H-tetrazol-5-yl)phenyl]-3-pyridinyl]methyl]-2H-imidazol-2-one;

1-(2-acetyl-6-methylphenyl)-4-propyl-1,3-dihydro-3-[[6-[2-(1H-tetrazol-5-yl)phenyl]-3-pyridinyl]methyl]-2H-imidazol-2-one;

1-(2-acetyl-6-ethylphenyl)-4-propyl-1,3-dihydro-3-[[6-[2-(1H-tetrazol-5-yl)phenyl]-3-pyridinyl]methyl]-2H-imidazol-2-one;

1-(2-acetyl-6-isopropylphenyl)-4-propyl-1,3-dihydro-3-[[6-[2-(1H-tetrazol-5-yl)phenyl]-3-pyridinyl]methyl]-2H-imidazol-2-one;

1-(2-acetyl-6-tertbutylphenyl)-4-propyl-1,3-dihydro-3-[[6-[2-(1H-tetrazol-5-yl)phenyl]-3-pyridinyl]methyl]-2H-imidazol-2-one;

1-(2-acetyl-6-fluorophenyl)-4-propyl-1,3-dihydro-3-[[6-[2-(1H-tetrazol-5-yl)phenyl]-3-pyridinyl]methyl]-2H-imidazol-2-one;

1-(2-acetyl-6-chlorophenyl)-4-propyl-1,3-dihydro-3-[[6-[2-(1H-tetrazol-5-yl)phenyl]-3-pyridinyl]methyl]-2H-imidazol-2-one;

1-(2-acetyl-6-methoxyphenyl)-4-propyl-1,3-dihydro-3-[[6-[2-(1H-tetrazol-5-yl)phenyl]-3-pyridinyl]methyl]-2H-imidazol-2-one;

1-(2,6-dimethylphenyl)-4-butyl-1,3-dihydro-3-[[6-[2-(1H-tetrazol-5-yl)phenyl]-3-pyridinyl]methyl]-2H-imidazol-2-one;

1-(2-methyl-6-ethylphenyl)-4-butyl-1,3-dihydro-3-[[6-[2-(1H-tetrazol-5-yl)phenyl]-3-pyridinyl]methyl]-2H-imidazol-2-one;

1-(2-methyl-6-isopropylphenyl)-4-butyl-1,3-dihydro-3-[[6-[2-(1H-tetrazol-5-yl)phenyl]-3-pyridinyl]methyl]-2H-imidazol-2-one;

1-(2-methyl-6-tertbutylphenyl)-4-butyl-1,3-dihydro-3-[[6-[2-(1H-tetrazol-5-yl)phenyl]-3-pyridinyl]methyl]-2H-imidazol-2-one;

1-(2-methyl-6-fluorophenyl)-4-butyl-1,3-dihydro-3-[[6-[2-(1H-tetrazol-5-yl)phenyl]-3-pyridinyl]methyl]-2H-imidazol-2-one;

1-(2-methyl-6-chlorophenyl)-4-butyl-1,3-dihydro-3-[[6-[2-(1H-tetrazol-5-yl)phenyl]-3-pyridinyl]methyl]-2H-imidazol-2-one;

1-(2-methyl-6-methoxyphenyl)-4-butyl-1,3-dihydro-3-[[6-[2-(1H-tetrazol-5-yl)phenyl]-3-pyridinyl]methyl]-2H-imidazol-2-one;

1-(2-ethyl-6-methylphenyl)-4-butyl-1,3-dihydro-3-[[6-[2-(1H-tetrazol-5-yl)phenyl]-3-pyridinyl]methyl]-2H-imidazol-2-one;

1-(2,6-diethylphenyl)-4-butyl-1,3-dihydro-3-[[6-[2-(1H-tetrazol-5-yl)phenyl]-3-pyridinyl]methyl]-2H-imidazol-2-one;

1-(2-ethyl-6-isopropylphenyl)-4-butyl-1,3-dihydro-3-[[6-[2-(1H-tetrazol-5-yl)phenyl]-3-pyridinyl]methyl]-2H-imidazol-2-one;

1-(2-ethyl-6-tertbutylphenyl)-4-butyl-1,3-dihydro-3-[[6-[2-(1H-tetrazol-5-yl)phenyl]-3-pyridinyl]methyl]-2H-imidazol-2-one;

1-(2-ethyl-6-fluorophenyl)-4-butyl-1,3-dihydro-3-[[6-[2-(1H-tetrazol-5-yl)phenyl]-3-pyridinyl]methyl]-2H-imidazol-2-one;

1-(2-ethyl-6-chlorophenyl)-4-butyl-1,3-dihydro-3-[[6-[2-(1H-tetrazol-5-yl)phenyl]-3-pyridinyl]methyl]-2H-imidazol-2-one;

1-(2-ethyl-6-methoxyphenyl)-4-butyl-1,3-dihydro-3-[[6-[2-(1H-tetrazol-5-yl)phenyl]-3-pyridinyl]methyl]-2H-imidazol-2-one;
1-(2-propyl-6-methylphenyl)-4-butyl-1,3-dihydro-3-[[6-[2-(1H-tetrazol-5-yl)phenyl]-3-pyridinyl]methyl]-2H-imidazol-2-one;
1-(2-propyl-6-ethylphenyl)-4-butyl-1,3-dihydro-3-[[6-[2-(1H-tetrazol-5-yl)phenyl]-3-pyridinyl]methyl]-2H-imidazol-2-one;
1-(2-propyl-6-isopropylphenyl)-4-butyl-1,3-dihydro-3-[[6-[2-(1H-tetrazol-5-yl)phenyl]-3-pyridinyl]methyl]-2H-imidazol-2-one;
1-(2-propyl-6-tertbutylphenyl)-4-butyl-1,3-dihydro-3-[[6-[2-(1H-tetrazol-5-yl)phenyl]-3-pyridinyl]methyl]-2H-imidazol-2-one;
1-(2-propyl-6-fluorophenyl)-4-butyl-1,3-dihydro-3-[[6-[2-(1H-tetrazol-5-yl)phenyl]-3-pyridinyl]methyl]-2H-imidazol-2-one;
1-(2-propyl-6-chlorophenyl)-4-butyl-1,3-dihydro-3-[[6-[2-(1H-tetrazol-5-yl)phenyl]-3-pyridinyl]methyl]-2H-imidazol-2-one;
1-(2-propyl-6-methoxyphenyl)-4-butyl-1,3-dihydro-3-[[6-[2-(1H-tetrazol-5-yl)phenyl]-3-pyridinyl]methyl]-2H-imidazol-2-one;
1-(2-isopropyl-6-methylphenyl)-4-butyl-1,3-dihydro-3-[[6-[2-(1H-tetrazol-5-yl)phenyl]-3-pyridinyl]methyl]-2H-imidazol-2-one;
1-(2-isopropyl-6-ethylphenyl)-4-butyl-1,3-dihydro-3-[[6-[2-(1H-tetrazol-5-yl)phenyl]-3-pyridinyl]methyl]-2H-imidazol-2-one;
1-(2,6-diisopropylphenyl)-4-butyl-1,3-dihydro-3-[[6-[2-(1H-tetrazol-5-yl)phenyl]-3-pyridinyl]methyl]-2H-imidazol-2-one;
1-(2-isopropyl-6-tertbutylphenyl)-4-butyl-1,3-dihydro-3-[[6-[2-(1H-tetrazol-5-yl)phenyl]-3-pyridinyl]methyl]-2H-imidazol-2-one;
1-(2-isopropyl-6-fluorophenyl)-4-butyl-1,3-dihydro-3-[[6-[2-(1H-tetrazol-5-yl)phenyl]-3-pyridinyl]methyl]-2H-imidazol-2-one;
1-(2-isopropyl-6-chlorophenyl)-4-butyl-1,3-dihydro-3-[[6-[2-(1H-tetrazol-5-yl)phenyl]-3-pyridinyl]methyl]-2H-imidazol-2-one;
1-(2-isopropyl-6-methoxyphenyl)-4-butyl-1,3-dihydro-3-[[6-[2-(1H-tetrazol-5-yl)phenyl]-3-pyridinyl]methyl]-2H-imidazol-2-one;
1-(2-tertbutyl-6-methylphenyl)-4-butyl-1,3-dihydro-3-[[6-[2-(1H-tetrazol-5-yl)phenyl]-3-pyridinyl]methyl]-2H-imidazol-2-one;
1-(2-tertbutyl-6-ethylphenyl)-4-butyl-1,3-dihydro-3-[[6-[2-(1H-tetrazol-5-yl)phenyl]-3-pyridinyl]methyl]-2H-imidazol-2-one;
1-(2-tertbutyl-6-isopropylphenyl)-4-butyl-1,3-dihydro-3-[[6-[2-(1H-tetrazol-5-yl)phenyl]-3-pyridinyl]methyl]-2H-imidazol-2-one;
1-(2,6-ditertbutylphenyl)-4-butyl-1,3-dihydro-3-[[6-[2-(1H-tetrazol-5-yl)phenyl]-3-pyridinyl]methyl]-2H-imidazol-2-one;
1-(2-tertbutyl-6-fluorophenyl)-4-butyl-1,3-dihydro-3-[[6-[2-(1H-tetrazol-5-yl)phenyl]-3-pyridinyl]methyl]-2H-imidazol-2-one;
1-(2-tertbutyl-6-chlorophenyl)-4-butyl-1,3-dihydro-3-[[6-[2-(1H-tetrazol-5-yl)phenyl]-3-pyridinyl]methyl]-2H-imidazol-2-one;
1-(2-tertbutyl-6-methoxyphenyl)-4-butyl-1,3-dihydro-3-[[6-[2-(1H-tetrazol-5-yl)phenyl]-3-pyridinyl]methyl]-2H-imidazol-2-one;
1-(2-fluoro-6-methylphenyl)-4-butyl-1,3-dihydro-3-[[6-[2-(1H-tetrazol-5-yl)phenyl]-3-pyridinyl]methyl]-2H-imidazol-2-one;
1-(2-fluoro-6-ethylphenyl)-4-butyl-1,3-dihydro-3-[[6-[2-(1H-tetrazol-5-yl)phenyl]-3-pyridinyl]methyl]-2H-imidazol-2-one;
1-(2-fluoro-6-isopropylphenyl)-4-butyl-1,3-dihydro-3-[[6-[2-(1H-tetrazol-5-yl)phenyl]-3-pyridinyl]methyl]-2H-imidazol-2-one;
1-(2-fluoro-6-tertbutylphenyl)-4-butyl-1,3-dihydro-3-[[6-[2-(1H-tetrazol-5-yl)phenyl]-3-pyridinyl]methyl]-2H-imidazol-2-one;
1-(2,6-difluorophenyl)-4-butyl-1,3-dihydro-3-[[6-[2-(1H-tetrazol-5-yl)phenyl]-3-pyridinyl]methyl]-2H-imidazol-2-one;
1-(2-fluoro-6-chlorophenyl)-4-butyl-1,3-dihydro-3-[[6-[2-(1H-tetrazol-5-yl)phenyl]-3-pyridinyl]methyl]-2H-imidazol-2-one;
1-(2-fluoro-6-methoxyphenyl)-4-butyl-1,3-dihydro-3-[[6-[2-(1H-tetrazol-5-yl)phenyl]-3-pyridinyl]methyl]-2H-imidazol-2-one;
1-(2-chloro-6-methylphenyl)-4-butyl-1,3-dihydro-3-[[6-[2-(1H-tetrazol-5-yl)phenyl]-3-pyridinyl]methyl]-2H-imidazol-2-one;

1-(2-chloro-6-ethylphenyl)-4-butyl-1,3-dihydro-3-[[6-[2-(1H-tetrazol-5-yl)phenyl]-3-pyridinyl]methyl]-2H-imidazol-2-one;

1-(2-chloro-6-isopropylphenyl)-4-butyl-1,3-dihydro-3-[[6-[2-(1H-tetrazol-5-yl)phenyl]-3-pyridinyl]methyl]-2H-imidazol-2-one;

1-(2-chloro-6-tertbutylphenyl)-4-butyl-1,3-dihydro-3-[[6-[2-(1H-tetrazol-5-yl)phenyl]-3-pyridinyl]methyl]-2H-imidazol-2-one;

1-(2-chloro-6-fluorophenyl)-4-butyl-1,3-dihydro-3-[[6-[2-(1H-tetrazol-5-yl)phenyl]-3-pyridinyl]methyl]-2H-imidazol-2-one;

1-(2,6-dichlorophenyl)-4-butyl-1,3-dihydro-3-[[6-[2-(1H-tetrazol-5-yl)phenyl]-3-pyridinyl]methyl]-2H-imidazol-2-one;

1-(2-chloro-6-methoxyphenyl)-4-butyl-1,3-dihydro-3-[[6-[2-(1H-tetrazol-5-yl)phenyl]-3-pyridinyl]methyl]-2H-imidazol-2-one;

1-(2-bromo-6-methylphenyl)-4-butyl-1,3-dihydro-3-[[6-[2-(1H-tetrazol-5-yl)phenyl]-3-pyridinyl]methyl]-2H-imidazol-2-one;

1-(2-bromo-6-ethylphenyl)-4-butyl-1,3-dihydro-3-[[6-[2-(1H-tetrazol-5-yl)phenyl]-3-pyridinyl]methyl]-2H-imidazol-2-one;

1-(2-bromo-6-isopropylphenyl)-4-butyl-1,3-dihydro-3-[[6-[2-(1H-tetrazol-5-yl)phenyl]-3-pyridinyl]methyl]-2H-imidazol-2-one;

1-(2-bromo-6-tertbutylphenyl)-4-butyl-1,3-dihydro-3-[[6-[2-(1H-tetrazol-5-yl)phenyl]-3-pyridinyl]methyl]-2H-imidazol-2-one;

1-(2-bromo-6-fluorophenyl)-4-butyl-1,3-dihydro-3-[[6-[2-(1H-tetrazol-5-yl)phenyl]-3-pyridinyl]methyl]-2H-imidazol-2-one;

1-(2-bromo-6-chlorophenyl)-4-butyl-1,3-dihydro-3-[[6-[2-(1H-tetrazol-5-yl)phenyl]-3-pyridinyl]methyl]-2H-imidazol-2-one;

1-(2-bromo-6-methoxyphenyl)-4-butyl-1,3-dihydro-3-[[6-[2-(1H-tetrazol-5-yl)phenyl]-3-pyridinyl]methyl]-2H-imidazol-2-one;

1-(2-methoxy-6-methylphenyl)-4-butyl-1,3-dihydro-3-[[6-[2-(1H-tetrazol-5-yl)phenyl]-3-pyridinyl]methyl]-2H-imidazol-2-one;

1-(2-methoxy-6-ethylphenyl)-4-butyl-1,3-dihydro-3-[[6-[2-(1H-tetrazol-5-yl)phenyl]-3-pyridinyl]methyl]-2H-imidazol-2-one;

1-(2-methoxy-6-isopropylphenyl)-4-butyl-1,3-dihydro-3-[[6-[2-(1H-tetrazol-5-yl)phenyl]-3-pyridinyl]methyl]-2H-imidazol-2-one;

1-(2-methoxy-6-tertbutylphenyl)-4-butyl-1,3-dihydro-3-[[6-[2-(1H-tetrazol-5-yl)phenyl]-3-pyridinyl]methyl]-2H-imidazol-2-one;

1-(2-methoxy-6-fluorophenyl)-4-butyl-1,3-dihydro-3-[[6-[2-(1H-tetrazol-5-yl)phenyl]-3-pyridinyl]methyl]-2H-imidazol-2-one;

1-(2-methoxy-6-chlorophenyl)-4-butyl-1,3-dihydro-3-[[6-[2-(1H-tetrazol-5-yl)phenyl]-3-pyridinyl]methyl]-2H-imidazol-2-one;

1-(2,6-dimethoxyphenyl)-4-butyl-1,3-dihydro-3-[[6-[2-(1H-tetrazol-5-yl)phenyl]-3-pyridinyl]methyl]-2H-imidazol-2-one;

1-(2-carboxy-6-methylphenyl)-4-butyl-1,3-dihydro-3-[[6-[2-(1H-tetrazol-5-yl)phenyl]-3-pyridinyl]methyl]-2H-imidazol-2-one;

1-(2-carboxy-6-ethylphenyl)-4-butyl-1,3-dihydro-3-[[6-[2-(1H-tetrazol-5-yl)phenyl]-3-pyridinyl]methyl]-2H-imidazol-2-one;

1-(2-carboxy-6-isopropylphenyl)-4-butyl-1,3-dihydro-3-[[6-[2-(1H-tetrazol-5-yl)phenyl]-3-pyridinyl]methyl]-2H-imidazol-2-one;

1-(2-carboxy-6-tertbutylphenyl)-4-butyl-1,3-dihydro-3-[[6-[2-(1H-tetrazol-5-yl)phenyl]-3-pyridinyl]methyl]-2H-imidazol-2-one;

1-(2-carboxy-6-fluorophenyl)-4-butyl-1,3-dihydro-3-[[6-[2-(1H-tetrazol-5-yl)phenyl]-3-pyridinyl]methyl]-2H-imidazol-2-one;

1-(2-carboxy-6-chlorophenyl)-4-butyl-1,3-dihydro-3-[[6-[2-(1H-tetrazol-5-yl)phenyl]-3-pyridinyl]methyl]-2H-imidazol-2-one;

1-(2-carboxy-6-methoxyphenyl)-4-butyl-1,3-dihydro-3-[[6-[2-(1H-tetrazol-5-yl)phenyl]-3-pyridinyl]methyl]-2H-imidazol-2-one;

1-(2-acetyl-6-methylphenyl)-4-butyl-1,3-dihydro-3-[[6-[2-(1H-tetrazol-5-yl)phenyl]-3-pyridinyl]methyl]-2H-imidazol-2-one;

1-(2-acetyl-6-ethylphenyl)-4-butyl-1,3-dihydro-3-[[6-[2-(1H-tetrazol-5-yl)phenyl]-3-pyridinyl]methyl]-2H-imidazol-2-one;

1-(2-acetyl-6-isopropylphenyl)-4-butyl-1,3-dihydro-3-[[6-[2-(1H-tetrazol-5-yl)phenyl]-3-pyridinyl]methyl]-2H-imidazol-2-one;

1-(2-acetyl-6-tertbutylphenyl)-4-butyl-1,3-dihydro-3-[[6-[2-(1H-tetrazol-5-yl)phenyl]-3-pyridinyl]methyl]-2H-imidazol-2-one;

1-(2-acetyl-6-fluorophenyl)-4-butyl-1,3-dihydro-3-[[6-[2-(1H-tetrazol-5-yl)phenyl]-3-pyridinyl]methyl]-2H-imidazol-2-one;

1-(2-acetyl-6-chlorophenyl)-4-butyl-1,3-dihydro-3-[[6-[2-(1H-tetrazol-5-yl)phenyl]-3-pyridinyl]methyl]-2H-imidazol-2-one; and

1-(2-acetyl-6-methoxyphenyl)-4-butyl-1,3-dihydro-3-[[6-[2-(1H-tetrazol-5-yl)phenyl]-3-pyridinyl]methyl]-2H-imidazol-2-one.

Within Formula I there is a first group of compounds of more interest as represented by Formula IV:

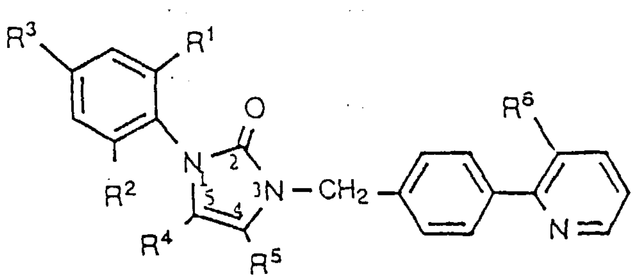

(IV)

wherein each of $R^1$, $R^2$ and $R^3$ is independently selected from methyl, ethyl, n-propyl, isopropyl, tert-butyl, methoxy, ethoxy, propoxy, isopropoxy, tert-butoxy, cyano, fluoro, chloro, iodo, bromo, hydroxy, nitro, amino, N-methylamino, N,N-dimethylamino, N-ethylamino, N,N-diethylamino, carboxyl, methoxycarbonyl, ethoxycarbonyl, formyl, methylcarbonyl, ethylcarbonyl and trifluoromethylcarbonyl; wherein further $R^3$ may be hydrogen wherein further when $R^1$ is ethyl or tert.-butyl then $R^2$ can be hydrogen with the proviso that $R^1$ and $R^2$ can not be fluoro or chloro simultaneously; wherein $R^4$ is selected from hydrogen methyl, fluoro, chloro, monofluoromethyl, difluoromethyl, trifluoromethyl, formyl, carboxyl and dimethoxymethyl; wherein $R^5$ is selected from methyl, ethyl, n-propyl, isopropyl, n-butyl, sec-butyl, isobutyl, tert-butyl, n-pentyl, isopentyl, neopentyl, phenyl, benzyl, phenethyl, cyclopropyl, cyclobutyl, cyclopentyl, cyclohexyl, cyclopropylmethyl, cyclopropylethyl, cyclobutylmethyl, cyclobutylethyl, cyclopentylmethyl, cyclopentylethyl, cyclohexylmethyl and cyclohexylethyl; wherein $R^6$ is an acidic group selected from COOH and

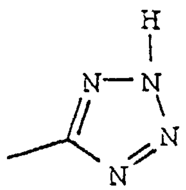

;

or a stereoisomer or a tautomer thereof or a pharmaceutically-acceptable salt thereof.

A family of specific compounds of particular interest within this first class of higher-interest compounds of the first group of compounds of Formula IV consists of compounds and their stereoisomers and tautomers and the pharmaceutically-acceptable salts thereof, said compounds consisting of:

1-(2-ethylphenyl)-4-propyl-1,3-dihydro-3-[[4-[3-(1H-tetrazol-5-yl)-2-pyridinyl]phenyl]methyl]-2H-imidazol-2-one;
1-(2-tertbutylphenyl)-4-propyl-1,3-dihydro-3-[[4-[3-(1H-tetrazol-5-yl)-2-pyridinyl]phenyl]methyl]-2H-imidazol-2-one;
1-(2-ethylphenyl)-4-butyl-1,3-dihydro-3-[[4-[3-(1H-tetrazol-5-yl)-2-pyridinyl]phenyl]methyl]-2H-imidazol-2-one;
1-(2-tertbutylphenyl)-4-butyl-1,3-dihydro-3-[[4-[3-(1H-tetrazol-5-yl)-2-pyridinyl]phenyl]methyl]-2H-imidazol-2-one;

Within this first group of compounds of Formula IV there is a second class, of higher-interest compounds wherein each of $R^1$ and $R^2$ is independently selected from methyl, ethyl, n-propyl, isopropyl, tert-butyl, methoxy, fluoro, chloro, iodo, bromo, carboxyl, formyl, methylcarbonyl and trifluoromethylcarbonyl; wherein each of $R^3$ and $R^4$ is hydrogen wherein $R^5$ is selected from methyl, ethyl, n-propyl, isopropyl, n-butyl, sec-butyl, isobutyl, tert-butyl, n-pentyl, iso-

pentyl and neopentyl; wherein $R^6$ is an acidic group selected from COOH and

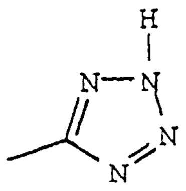

;

or a stereoisomer or a tautomer thereof or a pharmaceutically-acceptable salt thereof.

A family of specific compounds of particular interest within this second class of higher-interest compounds of the first group of compounds of Formula IV consists of compounds and their stereoisomers and tautomers and the pharmaceutically-acceptable salts thereof, said compounds consisting of

1-(2,6-dimethylphenyl)-4-propyl-1,3-dihydro-3-[[4-[3-(1H-tetrazol-5-yl)-2-pyridinyl]phenyl]methyl]-2H-imidazol-2-one;
1-(2-methyl-6-ethylphenyl)-4-propyl-1,3-dihydro-3-[[4-[3-(1H-tetrazol-5-yl)-2-pyridinyl]phenyl]methyl]-2H-imidazol-2-one;
1-(2-methyl-6-isopropylphenyl)-4-propyl-1,3-dihydro-3-[[4-[3-(1H-tetrazol-5-yl)-2-pyridinyl]phenyl]methyl]-2H-imidazol-2-one;
1-(2-methyl-6-tertbutylphenyl)-4-propyl-1,3-dihydro-3-[[4-[3-(1H-tetrazol-5-yl)-2-pyridinyl]phenyl]methyl]-2H-imidazol-2-one;
1-(2-methyl-6-fluorophenyl)-4-propyl-1,3-dihydro-3-[[4-[3-(1H-tetrazol-5-yl)-2-pyridinyl]phenyl]methyl]-2H-imidazol-2-one;
1-(2-methyl-6-chlorophenyl)-4-propyl-1,3-dihydro-3-[[4-[3-(1H-tetrazol-5-yl)-2-pyridinyl]phenyl]methyl]-2H-imidazol-2-one;
1-(2-methyl-6-methoxyphenyl)-4-propyl-1,3-dihydro-3-[[4-[3-(1H-tetrazol-5-yl)-2-pyridinyl]phenyl]methyl]-2H-imidazol-2-one;
1-(2-ethyl-6-methylphenyl)-4-propyl-1,3-dihydro-3-[[4-[3-(1H-tetrazol-5-yl)-2-pyridinyl]phenyl]methyl]-2H-imidazol-2-one;
1-(2,6-diethylphenyl)-4-propyl-1,3-dihydro-3-[[4-[3-(1H-tetrazol-5-yl)-2-pyridinyl]phenyl]methyl]-2H-imidazol-2-one;
1-(2-ethyl-6-isopropylphenyl)-4-propyl-1,3-dihydro-3-[[4-[3-(1H-tetrazol-5-yl)-2-pyridinyl]phenyl]methyl]-2H-imidazol-2-one;
1-(2-ethyl-6-tertbutylphenyl)-4-propyl-1,3-dihydro-3-[[4-[3-(1H-tetrazol-5-yl)-2-pyridinyl]phenyl]methyl]-2H-imidazol-2-one;
1-(2-ethyl-6-fluorophenyl)-4-propyl-1,3-dihydro-3-[[4-[3-(1H-tetrazol-5-yl)-2-pyridinyl]phenyl]methyl]-2H-imidazol-2-one;
1-(2-ethyl-6-chlorophenyl)-4-propyl-1,3-dihydro-3-[[4-[3-(1H-tetrazol-5-yl)-2-pyridinyl]phenyl]methyl]-2H-imidazol-2-one;
1-(2-ethyl-6-methoxyphenyl)-4-propyl-1,3-dihydro-3-[[4-[3-(1H-tetrazol-5-yl)-2-pyridinyl]phenyl]methyl]-2H-imidazol-2-one;
1-(2-propyl-6-methylphenyl)-4-propyl-1,3-dihydro-3-[[4-[3-(1H-tetrazol-5-yl)-2-pyridinyl]phenyl]methyl]-2H-imidazol-2-one;
1-(2-propyl-6-ethylphenyl)-4-propyl-1,3-dihydro-3-[[4-[3-(1H-tetrazol-5-yl)-2-pyridinyl]phenyl]methyl]-2H-imidazol-2-one;
1-(2-propyl-6-isopropylphenyl)-4-propyl-1,3-dihydro-3-[[4-[3-(1H-tetrazol-5-yl)-2-pyridinyl]phenyl]methyl]-2H-imidazol-2-one;
1-(2-propyl-6-tertbutylphenyl)-4-propyl-1,3-dihydro-3-[[4-[3-(1H-tetrazol-5-yl)-2-pyridinyl]phenyl]methyl]-2H-imidazol-2-one;
1-(2-propyl-6-fluorophenyl)-4-propyl-1,3-dihydro-3-[[4-[3-(1H-tetrazol-5-yl)-2-pyridinyl]phenyl]methyl]-2H-imidazol-2-one;
1-(2-propyl-6-chlorophenyl)-4-propyl-1,3-dihydro-3-[[4-[3-(1H-tetrazol-5-yl)-2-pyridinyl]phenyl]methyl]-2H-imidazol-2-one;
1-(2-propyl-6-methoxyphenyl)-4-propyl-1,3-dihydro-3-[[4-[3-(1H-tetrazol-5-yl)-2-pyridinyl]phenyl]methyl]-2H-imidazol-2-one;
1-(2-isopropyl-6-methylphenyl)-4-propyl-1,3-dihydro-3-[[4-[3-(1H-tetrazol-5-yl)-2-pyridinyl]phenyl]methyl]-2H-imidazol-2-one;

1-(2-isopropyl-6-ethylphenyl)-4-propyl-1,3-dihydro-3-[[4-[3-(1H-tetrazol-5-yl)-2-pyridinyl]phenyl]methyl]-2H-imidazol-2-one;

1-(2,6-diisopropylphenyl)-4-propyl-1,3-dihydro-3-[[4-[3-(1H-tetrazol-5-yl)-2-pyridinyl]phenyl]methyl]-2H-imidazol-2-one;

1-(2-isopropyl-6-tertbutylphenyl)-4-propyl-1,3-dihydro-3-[[4-[3-(1H-tetrazol-5-yl)-2-pyridinyl]phenyl]methyl]-2H-imidazol-2-one;

1-(2-isopropyl-6-fluorophenyl)-4-propyl-1,3-dihydro-3-[[4-[3-(1H-tetrazol-5-yl)-2-pyridinyl]phenyl]methyl]-2H-imidazol-2-one;

1-(2-isopropyl-6-chlorophenyl)-4-propyl-1,3-dihydro-3-[[4-[3-(1H-tetrazol-5-yl)-2-pyridinyl]phenyl]methyl]-2H-imidazol-2-one;

1-(2-isopropyl-6-methoxyphenyl)-4-propyl-1,3-dihydro-3-[[4-[3-(1H-tetrazol-5-yl)-2-pyridinyl]phenyl]methyl]-2H-imidazol-2-one;

1-(2-tertbutyl-6-methylphenyl)-4-propyl-1,3-dihydro-3-[[4-[3-(1H-tetrazol-5-yl)-2-pyridinyl]phenyl]methyl]-2H-imidazol-2-one;

1-(2-tertbutyl-6-ethylphenyl)-4-propyl-1,3-dihydro-3-[[4-[3-(1H-tetrazol-5-yl)-2-pyridinyl]phenyl]methyl]-2H-imidazol-2-one;

1-(2-tertbutyl-6-isopropylphenyl)-4-propyl-1,3-dihydro-3-[[4-[3-(1H-tetrazol-5-yl)-2-pyridinyl]phenyl]methyl]-2H-imidazol-2-one;

1-(2,6-ditertbutylphenyl)-4-propyl-1,3-dihydro-3-[[4-[3-(1H-tetrazol-5-yl)-2-pyridinyl]phenyl]methyl]-2H-imidazol-2-one;

1-(2-tertbutyl-6-fluorophenyl)-4-propyl-1,3-dihydro-3-[[4-[3-(1H-tetrazol-5-yl)-2-pyridinyl]phenyl]methyl]-2H-imidazol-2-one;

1-(2-tertbutyl-6-chlorophenyl)-4-propyl-1,3-dihydro-3-[[4-[3-(1H-tetrazol-5-yl)-2-pyridinyl]phenyl]methyl]-2H-imidazol-2-one;

1-(2-tertbutyl-6-methoxyphenyl)-4-propyl-1,3-dihydro-3-[[4-[3-(1H-tetrazol-5-yl)-2-pyridinyl]phenyl]methyl]-2H-imidazol-2-one;

1-(2-fluoro-6-methylphenyl)-4-propyl-1,3-dihydro-3-[[4-[3-(1H-tetrazol-5-yl)-2-pyridinyl]phenyl]methyl]-2H-imidazol-2-one;

1-(2-fluoro-6-ethylphenyl)-4-propyl-1,3-dihydro-3-[[4-[3-(1H-tetrazol-5-yl)-2-pyridinyl]phenyl]methyl]-2H-imidazol-2-one;

1-(2-fluoro-6-isopropylphenyl)-4-propyl-1,3-dihydro-3-[[4-[3-(1H-tetrazol-5-yl)-2-pyridinyl]phenyl]methyl]-2H-imidazol-2-one;

1-(2-fluoro-6-tertbutylphenyl)-4-propyl-1,3-dihydro-3-[[4-[3-(1H-tetrazol-5-yl)-2-pyridinyl]phenyl]methyl]-2H-imidazol-2-one;

1-(2-fluoro-6-chlorophenyl)-4-propyl-1,3-dihydro-3-[[4-[3-(1H-tetrazol-5-yl)-2-pyridinyl]phenyl]methyl]-2H-imidazol-2-one;

1-(2-fluoro-6-methoxyphenyl)-4-propyl-1,3-dihydro-3-[[4-[3-(1H-tetrazol-5-yl)-2-pyridinyl]phenyl]methyl]-2H-imidazol-2-one;

1-(2-chloro-6-methylphenyl)-4-propyl-1,3-dihydro-3-[[4-[3-(1H-tetrazol-5-yl)-2-pyridinyl]phenyl]methyl]-2H-imidazol-2-one;

1-(2-chloro-6-ethylphenyl)-4-propyl-1,3-dihydro-3-[[4-[3-(1H-tetrazol-5-yl)-2-pyridinyl]phenyl]methyl]-2H-imidazol-2-one;

1-(2-chloro-6-isopropylphenyl)-4-propyl-1,3-dihydro-3-[[4-[3-(1H-tetrazol-5-yl)-2-pyridinyl]phenyl]methyl]-2H-imidazol-2-one;

1-(2-chloro-6-tertbutylphenyl)-4-propyl-1,3-dihydro-3-[[4-[3-(1H-tetrazol-5-yl)-2-pyridinyl]phenyl]methyl]-2H-imidazol-2-one;

1-(2-chloro-6-fluorophenyl)-4-propyl-1,3-dihydro-3-[[4-[3-(1H-tetrazol-5-yl)-2-pyridinyl]phenyl]methyl]-2H-imidazol-2-one;

1-(2,6-dichlorophenyl)-4-propyl-1,3-dihydro-3-[[4-[3-(1H-tetrazol-5-yl)-2-pyridinyl]phenyl]methyl]-2H-imidazol-2-one;

1-(2-chloro-6-methoxyphenyl)-4-propyl-1,3-dihydro-3-[[4-[3-(1H-tetrazol-5-yl)-2-pyridinyl]phenyl]methyl]-2H-imidazol-2-one;

1-(2-bromo-6-methylphenyl)-4-propyl-1,3-dihydro-3-[[4-[3-(1H-tetrazol-5-yl)-2-pyridinyl]phenyl]methyl]-2H-imidazol-2-one;

1-(2-bromo-6-ethylphenyl)-4-propyl-1,3-dihydro-3-[[4-[3-(1H-tetrazol-5-yl)-2-pyridinyl]phenyl]methyl]-2H-imidazol-2-one;

1-(2-bromo-6-isopropylphenyl)-4-propyl-1,3-dihydro-3-[[4-[3-(1H-tetrazol-5-yl)-2-pyridinyl]phenyl]methyl]-2H-imidazol-2-one;

1-(2-bromo-6-tertbutylphenyl)-4-propyl-1,3-dihydro-3-[[4-[3-(1H-tetrazol-5-yl)-2-pyridinyl]phenyl]methyl]-2H-imidazol-2-one;

1-(2-bromo-6-fluorophenyl)-4-propyl-1,3-dihydro-3-[[4-[3-(1H-tetrazol-5-yl)-2-pyridinyl]phenyl]methyl]-2H-imidazol-2-one;

1-(2-bromo-6-chlorophenyl)-4-propyl-1,3-dihydro-3-[[4-[3-(1H-tetrazol-5-yl)-2-pyridinyl]phenyl]methyl]-2H-imidazol-2-one;

1-(2-bromo-6-methoxyphenyl)-4-propyl-1,3-dihydro-3-[[4-[3-(1H-tetrazol-5-yl)-2-pyridinyl]phenyl]methyl]-2H-imidazol-2-one;

1-(2-methoxy-6-methylphenyl)-4-propyl-1,3-dihydro-3-[[4-[3-(1H-tetrazol-5-yl)-2-pyridinyl]phenyl]methyl]-2H-imidazol-2-one;

1-(2-methoxy-6-ethylphenyl)-4-propyl-1,3-dihydro-3-[[4-[3-(1H-tetrazol-5-yl)-2-pyridinyl]phenyl]methyl]-2H-imidazol-2-one;

1-(2-methoxy-6-isopropylphenyl)-4-propyl-1,3-dihydro-3-[[4-[3-(1H-tetrazol-5-yl)-2-pyridinyl]phenyl]methyl]-2H-imidazol-2-one;

1-(2-methoxy-6-tertbutylphenyl)-4-propyl-1,3-dihydro-3-[[4-[3-(1H-tetrazol-5-yl)-2-pyridinyl]phenyl]methyl]-2H-imidazol-2-one;

1-(2-methoxy-6-fluorophenyl)-4-propyl-1,3-dihydro-3-[[4-[3-(1H-tetrazol-5-yl)-2-pyridinyl]phenyl]methyl]-2H-imidazol-2-one;

1-(2-methoxy-6-chlorophenyl)-4-propyl-1,3-dihydro-3-[[4-[3-(1H-tetrazol-5-yl)-2-pyridinyl]phenyl]methyl]-2H-imidazol-2-one;

1-(2,6-dimethoxyphenyl)-4-propyl-1,3-dihydro-3-[[4-[3-(1H-tetrazol-5-yl)-2-pyridinyl]phenyl]methyl]-2H-imidazol-2-one;

1-(2-carboxy-6-methylphenyl)-4-propyl-1,3-dihydro-3-[[4-[3-(1H-tetrazol-5-yl)-2-pyridinyl]phenyl]methyl]-2H-imidazol-2-one;

1-(2-carboxy-6-ethylphenyl)-4-propyl-1,3-dihydro-3-[[4-[3-(1H-tetrazol-5-yl)-2-pyridinyl]phenyl]methyl]-2H-imidazol-2-one;

1-(2-carboxy-6-isopropylphenyl)-4-propyl-1,3-dihydro-3-[[4-[3-(1H-tetrazol-5-yl)-2-pyridinyl]phenyl]methyl]-2H-imidazol-2-one;

1-(2-carboxy-6-tertbutylphenyl)-4-propyl-1,3-dihydro-3-[[4-[3-(1H-tetrazol-5-yl)-2-pyridinyl]phenyl]methyl]-2H-imidazol-2-one;

1-(2-carboxy-6-fluorophenyl)-4-propyl-1,3-dihydro-3-[[4-[3-(1H-tetrazol-5-yl)-2-pyridinyl]phenyl]methyl]-2H-imidazol-2-one;

1-(2-carboxy-6-chlorophenyl)-4-propyl-1,3-dihydro-3-[[4-[3-(1H-tetrazol-5-yl)-2-pyridinyl]phenyl]methyl]-2H-imidazol-2-one;

1-(2-carboxy-6-methoxyphenyl)-4-propyl-1,3-dihydro-3-[[4-[3-(1H-tetrazol-5-yl)-2-pyridinyl]phenyl]methyl]-2H-imidazol-2-one;

1-(2-acetyl-6-methylphenyl)-4-propyl-1,3-dihydro-3-[[4-[3-(1H-tetrazol-5-yl)-2-pyridinyl]phenyl]methyl]-2H-imidazol-2-one;

1-(2-acetyl-6-ethylphenyl)-4-propyl-1,3-dihydro-3-[[4-[3-(1H-tetrazol-5-yl)-2-pyridinyl]phenyl]methyl]-2H-imidazol-2-one;

1-(2-acetyl-6-isopropylphenyl)-4-propyl-1,3-dihydro-3-[[4-[3-(1H-tetrazol-5-yl)-2-pyridinyl]phenyl]methyl]-2H-imidazol-2-one;

1-(2-acetyl-6-tertbutylphenyl)-4-propyl-1,3-dihydro-3-[[4-[3-(1H-tetrazol-5-yl)-2-pyridinyl]phenyl]methyl]-2H-imidazol-2-one;

1-(2-acetyl-6-fluorophenyl)-4-propyl-1,3-dihydro-3-[[4-[3-(1H-tetrazol-5-yl)-2-pyridinyl]phenyl]methyl]-2H-imidazol-2-one;

1-(2-acetyl-6-chlorophenyl)-4-propyl-1,3-dihydro-3-[[4-[3-(1H-tetrazol-5-yl)-2-pyridinyl]phenyl]methyl]-2H-imidazol-2-one;

1-(2-acetyl-6-methoxyphenyl)-4-propyl-1,3-dihydro-3-[[4-[3-(1H-tetrazol-5-yl)-2-pyridinyl]phenyl]methyl]-2H-imidazol-2-one;

1-(2,6-dimethylphenyl)-4-butyl-1,3-dihydro-3-[[4-[3-(1H-tetrazol-5-yl)-2-pyridinyl]phenyl]methyl]-2H-imidazol-2-one;

1-(2-methyl-6-ethylphenyl)-4-butyl-1,3-dihydro-3-[[4-[3-(1H-tetrazol-5-yl)-2-pyridinyl]phenyl]methyl]-2H-imidazol-2-one;

1-(2-methyl-6-isopropylphenyl)-4-butyl-1,3-dihydro-3-[[4-[3-(1H-tetrazol-5-yl)-2-pyridinyl]phenyl]methyl]-2H-imidazol-2-one;

1-(2-methyl-6-tertbutylphenyl)-4-butyl-1,3-dihydro-3-[[4-[3-(1H-tetrazol-5-yl)-2-pyridinyl]phenyl]methyl]-2H-imidazol-2-one;

1-(2-methyl-6-fluorophenyl)-4-butyl-1,3-dihydro-3-[[4-[3-(1H-tetrazol-5-yl)-2-pyridinyl]phenyl]methyl]-2H-imidazol-2-one;
1-(2-methyl-6-chlorophenyl)-4-butyl-1,3-dihydro-3-[[4-[3-(1H-tetrazol-5-yl)-2-pyridinyl]phenyl]methyl]-2H-imidazol-2-one;
1-(2-methyl-6-methoxyphenyl)-4-butyl-1,3-dihydro-3-[[4-[3-(1H-tetrazol-5-yl)-2-pyridinyl]phenyl]methyl]-2H-imidazol-2-one;
1-(2-ethyl-6-methylphenyl)-4-butyl-1,3-dihydro-3-[[4-[3-(1H-tetrazol-5-yl)-2-pyridinyl]phenyl]methyl]-2H-imidazol-2-one;
1-(2,6-diethylphenyl)-4-butyl-1,3-dihydro-3-[[4-[3-(1H-tetrazol-5-yl)-2-pyridinyl]phenyl]methyl]-2H-imidazol-2-one;
1-(2-ethyl-6-isopropylphenyl)-4-butyl-1,3-dihydro-3-[[4-[3-(1H-tetrazol-5-yl)-2-pyridinyl]phenyl]methyl]-2H-imidazol-2-one;
1-(2-ethyl-6-tertbutylphenyl)-4-butyl-1,3-dihydro-3-[[4-[3-(1H-tetrazol-5-yl)-2-pyridinyl]phenyl]methyl]-2H-imidazol-2-one;
1-(2-ethyl-6-fluorophenyl)-4-butyl-1,3-dihydro-3-[[4-[3-(1H-tetrazol-5-yl)-2-pyridinyl]phenyl]methyl]-2H-imidazol-2-one;
1-(2-ethyl-6-chlorophenyl)-4-butyl-1,3-dihydro-3-[[4-[3-(1H-tetrazol-5-yl)-2-pyridinyl]phenyl]methyl]-2H-imidazol-2-one;
1-(2-ethyl-6-methoxyphenyl)-4-butyl-1,3-dihydro-3-[[4-[3-(1H-tetrazol-5-yl)-2-pyridinyl]phenyl]methyl]-2H-imidazol-2-one;
1-(2-propyl-6-methylphenyl)-4-butyl-1,3-dihydro-3-[[4-[3-(1H-tetrazol-5-yl)-2-pyridinyl]phenyl]methyl]-2H-imidazol-2-one;
1-(2-propyl-6-ethylphenyl)-4-butyl-1,3-dihydro-3-[[4-[3-(1H-tetrazol-5-yl)-2-pyridinyl]phenyl]methyl]-2H-imidazol-2-one;
1-(2-propyl-6-isopropylphenyl)-4-butyl-1,3-dihydro-3-[[4-[3-(1H-tetrazol-5-yl)-2-pyridinyl]phenyl]methyl]-2H-imidazol-2-one;
1-(2-propyl-6-tertbutylphenyl)-4-butyl-1,3-dihydro-3-[[4-[3-(1H-tetrazol-5-yl)-2-pyridinyl]phenyl]methyl]-2H-imidazol-2-one;
1-(2-propyl-6-fluorophenyl)-4-butyl-1,3-dihydro-3-[[4-[3-(1H-tetrazol-5-yl)-2-pyridinyl]phenyl]methyl]-2H-imidazol-2-one;
1-(2-propyl-6-chlorophenyl)-4-butyl-1,3-dihydro-3-[[4-[3-(1H-tetrazol-5-yl)-2-pyridinyl]phenyl]methyl]-2H-imidazol-2-one;
1-(2-propyl-6-methoxyphenyl)-4-butyl-1,3-dihydro-3-[[4-[3-(1H-tetrazol-5-yl)-2-pyridinyl]phenyl]methyl]-2H-imidazol-2-one;
1-(2-isopropyl-6-methylphenyl)-4-butyl-1,3-dihydro-3-[[4-[3-(1H-tetrazol-5-yl)-2-pyridinyl]phenyl]methyl]-2H-imidazol-2-one;
1-(2-isopropyl-6-ethylphenyl)-4-butyl-1,3-dihydro-3-[[4-[3-(1H-tetrazol-5-yl)-2-pyridinyl]phenyl]methyl]-2H-imidazol-2-one;
1-(2,6-diisopropylphenyl)-4-butyl-1,3-dihydro-3-[[4-[3-(1H-tetrazol-5-yl)-2-pyridinyl]phenyl]methyl]-2H-imidazol-2-one;
1-(2-isopropyl-6-tertbutylphenyl)-4-butyl-1,3-dihydro-3-[[4-[3-(1H-tetrazol-5-yl)-2-pyridinyl]phenyl]methyl]-2H-imidazol-2-one;
1-(2-isopropyl-6-fluorophenyl)-4-butyl-1,3-dihydro-3-[[4-[3-(1H-tetrazol-5-yl)-2-pyridinyl]phenyl]methyl]-2H-imidazol-2-one;
1-(2-isopropyl-6-chlorophenyl)-4-butyl-1,3-dihydro-3-[[4-[3-(1H-tetrazol-5-yl)-2-pyridinyl]phenyl]methyl]-2H-imidazol-2-one;
1-(2-isopropyl-6-methoxyphenyl)-4-butyl-1,3-dihydro-3-[[4-[3-(1H-tetrazol-5-yl)-2-pyridinyl]phenyl]methyl]-2H-imidazol-2-one;
1-(2-tertbutyl-6-methylphenyl)-4-butyl-1,3-dihydro-3-[[4-[3-(1H-tetrazol-5-yl)-2-pyridinyl]phenyl]methyl]-2H-imidazol-2-one;
1-(2-tertbutyl-6-ethylphenyl)-4-butyl-1,3-dihydro-3-[[4-[3-(1H-tetrazol-5-yl)-2-pyridinyl]phenyl]methyl]-2H-imidazol-2-one;
1-(2-tertbutyl-6-isopropylphenyl)-4-butyl-1,3-dihydro-3-[[4-[3-(1H-tetrazol-5-yl)-2-pyridinyl]phenyl]methyl]-2H-imidazol-2-one;
1-(2,6-ditertbutylphenyl)-4-butyl-1,3-dihydro-3-[[4-[3-(1H-tetrazol-5-yl)-2-pyridinyl]phenyl]methyl]-2H-imidazol-2-one;
1-(2-tertbutyl-6-fluorophenyl)-4-butyl-1,3-dihydro-3-[[4-[3-(1H-tetrazol-5-yl)-2-pyridinyl]phenyl]methyl]-2H-imidazol-2-one;
1-(2-tertbutyl-6-chlorophenyl)-4-butyl-1,3-dihydro-3-[[4-[3-(1H-tetrazol-5-yl)-2-pyridinyl)phenyl]methyl]-2H-imidazol-2-one;

1-(2-tertbutyl-6-methoxyphenyl)-4-butyl-1,3-dihydro-3-[[4-[3-(1H-tetrazol-5-yl)-2-pyridinyl]phenyl]methyl]-2H-imidazol-2-one;

1-(2-fluoro-6-methylphenyl)-4-butyl-1,3-dihydro-3-[[4-[3-(1H-tetrazol-5-yl)-2-pyridinyl]phenyl]methyl]-2H-imidazol-2-one;

1-(2-fluoro-6-ethylphenyl)-4-butyl-1,3-dihydro-3-[[4-[3-(1H-tetrazol-5-yl)-2-pyridinyl]phenyl]methyl]-2H-imidazol-2-one;

1-(2-fluoro-6-isopropylphenyl)-4-butyl-1,3-dihydro-3-[[4-[3-(1H-tetrazol-5-yl)-2-pyridinyl]phenyl]methyl]-2H-imidazol-2-one;

1-(2-fluoro-6-tertbutylphenyl)-4-butyl-1,3-dihydro-3-[[4-[3-(1H-tetrazol-5-yl)-2-pyridinyl]phenyl]methyl]-2H-imidazol-2-one;

1-(2-fluoro-6-chlorophenyl)-4-butyl-1,3-dihydro-3-[[4-[3-(1H-tetrazol-5-yl)-2-pyridinyl]phenyl]methyl]-2H-imidazol-2-one;

1-(2-fluoro-6-methoxyphenyl)-4-butyl-1,3-dihydro-3-[[4-[3-(1H-tetrazol-5-yl)-2-pyridinyl]phenyl]methyl]-2H-imidazol-2-one;

1-(2-chloro-6-methylphenyl)-4-butyl-1,3-dihydro-3-[[4-[3-(1H-tetrazol-5-yl)-2-pyridinyl]phenyl]methyl]-2H-imidazol-2-one;

1-(2-chloro-6-ethylphenyl)-4-butyl-1,3-dihydro-3-[[4-[3-(1H-tetrazol-5-yl)-2-pyridinyl]phenyl]methyl]-2H-imidazol-2-one;

1-(2-chloro-6-isopropylphenyl)-4-butyl-1,3-dihydro-3-[[4-[3-(1H-tetrazol-5-yl)-2-pyridinyl]phenyl]methyl]-2H-imidazol-2-one;

1-(2-chloro-6-tertbutylphenyl)-4-butyl-1,3-dihydro-3-[[4-[3-(1H-tetrazol-5-yl)-2-pyridinyl]phenyl]methyl]-2H-imidazol-2-one;

1-(2-chloro-6-fluorophenyl)-4-butyl-1,3-dihydro-3-[[4-[3-(1H-tetrazol-5-yl)-2-pyridinyl]phenyl]methyl]-2H-imidazol-2-one;

1-(2,6-dichlorophenyl)-4-butyl-1,3-dihydro-3-[[4-[3-(1H-tetrazol-5-yl)-2-pyridinyl]phenyl]methyl]-2H-imidazol-2-one;

1-(2-chloro-6-methoxyphenyl)-4-butyl-1,3-dihydro-3-[[4-[3-(1H-tetrazol-5-yl)-2-pyridinyl]phenyl]methyl]-2H-imidazol-2-one;

1-(2-bromo-6-methylphenyl)-4-butyl-1,3-dihydro-3-[[4-[3-(1H-tetrazol-5-yl)-2-pyridinyl]phenyl]methyl]-2H-imidazol-2-one;

1-(2-bromo-6-ethylphenyl)-4-butyl-1,3-dihydro-3-[[4-[3-(1H-tetrazol-5-yl)-2-pyridinyl]phenyl]methyl]-2H-imidazol-2-one;

1-(2-bromo-6-isopropylphenyl)-4-butyl-1,3-dihydro-3-[[4-[3-(1H-tetrazol-5-yl)-2-pyridinyl]phenyl]methyl]-2H-imidazol-2-one;

1-(2-bromo-6-tertbutylphenyl)-4-butyl-1,3-dihydro-3-[[4-[3-(1H-tetrazol-5-yl)-2-pyridinyl]phenyl]methyl]-2H-imidazol-2-one;

1-(2-bromo-6-fluorophenyl)-4-butyl-1,3-dihydro-3-[[4-[3-(1H-tetrazol-5-yl)-2-pyridinyl]phenyl]methyl]-2H-imidazol-2-one;

1-(2-bromo-6-chlorophenyl)-4-butyl-1,3-dihydro-3-[[4-[3-(1H-tetrazol-5-yl)-2-pyridinyl]phenyl]methyl]-2H-imidazol-2-one;

1-(2-bromo-6-methoxyphenyl)-4-butyl-1,3-dihydro-3-[[4-[3-(1H-tetrazol-5-yl)-2-pyridinyl]phenyl]methyl]-2H-imidazol-2-one;

1-(2-methoxy-6-methylphenyl)-4-butyl-1,3-dihydro-3-[[4-[3-(1H-tetrazol-5-yl)-2-pyridinyl]phenyl]methyl]-2H-imidazol-2-one;

1-(2-methoxy-6-ethylphenyl)-4-butyl-1,3-dihydro-3-[[4-[3-(1H-tetrazol-5-yl)-2-pyridinyl]phenyl]methyl]-2H-imidazol-2-one;

1-(2-methoxy-6-isopropylphenyl)-4-butyl-1,3-dihydro-3-[[4-[3-(1H-tetrazol-5-yl)-2-pyridinyl]phenyl]methyl]-2H-imidazol-2-one;

1-(2-methoxy-6-tertbutylphenyl)-4-butyl-1,3-dihydro-3-[[4-[3-(1H-tetrazol-5-yl)-2-pyridinyl]phenyl]methyl]-2H-imidazol-2-one;

1-(2-methoxy-6-fluorophenyl)-4-butyl-1,3-dihydro-3-[[4-[3-(1H-tetrazol-5-yl)-2-pyridinyl]phenyl]methyl]-2H-imidazol-2-one;

1-(2-methoxy-6-chlorophenyl)-4-butyl-1,3-dihydro-3-[[4-[3-(1H-tetrazol-5-yl)-2-pyridinyl]phenyl]methyl]-2H-imidazol-2-one;

1-(2,6-dimethoxyphenyl)-4-butyl-1,3-dihydro-3-[[4-[3-(1H-tetrazol-5-yl)-2-pyridinyl]phenyl]methyl]-2H-imidazol-2-one;

1-(2-carboxy-6-methylphenyl)-4-butyl-1,3-dihydro-3-[[4-[3-(1H-tetrazol-5-yl)-2-pyridinyl]phenyl]methyl]-2H-imidazol-2-one;

1-(2-carboxy-6-ethylphenyl)-4-butyl-1,3-dihydro-3-[[4-[3-(1H-tetrazol-5-yl)-2-pyridinyl]phenyl]methyl]-2H-imidazol-2-one;

1-(2-carboxy-6-isopropylphenyl)-4-butyl-1,3-dihydro-3-[[4-[3-(1H-tetrazol-5-yl)-2-pyridinyl]phenyl]methyl]-2H-imidazol-2-one;

1-(2-carboxy-6-tertbutylphenyl)-4-butyl-1,3-dihydro-3-[[4-[3-(1H-tetrazol-5-yl)-2-pyridinyl]phenyl]methyl]-2H-imidazol-2-one;

1-(2-carboxy-6-fluorophenyl)-4-butyl-1,3-dihydro-3-[[4-[3-(1H-tetrazol-5-yl)-2-pyridinyl]phenyl]methyl]-2H-imidazol-2-one;

1-(2-carboxy-6-chlorophenyl)-4-butyl-1,3-dihydro-3-[[4-[3-(1H-tetrazol-5-yl)-2-pyridinyl]phenyl]methyl]-2H-imidazol-2-one;

1-(2-carboxy-6-methoxyphenyl)-4-butyl-1,3-dihydro-3-[[4-[3-(1H-tetrazol-5-yl)-2-pyridinyl]phenyl]methyl]-2H-imidazol-2-one;

1-(2-acetyl-6-methylphenyl)-4-butyl-1,3-dihydro-3-[[4-[3-(1H-tetrazol-5-yl)-2-pyridinyl]phenyl]methyl]-2H-imidazol-2-one;

1-(2-acetyl-6-ethylphenyl)-4-butyl-1,3-dihydro-3-[[4-[3-(1H-tetrazol-5-yl)-2-pyridinyl]phenyl]methyl]-2H-imidazol-2-one;

1-(2-acetyl-6-isopropylphenyl)-4-butyl-1,3-dihydro-3-[[4-[3-(1H-tetrazol-5-yl)-2-pyridinyl]phenyl]methyl]-2H-imidazol-2-one;

1-(2-acetyl-6-tertbutylphenyl)-4-butyl-1,3-dihydro-3-[[4-[3-(1H-tetrazol-5-yl)-2-pyridinyl]phenyl]methyl]-2H-imidazol-2-one;

1-(2-acetyl-6-fluorophenyl)-4-butyl-1,3-dihydro-3-[[4-[3-(1H-tetrazol-5-yl)-2-pyridinyl]phenyl]methyl]-2H-imidazol-2-one;

1-(2-acetyl-6-chlorophenyl)-4-butyl-1,3-dihydro-3-[[4-[3-(1H-tetrazol-5-yl)-2-pyridinyl]phenyl]methyl]-2H-imidazol-2-one; and

1-(2-acetyl-6-methoxyphenyl)-4-butyl-1,3-dihydro-3-[[4-[3-(1H-tetrazol-5-yl)-2-pyridinyl]phenyl]methyl]-2H-imidazol-2-one.

Within Formula I there is a first group or compounds of more interest as represented by Formula V:

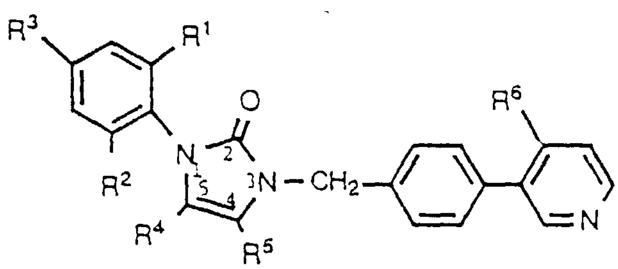

(V)

wherein each of $R^1$, $R^2$ and $R^3$ is independently selected from methyl, ethyl, n-propyl, isopropyl, tert-butyl, methoxy, ethoxy, propoxy, isopropoxy, tert-butoxy, cyano, fluoro, chloro, iodo, bromo, hydroxy, nitro, amino, N-methylamino, N,N-dimethylamino, N-ethylamino, N,N-diethylamino, carboxyl, methoxycarbonyl, ethoxycarbonyl, formyl, methylcarbonyl, ethylcarbonyl and trifluoromethylcarbonyl; wherein further $R^3$ may be hydrido, wherein further when $R^1$ is ethyl or tert.-butyl then $R^2$ can be hydrido, with the proviso that $R^1$ and $R^2$ can not be fluoro or chloro simultaneously; wherein $R^4$ is selected from hydrido, methyl, fluoro, chloro, monofluoromethyl, difluoromethyl, trifluoromethyl, formyl, carboxyl and dimethoxymethyl; wherein $R^5$ is selected from methyl, ethyl, n-propyl, isopropyl, n-butyl, sec-butyl, isobutyl, tert-butyl, n-pentyl, isopentyl, neopentyl, phenyl, benzyl, phenethyl, cyclopropyl, cyclobutyl, cyclopentyl, cyclohexyl, cyclopropylmethyl, cyclopropylethyl, cyclobutylmethyl, cyclobutylethyl, cyclopentylmethyl, cyclopentylethyl, cyclohexylmethyl and cyclohexylethyl; wherein $R^6$ is an acidic group selected from COOH and

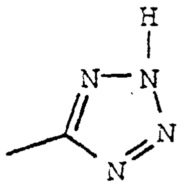

;

or a stereoisomer or a tautomer thereof or a pharmaceutically-acceptable salt thereof.

A family of specific compounds of particular interest within this first class of higher-interest compounds of the first group of compounds of Formula V consists of compounds and their stereoisomers and tautomers and the pharmaceutically-acceptable salts thereof, said compounds consisting of

1-(2-ethylphenyl)-4-propyl-1,3-dihydro-3-[[4-[4-(1H-tetrazol-5-yl)-3-pyridinyl]phenyl]methyl]-2H-imidazol-2-one;
1-(2-tertbutylphenyl)-4-propyl-1,3-dihydro-3-[[4-[4-(1H-tetrazol-5-yl)-3-pyridinyl]phenyl]methyl]-2H-imidazol-2-one;
1-(2-ethylphenyl)-4-butyl-1,3-dihydro-3-[[4-[4-(1H-tetrazol-5-yl)-3-pyridinyl]phenyl]methyl]-2H-imidazol-2-one;
1-(2-tertbutylphenyl)-4-butyl-1,3-dihydro-3-[[4-[4-(1H-tetrazol-5-yl)-3-pyridinyl]phenyl]methyl]-2H-imidazol-2-one;

A family of specific compounds of particular interest within this second class of higher-interest compounds of the first group of compounds of Formula V consists of compounds and their stereoisomers and tautomers and the pharmaceutically-acceptable salts thereof, said compounds consisting of

1-(2,6-dimethylphenyl)-4-propyl-1,3-dihydro-3-[[4-[4-(1H-tetrazol-5-yl)-3-pyridinyl]phenyl]methyl]-2H-imidazol-2-one;
1-(2-methyl-6-ethylphenyl)-4-propyl-1,3-dihydro-3-[[4-[4-(1H-tetrazol-5-yl)-3-pyridinyl]phenyl]methyl]-2H-imidazol-2-one;
1-(2-methyl-6-isopropylphenyl)-4-propyl-1,3-dihydro-3-[[4-[4-(1H-tetrazol-5-yl)-3-pyridinyl]phenyl]methyl]-2H-imidazol-2-one;
1-(2-methyl-6-tertbutylphenyl)-4-propyl-1,3-dihydro-3-[[4-[4-(1H-tetrazol-5-yl)-3-pyridinyl]phenyl]methyl]-2H-imidazol-2-one;
1-(2-methyl-6-fluorophenyl)-4-propyl-1,3-dihydro-3-[[4-[4-(1H-tetrazol-5-yl)-3-pyridinyl]phenyl]methyl]-2H-imidazol-2-one;
1-(2-methyl-6-chlorophenyl)-4-propyl-1,3-dihydro-3-[[4-[4-(1H-tetrazol-5-yl)-3-pyridinyl]phenyl]methyl]-2H-imidazol-2-one;
1-(2-methyl-6-methoxyphenyl)-4-propyl-1,3-dihydro-3-[[4-[4-(1H-tetrazol-5-yl)-3-pyridinyl]phenyl]methyl]-2H-imidazol-2-one;
1-(2-ethyl-6-methylphenyl)-4-propyl-1,3-dihydro-3-[[4-[4-(1H-tetrazol-5-yl)-3-pyridinyl]phenyl]methyl]-2H-imidazol-2-one;
1-(2,6-diethylphenyl)-4-propyl-1,3-dihydro-3-[[4-[4-(1H-tetrazol-5-yl)-3-pyridinyl]phenyl]methyl]-2H-imidazol-2-one;
1-(2-ethyl-6-isopropylphenyl)-4-propyl-1,3-dihydro-3-[[4-[4-(1H-tetrazol-5-yl)-3-pyridinyl]phenyl]methyl]-2H-imidazol-2-one;
1-(2-ethyl-6-tertbutylphenyl)-4-propyl-1,3-dihydro-3-[[4-[4-(1H-tetrazol-5-yl)-3-pyridinyl]phenyl]methyl]-2H-imidazol-2-one;
1-(2-ethyl-6-fluorophenyl)-4-propyl-1,3-dihydro-3-[[4-[4-(1H-tetrazol-5-yl)-3-pyridinyl]phenyl]methyl]-2H-imidazol-2-one;
1-(2-ethyl-6-chlorophenyl)-4-propyl-1,3-dihydro-3-[[4-[4-(1H-tetrazol-5-yl)-3-pyridinyl]phenyl]methyl]-2H-imidazol-2-one;
1-(2-ethyl-6-methoxyphenyl)-4-propyl-1,3-dihydro-3-[[4-[4-(1H-tetrazol-5-yl)-3-pyridinyl]phenyl]methyl]-2H-imidazol-2-one;
1-(2-propyl-6-methylphenyl)-4-propyl-1,3-dihydro-3-[[4-[4-(1H-tetrazol-5-yl)-3-pyridinyl]phenyl]methyl]-2H-imidazol-2-one;
1-(2-propyl-6-ethylphenyl)-4-propyl-1,3-dihydro-3-[[4-[4-(1H-tetrazol-5-yl)-3-pyridinyl]phenyl]methyl]-2H-imidazol-2-one;
1-(2-propyl-6-isopropylphenyl)-4-propyl-1,3-dihydro-3-[[4-[4-(1H-tetrazol-5-yl)-3-pyridinyl]phenyl]methyl]-2H-imidazol-2-one;
1-(2-propyl-6-tertbutylphenyl)-4-propyl-1,3-dihydro-3-[[4-[4-(1H-tetrazol-5-yl)-3-pyridinyl]phenyl]methyl]-2H-imidazol-2-one;

1-(2-propyl-6-fluorophenyl)-4-propyl-1,3-dihydro-3-[[4-[4-(1H-tetrazol-5-yl)-3-pyridinyl]phenyl]methyl]-2H-imidazol-2-one;

1-(2-propyl-6-chlorophenyl)-4-propyl-1,3-dihydro-3-[[4-[4-(1H-tetrazol-5-yl)-3-pyridinyl]phenyl]methyl]-2H-imidazol-2-one;

1-(2-propyl-6-methoxyphenyl)-4-propyl-1,3-dihydro-3-[[4-[4-(1H-tetrazol-5-yl)-3-pyridinyl]phenyl]methyl]-2H-imidazol-2-one;

1-(2-isopropyl-6-methylphenyl)-4-propyl-1,3-dihydro-3-[[4-[4-(1H-tetrazol-5-yl)-3-pyridinyl]phenyl]methyl]-2H-imidazol-2-one;

1-(2-isopropyl-6-ethylphenyl)-4-propyl-1,3-dihydro-3-[[4-[4-(1H-tetrazol-5-yl)-3-pyridinyl]phenyl]methyl]-2H-imidazol-2-one;

1-(2,6-isopropylphenyl)-4-propyl-1,3-dihydro-3-[[4-[4-(1H-tetrazol-5-yl)-3-pyridinyl]phenyl]methyl]-2H-imidazol-2-one;

1-(2-isopropyl-6-tertbutylphenyl)-4-propyl-1,3-dihydro-3-[[4-[4-(1H-tetrazol-5-yl)-3-pyridinyl]phenyl]methyl]-2H-imidazol-2-one;

1-(2-isopropyl-6-fluorophenyl)-4-propyl-1,3-dihydro-3-[[4-[4-(1H-tetrazol-5-yl)-3-pyridinyl]phenyl]methyl]-2H-imidazol-2-one;

1-(2-isopropyl-6-chlorophenyl)-4-propyl-1,3-dihydro-3-[[4-[4-(1H-tetrazol-5-yl)-3-pyridinyl]phenyl]methyl]-2H-imidazol-2-one;

1-(2-isopropyl-6-methoxyphenyl)-4-propyl-1,3-dihydro-3-[[4-[4-(1H-tetrazol-5-yl)-3-pyridinyl]phenyl]methyl]-2H-imidazol-2-one;

1-(2-tertbutyl-6-methylphenyl)-4-propyl-1,3-dihydro-3-[[4-[4-(1H-tetrazol-5-yl)-3-pyridinyl]phenyl]methyl]-2H-imidazol-2-one;

1-(2-tertbutyl-6-ethylphenyl)-4-propyl-1,3-dihydro-3-[[4-[4-(1H-tetrazol-5-yl)-3-pyridinyl]phenyl]methyl]-2H-imidazol-2-one;

1-(2-tertbutyl-6-isopropylphenyl)-4-propyl-1,3-dihydro-3-[[4-[4-(1H-tetrazol-5-yl)-3-pyridinyl]phenyl]methyl]-2H-imidazol-2-one;

1-(2,6-ditertbutylphenyl)-4-propyl-1,3-dihydro-3-[[4-[4-(1H-tetrazol-5-yl)-3-pyridinyl]phenyl]methyl]-2H-imidazol-2-one;

1-(2-tertbutyl-6-fluorophenyl)-4-propyl-1,3-dihydro-3-[[4-[4-(1H-tetrazol-5-yl)-3-pyridinyl]phenyl]methyl]-2H-imidazol-2-one;

1-(2-tertbutyl-6-chlorophenyl)-4-propyl-1,3-dihydro-3-[[4-[4-(1H-tetrazol-5-yl)-3-pyridinyl]phenyl]methyl]-2H-imidazol-2-one;

1-(2-tertbutyl-6-methoxyphenyl)-4-propyl-1,3-dihydro-3-[[4-[4-(1H-tetrazol-5-yl)-3-pyridinyl]phenyl]methyl]-2H-imidazol-2-one;

1-(2-fluoro-6-methylphenyl)-4-propyl-1,3-dihydro-3-[[4-[4-(1H-tetrazol-5-yl)-3-pyridinyl]phenyl]methyl]-2H-imidazol-2-one;

1-(2-fluoro-6-ethylphenyl)-4-propyl-1,3-dihydro-3-[[4-[4-(1H-tetrazol-5-yl)-3-pyridinyl]phenyl]methyl]-2H-imidazol-2-one;

1-(2-fluoro-6-isopropylphenyl)-4-propyl-1,3-dihydro-3-[[4-[4-(1H-tetrazol-5-yl)-3-pyridinyl]phenyl]methyl]-2H-imidazol-2-one;

1-(2-fluoro-6-tertbutylphenyl)-4-propyl-1,3-dihydro-3-[[4-[4-(1H-tetrazol-5-yl)-3-pyridinyl]phenyl]methyl]-2H-imidazol-2-one;

1-(2,6-difluorophenyl)-4-propyl-1,3-dihydro-3-[[4-[4-(1H-tetrazol-5-yl)-3-pyridinyl]phenyl]methyl]-2H-imidazol-2-one;

1-(2-fluoro-6-chlorophenyl)-4-propyl-1,3-dihydro-3-[[4-[4-(1H-tetrazol-5-yl)-3-pyridinyl]phenyl]methyl]-2H-imidazol-2-one;

1-(2-fluoro-6-methoxyphenyl)-4-propyl-1,3-dihydro-3-[[4-[4-(1H-tetrazol-5-yl)-3-pyridinyl]phenyl]methyl]-2H-imidazol-2-one;

1-(2-chloro-6-methylphenyl)-4-propyl-1,3-dihydro-3-[[4-[4-(1H-tetrazol-5-yl)-3-pyridinyl]phenyl]methyl]-2H-imidazol-2-one;

1-(2-chloro-6-ethylphenyl)-4-propyl-1,3-dihydro-3-[[4-[4-(1H-tetrazol-5-yl)-3-pyridinyl]phenyl]methyl]-2H-imidazol-2-one;

1-(2-chloro-6-isopropylphenyl)-4-propyl-1,3-dihydro-3-[[4-[4-(1H-tetrazol-5-yl)-3-pyridinyl]phenyl]methyl]-2H-imidazol-2-one;

1-(2-chloro-6-tertbutylphenyl)-4-propyl-1,3-dihydro-3-[[4-[4-(1H-tetrazol-5-yl)-3-pyridinyl]phenyl]methyl]-2H-imidazol-2-one;

1-(2-chloro-6-fluorophenyl)-4-propyl-1,3-dihydro-3-[[4-[4-(1H-tetrazol-5-yl)-3-pyridinyl]phenyl]methyl]-2H-imidazol-2-one;

1-(2-chloro-6-methoxyphenyl)-4-propyl-1,3-dihydro-3-[[4-[4-(1H-tetrazol-5-yl)-3-pyridinyl]phenyl]methyl]-2H-imidazol-2-one;

1-(2-bromo-6-methylphenyl)-4-propyl-1,3-dihydro-3-[[4-[4-(1H-tetrazol-5-yl)-3-pyridinyl]phenyl]methyl]-2H-imidazol-2-one;

1-(2-bromo-6-ethylphenyl)-4-propyl-1,3-dihydro-3-[[4-[4-(1H-tetrazol-5-yl)-3-pyridinyl]phenyl]methyl]-2H-imidazol-2-one;

1-(2-bromo-6-isopropylphenyl)-4-propyl-1,3-dihydro-3-[[4-[4-(1H-tetrazol-5-yl)-3-pyridinyl]phenyl]methyl]-2H-imidazol-2-one;

1-(2-bromo-6-tertbutylphenyl)-4-propyl-1,3-dihydro-3-[[4-[4-(1H-tetrazol-5-yl)-3-pyridinyl]phenyl]methyl]-2H-imidazol-2-one;

1-(2-bromo-6-fluorophenyl)-4-propyl-1,3-dihydro-3-[[4-[4-(1H-tetrazol-5-yl)-3-pyridinyl]phenyl]methyl]-2H-imidazol-2-one;

1-(2-bromo-6-chlorophenyl)-4-propyl-1,3-dihydro-3-[[4-[4-(1H-tetrazol-5-yl)-3-pyridinyl]phenyl]methyl]-2H-imidazol-2-one;

1-(2-bromo-6-methoxyphenyl)-4-propyl-1,3-dihydro-3-[[4-[4-(1H-tetrazol-5-yl)-3-pyridinyl]phenyl]methyl]-2H-imidazol-2-one;

1-(2-methoxy-6-methylphenyl)-4-propyl-1,3-dihydro-3-[[4-[4-(1H-tetrazol-5-yl)-3-pyridinyl]phenyl]methyl]-2H-imidazol-2-one;

1-(2-methoxy-6-ethylphenyl)-4-propyl-1,3-dihydro-3-[[4-[4-(1H-tetrazol-5-yl)-3-pyridinyl]phenyl]methyl]-2H-imidazol-2-one;

1-(2-methoxy-6-isopropylphenyl)-4-propyl-1,3-dihydro-3-[[4-[4-(1H-tetrazol-5-yl)-3-pyridinyl]phenyl]methyl]-2H-imidazol-2-one;

1-(2-methoxy-6-tertbutylphenyl)-4-propyl-1,3-dihydro-3-[[4-[4-(1H-tetrazol-5-yl)-3-pyridinyl]phenyl]methyl]-2H-imidazol-2-one;

1-(2-methoxy-6-fluorophenyl)-4-propyl-1,3-dihydro-3-[[4-[4-(1H-tetrazol-5-yl)-3-pyridinyl]phenyl]methyl]-2H-imidazol-2-one;

1-(2-methoxy-6-chlorophenyl)-4-propyl-1,3-dihydro-3-[[4-[4-(1H-tetrazol-5-yl)-3-pyridinyl]phenyl]methyl]-2H-imidazol-2-one;

1-(2,6-dimethoxyphenyl)-4-propyl-1,3-dihydro-3-[[4-[4-(1H-tetrazol-5-yl)-3-pyridinyl]phenyl]methyl]-2H-imidazol-2-one;

1-(2-carboxy-6-methylphenyl)-4-propyl-1,3-dihydro-3-[[4-[4-(1H-tetrazol-5-yl)-3-pyridinyl]phenyl]methyl]-2H-imidazol-2-one;

1-(2-carboxy-6-ethylphenyl)-4-propyl-1,3-dihydro-3-[[4-[4-(1H-tetrazol-5-yl)-3-pyridinyl]phenyl]methyl]-2H-imidazol-2-one;

1-(2-carboxy-6-isopropylphenyl)-4-propyl-1,3-dihydro-3-[[4-[4-(1H-tetrazol-5-yl)-3-pyridinyl]phenyl]methyl]-2H-imidazol-2-one;

1-(2-carboxy-6-tertbutylphenyl)-4-propyl-1,3-dihydro-3-[[4-[4-(1H-tetrazol-5-yl)-3-pyridinyl]phenyl]methyl]-2H-imidazol-2-one;

1-(2-carboxy-6-fluorophenyl)-4-propyl-1,3-dihydro-3-[[4-[4-(1H-tetrazol-5-yl)-3-pyridinyl]phenyl]methyl]-2H-imidazol-2-one;

1-(2-carboxy-6-chlorophenyl)-4-propyl-1,3-dihydro-3-[[4-[4-(1H-tetrazol-5-yl)-3-pyridinyl]phenyl]methyl]-2H-imidazol-2-one;

1-(2-carboxy-6-methoxyphenyl)-4-propyl-1,3-dihydro-3-[[4-[4-(1H-tetrazol-5-yl)-3-pyridinyl]phenyl]methyl]-2H-imidazol-2-one;

1-(2-acetyl-6-methylphenyl)-4-propyl-1,3-dihydro-3-[[4-[4-(1H-tetrazol-5-yl)-3-pyridinyl]phenyl]methyl]-2H-imidazol-2-one;

1-(2-acetyl-6-ethylphenyl)-4-propyl-1,3-dihydro-3-[[4-[4-(1H-tetrazol-5-yl)-3-pyridinyl]phenyl]methyl]-2H-imidazol-2-one;

1-(2-acetyl-6-isopropylphenyl)-4-propyl-1,3-dihydro-3-[[4-[4-(1H-tetrazol-5-yl)-3-pyridinyl]phenyl]methyl]-2H-imidazol-2-one;

1-(2-acetyl-6-tertbutylphenyl)-4-propyl-1,3-dihydro-3-[[4-[4-(1H-tetrazol-5-yl)-3-pyridinyl]phenyl]methyl]-2H-imidazol-2-one;

1-(2-acetyl-6-fluorophenyl)-4-propyl-1,3-dihydro-3-[[4-[4-(1H-tetrazol-5-yl)-3-pyridinyl]phenyl]methyl]-2H-imidazol-2-one;

1-(2-acetyl-6-chlorophenyl)-4-propyl-1,3-dihydro-3-[[4-[4-(1H-tetrazol-5-yl)-3-pyridinyl]phenyl]methyl]-2H-imidazol-2-one;

1-(2-acetyl-6-methoxyphenyl)-4-propyl-1,3-dihydro-3-[[4-[4-(1H-tetrazol-5-yl)-3-pyridinyl]phenyl]methyl]-2H-imidazol-2-one;

1-(2,6-dimethylphenyl)-4-butyl-1,3-dihydro-3-[[4-[4-(1H-tetrazol-5-yl)-3-pyridinyl]phenyl]methyl]-2H-imidazol-2-one;
1-(2-methyl-6-ethylphenyl)-4-butyl-1,3-dihydro-3-[[4-[4-(1H-tetrazol-5-yl)-3-pyridinyl]phenyl]methyl]-2H-imidazol-2-one;
1-(2-methyl-6-isopropylphenyl)-4-butyl-1,3-dihydro-3-[[4-[4-(1H-tetrazol-5-yl)-3-pyridinyl]phenyl]methyl]-2H-imidazol-2-one;
1-(2-methyl-6-tertbutylphenyl)-4-butyl-1,3-dihydro-3-[[4-[4-(1H-tetrazol-5-yl)-3-pyridinyl]phenyl]methyl]-2H-imidazol-2-one;
1-(2-methyl-6-fluorophenyl)-4-butyl-1,3-dihydro-3-[[4-[4-(1H-tetrazol-5-yl)-3-pyridinyl]phenyl]methyl]-2H-imidazol-2-one;
1-(2-methyl-6-chlorophenyl)-4-butyl-1,3-dihydro-3-[[4-[4-(1H-tetrazol-5-yl)-3-pyridinyl]phenyl]methyl]-2H-imidazol-2-one;
1-(2-methyl-6-methoxyphenyl)-4-butyl-1,3-dihydro-3-[[4-[4-(1H-tetrazol-5-yl)-3-pyridinyl]phenyl]methyl]-2H-imidazol-2-one;
1-(2-ethyl-6-methylphenyl)-4-butyl-1,3-dihydro-3-[[4-[4-(1H-tetrazol-5-yl)-3-pyridinyl]phenyl]methyl]-2H-imidazol-2-one;
1-(2,6-diethylphenyl)-4-butyl-1,3-dihydro-3-[[4-[4-(1H-tetrazol-5-yl)-3-pyridinyl]phenyl]methyl]-2H-imidazol-2-one;
1-(2-ethyl-6-isopropylphenyl)-4-butyl-1,3-dihydro-3-[[4-[4-(1H-tetrazol-5-yl)-3-pyridinyl]phenyl]methyl]-2H-imidazol-2-one;
1-(2-ethyl-6-tertbutylphenyl)-4-butyl-1,3-dihydro-3-[[4-[4-(1H-tetrazol-5-yl)-3-pyridinyl]phenyl]methyl]-2H-imidazol-2-one;
1-(2-ethyl-6-fluorophenyl)-4-butyl-1,3-dihydro-3-[[4-[4-(1H-tetrazol-5-yl)-3-pyridinyl]phenyl]methyl]-2H-imidazol-2-one;
1-(2-ethyl-6-chlorophenyl)-4-butyl-1,3-dihydro-3-[[4-[4-(1H-tetrazol-5-yl)-3-pyridinyl]phenyl]methyl]-2H-imidazol-2-one;
1-(2-ethyl-6-methoxyphenyl)-4-butyl-1,3-dihydro-3-[[4-[4-(1H-tetrazol-5-yl)-3-pyridinyl]phenyl]methyl]-2H-imidazol-2-one;
1-(2-propyl-6-methylphenyl)-4-butyl-1,3-dihydro-3-[[4-[4-(1H-tetrazol-5-yl)-3-pyridinyl]phenyl]methyl]-2H-imidazol-2-one;
1-(2-propyl-6-ethylphenyl)-4-butyl-1,3-dihydro-3-[[4-[4-(1H-tetrazol-5-yl)-3-pyridinyl]phenyl]methyl]-2H-imidazol-2-one;
1-(2-propyl-6-isopropylphenyl)-4-butyl-1,3-dihydro-3-[[4-[4-(1H-tetrazol-5-yl)-3-pyridinyl]phenyl]methyl]-2H-imidazol-2-one;
1-(2-propyl-6-tertbutylphenyl)-4-butyl-1,3-dihydro-3-[[4-[4-(1H-tetrazol-5-yl)-3-pyridinyl]phenyl]methyl]-2H-imidazol-2-one;
1-(2-propyl-6-fluorophenyl)-4-butyl-1,3-dihydro-3-[[4-[4-(1H-tetrazol-5-yl)-3-pyridinyl]phenyl]methyl]-2H-imidazol-2-one;
1-(2-propyl-6-chlorophenyl)-4-butyl-1,3-dihydro-3-[[4-[4-(1H-tetrazol-5-yl)-3-pyridinyl]phenyl]methyl]-2H-imidazol-2-one;
1-(2-propyl-6-methoxyphenyl)-4-butyl-1,3-dihydro-3-[[4-[4-(1H-tetrazol-5-yl)-3-pyridinyl]phenyl]methyl]-2H-imidazol-2-one;
1-(2-isopropyl-6-methylphenyl)-4-butyl-1,3-dihydro-3-[[4-[4-(1H-tetrazol-5-yl)-3-pyridinyl]phenyl]methyl]-2H-imidazol-2-one;
1-(2-isopropyl-6-ethylphenyl)-4-butyl-1,3-dihydro-3-[[4-[4-(1H-tetrazol-5-yl)-3-pyridinyl]phenyl]methyl]-2H-imidazol-2-one;
1-(2,6-isopropylphenyl)-4-butyl-1,3-dihydro-3-[[4-[4-(1H-tetrazol-5-yl)-3-pyridinyl]phenyl]methyl]-2H-imidazol-2-one;
1-(2-isopropyl-6-tertbutylphenyl)-4-butyl-1,3-dihydro-3-[[4-[4-(1H-tetrazol-5-yl)-3-pyridinyl]phenyl]methyl]-2H-imidazol-2-one;
1-(2-isopropyl-6-fluorophenyl)-4-butyl-1,3-dihydro-3-[[4-[4-(1H-tetrazol-5-yl)-3-pyridinyl]phenyl]methyl]-2H-imidazol-2-one;
1-(2-isopropyl-6-chlorophenyl)-4-butyl-1,3-dihydro-3-[[4-[4-(1H-tetrazol-5-yl)-3-pyridinyl]phenyl]methyl]-2H-imidazol-2-one;
1-(2-isopropyl-6-methoxyphenyl)-4-butyl-1,3-dihydro-3-[[4-[4-(1H-tetrazol-5-yl)-3-pyridinyl]phenyl]methyl]-2H-imidazol-2-one;
1-(2-tertbutyl-6-methylphenyl)-4-butyl-1,3-dihydro-3-[[4-[4-(1H-tetrazol-5-yl)-3-pyridinyl]phenyl]methyl]-2H-imidazol-2-one;
1-(2-tertbutyl-6-ethylphenyl)-4-butyl-1,3-dihydro-3-[[4-[4-(1H-tetrazol-5-yl)-3-pyridinyl]phenyl]methyl]-2H-imidazol-2-one;

1-(2-tertbutyl-6-isopropylphenyl)-4-butyl-1,3-dihydro-3-[[4-[4-(1H-tetrazol-5-yl)-3-pyridinyl]phenyl]methyl]-2H-imidazol-2-one;

1-(2,6-ditertbutylphenyl)-4-butyl-1,3-dihydro-3-[[4-[4-(1H-tetrazol-5-yl)-3-pyridinyl]phenyl]methyl]-2H-imidazol-2-one;

1-(2-tertbutyl-6-fluorophenyl)-4-butyl-1,3-dihydro-3-[[4-[4-(1H-tetrazol-5-yl)-3-pyridinyl]phenyl]methyl]-2H-imidazol-2-one;

1-(2-tertbutyl-6-chlorophenyl)-4-butyl-1,3-dihydro-3-[[4-[4-(1H-tetrazol-5-yl)-3-pyridinyl]phenyl]methyl]-2H-imidazol-2-one;

1-(2-tertbutyl-6-methoxyphenyl)-4-butyl-1,3-dihydro-3-[[4-[4-(1H-tetrazol-5-yl)-3-pyridinyl]phenyl]methyl]-2H-imidazol-2-one;

1-(2-fluoro-6-methylphenyl)-4-butyl-1,3-dihydro-3-[[4-[4-(1H-tetrazol-5-yl)-3-pyridinyl]phenyl]methyl]-2H-imidazol-2-one;

1-(2-fluoro-6-ethylphenyl)-4-butyl-1,3-dihydro-3-[[4-[4-(1H-tetrazol-5-yl)-3-pyridinyl]phenyl]methyl]-2H-imidazol-2-one;

1-(2-fluoro-6-isopropylphenyl)-4-butyl-1,3-dihydro-3-[[4-[4-(1H-tetrazol-5-yl)-3-pyridinyl]phenyl]methyl]-2H-imidazol-2-one;

1-(2-fluoro-6-tertbutylphenyl)-4-butyl-1,3-dihydro-3-[[4-[4-(1H-tetrazol-5-yl)-3-pyridinyl]phenyl]methyl]-2H-imidazol-2-one;

1-(2-fluoro-6-chlorophenyl)-4-butyl-1,3-dihydro-3-[[4-[4-(1H-tetrazol-5-yl)-3-pyridinyl]phenyl]methyl]-2H-imidazol-2-one;

1-(2-fluoro-6-methoxyphenyl)-4-butyl-1,3-dihydro-3-[[4-[4-(1H-tetrazol-5-yl)-3-pyridinyl]phenyl]methyl]-2H-imidazol-2-one;

1-(2-chloro-6-methylphenyl)-4-butyl-1,3-dihydro-3-[[4-[4-(1H-tetrazol-5-yl)-3-pyridinyl]phenyl]methyl]-2H-imidazol-2-one;

1-(2-chloro-6-ethylphenyl)-4-butyl-1,3-dihydro-3-[[4-[4-(1H-tetrazol-5-yl)-3-pyridinyl]phenyl]methyl]-2H-imidazol-2-one;

1-(2-chloro-6-isopropylphenyl)-4-butyl-1,3-dihydro-3-[[4-[4-(1H-tetrazol-5-yl)-3-pyridinyl]phenyl]methyl]-2H-imidazol-2-one;

1-(2-chloro-6-tertbutylphenyl)-4-butyl-1,3-dihydro-3-[[4-[4-(1H-tetrazol-5-yl)-3-pyridinyl]phenyl]methyl]-2H-imidazol-2-one;

1-(2-chloro-6-fluorophenyl)-4-butyl-1,3-dihydro-3-[[4-[4-(1H-tetrazol-5-yl)-3-pyridinyl]phenyl]methyl]-2H-imidazol-2-one;

1-(2,6-dichlorophenyl)-4-butyl-1,3-dihydro-3-[[4-[4-(1H-tetrazol-5-yl)-3-pyridinyl]phenyl]methyl]-2H-imidazol-2-one;

1-(2-chloro-6-methoxyphenyl)-4-butyl-1,3-dihydro-3-[[4-[4-(1H-tetrazol-5-yl)-3-pyridinyl]phenyl]methyl]-2H-imidazol-2-one;

1-(2-bromo-6-methylphenyl)-4-butyl-1,3-dihydro-3-[[4-[4-(1H-tetrazol-5-yl)-3-pyridinyl]phenyl]methyl]-2H-imidazol-2-one;

1-(2-bromo-6-ethylphenyl)-4-butyl-1,3-dihydro-3-[[4-[4-(1H-tetrazol-5-yl)-3-pyridinyl]phenyl]methyl]-2H-imidazol-2-one;

1-(2-bromo-6-isopropylphenyl)-4-butyl-1,3-dihydro-3-[[4-[4-(1H-tetrazol-5-yl)-3-pyridinyl]phenyl]methyl]-2H-imidazol-2-one;

1-(2-bromo-6-tertbutylphenyl)-4-butyl-1,3-dihydro-3-[[4-[4-(1H-tetrazol-5-yl)-3-pyridinyl]phenyl]methyl]-2H-imidazol-2-one;

1-(2-bromo-6-fluorophenyl)-4-butyl-1,3-dihydro-3-[[4-[4-(1H-tetrazol-5-yl)-3-pyridinyl]phenyl]methyl]-2H-imidazol-2-one;

1-(2-bromo-6-chlorophenyl)-4-butyl-1,3-dihydro-3-[[4-[4-(1H-tetrazol-5-yl)-3-pyridinyl]phenyl]methyl]-2H-imidazol-2-one;

1-(2-bromo-6-methoxyphenyl)-4-butyl-1,3-dihydro-3-[[4-[4-(1H-tetrazol-5-yl)-3-pyridinyl]phenyl]methyl]-2H-imidazol-2-one;

1-(2-methoxy-6-methylphenyl)-4-butyl-1,3-dihydro-3-[[4-[4-(1H-tetrazol-5-yl)-3-pyridinyl]phenyl]methyl]-2H-imidazol-2-one;

1-(2-methoxy-6-ethylphenyl)-4-butyl-1,3-dihydro-3-[[4-[4-(1H-tetrazol-5-yl)-3-pyridinyl]phenyl]methyl]-2H-imidazol-2-one;

1-(2-methoxy-6-isopropylphenyl)-4-butyl-1,3-dihydro-3-[[4-[4-(1H-tetrazol-5-yl)-3-pyridinyl]phenyl]methyl]-2H-imidazol-2-one;

1-(2-methoxy-6-tertbutylphenyl)-4-butyl-1,3-dihydro-3-[[4-[4-(1H-tetrazol-5-yl)-3-pyridinyl]phenyl]methyl]-2H-imidazol-2-one;

1-(2-methoxy-6-fluorophenyl)-4-butyl-1,3-dihydro-3-[[4-[4-(1H-tetrazol-5-yl)-3-pyridinyl]phenyl]methyl]-2H-imidazol-2-one;

1-(2-methoxy-6-chlorophenyl)-4-butyl-1,3-dihydro-3-[[4-[4-(1H-tetrazol-5-yl)-3-pyridinyl]phenyl]methyl]-2H-imidazol-2-one;

1-(2,6-dimethoxyphenyl)-4-butyl-1,3-dihydro-3-[[4-[4-(1H-tetrazol-5-yl)-3-pyridinyl]phenyl]methyl]-2H-imidazol-2-one;

1-(2-carboxy-6-methylphenyl)-4-butyl-1,3-dihydro-3-[[4-[4-(1H-tetrazol-5-yl)-3-pyridinyl]phenyl]methyl]-2H-imidazol-2-one;

1-(2-carboxy-6-ethylphenyl)-4-butyl-1,3-dihydro-3-[[4-[4-(1H-tetrazol-5-yl)-3-pyridinyl]phenyl]methyl]-2H-imidazol-2-one;

1-(2-carboxy-6-isopropylphenyl)-4-butyl-1,3-dihydro-3-[[4-[4-(1H-tetrazol-5-yl)-3-pyridinyl]phenyl]methyl]-2H-imidazol-2-one;

1-(2-carboxy-6-tertbutylphenyl)-4-butyl-1,3-dihydro-3-[[4-[4-(1H-tetrazol-5-yl)-3-pyridinyl]phenyl]methyl]-2H-imidazol-2-one;

1-(2-carboxy-6-fluorophenyl)-4-butyl-1,3-dihydro-3-[[4-[4-(1H-tetrazol-5-yl)-3-pyridinyl]phenyl]methyl]-2H-imidazol-2-one;

1-(2-carboxy-6-chlorophenyl)-4-butyl-1,3-dihydro-3-[[4-[4-(1H-tetrazol-5-yl)-3-pyridinyl]phenyl]methyl]-2H-imidazol-2-one;

1-(2-carboxy-6-methoxyphenyl)-4-butyl-1,3-dihydro-3-[[4-[4-(1H-tetrazol-5-yl)-3-pyridinyl]phenyl]methyl]-2H-imidazol-2-one;

1-(2-acetyl-6-methylphenyl)-4-butyl-1,3-dihydro-3-[[4-[4-(1H-tetrazol-5-yl)-3-pyridinyl]phenyl]methyl]-2H-imidazol-2-one;

1-(2-acetyl-6-ethylphenyl)-4-butyl-1,3-dihydro-3-[[4-[4-(1H-tetrazol-5-yl)-3-pyridinyl]phenyl]methyl]-2H-imidazol-2-one;

1-(2-acetyl-6-isopropylphenyl)-4-butyl-1,3-dihydro-3-[[4-[4-(1H-tetrazol-5-yl)-3-pyridinyl]phenyl]methyl]-2H-imidazol-2-one;

1-(2-acetyl-6-tertbutylphenyl)-4-butyl-1,3-dihydro-3-[[4-[4-(1H-tetrazol-5-yl)-3-pyridinyl]phenyl]methyl]-2H-imidazol-2-one;

1-(2-acetyl-6-fluorophenyl)-4-butyl-1,3-dihydro-3-[[4-[4-(1H-tetrazol-5-yl)-3-pyridinyl]phenyl]methyl]-2H-imidazol-2-one;

1-(2-acetyl-6-chlorophenyl)-4-butyl-1,3-dihydro-3-[[4-[4-(1H-tetrazol-5-yl)-3-pyridinyl]phenyl]methyl]-2H-imidazol-2-one; and

1-(2-acetyl-6-methoxyphenyl)-4-butyl-1,3-dihydro-3-[[4-[4-(1H-tetrazol-5-yl)-3-pyridinyl]phenyl]methyl]-2H-imidazol-2-one.

Within Formula I there is a first group of compounds of more interest as represented by Formula VI:

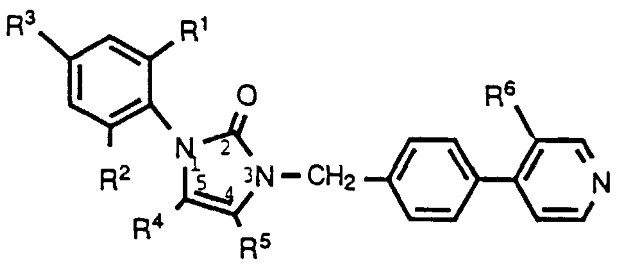

(VI)

wherein each of $R^1$, $R^2$ and $R^3$ is independently selected from methyl, ethyl, n-propyl, isopropyl, tert-butyl, methoxy, ethoxy, propoxy, isopropoxy, tert-butoxy, cyano, fluoro, chloro, iodo, bromo, hydroxy, nitro, amino, N-methylamino, N,N-dimethylamino, N-ethylamino, N,N-diethylamino, carboxyl, methoxycarbonyl, ethoxycarbonyl, formyl, methylcarbonyl, ethylcarbonyl and trifluoromethylcarbonyl; wherein $R^3$ may be hydrido, wherein when $R^1$ is ethyl or tert.-butyl, then $R^2$ can be hydrido, with the proviso that $R^1$ and $R^2$ can not be fluoro or chloro simultaneously; wherein $R^4$ is selected from hydrido, methyl, fluoro, chloro, monofluoromethyl, difluoromethyl, trifluoromethyl, formyl, carboxyl and dimethoxymethyl; wherein $R^5$ is selected from methyl, ethyl, n-propyl, isopropyl, n-butyl, sec-butyl, isobutyl, tert-butyl, n-pentyl, isopentyl, neopentyl, phenyl, benzyl, phenethyl, cyclopropyl, cyclobutyl, cyclopentyl, cyclohexyl, cyclopropylmethyl, cyclopropylethyl, cyclobutylmethyl, cyclobutylethyl, cyclopentylmethyl, cyclopentylethyl, cyclohexylmethyl and cyclohexylethyl; wherein $R^6$ is an acidic group selected from COOH and

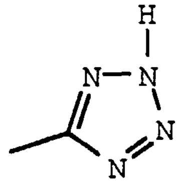

;

or a stereoisomer or a tautomer thereof or a pharmaceutically-acceptable salt thereof.

A family of specific compounds of particular interest within this first class of higher-interest compounds of the first group of compounds of Formula VI consists of compounds and their stereoisomers and tautomers and the pharmaceutically-acceptable salts thereof, said compounds consisting of

1-(2-ethylphenyl)-4-propyl-1,3-dihydro-3-[[4-[3-(1H-tetrazol-5-yl)-4-pyridinyl]phenyl]methyl]-2H-imidazol-2-one;
1-(2-tertbutylphenyl)-4-propyl-1,3-dihydro-3-[[4-[3-(1H-tetrazol-5-yl)-4-pyridinyl]phenyl]methyl]-2H-imidazol-2-one;
1-(2-ethylphenyl)-4-butyl-1,3-dihydro-3-[[4-[3-(1H-tetrazol-5-yl)-4-pyridinyl]phenyl]methyl]-2H-imidazol-2-one;
1-(2-tertbutylphenyl)-4-butyl-1,3-dihydro-3-[[4-[3-(1H-tetrazol-5-yl)-4-pyridinyl]phenyl]methyl]-2H-imidazol-2-one;

A family of specific compounds of particular interest within this second class of higher-interest compounds of the first group of compounds of Formula VI consists of compounds and their stereoisomers and tautomers and the pharmaceutically-acceptable salts thereof, said compounds consisting of

1-(2,6-dimethylphenyl)-4-propyl-1,3-dihydro-3-[[4-[3-(1H-tetrazol-5-yl)-4-pyridinyl]phenyl]methyl]-2H-imidazol-2-one;
1-(2-methyl-6-ethylphenyl)-4-propyl-1,3-dihydro-3-[[4-[3-(1H-tetrazol-5-yl)-4-pyridinyl]phenyl]methyl]-2H-imidazol-2-one;
1-(2-methyl-6-isopropylphenyl)-4-propyl-1,3-dihydro-3-[[4-[3-(1H-tetrazol-5-yl)-4-pyridinyl]phenyl]methyl]-2H-imidazol-2-one;
1-(2-methyl-6-tertbutylphenyl)-4-propyl-1,3-dihydro-3-[[4-[3-(1H-tetrazol-5-yl)-4-pyridinyl]phenyl]methyl]-2H-imidazol-2-one;
1-(2-methyl-6-fluorophenyl)-4-propyl-1,3-dihydro-3-[[4-[3-(1H-tetrazol-5-yl)-4-pyridinyl]phenyl]methyl]-2H-imidazol-2-one;
1-(2-methyl-6-chlorophenyl)-4-propyl-1,3-dihydro-3-[[4-[3-(1H-tetrazol-5-yl)-4-pyridinyl]phenyl]methyl]-2H-imidazol-2-one;
1-(2-methyl-6-methoxyphenyl)-4-propyl-1,3-dihydro-3-[[4-[3-(1H-tetrazol-5-yl)-4-pyridinyl]phenyl]methyl]-2H-imidazol-2-one;
1-(2-ethyl-6-methylphenyl)-4-propyl-1,3-dihydro-3-[[4-[3-(1H-tetrazol-5-yl)-4-pyridinyl]phenyl]methyl]-2H-imidazol-2-one;
1-(2,6-diethylphenyl)-4-propyl-1,3-dihydro-3-[[4-[3-(1H-tetrazol-5-yl)-4-pyridinyl]phenyl]methyl]-2H-imidazol-2-one;
1-(2-ethyl-6-isopropylphenyl)-4-propyl-1,3-dihydro-3-[[4-[3-(1H-tetrazol-5-yl)-4-pyridinyl]phenyl]methyl]-2H-imidazol-2-one;
1-(2-ethyl-6-tertbutylphenyl)-4-propyl-1,3-dihydro-3-[[4-[3-(1H-tetrazol-5-yl)-4-pyridinyl]phenyl]methyl]-2H-imidazol-2-one;
1-(2-ethyl-6-fluorophenyl)-4-propyl-1,3-dihydro-3-[[4-[3-(1H-tetrazol-5-yl)-4-pyridinyl]phenyl]methyl]-2H-imidazol-2-one;
1-(2-ethyl-6-chlorophenyl)-4-propyl-1,3-dihydro-3-[[4-[3-(1H-tetrazol-5-yl)-4-pyridinyl]phenyl]methyl]-2H-imidazol-2-one;
1-(2-ethyl-6-methoxyphenyl)-4-propyl-1,3-dihydro-3-[[4-[3-(1H-tetrazol-5-yl)-4-pyridinyl]phenyl]methyl]-2H-imidazol-2-one;
1-(2-propyl-6-methylphenyl)-4-propyl-1,3-dihydro-3-[[4-[3-(1H-tetrazol-5-yl)-4-pyridinyl]phenyl]methyl]-2H-imidazol-2-one;
1-(2-propyl-6-ethylphenyl)-4-propyl-1,3-dihydro-3-[[4-[3-(1H-tetrazol-5-yl)-4-pyridinyl]phenyl]methyl]-2H-imidazol-2-one;
1-(2-propyl-6-isopropylphenyl)-4-propyl-1,3-dihydro-3-[[4-[3-(1H-tetrazol-5-yl)-4-pyridinyl]phenyl]methyl]-2H-imidazol-2-one;
1-(2-propyl-6-tertbutylphenyl)-4-propyl-1,3-dihydro-3-[[4-[3-(1H-tetrazol-5-yl)-4-pyridinyl]phenyl]methyl]-2H-imidazol-2-one;

1-(2-propyl-6-fluorophenyl)-4-propyl-1,3-dihydro-3-[[4-[3-(1H-tetrazol-5-yl)-4-pyridinyl]phenyl]methyl]-2H-imidazol-2-one;

1-(2-propyl-6-chlorophenyl)-4-propyl-1,3-dihydro-3-[[4-[3-(1H-tetrazol-5-yl)-4-pyridinyl]phenyl]methyl]-2H-imidazol-2-one;

1-(2-propyl-6-methoxyphenyl)-4-propyl-1,3-dihydro-3-[[4-[3-(1H-tetrazol-5-yl)-4-pyridinyl]phenyl]methyl]-2H-imidazol-2-one;

1-(2-isopropyl-6-methylphenyl)-4-propyl-1,3-dihydro-3-[[4-[3-(1H-tetrazol-5-yl)-4-pyridinyl]phenyl]methyl]-2H-imidazol-2-one;

1-(2-isopropyl-6-ethylphenyl)-4-propyl-1,3-dihydro-3-[[4-[3-(1H-tetrazol-5-yl)-4-pyridinyl]phenyl]methyl]-2H-imidazol-2-one;

1-(2,6-diisopropylphenyl)-4-propyl-1,3-dihydro-3-[[4-[3-(1H-tetrazol-5-yl)-4-pyridinyl]phenyl]methyl]-2H-imidazol-2-one;

1-(2-isopropyl-6-tertbutylphenyl)-4-propyl-1,3-dihydro-3-[[4-[3-(1H-tetrazol-5-yl)-4-pyridinyl]phenyl]methyl]-2H-imidazol-2-one;

1-(2-isopropyl-6-fluorophenyl)-4-propyl-1,3-dihydro-3-[[4-[3-(1H-tetrazol-5-yl)-4-pyridinyl]phenyl]methyl]-2H-imidazol-2-one;

1-(2-isopropyl-6-chlorophenyl)-4-propyl-1,3-dihydro-3-[[4-[3-(1H-tetrazol-5-yl)-4-pyridinyl]phenyl]methyl]-2H-imidazol-2-one;

1-(2-isopropyl-6-methoxyphenyl)-4-propyl-1,3-dihydro-3-[[4-[3-(1H-tetrazol-5-yl)-4-pyridinyl]phenyl]methyl]-2H-imidazol-2-one;

1-(2-tertbutyl-6-methylphenyl)-4-propyl-1,3-dihydro-3-[[4-[3-(1H-tetrazol-5-yl)-4-pyridinyl]phenyl]methyl]-2H-imidazol-2-one;

1-(2-tertbutyl-6-ethylphenyl)-4-propyl-1,3-dihydro-3-[[4-[3-(1H-tetrazol-5-yl)-4-pyridinyl]phenyl]methyl]-2H-imidazol-2-one;

1-(2-tertbutyl-6-isopropylphenyl)-4-propyl-1,3-dihydro-3-[[4-[3-(1H-tetrazol-5-yl)-4-pyridinyl]phenyl]methyl]-2H-imidazol-2-one;

1-(2,6-ditertbutylphenyl)-4-propyl-1,3-dihydro-3-[[4-[3-(1H-tetrazol-5-yl)-4-pyridinyl]phenyl]methyl]-2H-imidazol-2-one;

1-(2-tertbutyl-6-fluorophenyl)-4-propyl-1,3-dihydro-3-[[4-[3-(1H-tetrazol-5-yl)-4-pyridinyl]phenyl]methyl]-2H-imidazol-2-one;

1-(2-tertbutyl-6-chlorophenyl)-4-propyl-1,3-dihydro-3-[[4-[3-(1H-tetrazol-5-yl)-4-pyridinyl]phenyl]methyl]-2H-imidazol-2-one;

1-(2-tertbutyl-6-methoxyphenyl)-4-propyl-1,3-dihydro-3-[[4-[3-(1H-tetrazol-5-yl)-4-pyridinyl]phenyl]methyl]-2H-imidazol-2-one;

1-(2-fluoro-6-methylphenyl)-4-propyl-1,3-dihydro-3-[[4-[3-(1H-tetrazol-5-yl)-4-pyridinyl]phenyl]methyl]-2H-imidazol-2-one;

1-(2-fluoro-6-ethylphenyl)-4-propyl-1,3-dihydro-3-[[4-[3-(1H-tetrazol-5-yl)-4-pyridinyl]phenyl]methyl]-2H-imidazol-2-one;

1-(2-fluoro-6-isopropylphenyl)-4-propyl-1,3-dihydro-3-[[4-[3-(1H-tetrazol-5-yl)-4-pyridinyl]phenyl]methyl]-2H-imidazol-2-one;

1-(2-fluoro-6-tertbutylphenyl)-4-propyl-1,3-dihydro-3-[[4-[3-(1H-tetrazol-5-yl)-4-pyridinyl]phenyl]methyl]-2H-imidazol-2-one;

1-(2,6-difluorophenyl)-4-propyl-1,3-dihydro-3-[[4-[3-(1H-tetrazol-5-yl)-4-pyridinyl]phenyl]methyl]-2H-imidazol-2-one;

1-(2-fluoro-6-chlorophenyl)-4-propyl-1,3-dihydro-3-[[4-[3-(1H-tetrazol-5-yl)-4-pyridinyl]phenyl]methyl]-2H-imidazol-2-one;

1-(2-fluoro-6-methoxyphenyl)-4-propyl-1,3-dihydro-3-[[4-[3-(1H-tetrazol-5-yl)-4-pyridinyl]phenyl]methyl]-2H-imidazol-2-one;

1-(2-chloro-6-methylphenyl)-4-propyl-1,3-dihydro-3-[[4-[3-(1H-tetrazol-5-yl)-4-pyridinyl]phenyl]methyl]-2H-imidazol-2-one;

1-(2-chloro-6-ethylphenyl)-4-propyl-1,3-dihydro-3-[[4-[3-(1H-tetrazol-5-yl)-4-pyridinyl]phenyl]methyl]-2H-imidazol-2-one;

1-(2-chloro-6-isopropylphenyl)-4-propyl-1,3-dihydro-3-[[4-[3-(1H-tetrazol-5-yl)-4-pyridinyl]phenyl]methyl]-2H-imidazol-2-one;

1-(2-chloro-6-tertbutylphenyl)-4-propyl-1,3-dihydro-3-[[4-[3-(1H-tetrazol-5-yl)-4-pyridinyl]phenyl]methyl]-2H-imidazol-2-one;

1-(2-chloro-6-fluorophenyl)-4-propyl-1,3-dihydro-3-[[4-[3-(1H-tetrazol-5-yl)-4-pyridinyl]phenyl]methyl]-2H-imidazol-2-one;

1-(2,6-dichlorophenyl)-4-propyl-1,3-dihydro-3-[[4-[3-(1H-tetrazol-5-yl)-4-pyridinyl]phenyl]methyl]-2H-imidazol-2-one;

1-(2-chloro-6-methoxyphenyl)-4-propyl-1,3-dihydro-3-[[4-[3-(1H-tetrazol-5-yl)-4-pyridinyl]phenyl]methyl]-2H-imidazol-2-one;

1-(2-bromo-6-methylphenyl)-4-propyl-1,3-dihydro-3-[[4-[3-(1H-tetrazol-5-yl)-4-pyridinyl]phenyl]methyl]-2H-imidazol-2-one;

1-(2-bromo-6-ethylphenyl)-4-propyl-1,3-dihydro-3-[[4-[3-(1H-tetrazol-5-yl)-4-pyridinyl]phenyl]methyl]-2H-imidazol-2-one;

1-(2-bromo-6-isopropylphenyl)-4-propyl-1,3-dihydro-3-[[4-[3-(1H-tetrazol-5-yl)-4-pyridinyl]phenyl]methyl]-2H-imidazol-2-one;

1-(2-bromo-6-tertbutylphenyl)-4-propyl-1,3-dihydro-3-[[4-[3-(1H-tetrazol-5-yl)-4-pyridinyl]phenyl]methyl]-2H-imidazol-2-one;

1-(2-bromo-6-fluorophenyl)-4-propyl-1,3-dihydro-3-[[4-[3-(1H-tetrazol-5-yl)-4-pyridinyl]phenyl]methyl]-2H-imidazol-2-one;

1-(2-bromo-6-chlorophenyl)-4-propyl-1,3-dihydro-3-[[4-[3-(1H-tetrazol-5-yl)-4-pyridinyl]phenyl]methyl]-2H-imidazol-2-one;

1-(2-bromo-6-methoxyphenyl)-4-propyl-1,3-dihydro-3-[[4-[3-(1H-tetrazol-5-yl)-4-pyridinyl]phenyl]methyl]-2H-imidazol-2-one;

1-(2-methoxy-6-methylphenyl)-4-propyl-1,3-dihydro-3-[[4-[3-(1H-tetrazol-5-yl)-4-pyridinyl]phenyl]methyl]-2H-imidazol-2-one;

1-(2-methoxy-6-ethylphenyl)-4-propyl-1,3-dihydro-3-[[4-[3-(1H-tetrazol-5-yl)-4-pyridinyl]phenyl]methyl]-2H-imidazol-2-one;

1-(2-methoxy-6-isopropylphenyl)-4-propyl-1,3-dihydro-3-[[4-[3-(1H-tetrazol-5-yl)-4-pyridinyl]phenyl]methyl]-2H-imidazol-2-one;

1-(2-methoxy-6-tertbutylphenyl)-4-propyl-1,3-dihydro-3-[[4-[3-(1H-tetrazol-5-yl)-4-pyridinyl]phenyl]methyl]-2H-imidazol-2-one;

1-(2-methoxy-6-fluorophenyl)-4-propyl-1,3-dihydro-3-[[4-[3-(1H-tetrazol-5-yl)-4-pyridinyl]phenyl]methyl]-2H-imidazol-2-one;

1-(2-methoxy-6-chlorophenyl)-4-propyl-1,3-dihydro-3-[[4-[3-(1H-tetrazol-5-yl)-4-pyridinyl]phenyl]methyl]-2H-imidazol-2-one;

1-(2,6-dimethoxyphenyl)-4-propyl-1,3-dihydro-3-[[4-[3-(1H-tetrazol-5-yl)-4-pyridinyl]phenyl]methyl]-2H-imidazol-2-one;

1-(2-carboxy-6-methylphenyl)-4-propyl-1,3-dihydro-3-[[4-[3-(1H-tetrazol-5-yl)-4-pyridinyl]phenyl]methyl]-2H-imidazol-2-one;

1-(2-carboxy-6-ethylphenyl)-4-propyl-1,3-dihydro-3-[[4-[3-(1H-tetrazol-5-yl)-4-pyridinyl]phenyl]methyl]-2H-imidazol-2-one;

1-(2-carboxy-6-isopropylphenyl)-4-propyl-1,3-dihydro-3-[[4-[3-(1H-tetrazol-5-yl)-4-pyridinyl]phenyl]methyl]-2H-imidazol-2-one;

1-(2-carboxy-6-tertbutylphenyl)-4-propyl-1,3-dihydro-3-[[4-[3-(1H-tetrazol-5-yl)-4-pyridinyl]phenyl]methyl]-2H-imidazol-2-one;

1-(2-carboxy-6-fluorophenyl)-4-propyl-1,3-dihydro-3-[[4-[3-(1H-tetrazol-5-yl)-4-pyridinyl]phenyl]methyl]-2H-imidazol-2-one;

1-(2-carboxy-6-chlorophenyl)-4-propyl-1,3-dihydro-3-[[4-[3-(1H-tetrazol-5-yl)-4-pyridinyl]phenyl]methyl]-2H-imidazol-2-one;

1-(2-carboxy-6-methoxyphenyl)-4-propyl-1,3-dihydro-3-[[4-[3-(1H-tetrazol-5-yl)-4-pyridinyl]phenyl]methyl]-2H-imidazol-2-one;

1-(2-acetyl-6-methylphenyl)-4-propyl-1,3-dihydro-3-[[4-[3-(1H-tetrazol-5-yl)-4-pyridinyl]phenyl]methyl]-2H-imidazol-2-one;

1-(2-acetyl-6-ethylphenyl)-4-propyl-1,3-dihydro-3-[[4-[3-(1H-tetrazol-5-yl)-4-pyridinyl]phenyl]methyl]-2H-imidazol-2-one;

1-(2-acetyl-6-isopropylphenyl)-4-propyl-1,3-dihydro-3-[[4-[3-(1H-tetrazol-5-yl)-4-pyridinyl]phenyl]methyl]-2H-imidazol-2-one;

1-(2-acetyl-6-tertbutylphenyl)-4-propyl-1,3-dihydro-3-[[4-[3-(1H-tetrazol-5-yl)-4-pyridinyl]phenyl]methyl]-2H-imidazol-2-one;

1-(2-acetyl-6-fluorophenyl)-4-propyl-1,3-dihydro-3-[[4-[3-(1H-tetrazol-5-yl)-4-pyridinyl]phenyl]methyl]-2H-imidazol-2-one;

1-(2-acetyl-6-chlorophenyl)-4-propyl-1,3-dihydro-3-[[4-[3-(1H-tetrazol-5-yl)-4-pyridinyl]phenyl]methyl]-2H-imidazol-2-one;

1-(2-acetyl-6-methoxyphenyl)-4-propyl-1,3-dihydro-3-[[4-[3-(1H-tetrazol-5-yl)-4-pyridinyl]phenyl]methyl]-2H-imidazol-2-one;

1-(2,6-dimethylphenyl)-4-butyl-1,3-dihydro-3-[[4-[3-(1H-tetrazol-5-yl)-4-pyridinyl]phenyl]methyl]-2H-imidazol-2-one;

1-(2-methyl-6-ethylphenyl)-4-butyl-1,3-dihydro-3-[[4-[3-(1H-tetrazol-5-yl)-4-pyridinyl]phenyl]methyl]-2H-imidazol-2-one;

1-(2-methyl-6-isopropylphenyl)-4-butyl-1,3-dihydro-3-[[4-[3-(1H-tetrazol-5-yl)-4-pyridinyl]phenyl]methyl]-2H-imidazol-2-one;

1-(2-methyl-6-tertbutylphenyl)-4-butyl-1,3-dihydro-3-[[4-[3-(1H-tetrazol-5-yl)-4-pyridinyl]phenyl]methyl]-2H-imidazol-2-one;

1-(2-methyl-6-fluorophenyl)-4-butyl-1,3-dihydro-3-[[4-[3-(1H-tetrazol-5-yl)-4-pyridinyl]phenyl]methyl]-2H-imidazol-2-one;

1-(2-methyl-6-chlorophenyl)-4-butyl-1,3-dihydro-3-[[4-[3-(1H-tetrazol-5-yl)-4-pyridinyl]phenyl]methyl]-2H-imidazol-2-one;

1-(2-methyl-6-methoxyphenyl)-4-butyl-1,3-dihydro-3-[[4-[3-(1H-tetrazol-5-yl)-4-pyridinyl]phenyl]methyl]-2H-imidazol-2-one;

1-(2-ethyl-6-methylphenyl)-4-butyl-1,3-dihydro-3-[[4-[3-(1H-tetrazol-5-yl)-4-pyridinyl]phenyl]methyl]-2H-imidazol-2-one;

1-(2,6-diethylphenyl)-4-butyl-1,3-dihydro-3-[[4-[3-(1H-tetrazol-5-yl)-4-pyridinyl]phenyl]methyl]-2H-imidazol-2-one;

1-(2-ethyl-6-isopropylphenyl)-4-butyl-1,3-dihydro-3-[[4-[3-(1H-tetrazol-5-yl)-4-pyridinyl]phenyl]methyl]-2H-imidazol-2-one;

1-(2-ethyl-6-tertbutylphenyl)-4-butyl-1,3-dihydro-3-[[4-[3-(1H-tetrazol-5-yl)-4-pyridinyl]phenyl]methyl]-2H-imidazol-2-one;

1-(2-ethyl-6-fluorophenyl)-4-butyl-1,3-dihydro-3-[[4-[3-(1H-tetrazol-5-yl)-4-pyridinyl]phenyl]methyl]-2H-imidazol-2-one;

1-(2-ethyl-6-chlorophenyl)-4-butyl-1,3-dihydro-3-[[4-[3-(1H-tetrazol-5-yl)-4-pyridinyl]phenyl]methyl]-2H-imidazol-2-one;

1-(2-ethyl-6-methoxyphenyl)-4-butyl-1,3-dihydro-3-[[4-[3-(1H-tetrazol-5-yl)-4-pyridinyl]phenyl]methyl]-2H-imidazol-2-one;

1-(2-propyl-6-methylphenyl)-4-butyl-1,3-dihydro-3-[[4-[3-(1H-tetrazol-5-yl)-4-pyridinyl]phenyl]methyl]-2H-imidazol-2-one;

1-(2-propyl-6-ethylphenyl)-4-butyl-1,3-dihydro-3-[[4-[3-(1H-tetrazol-5-yl)-4-pyridinyl]phenyl]methyl]-2H-imidazol-2-one;

1-(2-propyl-6-isopropylphenyl)-4-butyl-1,3-dihydro-3-[[4-[3-(1H-tetrazol-5-yl)-4-pyridinyl]phenyl]methyl]-2H-imidazol-2-one;

1-(2-propyl-6-tertbutylphenyl)-4-butyl-1,3-dihydro-3-[[4-[3-(1H-tetrazol-5-yl)-4-pyridinyl]phenyl]methyl]-2H-imidazol-2-one;

1-(2-propyl-6-fluorophenyl)-4-butyl-1,3-dihydro-3-[[4-[3-(1H-tetrazol-5-yl)-4-pyridinyl]phenyl]methyl]-2H-imidazol-2-one;

1-(2-propyl-6-chlorophenyl)-4-butyl-1,3-dihydro-3-[[4-[3-(1H-tetrazol-5-yl)-4-pyridinyl]phenyl]methyl]-2H-imidazol-2-one;

1-(2-propyl-6-methoxyphenyl)-4-butyl-1,3-dihydro-3-[[4-[3-(1H-tetrazol-5-yl)-4-pyridinyl]phenyl]methyl]-2H-imidazol-2-one;

1-(2-isopropyl-6-methylphenyl)-4-butyl-1,3-dihydro-3-[[4-[3-(1H-tetrazol-5-yl)-4-pyridinyl]phenyl]methyl]-2H-imidazol-2-one;

1-(2-isopropyl-6-ethylphenyl)-4-butyl-1,3-dihydro-3-[[4-[3-(1H-tetrazol-5-yl)-4-pyridinyl]phenyl]methyl]-2H-imidazol-2-one;

1-(2,6-diisopropylphenyl)-4-butyl-1,3-dihydro-3-[[4-[3-(1H-tetrazol-5-yl)-4-pyridinyl]phenyl]methyl]-2H-imidazol-2-one;

1-(2-isopropyl-6-tertbutylphenyl)-4-butyl-1,3-dihydro-3-[[4-[3-(1H-tetrazol-5-yl)-4-pyridinyl]phenyl]methyl]-2H-imidazol-2-one;

1-(2-isopropyl-6-fluorophenyl)-4-butyl-1,3-dihydro-3-[[4-[3-(1H-tetrazol-5-yl)-4-pyridinyl]phenyl]methyl]-2H-imidazol-2-one;

1-(2-isopropyl-6-chlorophenyl)-4-butyl-1,3-dihydro-3-[[4-[3-(1H-tetrazol-5-yl)-4-pyridinyl]phenyl]methyl]-2H-imidazol-2-one;

1-(2-isopropyl-6-methoxyphenyl)-4-butyl-1,3-dihydro-3-[[4-[3-(1H-tetrazol-5-yl)-4-pyridinyl]phenyl]methyl]-2H-imidazol-2-one;

1-(2-tertbutyl-6-methylphenyl)-4-butyl-1,3-dihydro-3-[[4-[3-(1H-tetrazol-5-yl)-4-pyridinyl]phenyl]methyl]-2H-imidazol-2-one;

1-(2-tertbutyl-6-ethylphenyl)-4-butyl-1,3-dihydro-3-[[4-[3-(1H-tetrazol-5-yl)-4-pyridinyl]phenyl]methyl]-2H-imidazol-2-one;

1-(2-tertbutyl-6-isopropylphenyl)-4-butyl-1,3-dihydro-3-[[4-[3-(1H-tetrazol-5-yl)-4-pyridinyl]phenyl]methyl]-2H-imidazol-2-one;

1-(2,6-ditertbutylphenyl)-4-butyl-1,3-dihydro-3-[[4-[3-(1H-tetrazol-5-yl)-4-pyridinyl]phenyl]methyl]-2H-imidazol-2-one;

1-(2-tertbutyl-6-fluorophenyl)-4-butyl-1,3-dihydro-3-[[4-[3-(1H-tetrazol-5-yl)-4-pyridinyl]phenyl]methyl]-2H-imidazol-2-one;

1-(2-tertbutyl-6-chlorophenyl)-4-butyl-1,3-dihydro-3-[[4-[3-(1H-tetrazol-5-yl)-4-pyridinyl]phenyl]methyl]-2H-imidazol-2-one;

1-(2-tertbutyl-6-methoxyphenyl)-4-butyl-1,3-dihydro-3-[[4-[3-(1H-tetrazol-5-yl)-4-pyridinyl]phenyl]methyl]-2H-imidazol-2-one;

1-(2-fluoro-6-methylphenyl)-4-butyl-1,3-dihydro-3-[[4-[3-(1H-tetrazol-5-yl)-4-pyridinyl]phenyl]methyl]-2H-imidazol-2-one;

1-(2-fluoro-6-ethylphenyl)-4-butyl-1,3-dihydro-3-[[4-[3-(1H-tetrazol-5-yl)-4-pyridinyl]phenyl]methyl]-2H-imidazol-2-one;

1-(2-fluoro-6-isopropylphenyl)-4-butyl-1,3-dihydro-3-[[4-[3-(1H-tetrazol-5-yl)-4-pyridinyl]phenyl]methyl]-2H-imidazol-2-one;

1-(2-fluoro-6-tertbutylphenyl)-4-butyl-1,3-dihydro-3-[[4-[3-(1H-tetrazol-5-yl)-4-pyridinyl]phenyl]methyl]-2H-imidazol-2-one;

1-(2-fluoro-6-chlorophenyl)-4-butyl-1,3-dihydro-3-[[4-[3-(1H-tetrazol-5-yl)-4-pyridinyl]phenyl]methyl]-2H-imidazol-2-one;

1-(2-fluoro-6-methoxyphenyl)-4-butyl-1,3-dihydro-3-[[4-[3-(1H-tetrazol-5-yl)-4-pyridinyl]phenyl]methyl]-2H-imidazol-2-one;

1-(2-chloro-6-methylphenyl)-4-butyl-1,3-dihydro-3-[[4-[3-(1H-tetrazol-5-yl)-4-pyridinyl]phenyl]methyl]-2H-imidazol-2-one;

1-(2-chloro-6-ethylphenyl)-4-butyl-1,3-dihydro-3-[[4-[3-(1H-tetrazol-5-yl)-4-pyridinyl]phenyl]methyl]-2H-imidazol-2-one;

1-(2-chloro-6-isopropylphenyl)-4-butyl-1,3-dihydro-3-[[4-[3-(1H-tetrazol-5-yl)-4-pyridinyl]phenyl]methyl]-2H-imidazol-2-one;

1-(2-chloro-6-tertbutylphenyl)-4-butyl-1,3-dihydro-3-[[4-[3-(1H-tetrazol-5-yl)-4-pyridinyl]phenyl]methyl]-2H-imidazol-2-one;

1-(2-chloro-6-fluorophenyl)-4-butyl-1,3-dihydro-3-[[4-[3-(1H-tetrazol-5-yl)-4-pyridinyl]phenyl]methyl]-2H-imidazol-2-one;

1-(2-chloro-6-methoxyphenyl)-4-butyl-1,3-dihydro-3-[[4-[3-(1H-tetrazol-5-yl)-4-pyridinyl]phenyl]methyl]-2H-imidazol-2-one;

1-(2-bromo-6-methylphenyl)-4-butyl-1,3-dihydro-3-[[4-[3-(1H-tetrazol-5-yl)-4-pyridinyl]phenyl]methyl]-2H-imidazol-2-one;

1-(2-bromo-6-ethylphenyl)-4-butyl-1,3-dihydro-3-[[4-[3-(1H-tetrazol-5-yl)-4-pyridinyl]phenyl]methyl]-2H-imidazol-2-one;

1-(2-bromo-6-isopropylphenyl)-4-butyl-1,3-dihydro-3-[[4-[3-(1H-tetrazol-5-yl)-4-pyridinyl]phenyl]methyl]-2H-imidazol-2-one;

1-(2-bromo-6-tertbutylphenyl)-4-butyl-1,3-dihydro-3-[[4-[3-(1H-tetrazol-5-yl)-4-pyridinyl]phenyl]methyl]-2H-imidazol-2-one;

1-(2-bromo-6-fluorophenyl)-4-butyl-1,3-dihydro-3-[[4-[3-(1H-tetrazol-5-yl)-4-pyridinyl]phenyl]methyl]-2H-imidazol-2-one;

1-(2-bromo-6-chlorophenyl)-4-butyl-1,3-dihydro-3-[[4-[3-(1H-tetrazol-5-yl)-4-pyridinyl]phenyl]methyl]-2H-imidazol-2-one;

1-(2-bromo-6-methoxyphenyl)-4-butyl-1,3-dihydro-3-[[4-[3-(1H-tetrazol-5-yl)-4-pyridinyl]phenyl]methyl]-2H-imidazol-2-one;

1-(2-methoxy-6-methylphenyl)-4-butyl-1,3-dihydro-3-[[4-[3-(1H-tetrazol-5-yl)-4-pyridinyl]phenyl]methyl]-2H-imidazol-2-one;

1-(2-methoxy-6-ethylphenyl)-4-butyl-1,3-dihydro-3-[[4-[3-(1H-tetrazol-5-yl)-4-pyridinyl]phenyl]methyl]-2H-imidazol-2-one;

1-(2-methoxy-6-isopropylphenyl)-4-butyl-1,3-dihydro-3-[[4-[3-(1H-tetrazol-5-yl)-4-pyridinyl]phenyl]methyl]-2H-imidazol-2-one;

1-(2-methoxy-6-tertbutylphenyl)-4-butyl-1,3-dihydro-3-[[4-[3-(1H-tetrazol-5-yl)-4-pyridinyl]phenyl]methyl]-2H-imidazol-2-one;
1-(2-methoxy-6-fluorophenyl)-4-butyl-1,3-dihydro-3-[[4-[3-(1H-tetrazol-5-yl)-4-pyridinyl]phenyl]methyl]-2H-imidazol-2-one;
1-(2-methoxy-6-chlorophenyl)-4-butyl-1,3-dihydro-3-[[4-[3-(1H-tetrazol-5-yl)-4-pyridinyl]phenyl]methyl]-2H-imidazol-2-one;
1-(2,6-dimethoxyphenyl)-4-butyl-1,3-dihydro-3-[[4-[3-(1H-tetrazol-5-yl)-4-pyridinyl]phenyl]methyl]-2H-imidazol-2-one;
1-(2-carboxy-6-methylphenyl)-4-butyl-1,3-dihydro-3-[[4-[3-(1H-tetrazol-5-yl)-4-pyridinyl]phenyl]methyl]-2H-imidazol-2-one;
1-(2-carboxy-6-ethylphenyl)-4-butyl-1,3-dihydro-3-[[4-[3-(1H-tetrazol-5-yl)-4-pyridinyl]phenyl]methyl]-2H-imidazol-2-one;
1-(2-carboxy-6-isopropylphenyl)-4-butyl-1,3-dihydro-3-[[4-[3-(1H-tetrazol-5-yl)-4-pyridinyl]phenyl]methyl]-2H-imidazol-2-one;
1-(2-carboxy-6-tertbutylphenyl)-4-butyl-1,3-dihydro-3-[[4-[3-(1H-tetrazol-5-yl)-4-pyridinyl]phenyl]methyl]-2H-imidazol-2-one;
1-(2-carboxy-6-fluorophenyl)-4-butyl-1,3-dihydro-3-[[4-[3-(1H-tetrazol-5-yl)-4-pyridinyl]phenyl]methyl]-2H-imidazol-2-one;
1-(2-carboxy-6-chlorophenyl)-4-butyl-1,3-dihydro-3-[[4-[3-(1H-tetrazol-5-yl)-4-pyridinyl]phenyl]methyl]-2H-imidazol-2-one;
1-(2-carboxy-6-methoxyphenyl)-4-butyl-1,3-dihydro-3-[[4-[3-(1H-tetrazol-5-yl)-4-pyridinyl]phenyl]methyl]-2H-imidazol-2-one;
1-(2-acetyl-6-methylphenyl)-4-butyl-1,3-dihydro-3-[[4-[3-(1H-tetrazol-5-yl)-4-pyridinyl]phenyl]methyl]-2H-imidazol-2-one;
1-(2-acetyl-6-ethylphenyl)-4-butyl-1,3-dihydro-3-[[4-[3-(1H-tetrazol-5-yl)-4-pyridinyl]phenyl]methyl]-2H-imidazol-2-one;
1-(2-acetyl-6-isopropylphenyl)-4-butyl-1,3-dihydro-3-[[4-[3-(1H-tetrazol-5-yl)-4-pyridinyl]phenyl]methyl]-2H-imidazol-2-one;
1-(2-acetyl-6-tertbutylphenyl)-4-butyl-1,3-dihydro-3-[[4-[3-(1H-tetrazol-5-yl)-4-pyridinyl]phenyl]methyl]-2H-imidazol-2-one;
1-(2-acetyl-6-fluorophenyl)-4-butyl-1,3-dihydro-3-[[4-[3-(1H-tetrazol-5-yl)-4-pyridinyl]phenyl]methyl]-2H-imidazol-2-one;
1-(2-acetyl-6-chlorophenyl)-4-butyl-1,3-dihydro-3-[[4-[3-(1H-tetrazol-5-yl)-4-pyridinyl]phenyl]methyl]-2H-imidazol-2-one; and
1-(2-acetyl-6-methoxyphenyl)-4-butyl-1,3-dihydro-3-[[4-[3-(1H-tetrazol-5-yl)-4-pyridinyl]phenyl]methyl]-2H-imidazol-2-one.

Within Formula I there is a first group of compounds of more interest as represented by Formula VII:

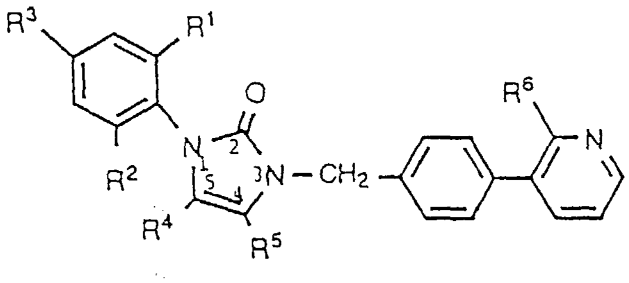

(VII)

wherein each of $R^1$, $R^2$ and $R^3$ is independently selected from methyl, ethyl, n-propyl, isopropyl, tert-butyl, methoxy, ethoxy, propoxy, isopropoxy, tert-butoxy, cyano, fluoro, chloro, iodo, bromo, hydroxy, nitro, amino, N-methylamino, N,N-dimethylamino, N-ethylamino, N,N-diethylamino, carboxyl, methoxycarbonyl, ethoxycarbonyl, formyl, methylcarbonyl ethylcarbonyl and trifluoromethylcarbonyl; wherein $R^3$ may be hydrogen wherein when $R^1$ is ethyl or tert.-butyl, then $R^2$ can be hydrogen, with the proviso that $R^1$ and $R^2$ can not be fluoro or chloro simultaneously; wherein $R^4$ is selected from hydrogen, methyl, fluoro, chloro, monofluoromethyl, difluoromethyl, trifluoromethyl, formyl, carboxyl and dimethoxymethyl; wherein $R^5$ is selected from methyl, ethyl, n-propyl, isopropyl, n-butyl, sec-butyl, isobutyl, tert-butyl, n-pentyl, isopentyl, neopentyl, phenyl, benzyl, phenethyl, cyclopropyl, cyclobutyl, cyclopentyl, cyclohexyl, cyclopropylmethyl, cyclopropylethyl, cyclobutylmethyl, cyclobutylethyl, cyclopentylmethyl, cyclopentylethyl, cyclohexylmethyl and

cyclohexylethyl; wherein $R^6$ is an acidic group selected tram COOH and

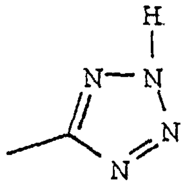

;

or a stereoisomer or a tautomer thereof or a pharmaceutically-acceptable salt thereof.

A family of specific compounds of particular interest within this first class of higher-interest compounds of the first group of compounds of Formula VII consists of compounds and their stereoisomers and tautomers and the pharmaceutically-acceptable salts thereof, said compounds consisting of

1-(2-ethylphenyl)-4-propyl-1,3-dihydro-3-[[4-[2-(1H-tetrazol-5-yl)-3-pyridinyl]phenyl]methyl]-2H-imidazol-2-one;
1-(2-tertbutylphenyl)-4-propyl-1,3-dihydro-3-[[4-[2-(1H-tetrazol-5-yl)-3-pyridinyl]phenyl]methyl]-2H-imidazol-2-one;
1-(2-ethylphenyl)-4-butyl-1,3-dihydro-3-[[4-[2-(1H-tetrazol-5-yl)-3-pyridinyl]phenyl]methyl]-2H-imidazol-2-one;
1-(2-tertbutylphenyl)-4-butyl-1,3-dihydro-3-[[4-[2-(1H-tetrazol-5-yl)-3-pyridinyl]phenyl]methyl]-2H-imidazol-2-one;

A family of specific compounds of particular interest within this second class of higher-interest compounds of the first group of compounds of Formula VII consists of compounds and their stereoisomers and tautomers and the pharmaceutically-acceptable salts thereof, said compounds consisting of

1-(2,6-dimethylphenyl)-4-propyl-1,3-dihydro-3-[[4-[2-(1H-tetrazol-5-yl)-3-pyridinyl]phenyl]methyl]-2H-imidazol-2-one;
1-(2-methyl-6-ethylphenyl)-4-propyl-1,3-dihydro-3-[[4-[2-(1H-tetrazol-5-yl)-3-pyridinyl]phenyl]methyl]-2H-imidazol-2-one;
1-(2-methyl-6-isopropylphenyl)-4-propyl-1,3-dihydro-3-[[4-[2-(1H-tetrazol-5-yl)-3-pyridinyl]phenyl]methyl]-2H-imidazol-2-one;
1-(2-methyl-6-tertbutylphenyl)-4-propyl-1,3-dihydro-3-[[4-[2-(1H-tetrazol-5-yl)-3-pyridinyl]phenyl]methyl]-2H-imidazol-2-one;
1-(2-methyl-6-fluorophenyl)-4-propyl-1,3-dihydro-3-[[4-[2-(1H-tetrazol-5-yl)-3-pyridinyl]phenyl]methyl]-2H-imidazol-2-one;
1-(2-methyl-6-chlorophenyl)-4-propyl-1,3-dihydro-3-[[4-[2-(1H-tetrazol-5-yl)-3-pyridinyl]phenyl]methyl]-2H-imidazol-2-one;
1-(2-methyl-6-methoxyphenyl)-4-propyl-1,3-dihydro-3-[[4-[2-(1H-tetrazol-5-yl)-3-pyridinyl]phenyl]methyl]-2H-imidazol-2-one;
1-(2-ethyl-6-methylphenyl)-4-propyl-1,3-dihydro-3-[[4-[2-(1H-tetrazol-5-yl)-3-pyridinyl]phenyl]methyl]-2H-imidazol-2-one;
1-(2,6-diethylphenyl)-4-propyl-1,3-dihydro-3-[[4-[2-(1H-tetrazol-5-yl)-3-pyridinyl]phenyl]methyl]-2H-imidazol-2-one;
1-(2-ethyl-6-isopropylphenyl)-4-propyl-1,3-dihydro-3-[[4-[2-(1H-tetrazol-5-yl)-3-pyridinyl]phenyl]methyl]-2H-imidazol-2-one;
1-(2-ethyl-6-tertbutylphenyl)-4-propyl-1,3-dihydro-3-[[4-[2-(1H-tetrazol-5-yl)-3-pyridinyl]phenyl]methyl]-2H-imidazol-2-one;
1-(2-ethyl-6-fluorophenyl)-4-propyl-1,3-dihydro-3-[[4-[2-(1H-tetrazol-5-yl)-3-pyridinyl]phenyl]methyl]-2H-imidazol-2-one;
1-(2-ethyl-6-chlorophenyl)-4-propyl-1,3-dihydro-3-[[4-[2-(1H-tetrazol-5-yl)-3-pyridinyl]phenyl]methyl]-2H-imidazol-2-one;
1-(2-ethyl-6-methoxyphenyl)-4-propyl-1,3-dihydro-3-[[4-[2-(1H-tetrazol-5-yl)-3-pyridinyl]phenyl]methyl]-2H-imidazol-2-one;
1-(2-propyl-6-methylphenyl)-4-propyl-1,3-dihydro-3-[[4-[2-(1H-tetrazol-5-yl)-3-pyridinyl]phenyl]methyl]-2H-imidazol-2-one;
1-(2-propyl-6-ethylphenyl)-4-propyl-1,3-dihydro-3-[[4-[2-(1H-tetrazol-5-yl)-3-pyridinyl]phenyl]methyl]-2H-imidazol-2-one;
1-(2-propyl-6-isopropylphenyl)-4-propyl-1,3-dihydro-3-[[4-[2-(1H-tetrazol-5-yl)-3-pyridinyl]phenyl]methyl]-2H-imidazol-2-one;
1-(2-propyl-6-tertbutylphenyl)-4-propyl-1,3-dihydro-3-[[4-[2-(1H-tetrazol-5-yl)-3-pyridinyl]phenyl]methyl]-2H-imidazol-2-one;

1-(2-propyl-6-fluorophenyl)-4-propyl-1,3-dihydro-3-[[4-[2-(1H-tetrazol-5-yl)-3-pyridinyl]phenyl]methyl]-2H-imidazol-2-one;

1-(2-propyl-6-chlorophenyl)-4-propyl-1,3-dihydro-3-[[4-[2-(1H-tetrazol-5-yl)-3-pyridinyl]phenyl]methyl]-2H-imidazol-2-one;

1-(2-propyl-6-methoxyphenyl)-4-propyl-1,3-dihydro-3-[[4-[2-(1H-tetrazol-5-yl)-3-pyridinyl]phenyl]methyl]-2H-imidazol-2-one;

1-(2-isopropyl-6-methylphenyl)-4-propyl-1,3-dihydro-3-[[4-[2-(1H-tetrazol-5-yl)-3-pyridinyl]phenyl]methyl]-2H-imidazol-2-one;

1-(2-isopropyl-6-ethylphenyl)-4-propyl-1,3-dihydro-3-[[4-[2-(1H-tetrazol-5-yl)-3-pyridinyl]phenyl]methyl]-2H-imidazol-2-one;

1-(2,6-diisopropylphenyl)-4-propyl-1,3-dihydro-3-[[4-[2-(1H-tetrazol-5-yl)-3-pyridinyl]phenyl]methyl]-2H-imidazol-2-one;

1-(2-isopropyl-6-tertbutylphenyl)-4-propyl-1,3-dihydro-3-[[4-[2-(1H-tetrazol-5-yl)-3-pyridinyl]phenyl]methyl]-2H-imidazol-2-one;

1-(2-isopropyl-6-fluorophenyl)-4-propyl-1,3-dihydro-3-[[4-[2-(1H-tetrazol-5-yl)-3-pyridinyl]phenyl]methyl]-2H-imidazol-2-one;

1-(2-isopropyl-6-chlorophenyl)-4-propyl-1,3-dihydro-3-[[4-[2-(1H-tetrazol-5-yl)-3-pyridinyl]phenyl]methyl]-2H-imidazol-2-one;

1-(2-isopropyl-6-methoxyphenyl)-4-propyl-1,3-dihydro-3-[[4-[2-(1H-tetrazol-5-yl)-3-pyridinyl]phenyl]methyl]-2H-imidazol-2-one;

1-(2-tertbutyl-6-methylphenyl)-4-propyl-1,3-dihydro-3-[[4-[2-(1H-tetrazol-5-yl)-3-pyridinyl]phenyl]methyl]-2H-imidazol-2-one;

1-(2-tertbutyl-6-ethylphenyl)-4-propyl-1,3-dihydro-3-[[4-[2-(1H-tetrazol-5-yl)-3-pyridinyl]phenyl]methyl]-2H-imidazol-2-one;

1-(2-tertbutyl-6-isopropylphenyl)-4-propyl-1,3-dihydro-3-[[4-[2-(1H-tetrazol-5-yl)-3-pyridinyl]phenyl]methyl]-2H-imidazol-2-one;

1-(2,6-ditertbutylphenyl)-4-propyl-1,3-dihydro-3-[[4-[2-(1H-tetrazol-5-yl)-3-pyridinyl]phenyl]methyl]-2H-imidazol-2-one;

1-(2-tertbutyl-6-fluorophenyl)-4-propyl-1,3-dihydro-3-[[4-[2-(1H-tetrazol-5-yl)-3-pyridinyl]phenyl]methyl]-2H-imidazol-2-one;

1-(2-tertbutyl-6-chlorophenyl)-4-propyl-1,3-dihydro-3-[[4-[2-(1H-tetrazol-5-yl)-3-pyridinyl]phenyl]methyl]-2H-imidazol-2-one;

1-(2-tertbutyl-6-methoxyphenyl)-4-propyl-1,3-dihydro-3-[[4-[2-(1H-tetrazol-5-yl)-3-pyridinyl]phenyl]methyl]-2H-imidazol-2-one;

1-(2-fluoro-6-methylphenyl)-4-propyl-1,3-dihydro-3-[[4-[2-(1H-tetrazol-5-yl)-3-pyridinyl]phenyl]methyl]-2H-imidazol-2-one;

1-(2-fluoro-6-ethylphenyl)-4-propyl-1,3-dihydro-3-[[4-[2-(1H-tetrazol-5-yl)-3-pyridinyl]phenyl]methyl]-2H-imidazol-2-one;

1-(2-fluoro-6-isopropylphenyl)-4-propyl-1,3-dihydro-3-[[4-[2-(1H-tetrazol-5-yl)-3-pyridinyl]phenyl]methyl]-2H-imidazol-2-one;

1-(2-fluoro-6-tertbutylphenyl)-4-propyl-1,3-dihydro-3-[[4-[2-(1H-tetrazol-5-yl)-3-pyridinyl]phenyl]methyl]-2H-imidazol-2-one;

1-(2-fluoro-6-chlorophenyl)-4-propyl-1,3-dihydro-3-[[4-[2-(1H-tetrazol-5-yl)-3-pyridinyl]phenyl]methyl]-2H-imidazol-2-one;

1-(2-fluoro-6-methoxyphenyl)-4-propyl-1,3-dihydro-3-[[4-[2-(1H-tetrazol-5-yl)-3-pyridinyl]phenyl]methyl]-2H-imidazol-2-one;

1-(2-chloro-6-methylphenyl)-4-propyl-1,3-dihydro-3-[[4-[2-(1H-tetrazol-5-yl)-3-pyridinyl]phenyl]methyl]-2H-imidazol-2-one;

1-(2-chloro-6-ethylphenyl)-4-propyl-1,3-dihydro-3-[[4-[2-(1H-tetrazol-5-yl)-3-pyridinyl]phenyl]methyl]-2H-imidazol-2-one;

1-(2-chloro-6-isopropylphenyl)-4-propyl-1,3-dihydro-3-[[4-[2-(1H-tetrazol-5-yl)-3-pyridinyl]phenyl]methyl]-2H-imidazol-2-one;

1-(2-chloro-6-tertbutylphenyl)-4-propyl-1,3-dihydro-3-[[4-[2-(1H-tetrazol-5-yl)-3-pyridinyl]phenyl]methyl]-2H-imidazol-2-one;

1-(2-chloro-6-fluorophenyl)-4-propyl-1,3-dihydro-3-[[4-[2-(1H-tetrazol-5-yl)-3-pyridinyl]phenyl]methyl]-2H-imidazol-2-one;

1-(2-chloro-6-methoxyphenyl)-4-propyl-1,3-dihydro-3-[[4-[2-(1H-tetrazol-5-yl)-3-pyridinyl]phenyl]methyl]-2H-imidazol-2-one;

1-(2-bromo-6-methylphenyl)-4-propyl-1,3-dihydro-3-[[4-[2-(1H-tetrazol-5-yl)-3-pyridinyl]phenyl]methyl]-2H-imidazol-2-one;

1-(2-bromo-6-ethylphenyl)-4-propyl-1,3-dihydro-3-[[4-[2-(1H-tetrazol-5-yl)-3-pyridinyl]phenyl]methyl]-2H-imidazol-2-one;

1-(2-bromo-6-isopropylphenyl)-4-propyl-1,3-dihydro-3-[[4-[2-(1H-tetrazol-5-yl)-3-pyridinyl]phenyl]methyl]-2H-imidazol-2-one;

1-(2-bromo-6-tertbutylphenyl)-4-propyl-1,3-dihydro-3-[[4-[2-(1H-tetrazol-5-yl)-3-pyridinyl]phenyl]methyl]-2H-imidazol-2-one;

1-(2-bromo-6-fluorophenyl)-4-propyl-1,3-dihydro-3-[[4-[2-(1H-tetrazol-5-yl)-3-pyridinyl]phenyl]methyl]-2H-imidazol-2-one;

1-(2-bromo-6-chlorophenyl)-4-propyl-1,3-dihydro-3-[[4-[2-(1H-tetrazol-5-yl)-3-pyridinyl]phenyl]methyl]-2H-imidazol-2-one;

1-(2-bromo-6-methoxyphenyl)-4-propyl-1,3-dihydro-3-[[4-[2-(1H-tetrazol-5-yl)-3-pyridinyl]phenyl]methyl]-2H-imidazol-2-one;

1-(2-methoxy-6-methylphenyl)-4-propyl-1,3-dihydro-3-[[4-[2-(1H-tetrazol-5-yl)-3-pyridinyl]phenyl]methyl]-2H-imidazol-2-one;

1-(2-methoxy-6-ethylphenyl)-4-propyl-1,3-dihydro-3-[[4-[2-(1H-tetrazol-5-yl)-3-pyridinyl]phenyl]methyl]-2H-imidazol-2-one;

1-(2-methoxy-6-isopropylphenyl)-4-propyl-1,3-dihydro-3-[[4-[2-(1H-tetrazol-5-yl)-3-pyridinyl]phenyl]methyl]-2H-imidazol-2-one;

1-(2-methoxy-6-tertbutylphenyl)-4-propyl-1,3-dihydro-3-[[4-[2-(1H-tetrazol-5-yl)-3-pyridinyl]phenyl]methyl]-2H-imidazol-2-one;

1-(2-methoxy-6-fluorophenyl)-4-propyl-1,3-dihydro-3-[[4-[2-(1H-tetrazol-5-yl)-3-pyridinyl]phenyl]methyl]-2H-imidazol-2-one;

1-(2-methoxy-6-chlorophenyl)-4-propyl-1,3-dihydro-3-[[4-[2-(1H-tetrazol-5-yl)-3-pyridinyl]phenyl]methyl]-2H-imidazol-2-one;

1-(2,6-dimethoxyphenyl)-4-propyl-1,3-dihydro-3-[[4-[2-(1H-tetrazol-5-yl)-3-pyridinyl]phenyl]methyl]-2H-imidazol-2-one;

1-(2-carboxy-6-methylphenyl)-4-propyl-1,3-dihydro-3-[[4-[2-(1H-tetrazol-5-yl)-3-pyridinyl]phenyl]methyl]-2H-imidazol-2-one;

1-(2-carboxy-6-ethylphenyl)-4-propyl-1,3-dihydro-3-[[4-[2-(1H-tetrazol-5-yl)-3-pyridinyl]phenyl]methyl]-2H-imidazol-2-one;

1-(2-carboxy-6-isopropylphenyl)-4-propyl-1,3-dihydro-3-[[4-[2-(1H-tetrazol-5-yl)-3-pyridinyl]phenyl]methyl]-2H-imidazol-2-one;

1-(2-carboxy-6-tertbutylphenyl)-4-propyl-1,3-dihydro-3-[[4-[2-(1H-tetrazol-5-yl)-3-pyridinyl]phenyl]methyl]-2H-imidazol-2-one;

1-(2-carboxy-6-fluorophenyl)-4-propyl-1,3-dihydro-3-[[4-[2-(1H-tetrazol-5-yl)-3-pyridinyl]phenyl]methyl]-2H-imidazol-2-one;

1-(2-carboxy-6-chlorophenyl)-4-propyl-1,3-dihydro-3-[[4-[2-(1H-tetrazol-5-yl)-3-pyridinyl]phenyl]methyl]-2H-imidazol-2-one;

1-(2-carboxy-6-methoxyphenyl)-4-propyl-1,3-dihydro-3-[[4-[2-(1H-tetrazol-5-yl)-3-pyridinyl]phenyl]methyl]-2H-imidazol-2-one;

1-(2-acetyl-6-methylphenyl)-4-propyl-1,3-dihydro-3-[[4-[2-(1H-tetrazol-5-yl)-3-pyridinyl]phenyl]methyl]-2H-imidazol-2-one;

1-(2-acetyl-6-ethylphenyl)-4-propyl-1,3-dihydro-3-[[4-[2-(1H-tetrazol-5-yl)-3-pyridinyl]phenyl]methyl]-2H-imidazol-2-one;

1-(2-acetyl-6-isopropylphenyl)-4-propyl-1,3-dihydro-3-[[4-[2-(1H-tetrazol-5-yl)-3-pyridinyl]phenyl]methyl]-2H-imidazol-2-one;

1-(2-acetyl-6-tertbutylphenyl)-4-propyl-1,3-dihydro-3-[[4-[2-(1H-tetrazol-5-yl)-3-pyridinyl]phenyl]methyl]-2H-imidazol-2-one;

1-(2-acetyl-6-fluorophenyl)-4-propyl-1,3-dihydro-3-[[4-[2-(1H-tetrazol-5-yl)-3-pyridinyl]phenyl]methyl]-2H-imidazol-2-one;

1-(2-acetyl-6-chlorophenyl)-4-propyl-1,3-dihydro-3-[[4-[2-(1H-tetrazol-5-yl)-3-pyridinyl]phenyl]methyl]-2H-imidazol-2-one;

1-(2-acetyl-6-methoxyphenyl)-4-propyl-1,3-dihydro-3-[[4-[2-(1H-tetrazol-5-yl)-3-pyridinyl]phenyl]methyl]-2H-imidazol-2-one;

1-(2,6-dimethylphenyl)-4-butyl-1,3-dihydro-3-[[4-[2-(1H-tetrazol-5-yl)-3-pyridinyl]phenyl]methyl]-2H-imidazol-2-one;

1-(2-methyl-6-ethylphenyl)-4-butyl-1,3-dihydro-3-[[4-[2-(1H-tetrazol-5-yl)-3-pyridinyl]phenyl]methyl]-2H-imidazol-2-one;

1-(2-methyl-6-isopropylphenyl)-4-butyl-1,3-dihydro-3-[[4-[2-(1H-tetrazol-5-yl)-3-pyridinyl]phenyl]methyl]-2H-imidazol-2-one;

1-(2-methyl-6-tertbutylphenyl)-4-butyl-1,3-dihydro-3-[[4-[2-(1H-tetrazol-5-yl)-3-pyridinyl]phenyl]methyl]-2H-imidazol-2-one;

1-(2-methyl-6-fluorophenyl)-4-butyl-1,3-dihydro-3-[[4-[2-(1H-tetrazol-5-yl)-3-pyridinyl]phenyl]methyl]-2H-imidazol-2-one;

1-(2-methyl-6-chlorophenyl)-4-butyl-1,3-dihydro-3-[[4-[2-(1H-tetrazol-5-yl)-3-pyridinyl]phenyl]methyl]-2H-imidazol-2-one;

1-(2-methyl-6-methoxyphenyl)-4-butyl-1,3-dihydro-3-[[4-[2-(1H-tetrazol-5-yl)-3-pyridinyl]phenyl]methyl]-2H-imidazol-2-one;

1-(2-ethyl-6-methylphenyl)-4-butyl-1,3-dihydro-3-[[4-[2-(1H-tetrazol-5-yl)-3-pyridinyl]phenyl]methyl]-2H-imidazol-2-one;

1-(2,6-diethylphenyl)-4-butyl-1,3-dihydro-3-[[4-[2-(1H-tetrazol-5-yl)-3-pyridinyl]phenyl]methyl]-2H-imidazol-2-one;

1-(2-ethyl-6-isopropylphenyl)-4-butyl-1,3-dihydro-3-[[4-[2-(1H-tetrazol-5-yl)-3-pyridinyl]phenyl]methyl]-2H-imidazol-2-one;

1-(2-ethyl-6-tertbutylphenyl)-4-butyl-1,3-dihydro-3-[[4-[2-(1H-tetrazol-5-yl)-3-pyridinyl]phenyl]methyl]-2H-imidazol-2-one;

1-(2-ethyl-6-fluorophenyl)-4-butyl-1,3-dihydro-3-[[4-[2-(1H-tetrazol-5-yl)-3-pyridinyl]phenyl]methyl]-2H-imidazol-2-one;

1-(2-ethyl-6-chlorophenyl)-4-butyl-1,3-dihydro-3-[[4-[2-(1H-tetrazol-5-yl)-3-pyridinyl]phenyl]methyl]-2H-imidazol-2-one;

1-(2-ethyl-6-methoxyphenyl)-4-butyl-1,3-dihydro-3-[[4-[2-(1H-tetrazol-5-yl)-3-pyridinyl]phenyl]methyl]-2H-imidazol-2-one;

1-(2-propyl-6-methylphenyl)-4-butyl-1,3-dihydro-3-[[4-[2-(1H-tetrazol-5-yl)-3-pyridinyl]phenyl]methyl]-2H-imidazol-2-one;

1-(2-propyl-6-ethylphenyl)-4-butyl-1,3-dihydro-3-[[4-[2-(1H-tetrazol-5-yl)-3-pyridinyl]phenyl]methyl]-2H-imidazol-2-one;

1-(2-propyl-6-isopropylphenyl)-4-butyl-1,3-dihydro-3-[[4-[2-(1H-tetrazol-5-yl)-3-pyridinyl]phenyl]methyl]-2H-imidazol-2-one;

1-(2-propyl-6-tertbutylphenyl)-4-butyl-1,3-dihydro-3-[[4-[2-(1H-tetrazol-5-yl)-3-pyridinyl]phenyl]methyl]-2H-imidazol-2-one;

1-(2-propyl-6-fluorophenyl)-4-butyl-1,3-dihydro-3-[[4-[2-(1H-tetrazol-5-yl)-3-pyridinyl]phenyl]methyl]-2H-imidazol-2-one;

1-(2-propyl-6-chlorophenyl)-4-butyl-1,3-dihydro-3-[[4-[2-(1H-tetrazol-5-yl)-3-pyridinyl]phenyl]methyl]-2H-imidazol-2-one;

1-(2-propyl-6-methoxyphenyl)-4-butyl-1,3-dihydro-3-[[4-[2-(1H-tetrazol-5-yl)-3-pyridinyl]phenyl]methyl]-2H-imidazol-2-one;

1-(2-isopropyl-6-methylphenyl)-4-butyl-1,3-dihydro-3-[[4-[2-(1H-tetrazol-5-yl)-3-pyridinyl]phenyl]methyl]-2H-imidazol-2-one;

1-(2-isopropyl-6-ethylphenyl)-4-butyl-1,3-dihydro-3-[[4-[2-(1H-tetrazol-5-yl)-3-pyridinyl]phenyl]methyl]-2H-imidazol-2-one;

1-(2,6-diisopropylphenyl)-4-butyl-1,3-dihydro-3-[[4-[2-(1H-tetrazol-5-yl)-3-pyridinyl]phenyl]methyl]-2H-imidazol-2-one;

1-(2-isopropyl-6-tertbutylphenyl)-4-butyl-1,3-dihydro-3-[[4-[2-(1H-tetrazol-5-yl)-3-pyridinyl]phenyl]methyl]-2H-imidazol-2-one;

1-(2-isopropyl-6-fluorophenyl)-4-butyl-1,3-dihydro-3-[[4-[2-(1H-tetrazol-5-yl)-3-pyridinyl]phenyl]methyl]-2H-imidazol-2-one;

1-(2-isopropyl-6-chlorophenyl)-4-butyl-1,3-dihydro-3-[[4-[2-(1H-tetrazol-5-yl)-3-pyridinyl]phenyl]methyl]-2H-imidazol-2-one;

1-(2-isopropyl-6-methoxyphenyl)-4-butyl-1,3-dihydro-3-[[4-[2-(1H-tetrazol-5-yl)-3-pyridinyl]phenyl]methyl]-2H-imidazol-2-one;

1-(2-tertbutyl-6-methylphenyl)-4-butyl-1,3-dihydro-3-[[4-[2-(1H-tetrazol-5-yl)-3-pyridinyl]phenyl]methyl]-2H-imidazol-2-one;

1-(2-tertbutyl-6-ethylphenyl)-4-butyl-1,3-dihydro-3-[[4-[2-(1H-tetrazol-5-yl)-3-pyridinyl]phenyl]methyl]-2H-imidazol-2-one;

1-(2-tertbutyl-6-isopropylphenyl)-4-butyl-1,3-dihydro-3-[[4-[2-(1H-tetrazol-5-yl)-3-pyridinyl]phenyl]methyl]-2H-imidazol-2-one;
1-(2,6-ditertbutylphenyl)-4-butyl-1,3-dihydro-3-[[4-[2-(1H-tetrazol-5-yl)-3-pyridinyl]phenyl]methyl]-2H-imidazol-2-one;
1-(2-tertbutyl-6-fluorophenyl)-4-butyl-1,3-dihydro-3-[[4-[2-(1H-tetrazol-5-yl)-3-pyridinyl]phenyl]methyl]-2H-imidazol-2-one;
1-(2-tertbutyl-6-chlorophenyl)-4-butyl-1,3-dihydro-3-[[4-[2-(1H-tetrazol-5-yl)-3-pyridinyl]phenyl]methyl]-2H-imidazol-2-one;
1-(2-tertbutyl-6-methoxyphenyl)-4-butyl-1,3-dihydro-3-[[4-[2-(1H-tetrazol-5-yl)-3-pyridinyl]phenyl]methyl]-2H-imidazol-2-one;
1-(2-fluoro-6-methylphenyl)-4-butyl-1,3-dihydro-3-[[4-[2-(1H-tetrazol-5-yl)-3-pyridinyl]phenyl]methyl]-2H-imidazol-2-one;
1-(2-fluoro-6-ethylphenyl)-4-butyl-1,3-dihydro-3-[[4-[2-(1H-tetrazol-5-yl)-3-pyridinyl]phenyl]methyl]-2H-imidazol-2-one;
1-(2-fluoro-6-isopropylphenyl)-4-butyl-1,3-dihydro-3-[[4-[2-(1H-tetrazol-5-yl)-3-pyridinyl]phenyl]methyl]-2H-imidazol-2-one;
1-(2-fluoro-6-tertbutylphenyl)-4-butyl-1,3-dihydro-3-[[4-[2-(1H-tetrazol-5-yl)-3-pyridinyl]phenyl]methyl]-2H-imidazol-2-one;
1-(2,6-difluorophenyl)-4-butyl-1,3-dihydro-3-[[4-[2-(1H-tetrazol-5-yl)-3-pyridinyl]phenyl]methyl]-2H-imidazol-2-one;
1-(2-fluoro-6-chlorophenyl)-4-butyl-1,3-dihydro-3-[[4-[2-(1H-tetrazol-5-yl)-3-pyridinyl]phenyl]methyl]-2H-imidazol-2-one;
1-(2-fluoro-6-methoxyphenyl)-4-butyl-1,3-dihydro-3-[[4-[2-(1H-tetrazol-5-yl)-3-pyridinyl]phenyl]methyl]-2H-imidazol-2-one;
1-(2-chloro-6-methylphenyl)-4-butyl-1,3-dihydro-3-[[4-[2-(1H-tetrazol-5-yl)-3-pyridinyl]phenyl]methyl]-2H-imidazol-2-one;
1-(2-chloro-6-ethylphenyl)-4-butyl-1,3-dihydro-3-[[4-[2-(1H-tetrazol-5-yl)-3-pyridinyl]phenyl]methyl]-2H-imidazol-2-one;
1-(2-chloro-6-isopropylphenyl)-4-butyl-1,3-dihydro-3-[[4-[2-(1H-tetrazol-5-yl)-3-pyridinyl]phenyl]methyl]-2H-imidazol-2-one;
1-(2-chloro-6-tertbutylphenyl)-4-butyl-1,3-dihydro-3-[[4-[2-(1H-tetrazol-5-yl)-3-pyridinyl]phenyl]methyl]-2H-imidazol-2-one;
1-(2-chloro-6-fluorophenyl)-4-butyl-1,3-dihydro-3-[[4-[2-(1H-tetrazol-5-yl)-3-pyridinyl]phenyl]methyl]-2H-imidazol-2-one;
1-(2,6-dichlorophenyl)-4-butyl-1,3-dihydro-3-[[4-[2-(1H-tetrazol-5-yl)-3-pyridinyl]phenyl]methyl]-2H-imidazol-2-one;
1-(2-chloro-6-methoxyphenyl)-4-butyl-1,3-dihydro-3-[[4-[2-(1H-tetrazol-5-yl)-3-pyridinyl]phenyl]methyl]-2H-imidazol-2-one;
1-(2-bromo-6-methylphenyl)-4-butyl-1,3-dihydro-3-[[4-[2-(1H-tetrazol-5-yl)-3-pyridinyl]phenyl]methyl]-2H-imidazol-2-one;
1-(2-bromo-6-ethylphenyl)-4-butyl-1,3-dihydro-3-[[4-[2-(1H-tetrazol-5-yl)-3-pyridinyl]phenyl]methyl]-2H-imidazol-2-one;
1-(2-bromo-6-isopropylphenyl)-4-butyl-1,3-dihydro-3-[[4-[2-(1H-tetrazol-5-yl)-3-pyridinyl]phenyl]methyl]-2H-imidazol-2-one;
1-(2-bromo-6-tertbutylphenyl)-4-butyl-1,3-dihydro-3-[[4-[2-(1H-tetrazol-5-yl)-3-pyridinyl]phenyl]methyl]-2H-imidazol-2-one;
1-(2-bromo-6-fluorophenyl)-4-butyl-1,3-dihydro-3-[[4-[2-(1H-tetrazol-5-yl)-3-pyridinyl]phenyl]methyl]-2H-imidazol-2-one;
1-(2-bromo-6-chlorophenyl)-4-butyl-1,3-dihydro-3-[[4-[2-(1H-tetrazol-5-yl)-3-pyridinyl]phenyl]methyl]-2H-imidazol-2-one;
1-(2-bromo-6-methoxyphenyl)-4-butyl-1,3-dihydro-3-[[4-[2-(1H-tetrazol-5-yl)-3-pyridinyl]phenyl]methyl]-2H-imidazol-2-one;
1-(2-methoxy-6-methylphenyl)-4-butyl-1,3-dihydro-3-[[4-[2-(1H-tetrazol-5-yl)-3-pyridinyl]phenyl]methyl]-2H-imidazol-2-one;
1-(2-methoxy-6-ethylphenyl)-4-butyl-1,3-dihydro-3-[[4-[2-(1H-tetrazol-5-yl)-3-pyridinyl]phenyl]methyl]-2H-imidazol-2-one;
1-(2-methoxy-6-isopropylphenyl)-4-butyl-1,3-dihydro-3-[[4-[2-(1H-tetrazol-5-yl)-3-pyridinyl]phenyl]methyl]-2H-imidazol-2-one;
1-(2-methoxy-6-tertbutylphenyl)-4-butyl-1,3-dihydro-3-[[4-[2-(1H-tetrazol-5-yl)-3-pyridinyl]phenyl]methyl]-2H-imidazol-2-one;

1-(2-methoxy-6-fluorophenyl)-4-butyl-1,3-dihydro-3-[[4-[2-(1H-tetrazol-5-yl)-3-pyridinyl]phenyl]methyl]-2H-imidazol-2-one;

1-(2-methoxy-6-chlorophenyl)-4-butyl-1,3-dihydro-3-[[4-[2-(1H-tetrazol-5-yl)-3-pyridinyl]phenyl]methyl]-2H-imidazol-2-one;

1-(2,6-dimethoxyphenyl)-4-butyl-1,3-dihydro-3-[[4-[2-(1H-tetrazol-5-yl)-3-pyridinyl]phenyl]methyl]-2H-imidazol-2-one;

1-(2-carboxy-6-methylphenyl)-4-butyl-1,3-dihydro-3-[[4-[2-(1H-tetrazol-5-yl)-3-pyridinyl]phenyl]methyl]-2H-imidazol-2-one;

1-(2-carboxy-6-ethylphenyl)-4-butyl-1,3-dihydro-3-[[4-[2-(1H-tetrazol-5-yl)-3-pyridinyl]phenyl]methyl]-2H-imidazol-2-one;

1-(2-carboxy-6-isopropylphenyl)-4-butyl-1,3-dihydro-3-[[4-[2-(1H-tetrazol-5-yl)-3-pyridinyl]phenyl]methyl]-2H-imidazol-2-one;

1-(2-carboxy-6-tertbutylphenyl)-4-butyl-1,3-dihydro-3-[[4-[2-(1H-tetrazol-5-yl)-3-pyridinyl]phenyl]methyl]-2H-imidazol-2-one;

1-(2-carboxy-6-fluorophenyl)-4-butyl-1,3-dihydro-3-[[4-[2-(1H-tetrazol-5-yl)-3-pyridinyl]phenyl]methyl]-2H-imidazol-2-one;

1-(2-carboxy-6-chlorophenyl)-4-butyl-1,3-dihydro-3-[[4-[2-(1H-tetrazol-5-yl)-3-pyridinyl]phenyl]methyl]-2H-imidazol-2-one;

1-(2-carboxy-6-methoxyphenyl)-4-butyl-1,3-dihydro-3-[[4-[2-(1H-tetrazol-5-yl)-3-pyridinyl]phenyl]methyl]-2H-imidazol-2-one;

1-(2-acetyl-6-methylphenyl)-4-butyl-1,3-dihydro-3-[[4-[2-(1H-tetrazol-5-yl)-3-pyridinyl]phenyl]methyl]-2H-imidazol-2-one;

1-(2-acetyl-6-ethylphenyl)-4-butyl-1,3-dihydro-3-[[4-[2-(1H-tetrazol-5-yl)-3-pyridinyl]phenyl]methyl]-2H-imidazol-2-one;

1-(2-acetyl-6-isopropylphenyl)-4-butyl-1,3-dihydro-3-[[4-[2-(1H-tetrazol-5-yl)-3-pyridinyl]phenyl]methyl]-2H-imidazol-2-one;

1-(2-acetyl-6-tertbutylphenyl)-4-butyl-1,3-dihydro-3-[[4-[2-(1H-tetrazol-5-yl)-3-pyridinyl]phenyl]methyl]-2H-imidazol-2-one;

1-(2-acetyl-6-fluorophenyl)-4-butyl-1,3-dihydro-3-[[4-[2-(1H-tetrazol-5-yl)-3-pyridinyl]phenyl]methyl]-2H-imidazol-2-one;

1-(2-acetyl-6-chlorophenyl)-4-butyl-1,3-dihydro-3-[[4-[2-(1H-tetrazol-5-yl)-3-pyridinyl]phenyl]methyl]-2H-imidazol-2-one; and

1-(2-acetyl-6-methoxyphenyl)-4-butyl-1,3-dihydro-3-[[4-[2-(1H-tetrazol-5-yl)-3-pyridinyl]phenyl]methyl]-2H-imidazol-2-one.

The term "hydrogen" denotes a single hydrogen atom (H). This hydrido group may be attached, for example, to an oxygen atom to form a hydroxyl group; or, as another example, one hydrido group may be attached to a carbon atom to form a >CH- group; or, as another example, two hydrido groups may be attached to a carbon atom to form a $-CH_2-$ group. Where the term "alkyl" is used, either alone or within other terms such as "haloalkyl", the term "alkyl" embraces linear or branched radicals having one to about twenty carbon atoms or, preferably, one to about twelve carbon atoms. More preferred alkyl radicals are "lower alkyl" radicals having one to about ten carbon atoms. Most preferred are lower alkyl radicals having one to about five carbon atoms. The term "cycloalkyl" embraces cyclic radicals having three to about ten ring carbon atoms, preferably three to about six carbon atoms, such as cyclopropyl, cyclobutyl, cyclopentyl and cyclohexyl. The term "haloalkyl" embraces radicals wherein any one or more of the alkyl carbon atoms is substituted with one or more halo groups, preferably selected from bromo, chloro and fluoro. Specifically embraced by the term "haloalkyl" are monohaloalkyl, dihaloalkyl and polyhaloalkyl groups. A monohaloalkyl group, for example, may have either a bromo, a chloro, or a fluoro atom within the group. Dihaloalkyl and polyhaloalkyl groups may be substituted with two or more of the same halo groups, or may have a combination of different halo groups. A dihaloalkyl group, for example, may have two fluoro atoms, such as difluoromethyl and difluorobutyl groups, or two chloro atoms, such as a dichloromethyl group, or one fluoro atom and one chloro atom, such as a fluoro-chloromethyl group. Examples of a polyhaloalkyl are trifluoromethyl, 1,1-difluoroethyl, 2,2,2-trifluoroethyl, perfluoroethyl and 2,2,3,3-tetrafluoropropyl groups. The term "difluoroalkyl" embraces alkyl groups having two fluoro atoms substituted on any one or two of the alkyl group carbon atoms. The terms "alkoxy" and "alkoxyalkyl" embrace linear or branched oxy-containing radicals each having alkyl portions of one to about ten carbon atoms, such as methoxy group. The term "alkoxyalkyl" also embraces alkyl radicals having two or more alkoxy groups attached to the alkyl radical, that is, to form monoalkoxyalkyl and dialkoxyalkyl groups. The "alkoxy" or "alkoxyalkyl" radicals may be further substi-tuted with one or more halo atoms, such as fluoro, chloro or bromo, to provide haloalkoxy or haloalkoxyalkyl groups. The terms "benzyl" and "phenylmethyl" are interchangeable. For any of the foregoing defined radicals, preferred radicals are those containing from one to about

ten carbon atoms.

The term "arylheteroarylalkyl" embraces moieties, having an aryl fragment, a heteroaryl fragment and an alkyl fragment, of the type exemplified by moieties shown for the "A" substituent of Formula I, herein.

Specific examples of alkyl groups are methyl, ethyl, n-propyl, isopropyl, n-butyl, sec-butyl, isobutyl, tert-butyl, n-pentyl, isopentyl, methylbutyl, dimethylbutyl and neopentyl.

Compounds of Formula I have been found to inhibit the action of angiotensin II in mammals. Angiotensin II is a potent vasoconstrictor and participates in the formation of aldosterone which regulates sodium and water balance in mammals. Thus, compounds of Formula I are therapeutically useful in methods for treating hypertension by administering to a hypertensive patient a therapeutically-effective amount of a compound of Formula I. The phrase "hypertensive patient" means, in this context, a mammalian subject suffering from or afflicted by the effects of hypertension or susceptible to a hypertensive condition if not treated to prevent or control such hypertension.

Also included in the family of compounds of Formula I are isomeric forms including diastereoisomers and the pharmaceutically-acceptable salts thereof. The term "pharmaceutically-acceptable salts" embraces salts commonly used to form alkali metal salts and to form addition salts of free acids or free bases. The nature of the salt is not critical, provided that it is pharmaceutically-acceptable. Suitable pharmaceutically-acceptable acid addition salts of compounds of Formula I may be prepared from an inorganic acid or from an organic acid. Examples of such inorganic acids are hydrochloric, hydrobromic, hydroiodic, nitric, carbonic, sulfuric and phosphoric acid. Appropriate organic acids may be selected from aliphatic, cycloaliphatic, aromatic, araliphatic, heterocyclic, carboxylic and sulfonic classes of organic acids, example of which are formic, acetic, propionic, succinic, glycolic, gluconic, lactic, malic, tartaric, citric, ascorbic, glucuronic, maleic, fumaric, pyruvic, aspartic, glutamic, benzoic, anthranilic, p-hydroxybenzoic, salicyclic, phenylacetic, mandelic, embonic (pamoic), methansulfonic, ethanesulfonic, 2-hydroxyethanesulfonic, pantothenic, benzenesulfonic, toluenesulfonic, sulfanilic, mesylic, cyclohexylaminosulfonic, stearic, algenic, β-hydroxybutyric, malonic, galactaric and galacturonic acid. Suitable pharmaceutically-acceptable base addition salts of compounds of Formula I include metallic salts made from aluminium, calcium, lithium, magnesium, potassium, sodium and zinc or organic salts made from N,N'-dibenzylethylenediamine, chloroprocaine, choline, diethanolamine, ethylenediamine, meglumine (N-methylglucamine) and procaine. All of these salts may be prepared by conventional means from the corresponding compound of Formula I by reacting, for example, the appropriate acid or base with the compound of Formula I.

## General Synthetic Procedures

The compounds of the invention can be synthesized according to the following procedures of Schemes I-XXIX, wherein the R substituents are as defined for Formula I, above, except where further noted.

# Scheme I

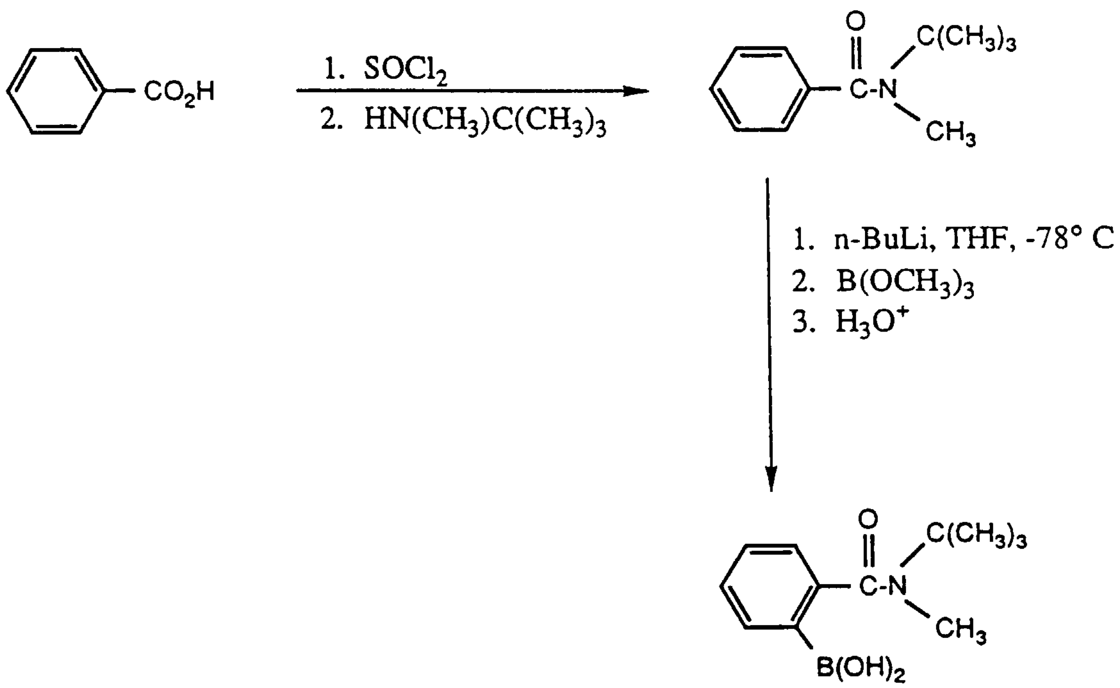


**1**

[$R^6$ =$CON(CH_3)C(CH_3)_3$]

Synthetic Scheme I shows the preparation of the boronic acid 1 where $R^6$ equals N-tertbutyl-N-methylcarboxamide. In step 1, benzoic acid is treated with thionyl chloride to give the corresponding acid chloride which is subsequently reacted with N-tertbutyl-N-methylamine to give N-tertbutyl-N-methylbenzamide. In step 2, the amide is orthometalated and subsequently reacted with trimethyl borate. The tree boronic acid 1 is produced on hydroylsis.

# Scheme II

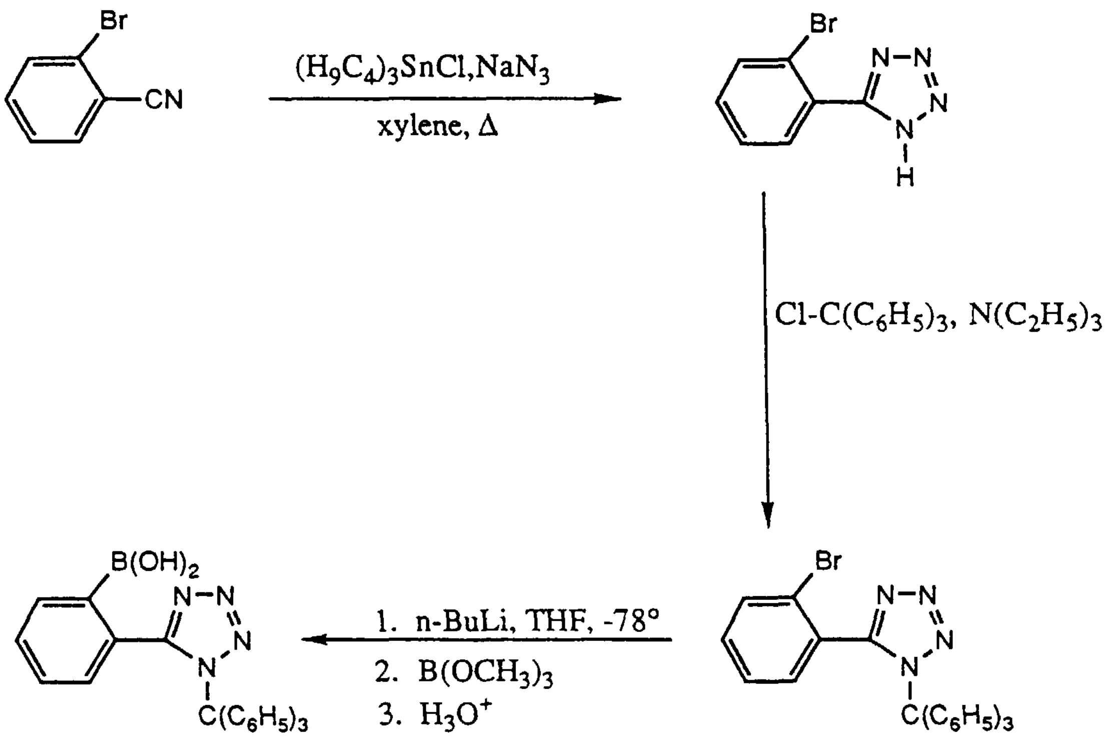


**1**

[$R^6$=$CN_4C(C_6H_5)_3$]

Synthetic Scheme II shows the preparation of the boronic acid 1 where $R^6$ equals N-triphenylmethyl-1H-tetrazole. In step 1, 2-bromobenzonitrile (Aldrich) is reacted with tributyltin azide to give the corresponding tetrazole. In step 2, the tetrazole is reacted with triphenylmethyl chloride in the presence of triethylamine to give the protected bromophenyltetrazole. In step 3, halogen-metal interchange with n-butyllithium generates the corresponding ortho-lithiated species which is reacted with trimethyl borate. The free boronic acid 1 is produced on hydrolysis.

# Scheme III

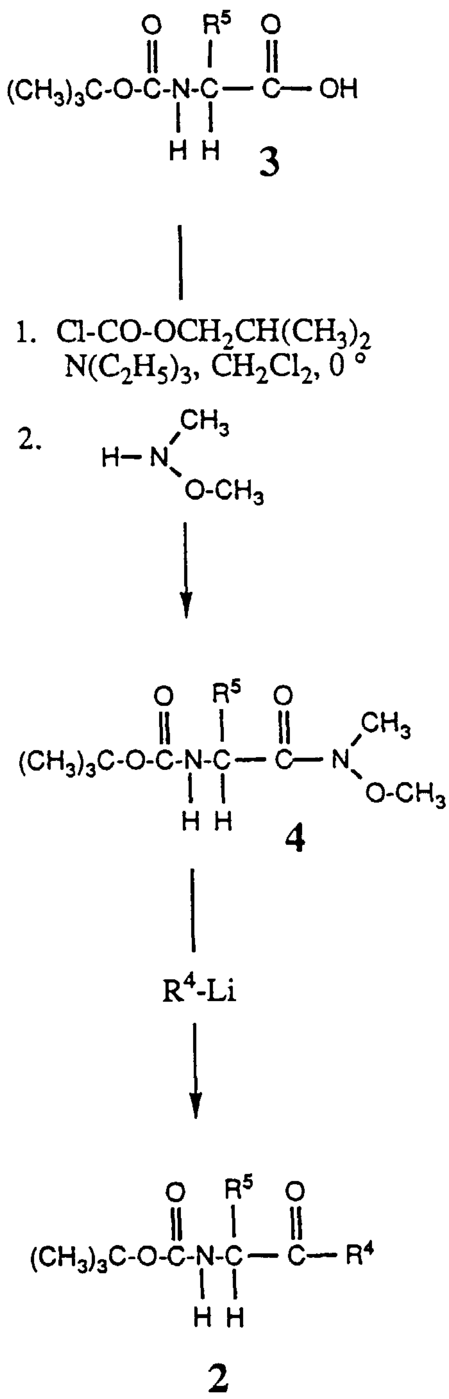


Synthetic Scheme III shows the preparation of N-Boc-amino ketones 2 (or aldehydes when $R^4$ = H) from the corresponding N-Boc-amino acides 3. In step 1, the amino acid 3 is reacted with isobutyl chloroformate in the presence of triethylamine and subsequently with N,O-dimethylhydroxylamine to give the corresponding N-methoxy-N-methylamide 4. In step 2, the amide 4 is reacted with an organolithium reagent $R^4$-Li (or lithium aluminum hydride (LAH) when $R^4$ = H) to give the desired ketone 2 (or aldehyde when $R^4$ = H).

# Scheme IV

## METHOD A:

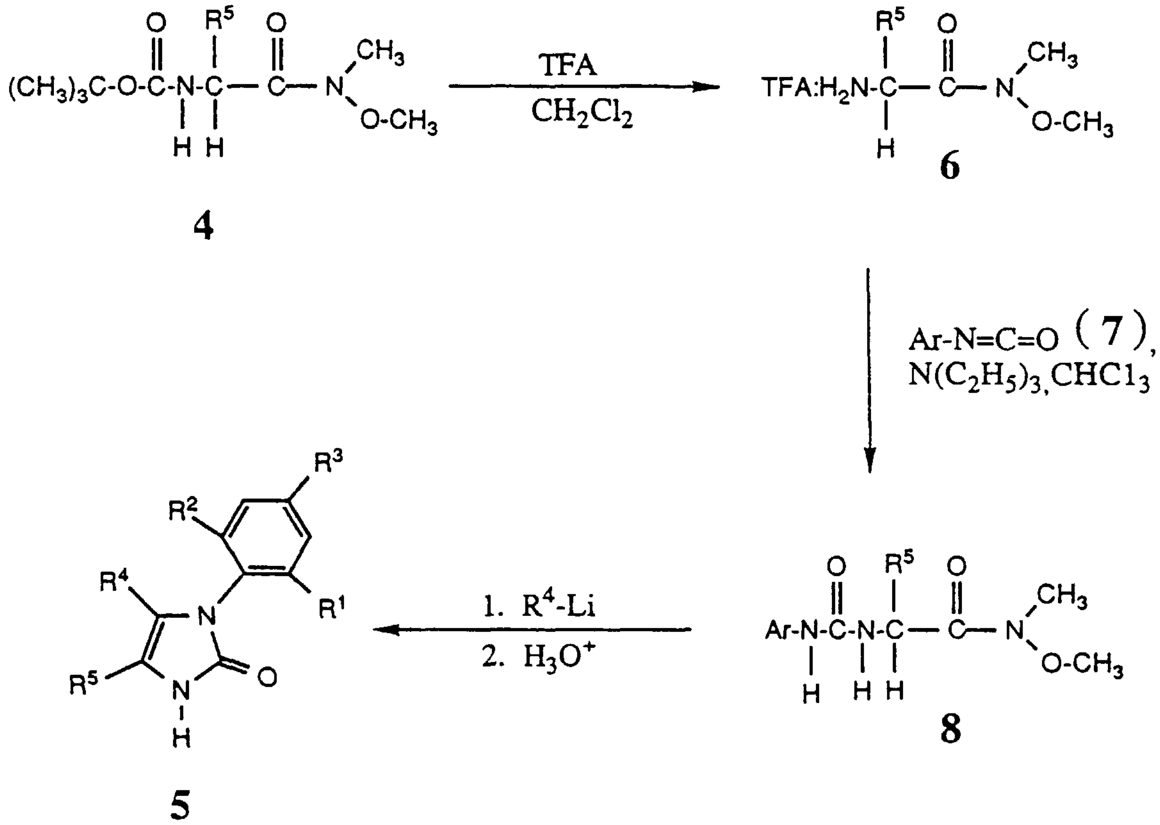


Synthetic Scheme IV shows the preparation of imidazol-2-ones 5 from the corresponding amides 4 via Method A. In step 1, the protected amide 4 (prepared in Scheme III) is reacted with trifluoroacetic acid (TFA) to give the TFA salt 6 of the free amine. In step 2, the salt 6 is reacted with the appropriate isocyanate 7 in the presence of triethylamine to give the urea 8. In step 3, the urea 8 is reacted with an organolithium reagent $R^4$-Li (or lithium aluminum hydride (LAH) when $R^4$ = H) and subsequently cyclized to the imidazole-2-one 5 on treatment with dilute acid during the work-up procedure.

# Scheme V

**METHOD B:**

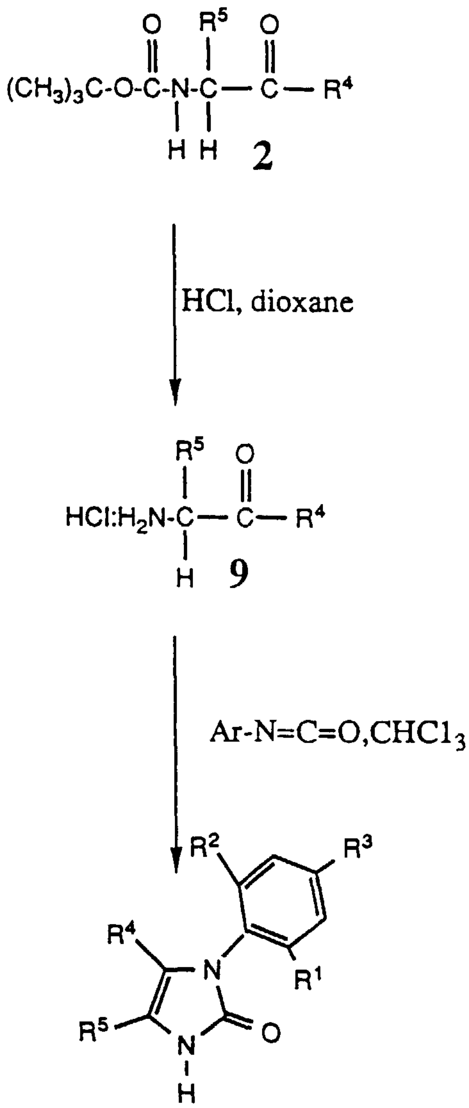


2

9

5

Synthetic Scheme V shows the preparation of imidazol-2-ones 5 from the corresponding N-Boc-protected amino ketones 2 (or aldehydes when $R^4$ = H) via Method B. In step 1, the carbonyl compound 2 (prepared in Scheme III) is reacted with anhydrous hydrogen chloride in dioxane to give the HCl salt 9. In step 2, the salt 9 is reacted with the

appropriate isocyanate 7 in chloroform to give the imidazol-2-one 5 directly.

## Scheme VI

### METHOD C:

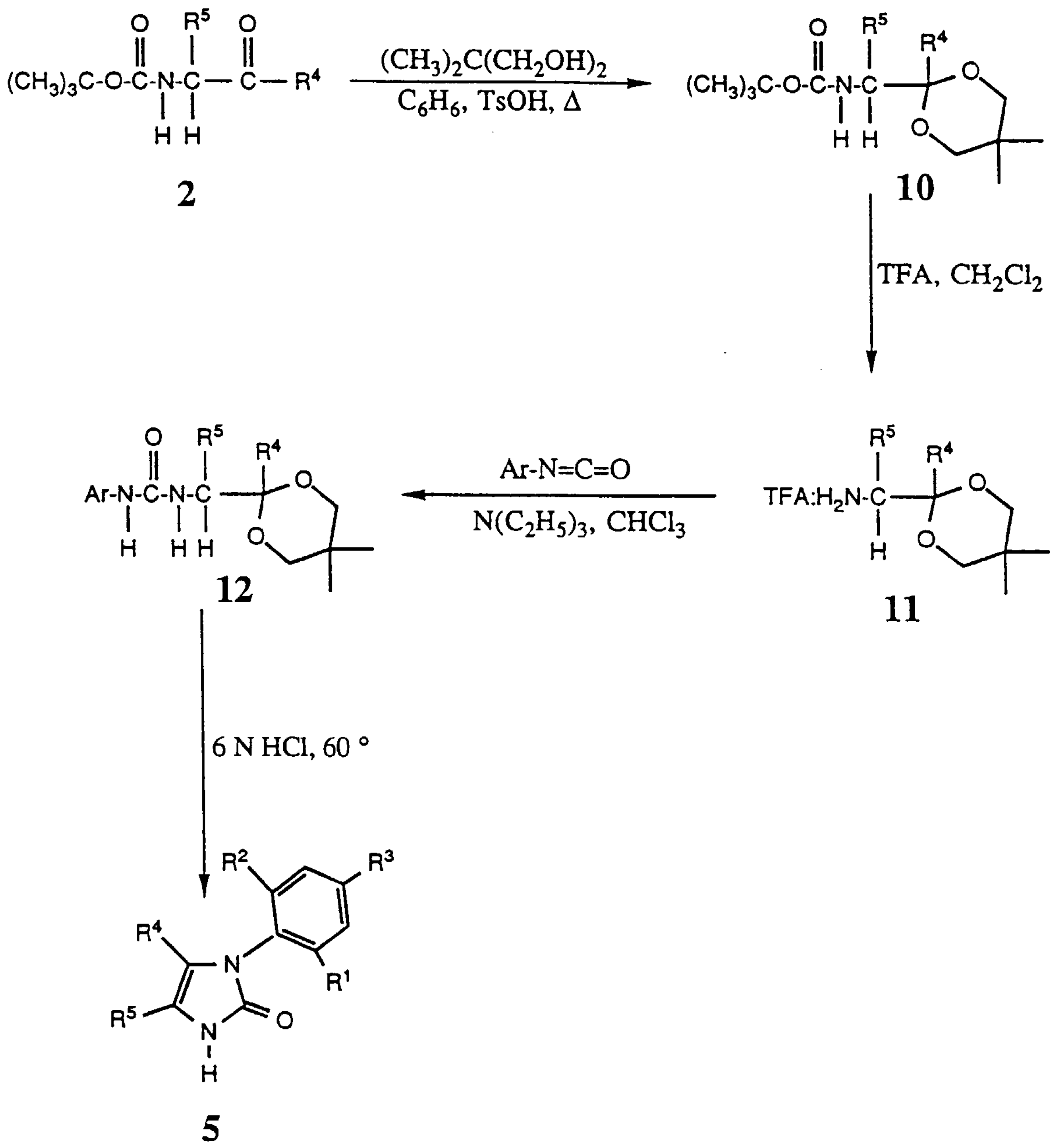


Synthetic Scheme VI shows the preparation of imidazol-2-ones 5 from the corresponding N-Boc-protected amino ketones 2 (or aldehydes when $R^4$ = H) via Method C. In step 1, the carbonyl compound 2 (prepared in Scheme III) is reacted with 2,2-dimethyl-1,3-propandiol to give the cyclic ketal 10. In step 2, the ketal 10 is reacted with TFA to give the TFA salt 11 of the free amine. In step 3, the salt 11 is reacted with the appropriate isocyanate 7 in the presence

of triethylamine to give the urea ketal 12. In step 4, the urea ketal 12 is reacted with 6N hydrochloric acid at 60°C to give the desired imidazol-2-one 5 directly.

# Scheme VII

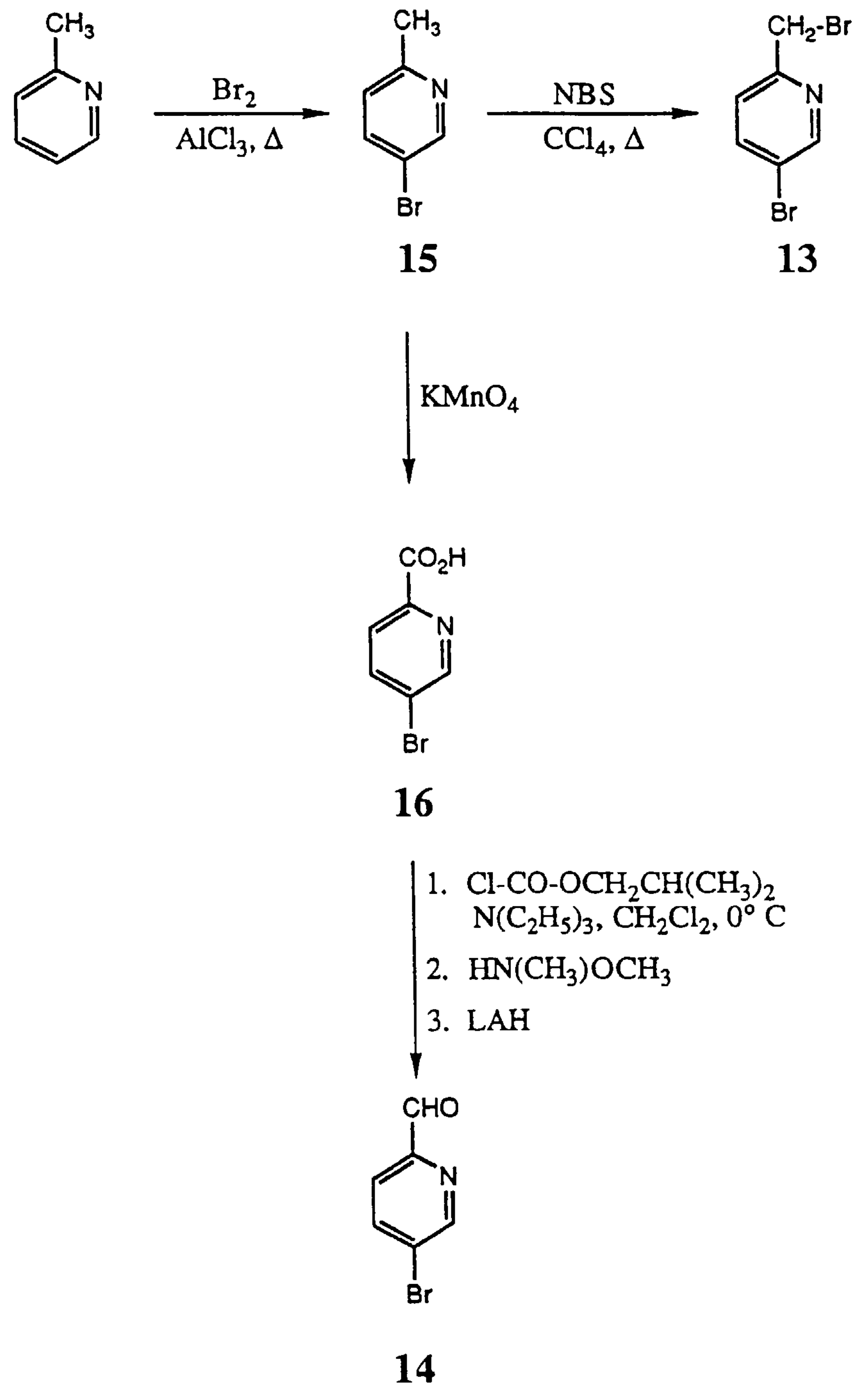


15

13

16

14

Synthetic Scheme VII shows the preparation of 2-bromomethyl-5-bromopyridine (13) and 5-bromo-2-pyridinecarboxaldehyde (14) from 2-picoline (Aldrich). In step 1, 2-picoline is reacted with bromine in the presence of a large excess of aluminum chloride at elevated temperatures to give 5-bromo-2-picoline (15). In step 2a, 15 is reacted with NBS to give the 2-pyridinylmethyl bromide 13. In step 2b, the intermediate 15 is treated with potassium permanganate to give the corresponding picolinic acid 16. In step 3b, the acid 16 is first converted to its N-methoxy-N-methylamide and subsequently reduced with LAH to provide 5-bromo-2-pyridinecarboxaldehyde (14).

# Scheme VIII

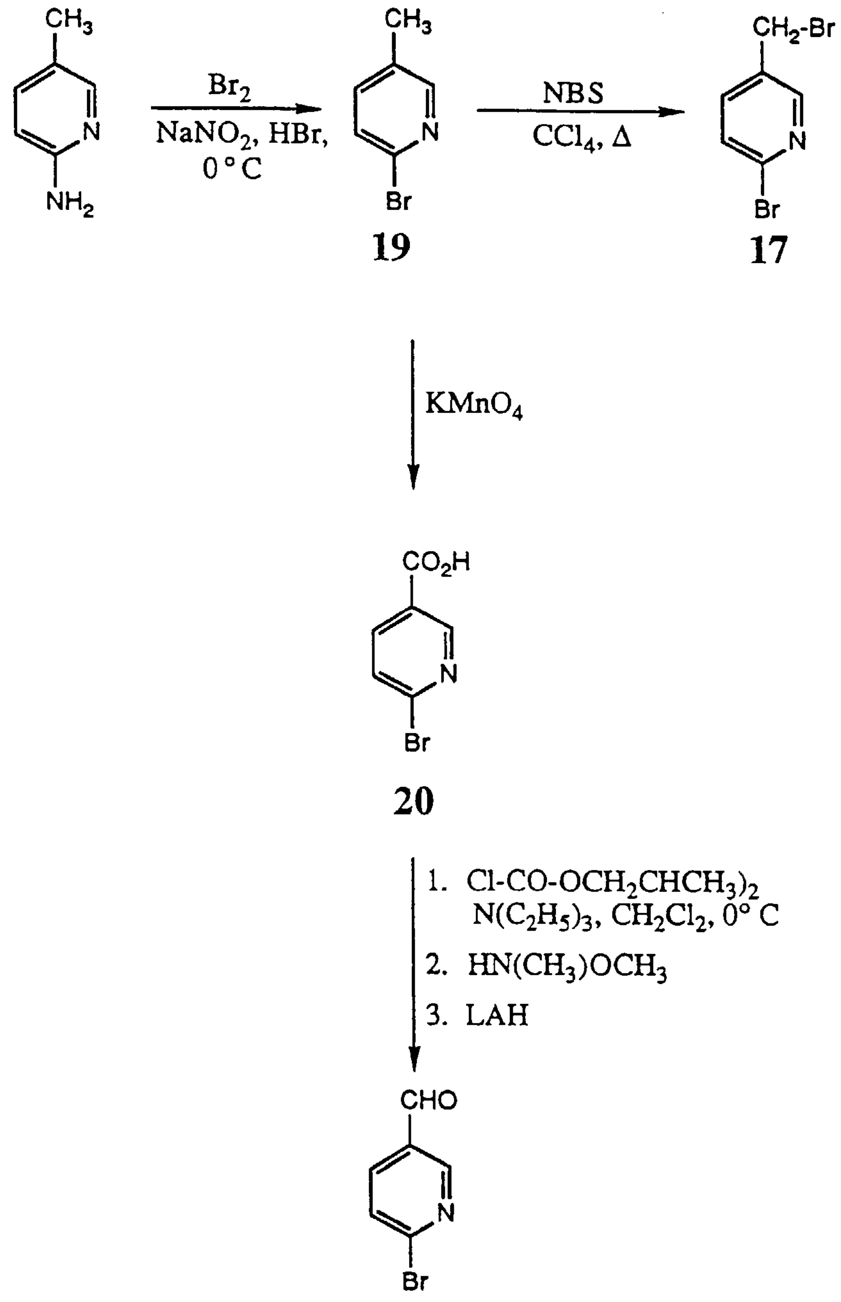


19

17

20

18

Synthetic Scheme VIII shows the preparation of 2-bromo-5-bromomethylpyridine (17) and 2-bromo-5-pyridinecarboxaldehyde (18) from 2-amino-5-picoline (Aldrich). In step 1, 2-amino-5-picoline is reacted with bromine in the presence of hydrobromic acid and sodium nitrite at 0°C to give 2-bromo-5-picoline (19). In step 2a, 19 is reacted with

NBS to give the 3-pyridinylmethyl bromide 17. In step 2b, the intermediate 19 is treated with potassium permanganate to give the corresponding nicotinic acid 20. In step 3b, the acid 20 is first converted to its N-methoxy-N-methylamide and subsequently reduced with LAH to provide 2-bromo-5-pyridinecarboxaldehyde (18).

## Scheme IX

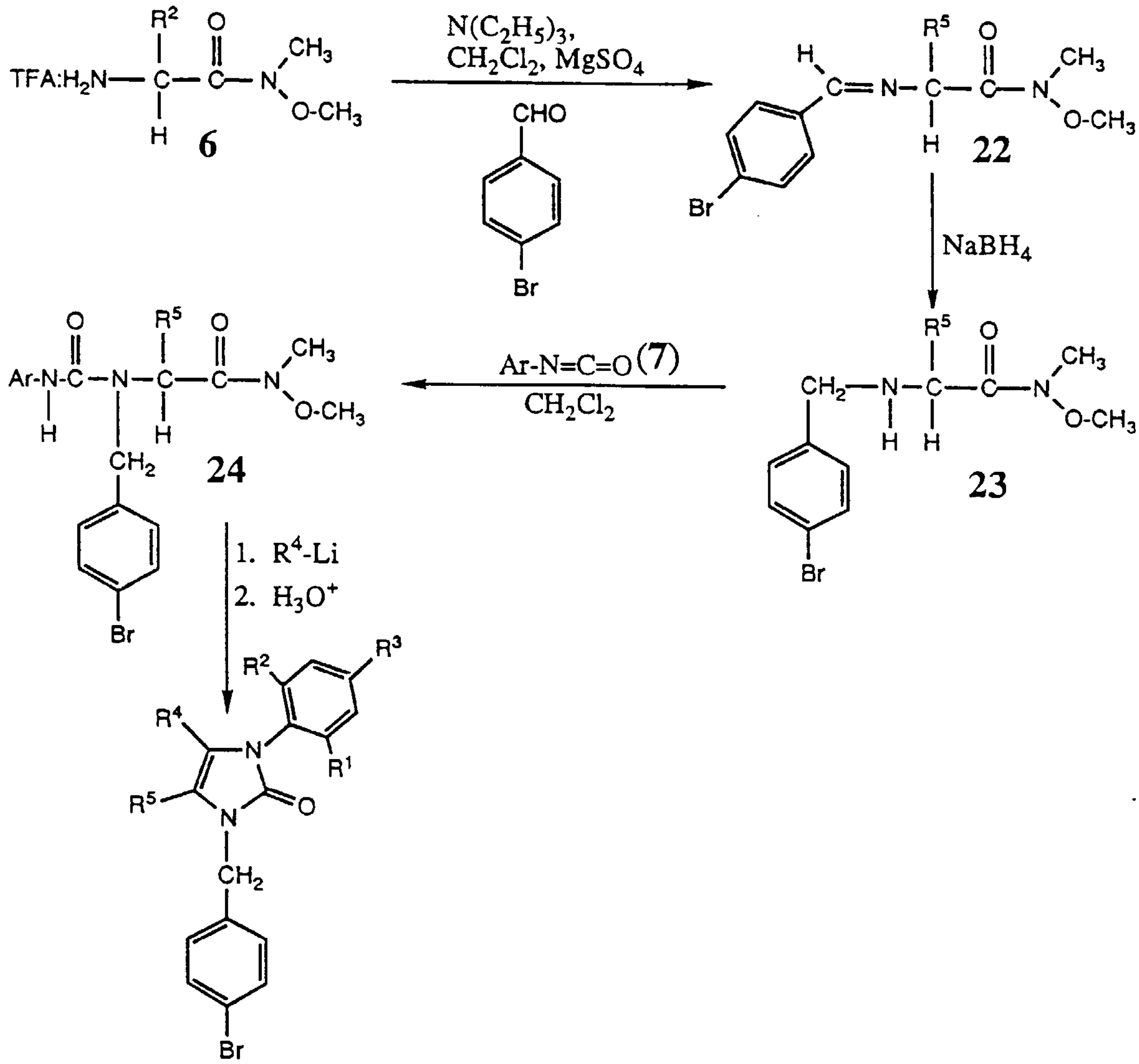


21

Synthetic Scheme IX shows the preparation of (4-bromobenzyl)imidazol-2-ones 21 from the TFA salt of the amino amide 6 (prepared in Scheme III). In step 1, the TFA salt 6 is allowed to react with the 4-bromobenzaldehyde in the presence of triethylamine and anhydrous magnesium sulfate to give the imine 22. In step 2, the imine 22 is allowed to react with sodium borohydride to give the substituted benzylamine 23. In step 3, the benzylamine 23 is allowed to react with the appropriate isocyanate 7 to give the substituted benzylurea 24. In step 4, the urea 24 is first allowed to react with an organolithium reagent $R^4$-Li (or lithium aluminum hydride (LAH) when $R^4$ = H) and subsequently with dilute aqueous acid to give the desired 3-(4-bromobenzyl)imidazol-2-ones.

# Scheme X

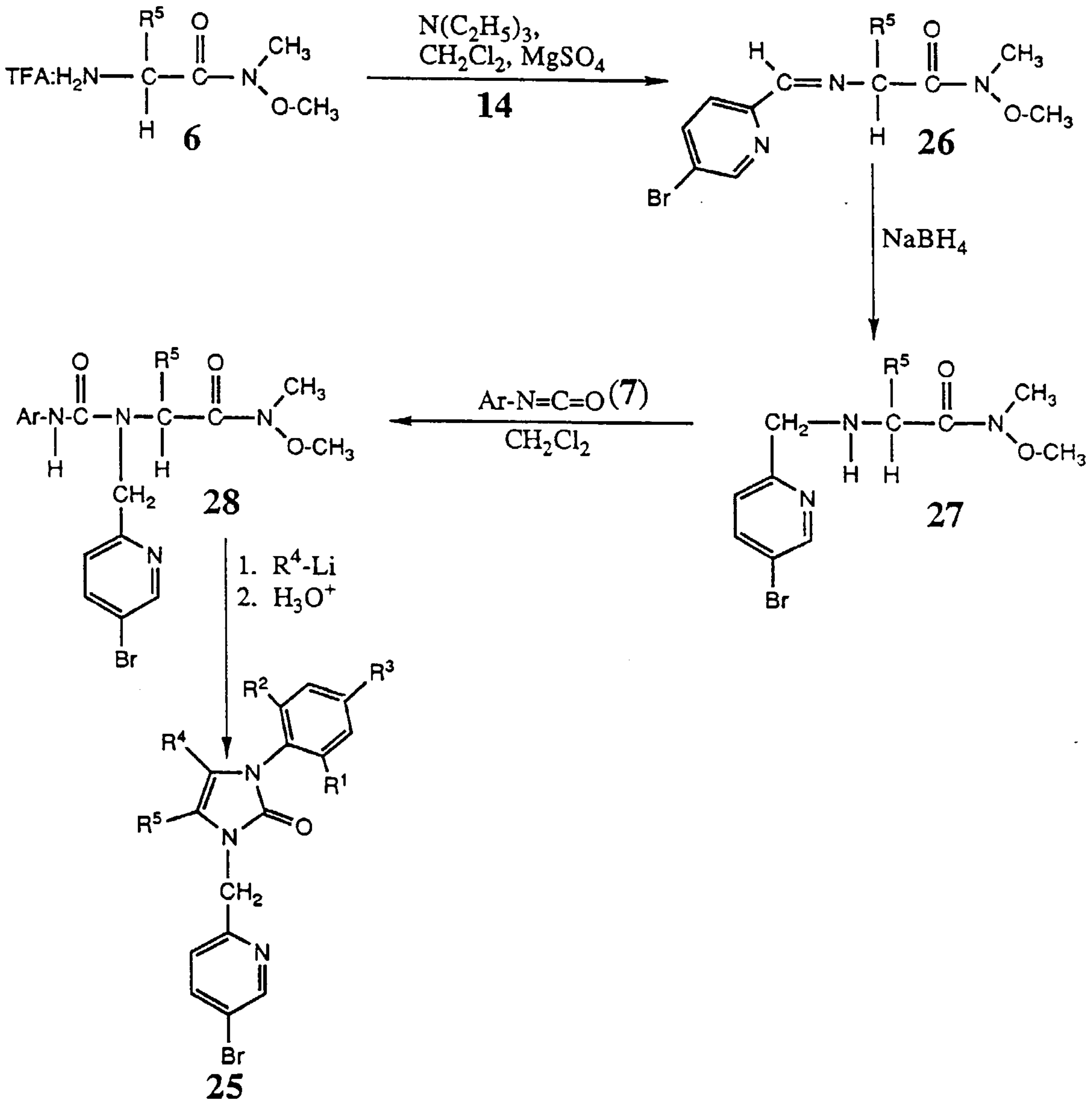


Synthetic Scheme X shows the preparation of 3-(5-bromo-2-pyridinylmethyl)imidazol-2-ones 25 from the TFA salt of the amino amide 6 (prepared in Scheme III). In step 1, the TFA salt 6 is allowed to react with the 5-bromo-2-pyridinylaldehyde 14 (prepared in Scheme VII) in the presence of triethylamine and anhydrous magnesium sulfate to give the imine 26. In step 2, the imine 26 is allowed to react with sodium borohydride to give the substituted benzylamine 27. In step 3, the benzylamine 27 is allowed to react with the appropriate isocyanate 7 to give the substituted benzylurea 28. In step 4, the urea 28 is first allowed to react with an organolithium reagent $R^4$-Li (or lithium aluminum hydride (LAH) when $R^4$ = H) and subsequently with dilute aqueous acid to give the desired 3-(5-bromo-2-pyridinylmethyl)imidazol-2-ones 25.

## Scheme XI

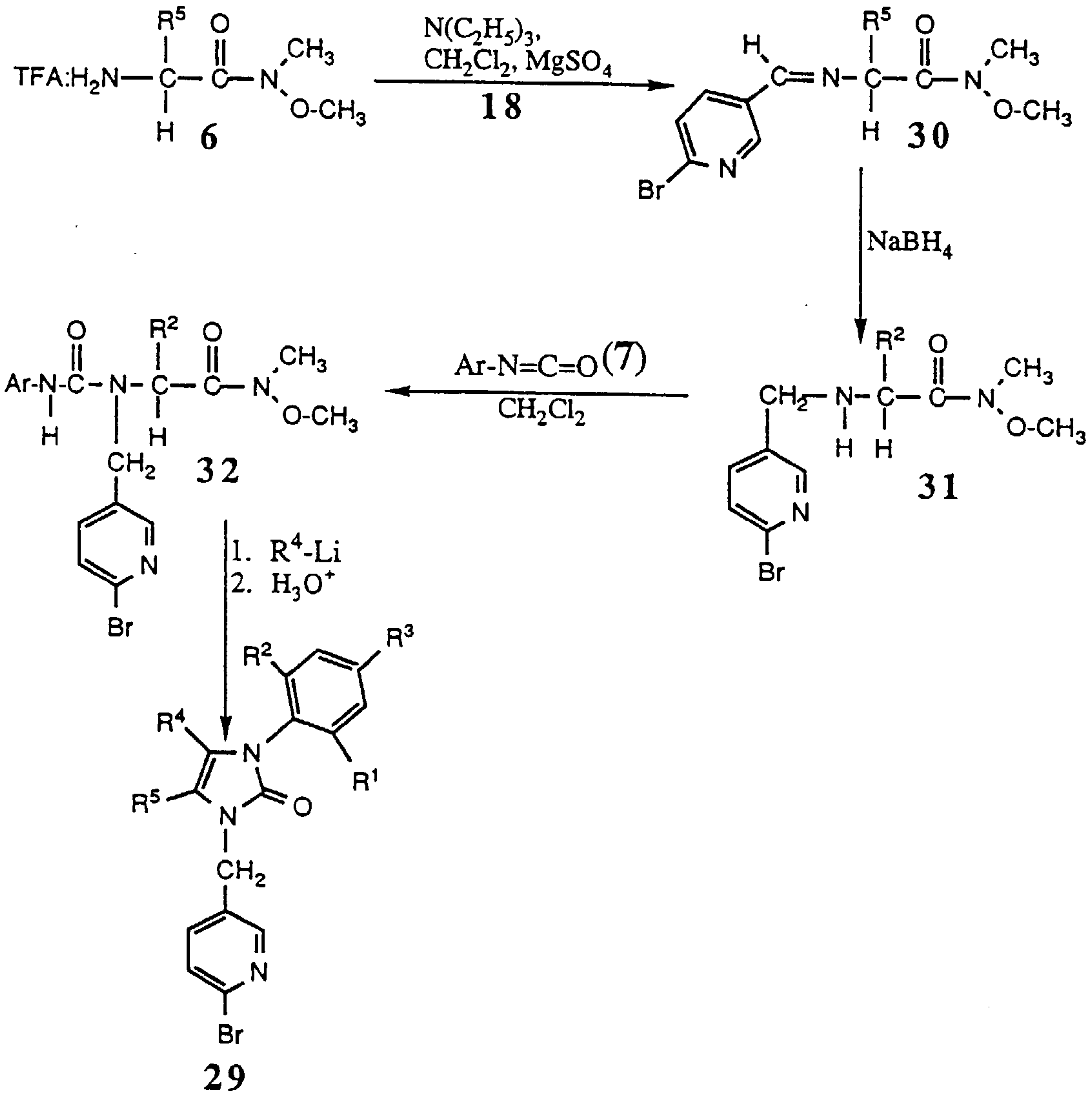


Synthetic Scheme XI shows the preparation of 3-(2-bromo-5-pyridinylmethyl)imidazol-2-ones 29 from the TFA salt of the amino amide 6 (prepared in Scheme III). In step 1, the TFA salt 6 is allowed to react with 2-bromo-5-pyridinylaldehyde 18 (prepared in Scheme VIII) in the presence of triethylamine and anhydrous magnesium sulfate to give the imine 30. In step 2, the imine 30 is allowed to react with sodium borohydride to give the substituted benzylamine 31. In step 3, the benzylamine 31 is allowed to react with the appropriate isocyanate 7 to give the substituted benzylurea 32. In step 4, the urea 32 is first allowed to react with an organolithium reagent $R^4$-Li (or lithium aluminum hydride (LAH) when $R^4$ = H) and subsequently with dilute aqueous acid to give the desired 3-(2-bromo-5-pyridinylmethyl)imidazol-2-ones 29.

# Scheme XII

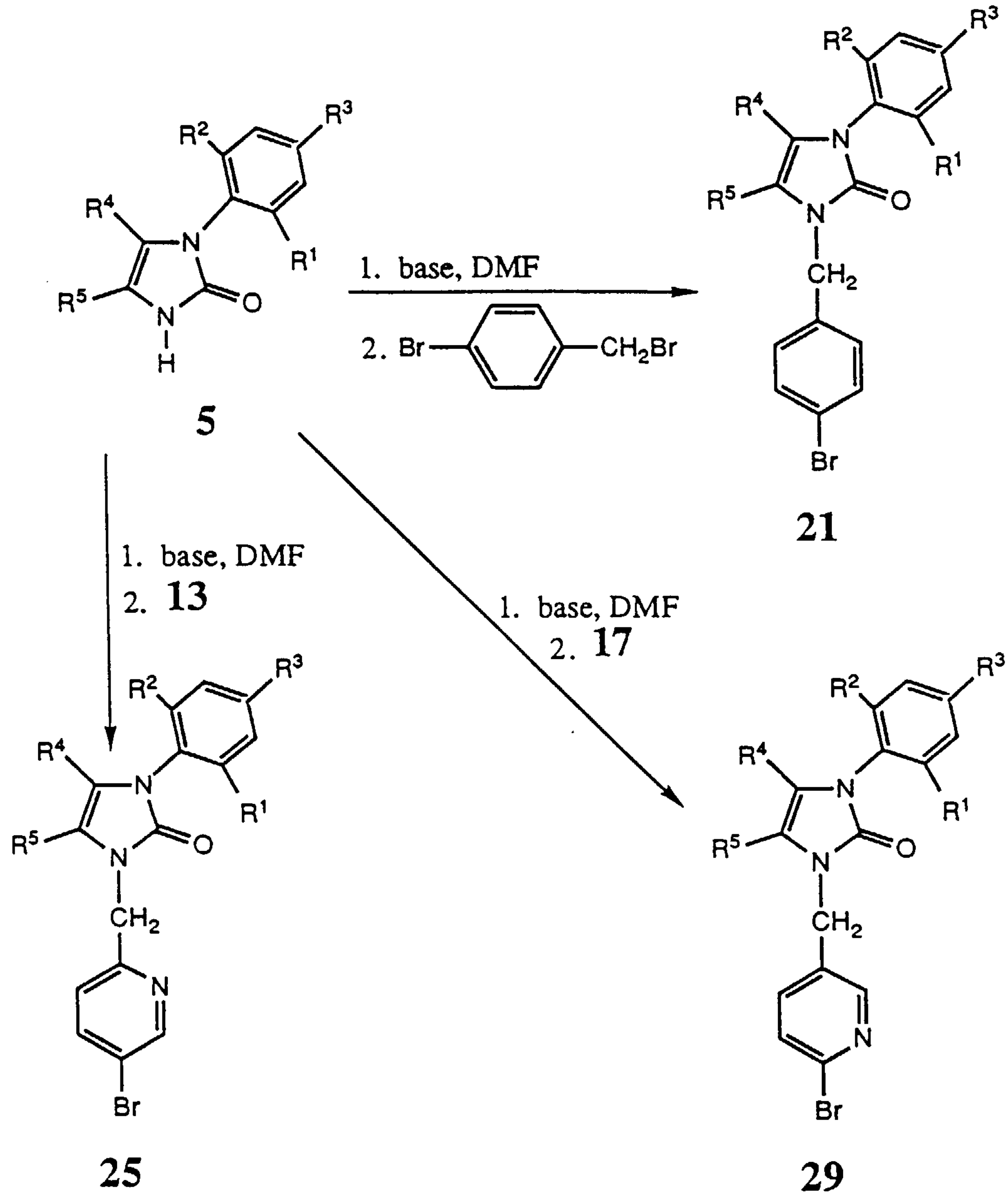


5 21 25 29

Synthetic Scheme XII shows the preparation of 3-(4-bromobenzyl)imidazol-2-ones 21, 3-(5-bromo-2-pyridinylmethyl)imidazol-2-ones 25, and 3-(2-bromo-5-pyridinylmethyl)imidazol-2-ones 29 from the parent imidazol-2-ones 5 (prepared in Scheme IV, Scheme V, or Scheme VI). The imidazol-2-one 5 is first treated with a base, such as potassium t-butoxide, and subsequently with the alkylating agent 4-bromobenzyl bromide, 13 (prepared in Scheme VII), and 17 (prepared in Scheme VIII) to give 3-(4-bromobenzyl)imidazol-2-ones 21, 3-(5-bromo-2-pyridinylmethyl)imidazol-2-ones 25, and 3-(2-bromo-5-pyridinylmethyl)imidazol-2-ones 29, respectively.

# Scheme XIII

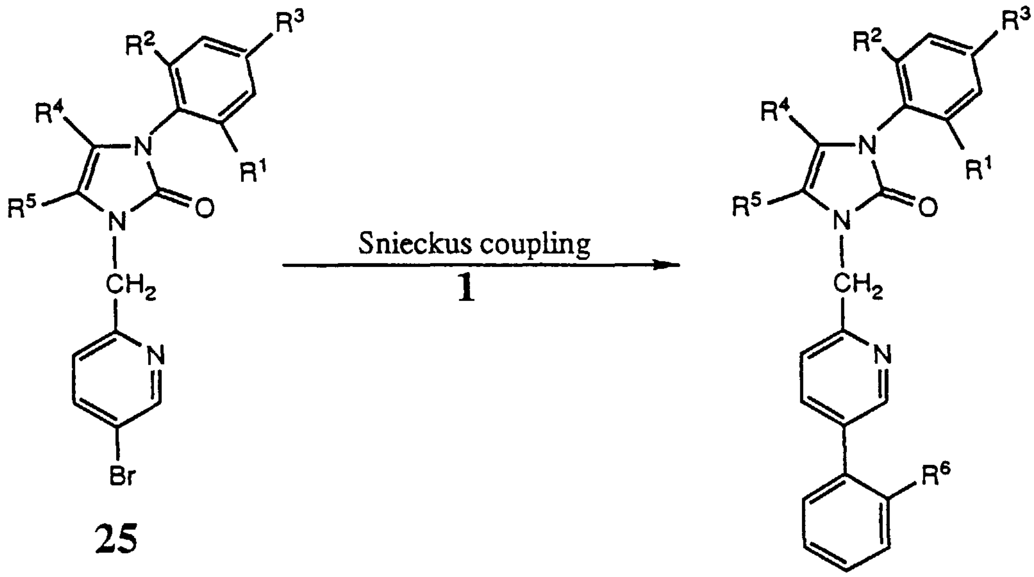


25

30

Synthetic Scheme XIII shows the preparation of 3-[[5-[2-(1H-tetrazol-5-yl)phenyl]-2-pyridinyl]methyl]-2H-imidazol-2-ones 30 from the boronic acid 1 (prepared in Scheme I and Scheme II) and the bromoimidazol-2-one coupling reagent 25 (prepared in Scheme X and Scheme XII). The boronic acid 1 is reacted with the bromoimidazol-2-one coupling reagent 25 in the presence of a palladium zero catalyst via a Snieckus coupling [see M. J. Sharp and V. Snieckus, Tetrahedron Lett.., 5997(1985)] to give the angiotensin II antagonists 30 of this invention.

# Scheme XIV

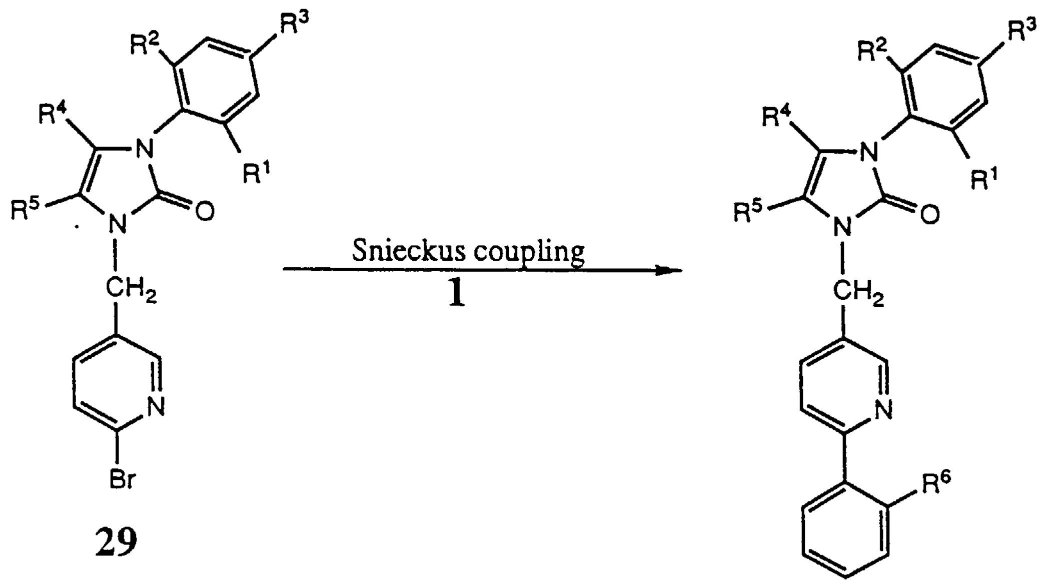


29

31

Synthetic Scheme XIV shows the preparation of 3-[[6-[2-(1H-tetrzol-5-yl)phenyl]-3-pyridinyl]methyl]-2H-imidazol-2-ones 31 from the boronic acid 1 (prepared in Scheme I and Scheme II) and the bromoimidazol-2-one coupling reagent 29 (prepared in Scheme XI and Scheme XII). The boronic acid 1 is reacted with the bromoimidazol-2-one coupling reagent 29 in the presence of a palladium zero catalyst via a Snieckus coupling [see M. J. Sharp and V. Snieckus, Tetrahedron Lett., 5997(1985)] to give the angiotensin II antagonists 31 of this invention.

# Scheme XV

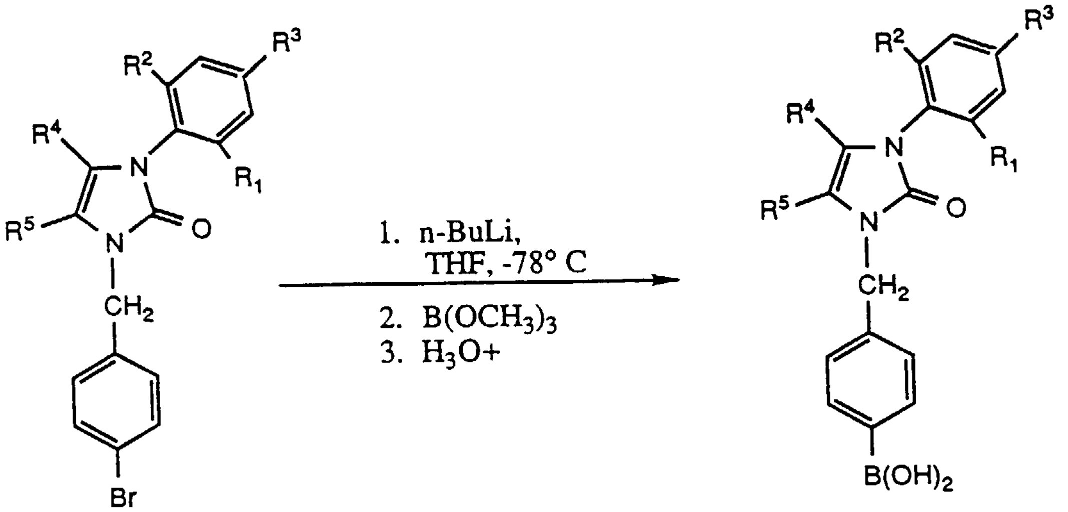


21

32

Synthetic Scheme XV shows the preparation of the imidazol-2-one boronic acid coupling reagents 32 from the corresponding 3-(4-bromobenzyl)imidazol-2-ones 21 (prepared in Scheme IX and Scheme XII). Halogen-metal interchange generates the corresponding lithiated species from 21 which is reacted with trimethyl borate. The free imidazol-2-one boronic acid coupling reagents 32 are produced on acid hydrolysis.

# Scheme XVI

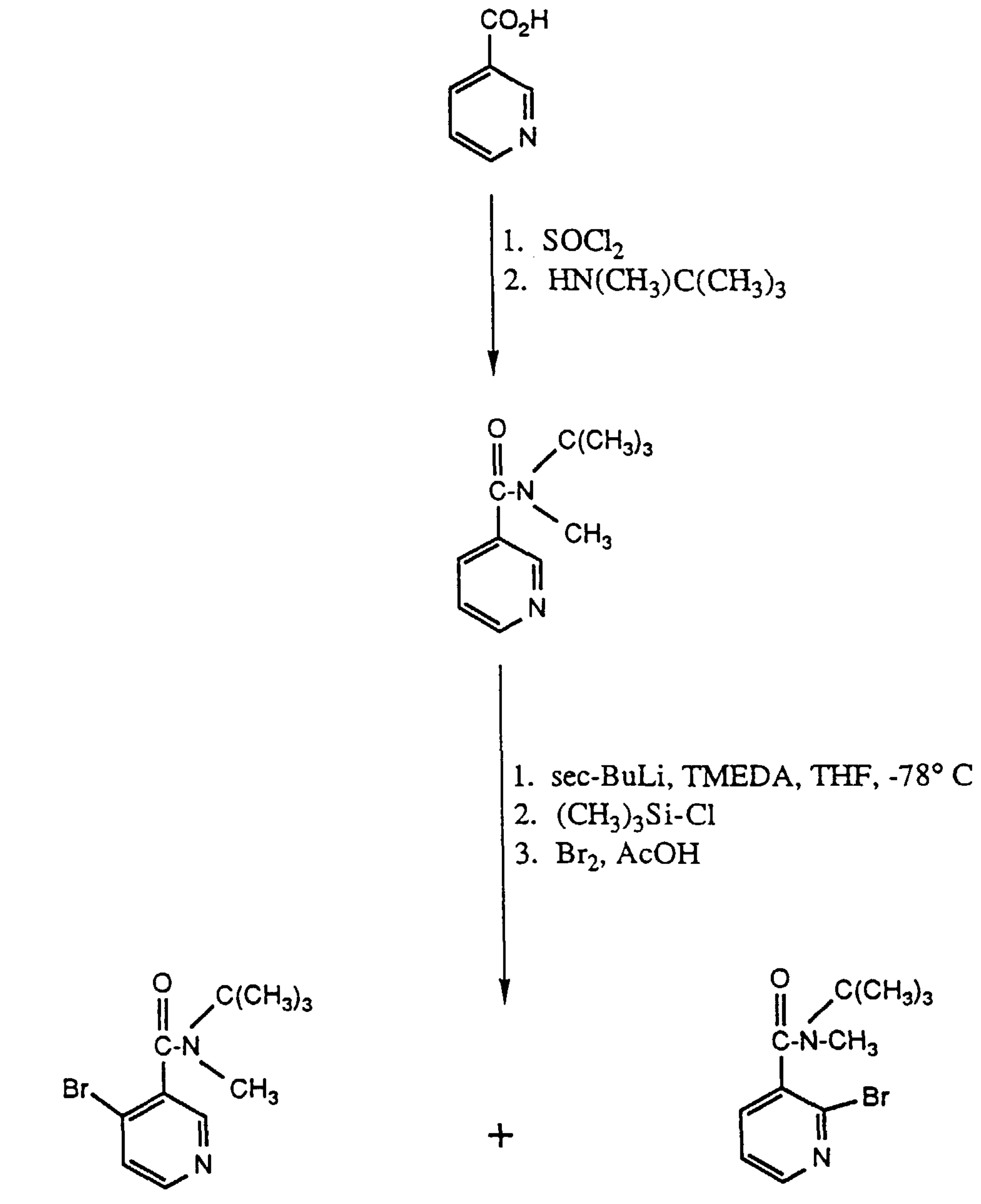


**33** $[R^6=CON(CH_3)C(CH_3)_3]$

**34** $[R^6=CON(CH_3)C(CH_3)_3]$

Synthetic Scheme XVI shows the preparation of the 4-bromopyridine coupling reagent 33 $[R^6 = CON(CH_3)C(CH_3)_3]$ and the 2-bromopyridine coupling reagent 34 $[R^6 = CON(CH_3)C(CH_3)_3]$ from nicotinic acid. In step 1, N-tertbuty-N-methylnicotinamide is prepared from nicotinoyl chloride and N-tertbutyl-N-methylamine. In step 2, ortho-metalalion with sec-butyllithium gives a mixture of regioanions which are reacted with trimethylsilyl chloride; subsequent conversion to the corresponding bromides on treatment with bromine in acetic acid and separation of the regioisomers by chromatography provides 33 and 34.

## Scheme XVII

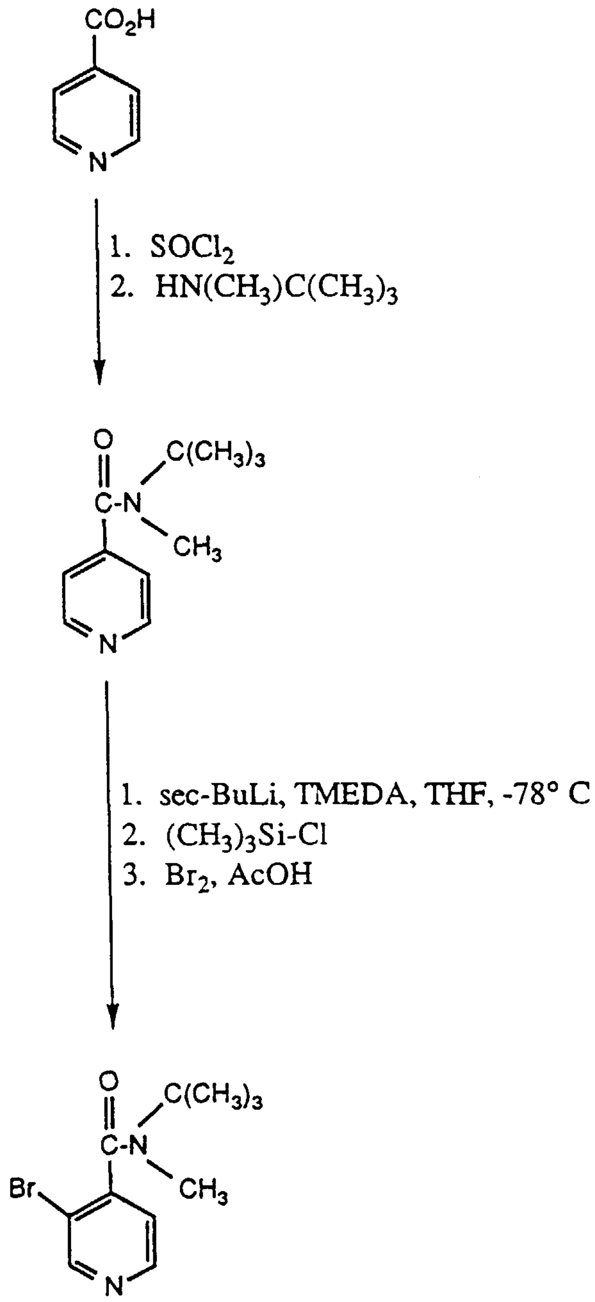


35 [$R^6$=$CON(CH_3)C(CH_3)_3$]

Synthetic Scheme XVII shows the preparation of the 3-bromopyridine coupling reagent 35 [$R^6$ = $CON(CH_3)C(CH_3)_3$] from isonicotinic acid. In step 1, N-tertbutyl-N-methylisonicotinamide is prepared from isonicotinoyl

chloride and N-tertbutyl-N-methylamine. In step 2, reaction with sec-butyllithium gives the ortho-lithiated species which is reacted with trimethylsilyl chloride and subsequently converted to the corresponding bromide 35 on treatment with bromine in acetic acid.

## Scheme XVIII

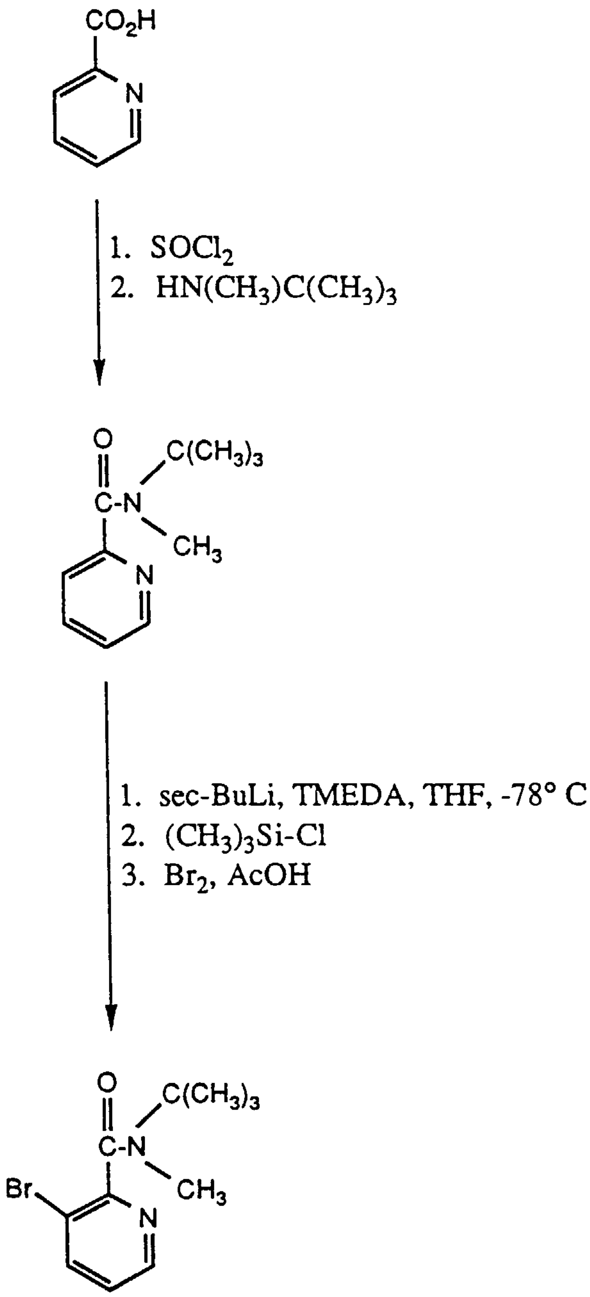


**36** [$R^6$=CON($CH_3$)C($CH_3$)$_3$]

Synthetic Scheme XVIII shows the preparation of the 3-bromopyridine coupling reagent 36 [$R^6$ = $CON(CH_3)C(CH_3)_3$] from picolinic acid. In step 1, N-tertbutyl-N-methylpicolinamide is prepared from picolinoyl chloride and N-tertbutyl-N-methylamine. In step 2, reaction with sec-butyllithium gives the ortho-lithiated species which is reacted with trimethylsilyl chloride and subsequently converted to the corresponding bromide 36 on treatment with bromine in acetic acid.

## Scheme XIX

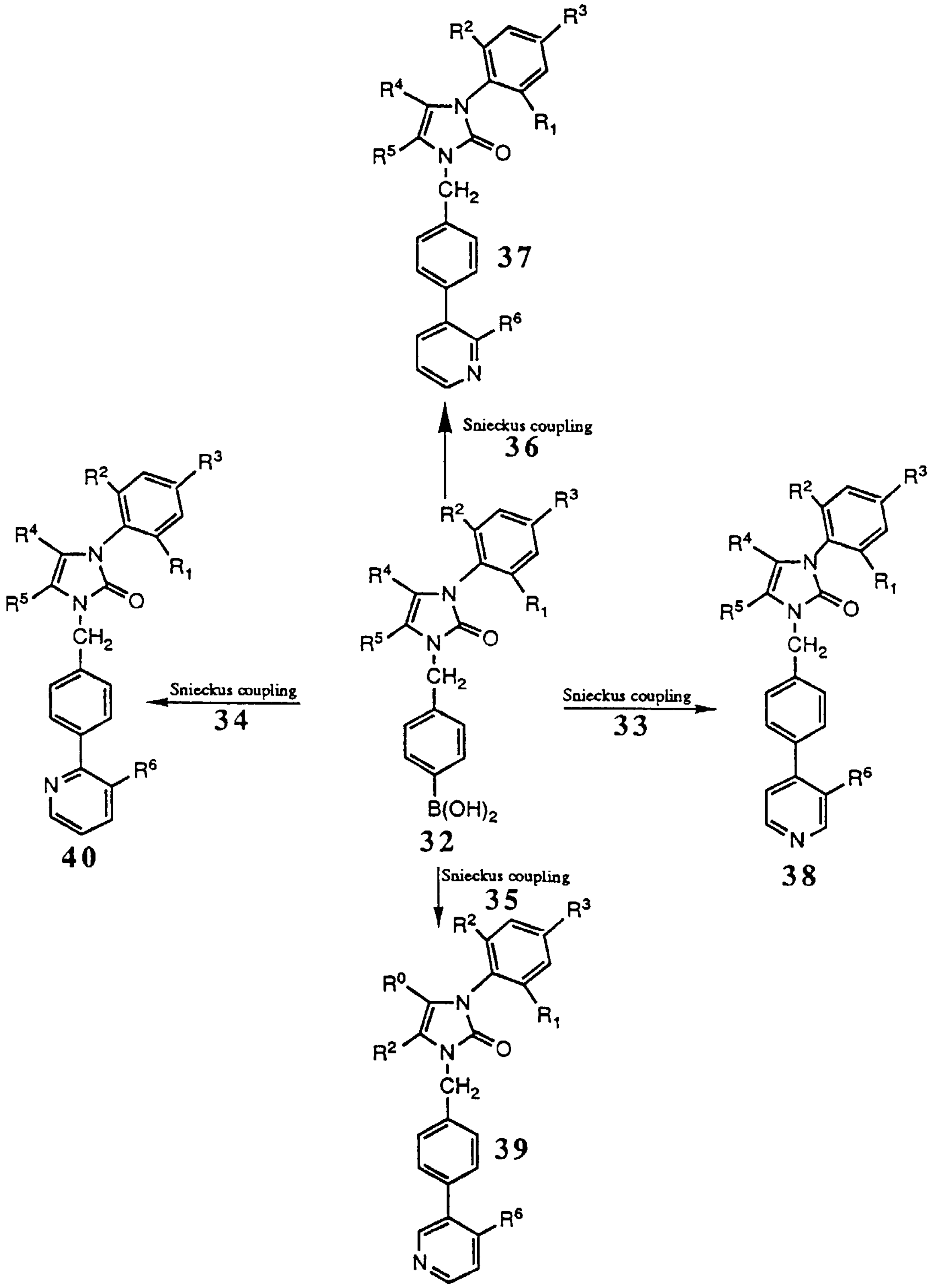


37, 38, 39, 40, 32

Synthetic Scheme XIX shows the preparation of 3-(pyridinylbenzyl)imidazol-2-ones 37, 38, 39 and 40 from the common imidazol-2-one boronic acids 32 (Scheme XV) and the corresponding bromo coupling reagents 36 (Scheme XVIII), 33 (Scheme XVI, 35 (Scheme XVII), and 34 (Scheme XVI), respectively. The boronic acids 32 are reacted with

the bromo coupling reagents 36, 33, 35 and 34 in the presence of a palladium zero catalyst via a Snieckus coupling [see M. J. Sharp and V. Snieckus, Tetrahedron Lett., 5997 (1985)] to give the angiotensin II antagonists 37, 38, 39 and 40, respectively, of this invention.

## Scheme XX

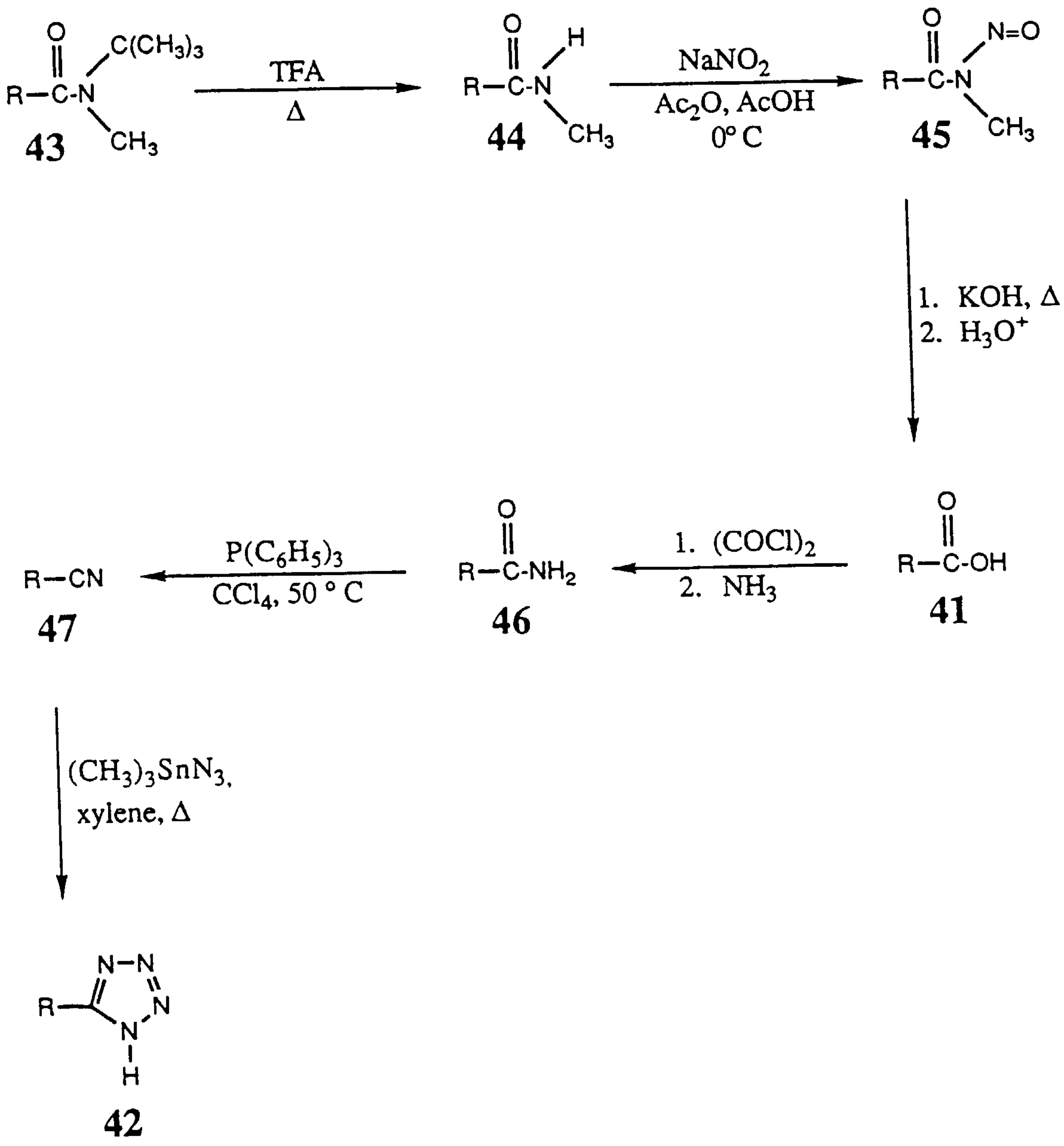


43 44 45 41 46 47 42

Synthetic Scheme XX shows the preparation of carboxylic acid analogs 41 and 1H-tetrazole analogs 42 from analogs which have $R^6$ = $CON(CH_3)C(CH_3)_3$. In step 1, the N-tertbutyl-N-methylamide analog 43 is reacted with trifluoroacetic acid at reflux to give the N-methylamide 44. In step 2, the N-methylamide 44 is reacted with sodium nitrite in acetic anhydride/acetic acid at 0°C to give the corresponding N-methyl-N-nitrosoamide 45. In step 3, the N-methyl-N-nitrosoamide 45 is hydrolyzed in base to give the corresponding carboxylic acid angiotensin II antagonists of this invention. In step 4, the acid analog 41 is reacted with oxalyl chloride and subsequently with anhydrous ammonia to give

the primary amide 46. In step 5, the amide 46 is reacted with triphenylphosphine in carbon tetrachloride at 50°C to give the corresponding nitrile 47. In step 6, the nitrile 47 is reacted with trimethyltin azide in xylene at reflux to provide the 1H-tetrazole angiotensin II antagonists of this invention.

## Scheme XXI

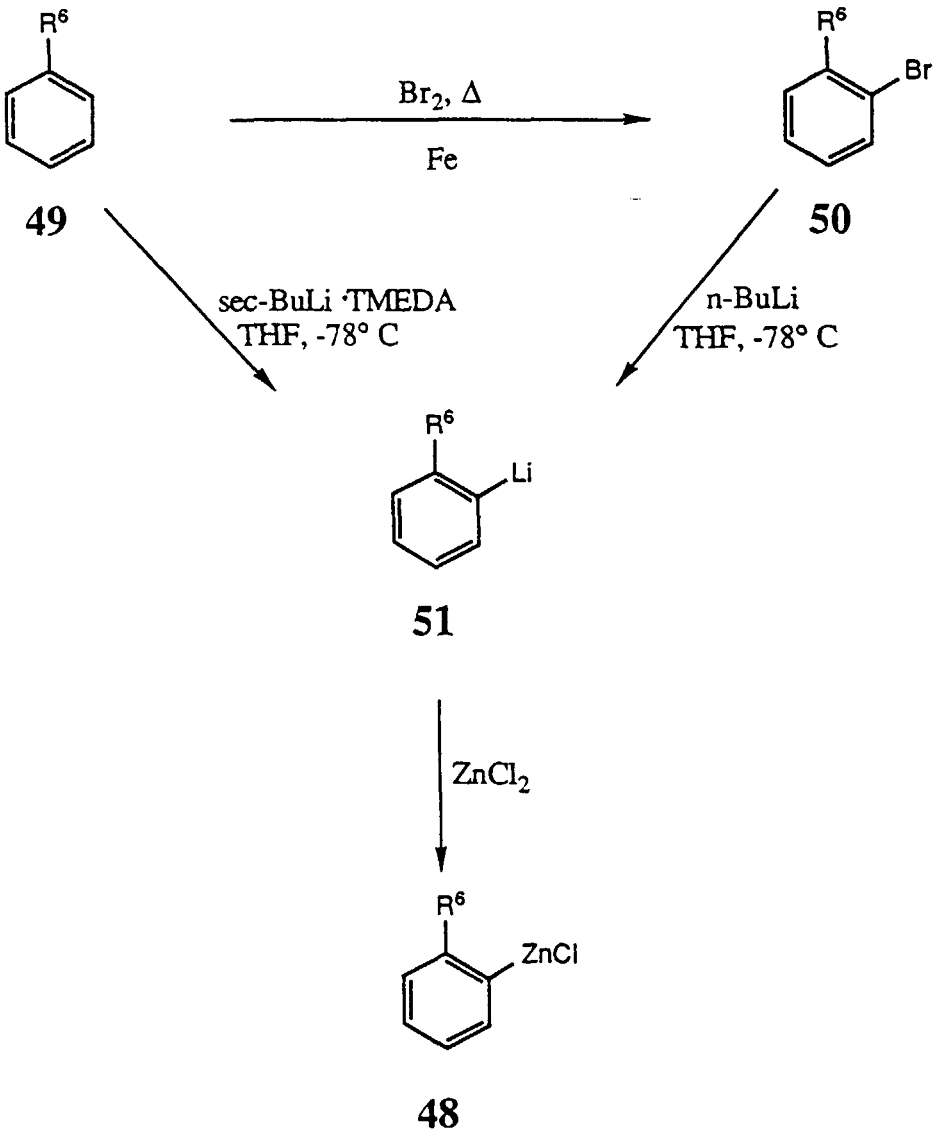


49

50

51

48

$R^6$= $CO_2R$, CN, $CN_4C(C_6H_5)_3$, or $CN_4H$

Synthetic Scheme XXI shows the preparation of the organozinc reagent 48 from the appropriate benzoic acid analog 49. In step 1, the analog 49 is brominated with bromine in the presence of a suitable catalyst, e.g., iron, to give the 2-bromo analog 50. In step 2, the 2-bromo analog 50 was converted to the organolithium reagent 51 by reaction with n-butyllithium in THF at -78°C, a process known as halogen-metal interchange. Alternatively, the organolithium reagent 51 can be generated directly by the reaction of 49 with an alkyllithium reagent in the presence or absence of a suitable complexing agent in THF at -78°C, e.g., sec-butyllithium/TMEDA (N,N,N',N'-tetramethylethylenediamine). In step 3, the organolithium reagent 51 was treated with anhydrous zinc chloride at -78°C and subsequently allowed to warm to ambient temperature. The organozinc reagent 48 was generated and used in situ.

## Scheme XXII

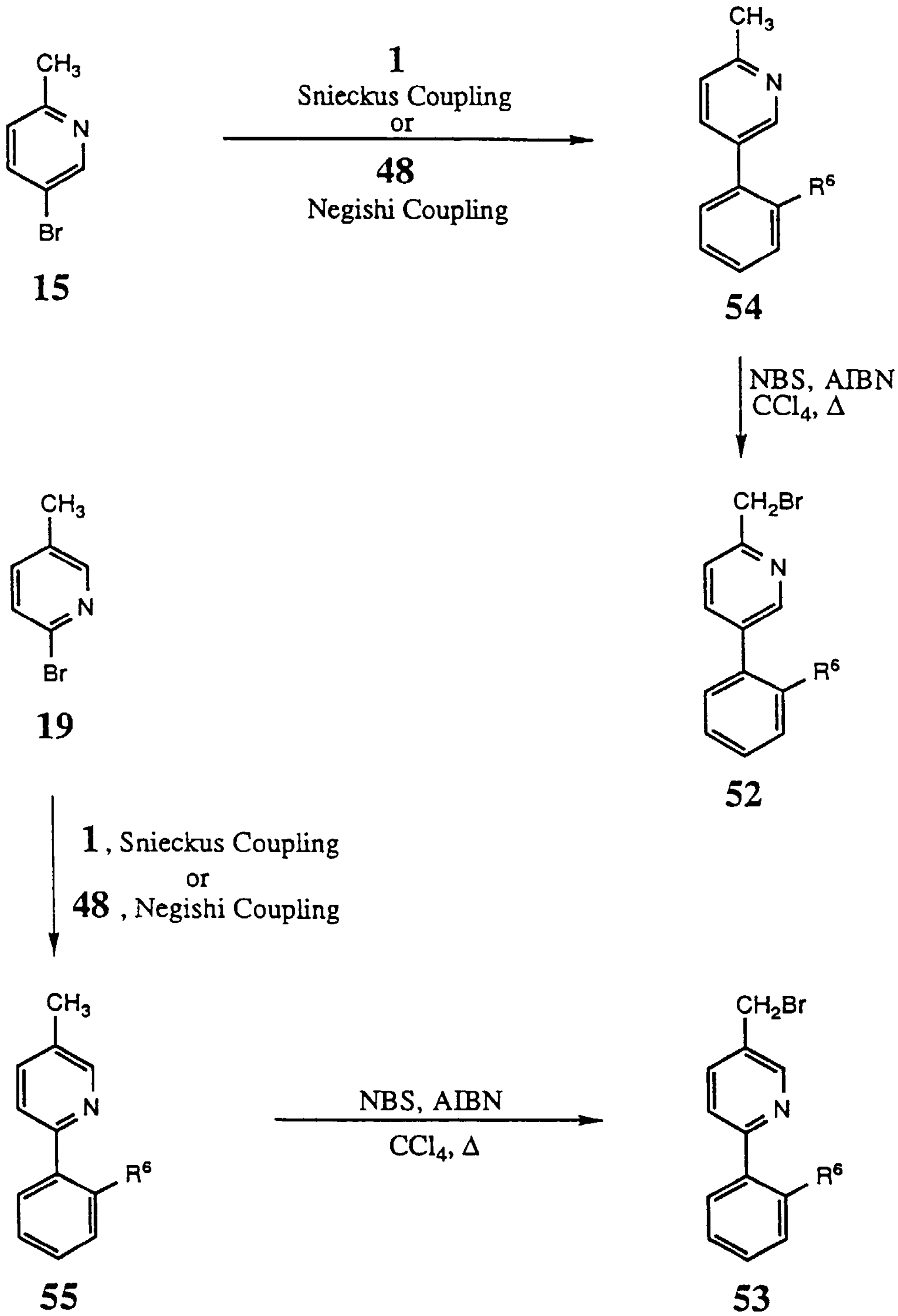


15 54 52 19 55 53

Synthetic Scheme XXII shows the preparation of the 2-pyridinyl alkylating reagent 52 and the 3-pyridinyl alkylating 53 from 15 (scheme VII) and 19 (Scheme VIII), respectively. In step 1, 15 and 19 were coupled with 1 using Snieckus conditions (Scheme XIII) or 48 using Negishi conditions [see E. Negishi, A. O. King, and N. Okukado, J. Org. Chem., 42, 1821 (1977)] to give 54 and 55, respectively. In step 2, the coupled biaryl compounds 54 and 55 were brominated using NBS/AIBN to give the 2-pyridinyl alkylating reagent 52 and the 3-pyridinyl alkylating reagent 53, respectively.

## Scheme XXIII

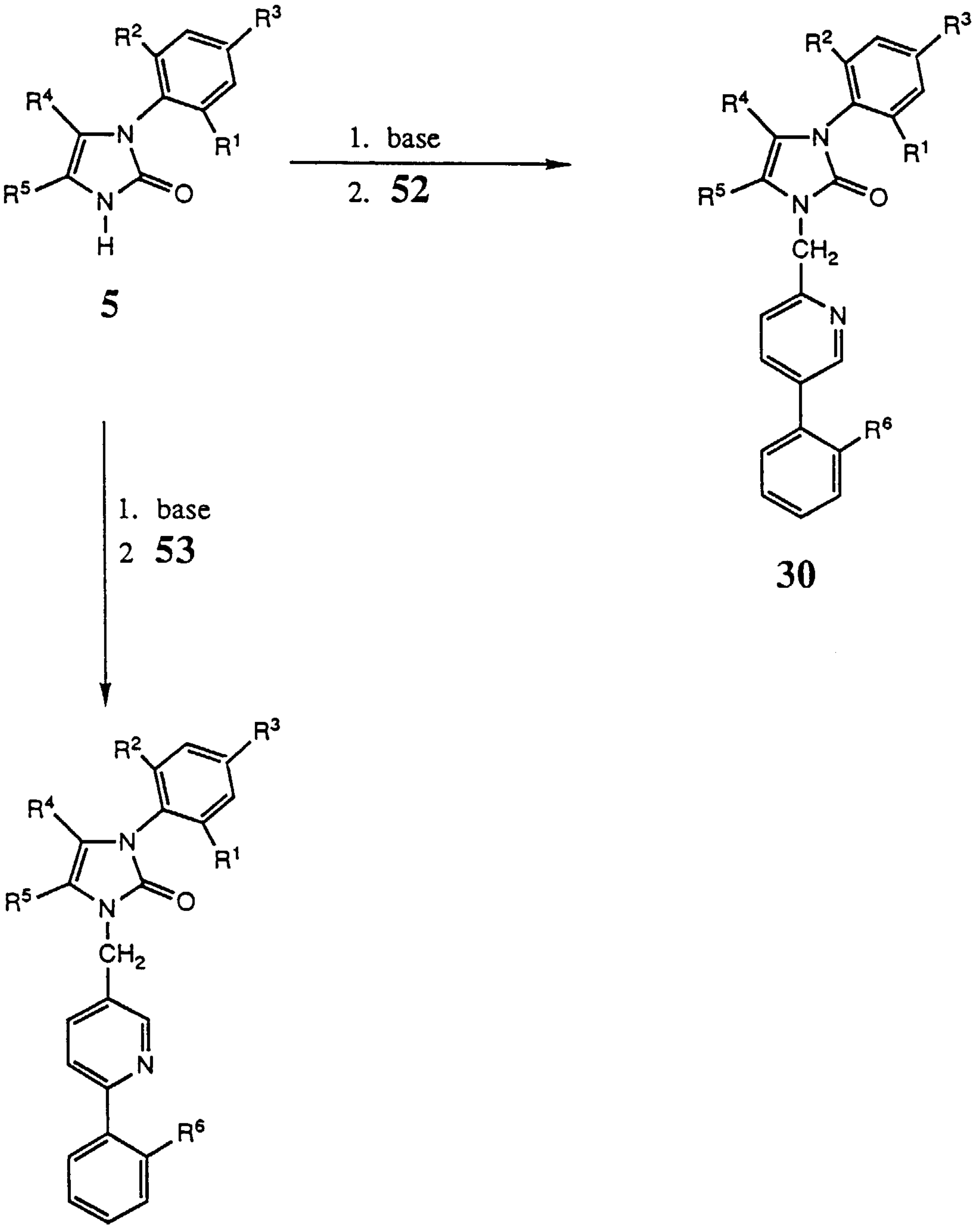


5

30

31

Synthetic Scheme XXIII shows the preparation of 3-[[5-[2-(1H-tetrazol-5-yl)phenyl]-2-pyridinyl]methyl]-2H-imidazol-2-ones 30 ($R^6$=$CN_4H$) and 3-[[6-[2-(1H-tetrazol-5-yl)phenyl]-3-pyridinyl]methyl]-2H-imidazol-2-ones 31 ($R^6$=$CN_4H$) from the parent imidazol-2-ones 5 (prepared in Scheme IV, Scheme V, or Scheme VI). The imidazol-2-one 5 was first treated with a base, such as potassium t-butoxide, and subsequently with the alkylating reagent 52 or 53

(Scheme XXII) to give 3-[[5-[2-(1H-tetrazol-5-yl)phenyl]-2-pyridinyl]methyl]-2H-imidazol-2-ones 30 ($R^6$=$CN_4H$) and 3-[[6-[2-(1H-tetrazol-5-yl)phenyl]-3-pyridinyl]methyl]-2H-imidazol-2-ones 31 ($R^6$=$CN_4H$), respectively.

## Scheme XXIV

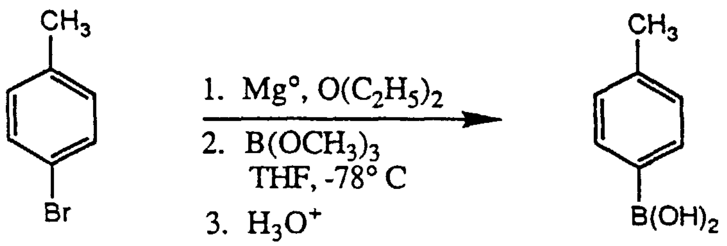


56

Synthetic Scheme XXIV shows the preparation of 4-methylphenylboronic acid (56) from 4-bromotoluene. In step 1, the Grignard reagent was generated by the reaction of 4-bromotoluene with metallic magnesium in ether at reflux. In step 2, a THF solution of trimethoxyborane was cooled to -78° C and slowly treated with the Grignard reagent. In step 3, the boronic ester was hydrolyzed with aqueous hydrochloric acid to give 4-methylphenylboronic acid (56).

# Scheme XXV

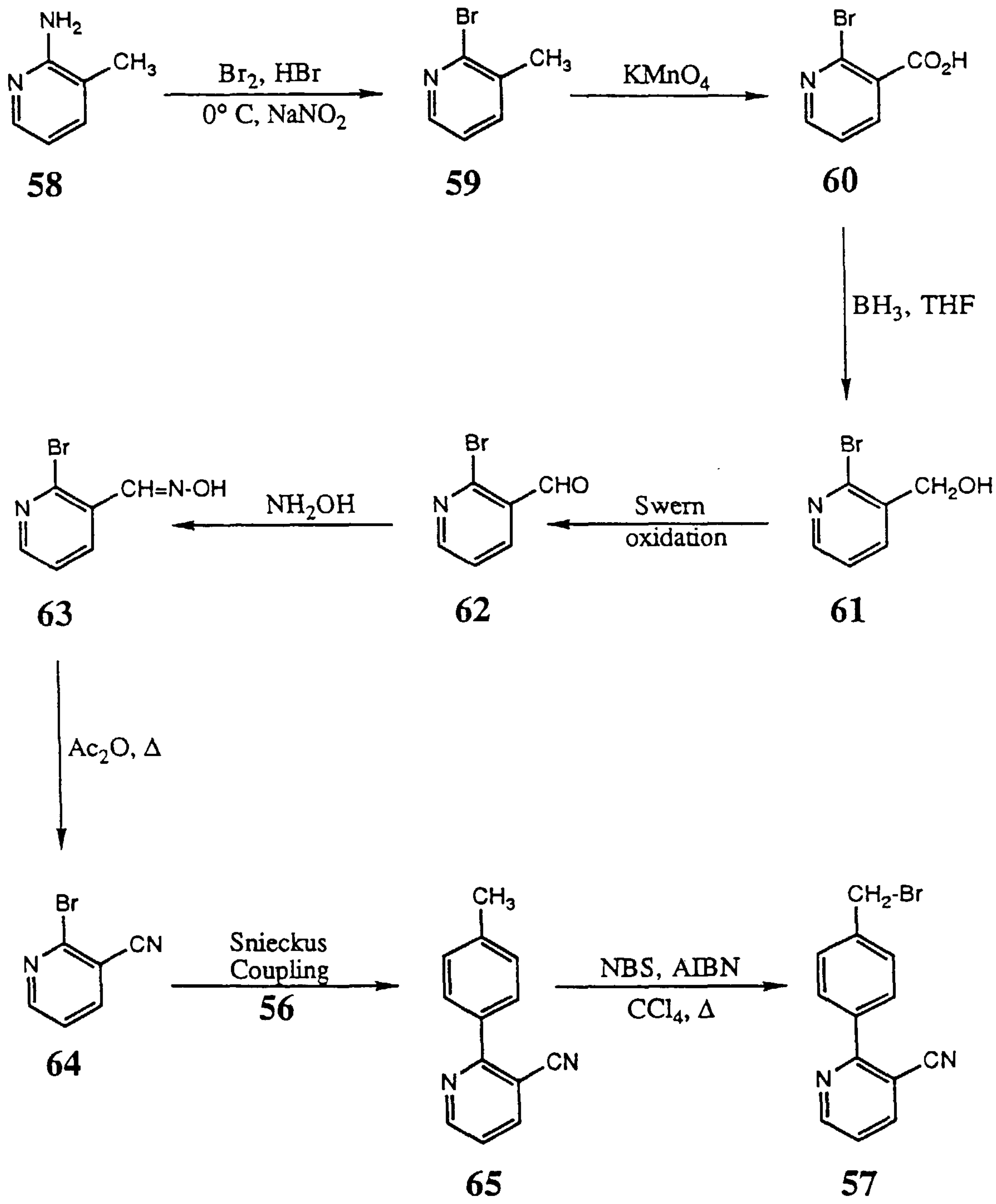


Synthetic Scheme XXV shows the 8-step preparation of the alkylating reagent 2-(4-bromomethylphenyl)-3-cyanopyridine (57) from 2-amino-3-picoline (58) (Aldrich). In step 1, the aminopicoline 58 was converted to the bromopicoline 59 by reaction with bromine, concentrated hydrobromic acid, and sodium nitrite at 0°C. In step 2, the picoline 59

was oxidized with $KMNO_4$ to give the corresponding carboxylic acid 60. In step 3, the acid 60 was reduced to the alcohol 61 with borane/THF. In step 4, the alcohol 61 was oxidized to the aldelyde 62 under Swern conditions or by using $MnO_2$. In step 5, the aldehyde 62 was reacted with hydroxylamine to give the oxime 63. In step 6, the oxime 63 was converted to 2-bromo-3-cyanopyridine (64) with acetic anhydride at reflux. In step 7, the nitrile 64 was coupled with 4-methylphenylboronic acid (56) (Scheme XXIV) using Snieckus conditions (Scheme XIII) to give 3-cyano-2-(4-methylphenyl)pyridine (65). In step 8, 65 was brominated with NBS/AIBN in carbon tetrachloride at reflux to give the desired alkylating reagent 57.

# Scheme XXVI

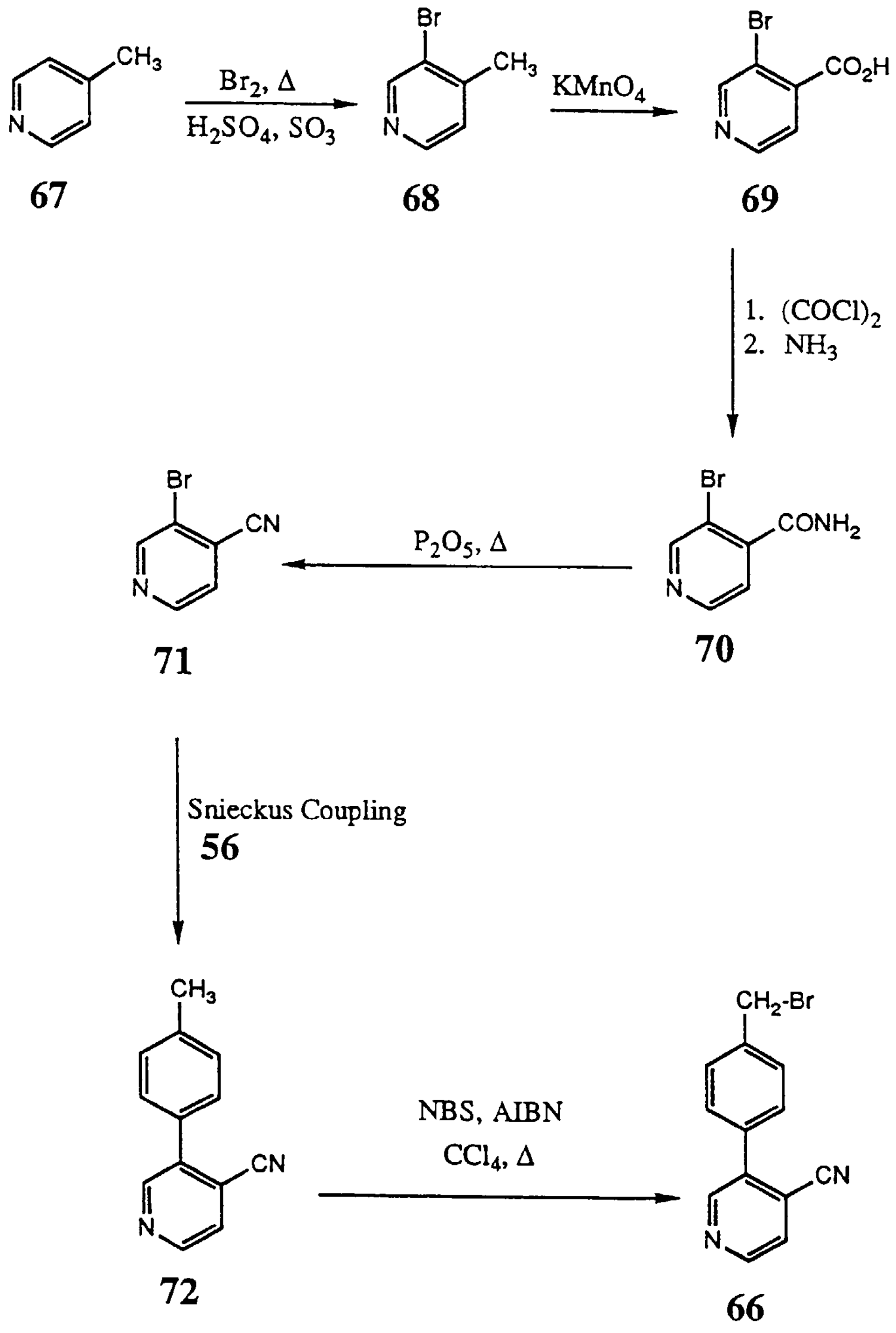


67 68 69 70 71 72 66

Synthetic Scheme XXVI shows the 6-step preparation of the alkylating reagent 3-(4-bromomethylphenyl)-4-cyanopyridine (66) from 4-picoline (67) (Aldrich). In step 1, 4-picoline was brominated with bromine in fuming sulfuric

acid at high temperatures to give 3-bromo-4-picoline (68). In step 2, the picoline 68 was oxidized to the corresponding carboxylic acid 69 with $KMnO_4$. In step 3, the acid 69 was first converted to its acid chloride with oxalyl chloride and subsequently treated with condensed ammonia to give the amide 70. In step 4, the amide 70 was converted to 3-bromo-4-cyanopyridine (71) by treatment with $P_2O_5$ at high temperatures. In step 5, the nitrile 71 was coupled with 4-methylphenylboronic acid (56) (Scheme XXIV) using Snieckus conditions (Scheme XIII) to give 4-cyano-3-(4-methylphenyl)pyridine (72). In step 6, 72 was brominated with NBS/AIBN in carbon tetrachloride at reflux to give the desired alkylating reagent 66.

## Scheme XXVII

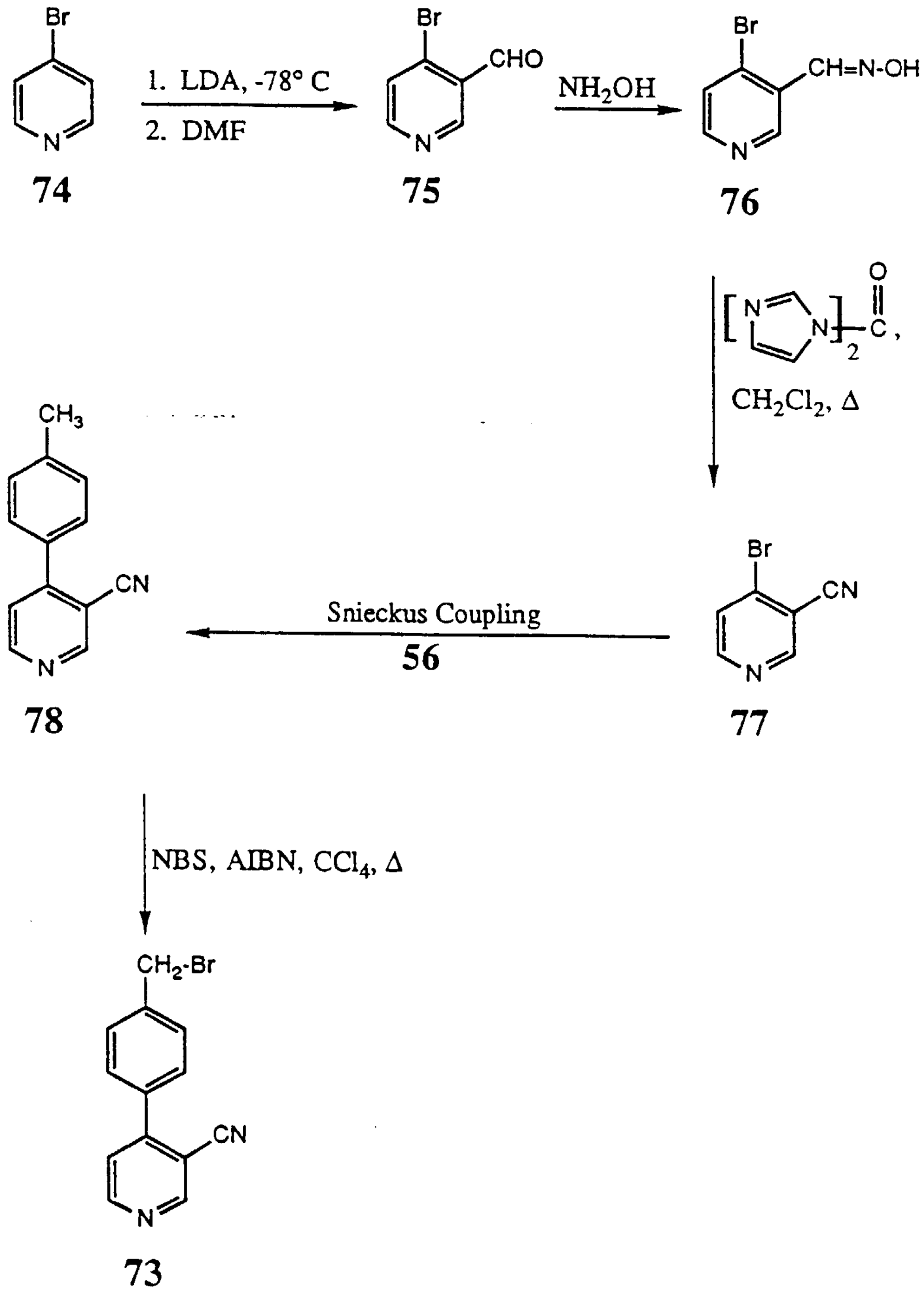


74 75 76 77 78 73

Synthetic Scheme XXVII shows the 5-step preparation of the alkylating reagent 4-(4-bromomethylphenyl)-3-cyanopyridine (73) from 4-bromopyridine (74) (Aldrich). In step 1, the ortho-bromo carbanion was generated with LDA

in THF at -78° C and reacted with anhydrous DMF to give 4-bromo-3-carboxaldehyde 75. In step 2, the aldehyde 75 was reacted with hydroxylamine to give the oxime 76. In step 3, the oxime 76 was dehydrated with 1,1'-carbonyldiimidazole in methylene chloride at reflux to give 4-bromo-3-cyanopyridine (77). In step 4, the nitrile 77 was coupled with 4-methylphenylboronic acid (56) (Scheme XXIV) using Snieckus conditions (Scheme XIII) to give 3-cyano-4-(4-methylphenyl)pridine (78). In step 5, 78 was brominated with NBS/AIBN in carbon tetrachloride at reflux to give the desired alkylating reagent 73.

## Scheme XXVIII

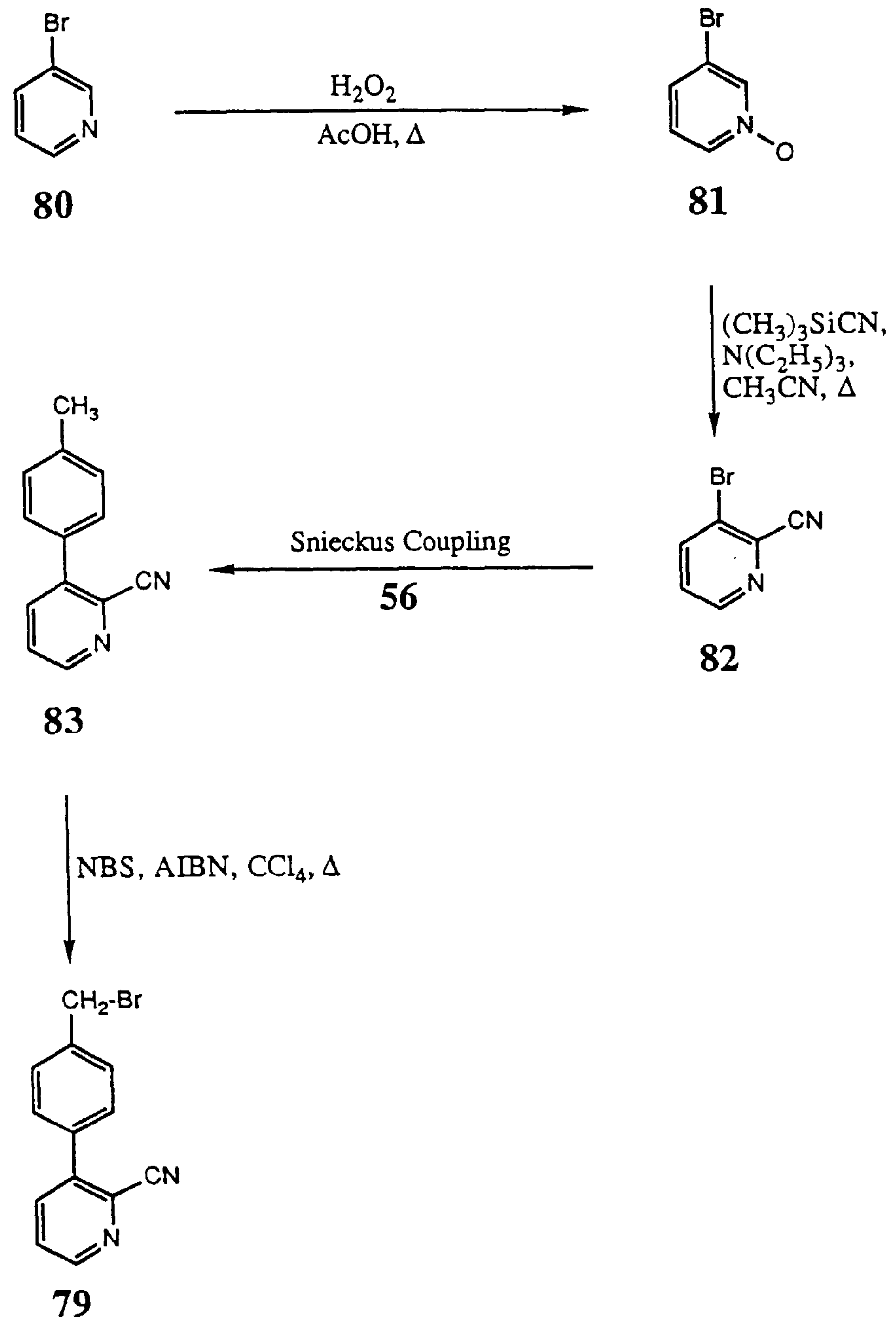


80 81 82 83 79

Synthetic Scheme XXVIII shows the 4-step preparation of the alkylating reagent 2-cyano-3-(4-bromomethylphenyl)pyridine (79) from 3-bromopyridine (80) (Aldrich). In step 1, the pyridine 80 was reacted with hydrogen peroxide in acetic acid at reflux to give the pyridine N-oxide 81. In step 2, the N-oxide 81 was converted to 3-bromo-2-cyanopyridine (82) by reaction with trimethysilylcyanide and triethyl anine in acetonitrile at reflux. In step 3, the nitrile 82 was coupled with 4-methylphenylboronic acid (56) (Scheme XXIV) using Snieckus conditions (Scheme XIII) to give 2-cyano-3-(4-methylphenyl) pyridine (83). In step 4, 83 was brominated with NBS/AIBN in carbon tetrachloride at reflux to give the desired alkylating reagent 79.

## Scheme XXIX

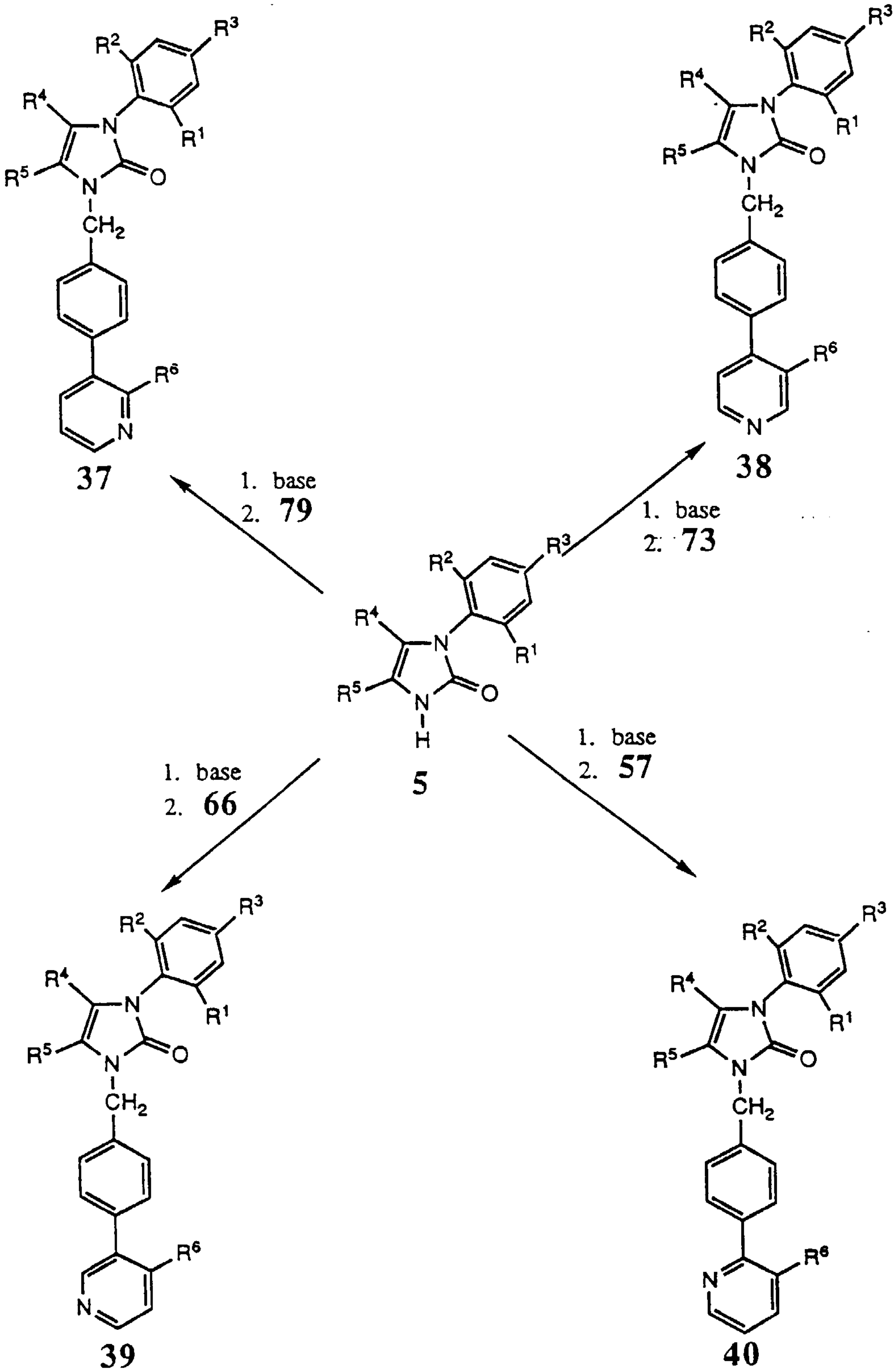


37

38

5

39

40

Synthetic Scheme XXIX shows the preparation of 1,4,5-trisubstituted-1,3-dihydro-3-[[4-(2-cyano-3-pyridinyl)phenyl]methyl]-2H-imidazol-2-ones 37 ($R^6$=CN), 1,4,5-trisubstituted-1,3-dihydro-3-[[4-(3-cyano-4-pyridinyl)phenyl]methyl]-2H-imidazol-2-ones 38 ($R^6$=CN), 1,4,5-trisubstituted-1,3-dihydro-3-[[4-(4-cyano-3-pyridinyl)phenyl]methyl]-2H-imidazol-2-ones 39 ($R^6$=CN), and 1,4,5-trisubstituted-1,3,-dihydro-3-[[4-(3-cyano-2-pyridinyl)phenyl]methyl]-2H-imidazol-2-ones 40 ($R^6$=CN) from the parent 1,4,5-trisubstituted-1,3-dihydro-2H-imidazol-2-ones 5 (prepared in Scheme IV, Scheme V, or Scheme VI). The imidazol-2-one 5 was first treated with a base, such as potassium t-butoxide, and subsequently with the alkylating reagents 79 (Scheme XXVIII), 73 (Scheme XXVII), 66 (Scheme XXVI), and 57 (Scheme XXV) to give the alkylated products 37 ($R^6$=CN), 38 ($R^6$=CN, 39 ($R^6$=CN), and 40 ($R^6$=CN), respectively.

The following Examples contain detailed descriptions of the methods of preparation of compounds of Formula I. These detailed descriptions fall within the scope of, and serve to exemplify, the above described General Synthetic Procedures which form part of the invention. These detailed descriptions are presented for illustrative purposes only and are no intended as a restriction on the scope of the invention. All parts are by weight and temperatures are in degrees Centigrade, unless otherwise indicated.

Example 1

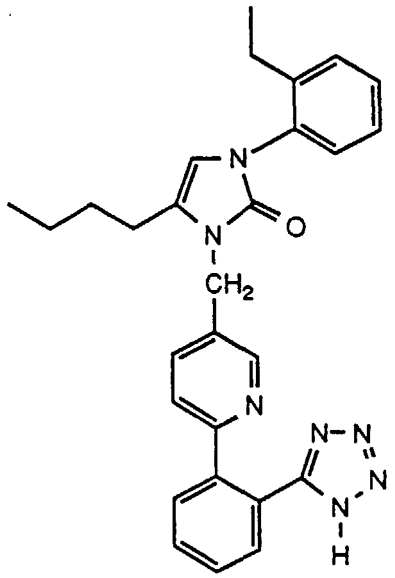

1-(2-ethylphenyl)-4-butyl-1,3-dihydro-3-[[6-[2-(1H-tetrazol-5-yl)phenyl]-3-pyridinyl]methyl]-2H-imidazol-2-one

Step 1: Preparation of 1-(2-ethylphenyl)-4-butyl-1,3-dihydro-2H-imidazol-2-one.

Following General Synthetic Schemes III and IV, 1-(2-ehtylphenyl)-4-butyl-1,3-dihydro-2H-imidazol-2-one was prepared: NMR ($CDCl_3$) δ 0.92 (t, J=7 Hz, 3H), 1.17 (t, J=7 Hz, 3H), 1.38 (m, J = 7 Hz, 2H), 1.58(m, J=7 Hz, 2H), 2.46 (t, J=7 Hz, 2H), 2.58 (q, J=7 Hz, 2H), 6.06 (s, 1H), 7.16-7.30 (m, 2H), 7.30-7.40 (m, 2H).

Step 2: Preparation of 1-(2-ethylphenyl)-4-butyl-1,3-dihydro-3-[[6-[2-(1H-tetrazol-5-yl)phenyl]-3-pyridinyl]methyl]-2H-imidazol-2-one.

Following General Synthetic Schemes XXII and XXIII, the imidazol-2-one from Step 1 was converted to 1-(2-ethylphenyl)-4-butyl-1,3-dihydro-3-[[6-[2-(1H-tetrazol-5-yl)phenyl]-3-pyridinyl]methyl]-2H-imidazol-2-one as a colorless solid: mp 151-152°C; NMR ($CDCl_3$)δ 0.91 (t, J=8 Hz, 3H), 1.17 (t, J=8 Hz, 3H), 1.33-1.43 (m, 2H), 1.50-1.60 (m, 2H), 2.41 (t, J=7 Hz, 2H), 2.60 (q, J=8 Hz, 2H), 5.07 (s,2H), 6.10 (s,1H), 7.22 (d, J=3 Hz, 2H), 7.34 (d, J= 3 Hz, 2H), 7.51 (d, J=8 Hz, 1H), 7.55-7.65 (m,3H), 8.01(dd, J=8 and 2 Hz, 1H), 8.06-8.13 (m,1H), 8.84 (br s, 1H); MS(FAB) m/e (rel intensity) 480 (33), 452 (15), 424 (5), 243 (15), 237 (18), 209 (100), 180 (88), 152 (10), 130 (17); HMRS: Calc'd for M+H:

480.2512. Found: 480.2518.

Example 2

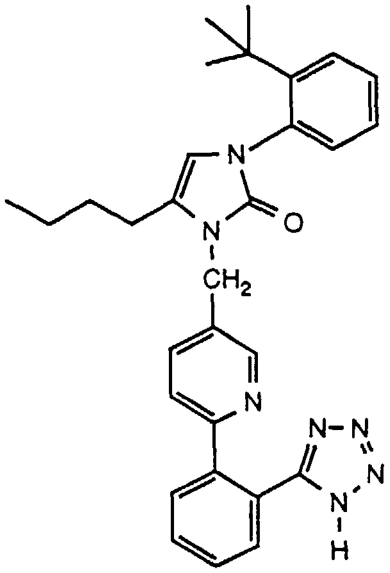

1-(2-tertbutylphenyl)-4-butyl-1,3-dihydro-3-[[6-[2-(1H-tetrazol-5-yl)phenyl]-3-pyridinyl]methyl]-2H-imidazol-2-one

Step 1: Preparation of 1-(2-tertbutylphenyl)-4-butyl-1,3-dihydro-2H-imidazol-2-one.

Following General Synthetic Schemes III and IV, 1-(2-tertbutylphenyl)-4-butyl-1,3-dihydro-2H-imidazol-2-one was prepared: NMR ($CDCl_3$) δ 0.95 (t, J=8 Hz, 3H), 1.30 (s, 9H), 1.35-1.45 (m, 2H), 1.53-1.65 (m, 2H), 2.50 (t, J=8 Hz, 2H), 6.12-6.14 (m, 1H), 7.05(dd, J=8 and 2 Hz, 1H), 7.27 (td, J=7 and 2 Hz, 1H), 7.57 (dd, J=8 and 2 Hz, 1H).

Step 2: Preparation of 1-(2-tertbutylphenyl)-4-butyl-1,3-dihydro-3-[[6-[2-(1H-tetrazol-5-yl)phenyl]-3-pyridinyl]methyl]-2H-imidazol-2-one.

Following General Synthetic Schemes XXII and XXIII, the imidazol-2-one from Step 1 was converted to 1-(2-tertbutylphenyl)-4-butyl-1,3-dihydro-3-[[6-[2-(1H-tetrazol-5-yl)phenyl]-3-pyridinyl]methyl]-2H-imidazol-2-one as a colorless solid: NMR ($CDCl_3$) δ 0.85 (t, J=7 Hz, 3H), 1.30 (s, 9H), 1.33-1.45 (m, 2H), 1.45-1.58 (m, 2H), 2.39 (t, J=7 Hz, 2H), 4.97(d, J=16 Hz, 1H), 5.06 (d, J=16 Hz, 1H), 6.07 (br s, 1H), 7.06 (dd, J=7 and 2 Hz, 1H), 7.22 (td, J=8 and 2 Hz, 1H), 7.35 (td, J=8 and 2 Hz, 1H), 7.43 (d, J=8 Hz, 1H), 7.50-7.61 (m, 4H), 7.88-7.92 (m, 1H), 8.03-8.08 (m, 1H), 8.61 (br s, 1H); MS (FAB) m/e (rel intensity) 508 (40), 480 (20), 452 (5), 237 (20), 209 (100), 180 (85), 130 (15); HMRS: Calc'd for M+H: 508.2825. Found: 508.2792.

Example 3

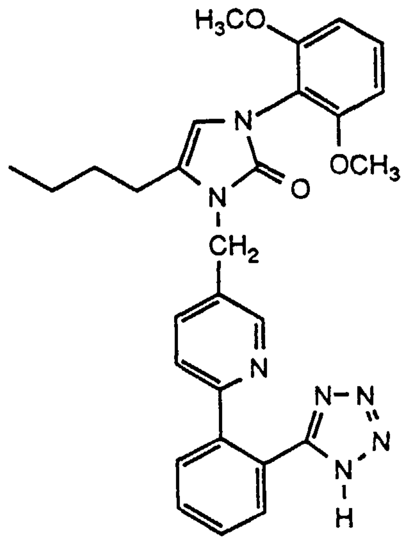

1-(2,6-dimethoxyphenyl)-4-butyl-1,3-dihydro-3-[[6-[2-(1H-tetrazol-5-yl)phenyl]-3-pyridinyl]methyl]-2H-imidazol-2-one

Step 1: Preparation of 1-(2,6-dimethoxyphenyl)-4-butyl-1,3-dihydro-2H-imidazol-2-one.

Following General Synthetic Schemes III and IV, 1-(2,6-dimethylphenyl)-4-butyl-1,3-dihydro-2H-imidazol-2-one was prepared: NMR (DMSO-$d_6$) δ 0.89 (t, J=8 Hz, 3H), 1.27-1.40 (m, 2H), 1.42-1.57 (m, 2H), 2.26 (t, J=8 Hz, 2H), 3.71 (s, 6H), 5.91 (t, J=1 Hz, 1H), 6.72 (d, J=8 Hz, 2H), 7.31 (t, J=8 Hz, 2H), 9.85 (br s, 1H).

Step 2: Preparation of 1-(2,6-dimethoxyphenyl)-4-butyl-1,3-dihydro-3-[[6-[2-(1H-tetrazol-5-yl)phenyl]-3-pyridinyl]methyl]-2H-imidazol-2-one.

Following General Synthetic Schemes XXII and XXIII, the imidazol-2-one from Step 1 was converted to 1-(2,6-dimethoxyphenyl)-4-butyl-1,3-dihydro-3-[[6-[2-(1H-tetrazol-5-yl)phenyl]-3-pyridinyl]methyl]-2H- imidazol-2-one as a colorless visious oil: NMR (DMSO-$d_6$) δ 0.82 (t, J=7 Hz, 3H), 1.21-1.44 (m, 4H), 2.26 (t, J=7 Hz, 2H), 3.73 (s, 6H), 4.86 (s, 2H), 6.10 (t, J=1 Hz, 1H), 6.76 (d, J=8 Hz, 2H), 7.35(t, J=8 Hz, 1H), 7.48 (d, J=9 Hz, 1H) 7.57-7.82 (m, 5H), 8.25 (d, J=2 Hz, 1H); MS (FAB) m/e (rel intensity) 512 (30), 484 (20), 180 (90); HRMS: Calc'd for M+H: 512.2410. Found: 512.2465.

Example 4

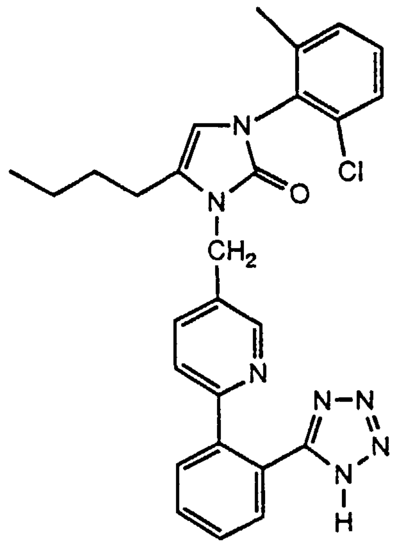

1-(2-chloro-6-methylphenyl)-4-butyl-1,3-dihydro-3-[[6-[2-(1H-tetrazol-5-yl)phenyl]-3-pyridinyl]methyl]-2H-imidazol-2-one

Step 1: Preparation of 1-(2-chloro-6-methylyphenyl)-4-butyl-1,3-dihydro-2H-imidazol-2-one.

Following General Synthetic Schemes III and IV, 1-(2-chloro-6-methylyphenyl)-4-butyl-1,3-dihydro-2H-imidazol-2-one was prepared: NMR ($CDCl_3$) δ 0.90 (t, J=7 Hz, 3H), 1.35 (m, J=7 Hz, 2H), 1.55 (m, J=7 Hz, 2H), 2.26 (s, 3H), 2.40 (t, J=7 Hz, 2H), 5.85-5.88 (m, 1H), 7.17-7.25 (m,2H), 7.33 (dd, J=7 and 2 Hz, 1H), 10.20(br s, 1H); MS (FAB) m/e (rel intensity) 265 (100), 249 (3), 221 (10), 201 (2), 187 (8); HMRS: Calc'd for M+H: 265.1108. Found: 265.1126.

Step 2: Preparation of 1-(2-chloro-6-methylyphenyl)-4-butyl-1,3-dihydro-3-[[6-[2-(1H-tetrazol-5-yl)phenyl]-3-pyridinyl]methyl]-2H-imidazol-2-one.

Following General Synthetic Schemes XXII and XXIII, the imidazol-2-one from Step 1 was converted to 1-(2-chloro-6-methylyphenyl)-4-butyl-1,3-dihydro-3-[[6-[2-(1H-tetrazol-5-yl)phenyl]-3-pyridinyl]methyl]-2H-imidazol-2-one as a colorless solid: NMR ($CDCl_3$) δ 0.86 (t, J=7 Hz, 3H), 1.34 (m, J=7 Hz, 2H), 1.49 (m, J=8 Hz, 2H), 2.24 (s, 3H), 2.31 (t, J=8 Hz, 2H), 5.05 (d, J=13 Hz, 1H), 5.15 (d, J=13 Hz, 1H), 5.98-601 (m, 1H), 7.14 (d, J=8 Hz, 1H), 7.18-7.25 (m, 2H), 7.33 (dd, J=7 and 2 Hz, 1H), 7,41-7.48 (m, 3H), 7.61 (dd, J=8 and 2 Hz, 1H), 7.82-7.87 (m, 1H), 8.44 (d, J=2 Hz, 1H); MS (FAB) m/e (rel intensity) 500 (55), 472 (15), 457 (5), 237 (15), 209 (100), 194 (36), 180 (96); HMRS: Calc'd for M+H: 500.1966. Found: 500.2028.

Example 5

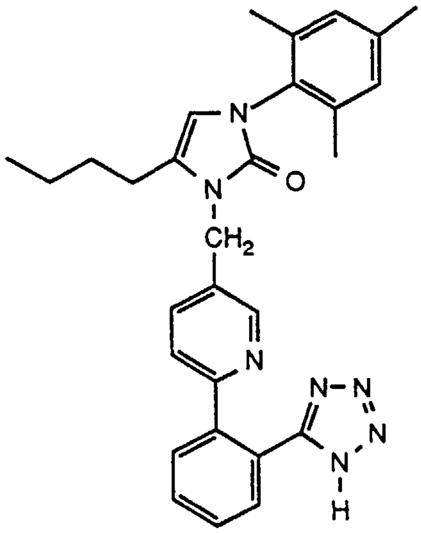

1-(2,4,6-trimethylphenyl)-4-butyl-1,3-dihydro-3-[[6-[2-(1H-tetrazol-5-yl)phenyl]-3-pyridinyl]methyl]-2H-imidazol-2-one

Step 1: Preparation of 1-(2,4,6-trimethyphenyl)-4-butyl-1,3-dihydro-2H-imidazol-2-one.

Following General Synthetic Schemes III and IV, 1-(2,4,6-trimethylphenyl)-4-butyl-1, 3-dihydro-2H-imidazol-2-one was prepared: NMR ($CDCl_3$) δ 0.91 (t, J=8 Hz, 3H), 1.28-1.42 (m, 2H), 1.47-1.60 (m, 2H), 2.14 (s, 6H), 2.30 (s, 3H), 2,40 (t, J=8 Hz, 2H), 5.80-5.83 (m, 1H), 6.93 (s, 2H), 10.20 (br s, 1H).

Step 2: Preparation of 1-(2,4,6-trimethylphenyl)-4-butyl-1,3-dihydro-3-[[6-[2-(1H-tetrazol-5-yl)phenyl]-3-pyridinyl]methyl]-2H-imidazol-2-one.

Following General Synthetic Schemes XXII and XXIII, the imidazol-2-one from Step 1 was converted to 1-(2,4,6-trimethylphenyl)-4-butyl-1,3-dihydro-3-[[6-[2-(1H-tetrazol-5-yl)phenyl]-3-pyridinyl]methyl]-2H-imidazol-2-one as a colorless solid: NMR ($CDCl_3$) δ 0.85 (t, J=8 Hz, 3H), 1.24-1.38 (m, 2H), 1.39-1.52 (m, 2H), 2.05 (s, 6H), 2.22-2.31 (m, 2H), 2.26 (s, 3H), 5.02 (s, 2H), 5.90 (s, 1H), 6.82 (s, 2H), 7.16 (d, J=8, 1H), 7.46-7.55 (m, 3H), 7.60 (dd, J=8 and 2 Hz, 1H), 7.82-7.89 (m, 1H), 8.47 (d, J=2 Hz, 1H); MS (FAB) m/e (rel intensity) 494 (83), 466 (22), 259 (25), 237 (37), 209 (100), 180 (79); HMRS: Calc'd for M+H: 494.2668. Found: 494.2615.

Example 6

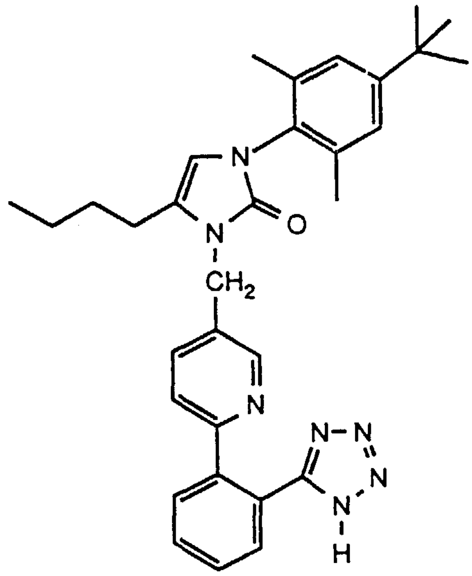

1-(2,6-dimethyl-4-tertbutylphenyl)-4-butyl-1,3-dihydro-3-[[6-[2-(1H-tetrazol-5-yl)phenyl]-3-pyridinyl]methyl]-2H-imidazol-2-one

Step 1: Preparation of 1-(2,6-dimethyl-4-tertbutylphenyl)-4-butyl-1,3-dihydro-2H-imidazol-2-one.

Following General Synthetic Schemes III and IV, 1-(2,6-dimethyl-4-tertbutylphenyl)-4-butyl-1, 3-dihydro-2H-imidazol-2-one was prepared: NMR ($CDCl_3$) δ 0.90 (t, J=8 Hz, 3H), 1.26-1.42 (m, 2H), 1.30 (s, 9H), 1.48-1.60 (m, 2H), 2.18 (s, 6H), 2.40 (t, J=8 Hz, 2H), 5.83 (s, 1H), 7.11 (s, 2H), 10.30 (br s, 1H).

Step 2: Preparation of 1-(2,6-dimethyl-4-tertbutylphenyl)-4-butyl-1,3-dihydro-3-[[6-[2-(1H-tetrazol-5-yl)phenyl]-3-pyridinyl]methyl]-2H-imidazol-2-one.

Following General Synthetic Schemes XXII and XXIII, the imidazol-2-one from Step 1 was converted to 1-(2,6-dimethyl-4-tertbutylphenyl)-4-butyl-1,3-dihydro-3-[[6-[2-(1H-tetrazol-5-yl)phenyl]-3-pyridinyl]methyl]-2H-imidazol-2-one as a colorless solid: NMR ($CDCl_3$) δ 0.87 (t, J=8 Hz, 3H), 1.25-1.41 (m, 2H), 1.32 (s, 9H), 1.43-1.55 (m, 2H), 2.15 (s, 6H), 2.32 (t, J=8, 2H), 5.09 (s, 2H), 5.96 (s, 1H), 7.13 (s, 2H), 7.19 (d, J=8 Hz, 1H) 7.45-7.54 (m, 3H), 7.63 (dd, J=8 and 2 Hz, 1H), 7.90-7.97 (m, 1H), 8.49 (d, J=2 Hz, 1H); MS (FAB) m/e (rel intensity) 536 (36), 508 (18), 301 (19), 243 (26), 208 (100), 180 (90); HMRS: Calc'd for M+H: 536.3138. Found: 536.3098.

Example 7

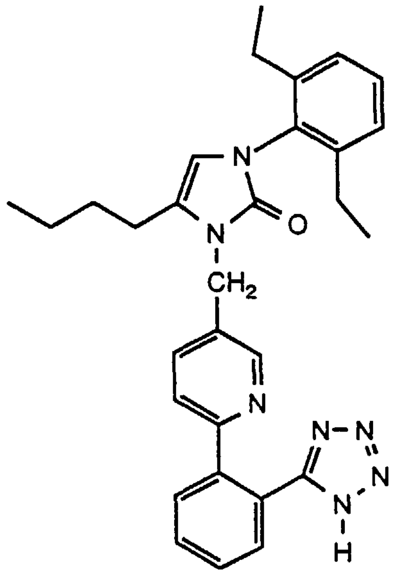

1-(2,6-diethylphenyl)-4-butyl-1,3-dihydro-3-[[6-[2-(1H-tetrazol-5-yl)phenyl]-3-pyridinyl]methyl]-2H-imidazol-2-one

Step 1: Preparation of 1-(2,6-diethylphenyl)-4-butyl-1,3-dihydro-2H-imidazol-2-one.

Following General Synthetic Schemes III and IV, 1-(2,6-diethylphenyl)-4-butyl-1, 3-dihydro-2H-imidazol-2-one was prepared: NMR ($CDCl_3$) δ 0.89 (t, J=7 Hz, 3H), 1.17 (t, J=7 Hz, 6H), 1.33 (m, J=7 Hz, 2H), 1.53 (m, J=8 Hz, 2H), 2.40 (t, J=7 Hz, 2H), 2.50 (q, J=8 Hz, 2H), 2.52 (q, J=8 Hz, 2H), 5.82-5.84 (m, 1H), 7.16 (d, J=8 Hz, 2H), 7.26-7.33 (m, 1H), 10.71 (br s, 1H); MS (FAB) m/e (rel intensity) 273 (100), 255 (15); HMRS: Calc'd for M+H: 273.1967 Found: 273.1980.

Step 2: Preparation of 1-(2,6-diethylphenyl)-4-butyl-1,3-dihydro-3-[[6-[2-(1H-tetrazol-5-yl)phenyl]-3-pyridinyl]methyl]-2H-imidazol-2-one.

Following General Synthetic Schemes XXII and XXIII, the imidazol-2-one from Step 1 was converted to 1-(2,6-diethylphenyl)-4-butyl-1,3-dihydro-3-[[6-[2-(1H-tetrazol-5-yl)phenyl]-3-pyridinyl]methyl]-2H-imidazol-2-one as a colorless solid: NMR ($CDCl_3$) δ 0.86 (t, J=7 Hz, 3H), 1.12 (t, J=8 Hz, 6H), 1.33 (m, J=8 Hz, 2H), 1.48 (m, J=8 Hz, 2H), 2.33 (t, J=7 Hz, 2H), 2.37-2.54 (m, 4H), 5.08 (s, 2H), 5.95 (s, 1H), 7.12 (d, J=8 Hz, 2H), 7.24-7.31 (m, 1H), 7.43-7.49 (m, 3H), 7.63 (dd, J=6 and 2 Hz, 1H), 7.80-7.87 (m, 1H), 8.44-8.48 (m, 1H); MS (FAB) m/e (rel intensity) 508 (53), 480 (27), 465 (7), 237 (17), 209 (100), 194 (26), 180 (60); HRMS: Calc'd for M+H: 508.2825. Found: 508.2833.

Example 8

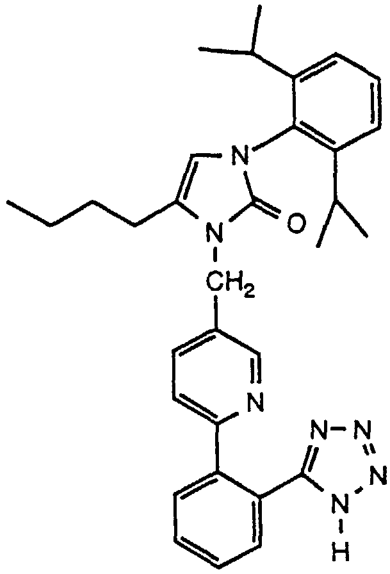

1-(2,6-diisopropylphenyl)-4-butyl-1,3-dihydro-3-[[6-[2-(1H-tetrazol-5-yl)phenyl]-3-pyridinyl]methyl]-2H-imidazol-2-one

Step 1: Preparation of 1-(2,6-diisopropylphenyl)-4-butyl-1,3-dihydro-2H-imidazol-2-one.

Following General Synthetic Schemes III and IV, 1-(2,6-diisopropylphenyl)-4-butyl-1, 3-dihydro-2H-imidazol-2-one was prepared: NMR ($CDCl_3$) δ 0.92 (t, J=8 Hz, 3H), 1.16 (d, J=8 Hz, 6H), 1.23 (d, J=8 Hz, 6H), 1.29-1.44 (m, 2H), 1.50-1.61 (m, 2H), 2.41 (t, J=8 Hz, 2H), 2.75-2.91 (m, 2H), 5.85 (t, J=1 Hz, 1H), 7.22 (d, J=8 Hz, 2H), 7.36 (t, J=8 Hz, 1H).

Step 2: Preparation of 1-(2,6-diisopropylphenyl)-4-butyl-1,3-dihydro-3-[[6-[2-(1H-tetrazol-5-yl)phenyl]-3-pyridinyl]methyl]-2H-imidazol-2-one.

Following General Synthetic Schemes XXII and XXIII, the imidazol-2-one from Step 1 was converted to 1-(2,6-diisopropylphenyl)-4-butyl-1,3-dihydro-3-[[6-[2-(1H-tetrazol-5-yl)phenyl]-3-pyridinyl]methyl]-2H- imidazol-2-one as a colorless solid: NMR ($CDCl_3$) δ 0.87 (t, J=7 Hz, 3H), 1.19 (d, J=7 Hz, 6H), 1.21 (t, J=7 Hz, 3H), 1.28-1.42 (m, 2H), 1.44-1.56 (m, 2H), 2.35 (t, J=8 Hz, 2H), 2.69-2.84 (m, 2H), 5.08 (s, 2H), 5.96 (s, 1H), 7.20-7.29 (m, 3H), 7.34-7.53 (m, 4H), 7.69 (dd, J=8 and 2 Hz, 1H), 7.92-7.97 (m, 1H), 8.49 (d, J=2 Hz, 1H); MS (FAB) m/e (rel intensity) 536 (28), 237 (22), 209 (23); HRMS: Calc'd for M+H: 536.3138 Found: 536.3102.

## BIOLOGICAL EVALUATION

Assay A: Angiotensin II Binding Activity

Compounds of the invention were tested for ability to bind to the smooth muscle angiotensin II receptor using a rat uterine membrane preparation. Angiotensin II (AII) was purchased from Peninsula Labs. $^{125}$I-angiotensin II (specific activity of 2200 Ci/mmol) was purchased from Du Pont-New England Nuclear. Other chemicals were obtained from Sigma Chemical Co. This assay was carried out according to the method of Douglas et al [Endocrinology, 106, 120-124 (1980)]. Rat uterine membranes were prepared from fresh tissue. All procedures were carried out at 4°C. Uteri were stripped of fat and homogenized in phosphate-buffered saline at pH 7.4 containing 5 mM EDTA. The homogenate was centrifuged at 1500 x g for 20 min., and the supernatant was recentrifuged at 100,000 x g for 60 min. The pellet was resuspended in buffer consisting of 2 mM EDTA and 50 mM Tris-HCl (pH 7.5) to a final protein concentration of 4 mg/ml. Assay tubes were charged with 0.25 ml of a solution containing 5 mM $MgCl_2$, 2 mM EDTA, 0.5% bovine serum albumin,

50 mM Tris-HCl, pH 7.5 and $^{125}$I-AII (approximately $10^5$ cpm) in the absence or in the presence of unlabelled ligand. The reaction was initiated by the addition of membrane protein and the mixture was incubated at 25°C for 60 min. The incubation was terminated with ice-cold 50 mM Tris-HCl (pH 7.5) and the mixture was filtered to separate membrane-bound labelled peptide from the free ligand. The incubation tube and filter were washed with ice-cold buffer. Filters were assayed for radioactivity in a Micromedic gamma counter. Nonspecific binding was defined as binding in the presence of 10 µM of unlabelled AII. Specific binding was calculated as total binding minus nonspecific binding. The receptor binding affinity of an AII antagonist compound was indicated by the concentration ($IC_{50}$) of the tested AII antagonist which gives 50% displacement of the total specifically bound $^{125}$I-AII from the high affinity AII receptor. Binding data were analyzed by a nonlinear least-squares curve fitting program. Results are reported in Table I.

Assay B: In Vitro Vascular Smooth Muscle-Response for AII

The compounds of the invention were tested for antagonist activity in rabbit aortic rings. Male New Zealand white rabbits (2-2.5 kg) were sacrificed using an overdose of pentobarbital and exsanguinated via the carotid arteries. The thoracic aorta was removed, cleaned of adherent fat and connective tissue and then cut into 3-mm ring segments. The endothelium was removed from the rings by gently sliding a rolled-up piece of filter paper into the vessel lumen. The rings were then mounted in a water-jacketed tissue bath, maintained at 37°C, between moveable and fixed ends of a stainless steel wire with the moveable end attached to an FT03 Grass transducer coupled to a Model 7D Grass Polygraph for recording isometric force responses. The bath was filled with 20 ml of oxygenated (95% oxygen/5% carbon dioxide) Krebs solution of the following composition (mM): 130 NaCl, 15 $NaHC0_3$, 15 KCl, 1.2 $NaH_2P0_4$, 1.2 $MgS0_4$, 2.5 $CaC1_2$, and 11.4 glucose. The preparations were equilibrated for one hour before approximately one gram of passive tension was placed on the rings. Angiotensin II concentration-response curves were then recorded (3 X $10^{-10}$ to 1 x $10^{-5}$ M). Each concentration of AII was allowed to elicit its maximal contraction, and then AII was washed out repeatedly for 30 minutes before rechallenging with a higher concentration of AII. Aorta rings were exposed to the test antagonist at $10^{-5}$ M for 5 minutes before challenging with AII. Adjacent segments of the same aorta ring were used for all concentration-response curves in the presence or absence of the test antagonist. The effectiveness of the test compound was expressed in terms of $pA_2$ values and were calculated according to H.O. Schild [Br. J. Pharmacol. Chemother., 2,189-206 (1947)]. The $pA_2$ value is the concentration of the antagonist which increases the $EC_{50}$ value for AII by a factor of two. Each test antagonist was evaluated in aorta rings from two rabbits. Results are reported in Table I.

Assay C: In Vivo Intragastric Pressor Assay Response for All Antagonists

Male Sprague-Dawley rats weighing 225-300 grams were anesthetized with methohexital (30 mg/kg, i.p.) and catheters were implanted into the femoral artery and vein. The catheters were tunneled subcutaneously to exit dorsally, posterior to the head and between the scapulae. The catheters were filled with heparin (1000 units/ml of saline). The rats were returned to their cage and allowed regular rat chow and water ad libitum. After full recovery from surgery (3-4 days), rats were placed in Lucite holders and the arterial line was connected to a pressure transducer. Arterial pressure was recorded on a Gould polygraph (mmHg). Angiotensin II was administered as a 30 ng/kg bolus via the venous catheter delivered in a 50 µl volume with a 0.2 ml saline flush. The pressor response in mm Hg was measured by the difference from pre-injection arterial pressure to the maximum pressure achieved. The AII injection was repeated every 10 minutes until three consecutive injections yielded responses within 4 mmHg of each other. These three responses were then averaged and represented the control response to AII. The test compound was suspended in 0.5% methylcellulose in water and was administered by gavage. The volume administered was 2 ml/kg body weight. The standard dose was 3 mg/kg. Angiotensin II bolus injections were given at 30, 45, 60, 75, 120, 150, and 180 minutes after gavage. The pressor response to AII was measured at each time point. The rats were then returned to their cage for future testing. A minimum of 3 days was allowed between tests. Percent inhibition was calculated for each time point following gavage by the following formula: [(Control Response - Response at time point)/Control Response] X 100 . Results are shown in Table I.

TABLE I

| In Vitro and In Vivo Angiotensin II Activity of Compounds of the Invention | | | | |
|---|---|---|---|---|
| Test Compound Example # | [1]Assay A $IC_{50}$ (nM) | [2]Assay B $pA_2$ | [3]Assay C Dose: 3 mg/kg (i.g.) | |
| | | | Inhibition (%) | Duration (min.) |
| 1 | 4.5 | 9.17/8.25 | 80 | >180 |
| 2 | 19 | NT | 40 | >180 |
| 3 | 2.6 | 8.23/8.69 | 25 | >180 |
| 4 | 3.6 | NT | 75 | >180 |
| 5 | 4.4 | 8.59/8.89 | 70 | >180 |
| 6 | 84 | 8.51/8.78 | NT | - |
| 7 | 5.0 | 8.49/9.00 | 20 | - |
| 8 | 34 | 7.14/7.07 | NT | - |

[1]Assay A: Angiotensin II Binding Activity
[2]Assay B: In Vitro Vascular Smooth Muscle Response
[3]Assay C: In Vivo Pressor Response (all test compounds administered intragastrically at 3 mg/kg).
*NT= Not Tested

Also embraced within this invention is a class of pharmaceutical compositions comprising one or more compounds of Formula I in association with one or more non-toxic, pharmaceutically acceptable carriers and/or diluents and/or adjuvants (collectively referred to herein as "carrier" materials) and, if desired, other active ingredients. The compounds of the present invention may be administered by any suitable route, preferably in the form of a pharmaceutical composition adapted to such a route, and in a dose effective for the treatment intended. Therapeutically effective doses of the compounds of the present invention required to prevent or arrest the progress of the medical condition are readily ascertained by one of ordinary skill in the art. The compounds and composition may, for example, be administered intravascularly, intraperitoneally, subcutaneously, intramuscularly or topically.

For oral administration, the pharmaceutical composition may be in the form of, for example, a tablet, capsule, suspension or liquid. The pharmaceutical composition is preferably made in the form of a dosage unit containing a particular amount of the active ingredient. Examples of such dosage units are tablets or capsules. These may with advantage contain an amount of active ingredient from about 1 to 250 mg, preferably from about 25 to 150 mg. A suitable daily dose for a mammal may vary widely depending on the condition of the patient and other factors. However, a dose of from about 0.1 to 3000 mg/kg body weight, particularly from about 1 to 100 mg/kg body weight, may be appropriate.

The active ingredient may also be administered by injection as a composition wherein, for example, saline, dextrose or water may be used as a suitable carrier. A suitable daily dose is from about 0.1 to 100 mg/kg body weight injected per day in multiple doses depending on the disease being treated. A preferred daily dose would be from about 1 to 30 mg/kg body weight. Compounds indicated for prophylactic therapy will preferably be administered in a daily dose generally in a range from about 0.1 mg to about 100 mg per kilogram of body weight per day. A more preferred dosage will be a range from about 1 mg to about 100 mg per kilogram of body weight. Most preferred is a dosage in a range from about 1 to about 50 mg per kilogram of body weight per day. A suitable dose can be administered, in multiple sub-doses per day. These sub-doses may be administered in unit dosage forms. Typically, a dose or sub-dose may contain from about 1 mg to about 100 mg of active compound per unit dosage form. A more preferred dosage will contain from about 2 mg to about 50 mg of active compound per unit dosage form. Most preferred is a dosage form containing from about 3 mg to about 25 mg of active compound per unit dose.

The dosage regimen for treating a disease condition with the compounds and/or compositions of this invention is selected in accordance with a variety of factors, including the type, age, weight, sex and medical condition of the patient, the severity of the disease, the route of administration, and the particular compound employed, and thus may vary widely.

For therapeutic purposes, the compounds of this invention are ordinarily combined with one or more adjuvants appropriate to the indicated route of administration. If administered per os, the compounds may be admixed with lactose, sucrose, starch powder, cellulose esters of alkanoic acids, cellulose alkyl esters, talc, stearic acid, magnesium stearate, magnesium oxide, sodium and calcium salts of phosphoric and sulfuric acids, gelatin, acacia gum, sodium

alginate, polyvinylpyrrolidone, and/or polyvinyl alcohol, and then tableted or encapsulated for convenient administration. Such capsules or tablets may contain a controlled-release formulation as may be provided in a dispersion of active compound in hydroxypropylmethyl cellulose. Formulations for parenteral administration may be in the form of aqueous or non-aqueous isotonic sterile injection solutions or suspensions. These solutions and suspensions may be prepared from sterile powders or granules having one or more of the carriers or diluents mentioned for use in the formulations for oral administration. The compounds may be dissolved in water, polyethylene glycol, propylene glycol, ethanol, corn oil, cottonseed oil, peanut oil, sesame oil, benzyl alcohol, sodium chloride, and/or various buffers. Other adjuvants and modes of administration, are well and widely known in the pharmaceutical art.

## Claims

**1.** A compound of Formula I

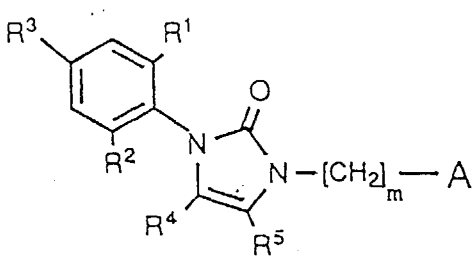

wherein A is selected from

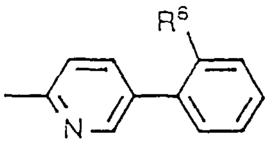 , 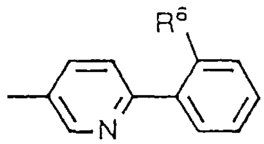 , 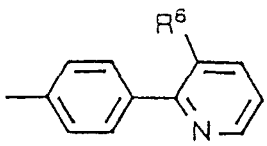

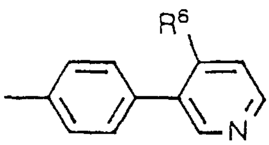 , 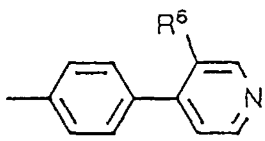 and 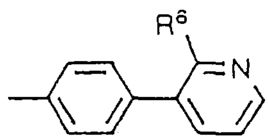 ;

wherein m is a number selected from one to four, inclusive;

wherein each of $R^1$, $R^2$ and $R^3$ is independently selected from alkyl, alkoxy, cyano, halo, hydroxy, nitro, amino, carboxyl, alkoxycarbonyl, formyl, alkylcarbonyl and haloalkylcarbonyl; wherein further $R^3$ may be hydrogen; wherein further when $R^1$ is ethyl or tertbutyl, then $R^2$ can be hydrogen; with the proviso that $R^1$ and $R^2$ cannot be fluoro simultaneously; and with the further proviso that $R^1$ and $R^2$ cannot be chloro simultaneously; wherein $R^4$ is selected from hydrogen, alkyl, halo, haloalkyl, formyl, carboxyl and alkoxyalkyl; wherein $R^5$ is selected from alkylphenyl, phenylalkyl, cycloalkyl and cycloalkylalkyl; wherein $R^6$ is an acidic group selected from COOH and

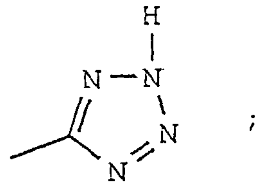

or a stereoisomer or a tautomer thereof or a pharmaceutically-acceptable salt thereof.

**2.** Compound of Claim 1 of Formula II

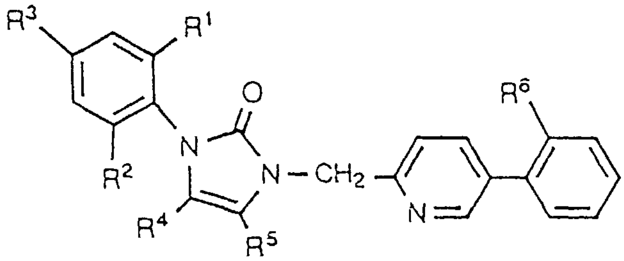

(II)

wherein each of $R^1$, $R^2$ and $R^3$ is independently selected from methyl, ethyl, n-propyl, isopropyl, tert-butyl, methoxy, ethoxy, propoxy, isopropoxy, tert-butoxy, cyano, fluoro, chloro, iodo, bromo, hydroxy, nitro, amino, N-methylamino, N,N-dimethylamino, N-ethylamino, N,N-diethylamino, carboxyl, methoxycarbonyl, ethoxycarbonyl, formyl, methylcarbonyl, ethylcarbonyl and trifluoromethylcarbonyl; wherein further $R^3$ may be hydrogen; wherein further when $R^1$ is ethyl or tertbutyl, then $R^2$ can be hydrogen; with the proviso that $R^1$ and $R^2$ cannot be fluoro simultaneously; and with the further proviso that $R^1$ and $R^2$ cannot be chloro simultaneously; wherein $R^4$ is selected from hydrogen, methyl, fluoro, chloro, monofluoromethyl, difluoromethyl, trifluoromethyl, formyl, carboxyl and dimethoxymethyl; wherein $R^5$ is selected from methyl, ethyl, n-propyl, isopropyl, n-butyl, sec-butyl, isobutyl, tert-butyl, n-pentyl, isopentyl, neopentyl, phenyl, benzyl, phenethyl, cyclopropyl, cyclobutyl, cyclopentyl, cyclohexyl, cyclopropylmethyl, cyclopropylethyl, cyclobutylmethyl, cyclobutylethyl, cyclopentylmethyl, cyclopentylethyl, cyclohexylmethyl and cyclohexylethyl; wherein $R^6$ is an acidic group selected from COOH and

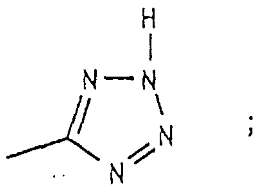

or a stereoisomer or a tautomer thereof or a pharmaceutically-acceptable salt thereof.

**3.** Compound of Claim 1 of Formula III

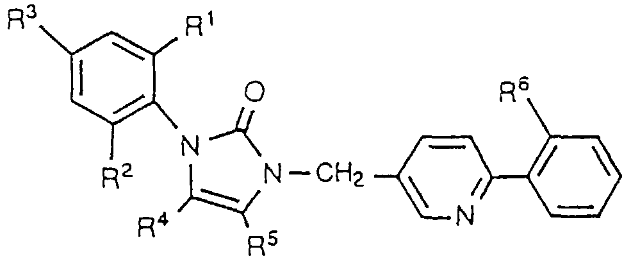

(III)

wherein each of $R^1$, $R^2$ and $R^3$ is independently selected from methyl, ethyl, n-propyl, isopropyl, tert-butyl, methoxy, ethoxy, propoxy, isopropoxy, tert-butoxy, cyano, fluoro, chloro, iodo, bromo, hydroxy, nitro, amino, N-methylamino, N,N-dimethylamino, N-ethylamino, N,N-diethylamino, carboxyl, methoxycarbonyl, ethoxycarbonyl, formyl, methylcarbonyl, ethylcarbonyl and trifluoromethylcarbonyl; wherein further $R^3$ may be hydrogen; wherein further when $R^1$ is ethyl or tertbutyl, then $R^2$ can be hydrogen; with the proviso that $R^1$ and $R^2$ cannot, be fluoro simultaneously; and with the further proviso that $R^1$ and $R^2$ cannot be chloro simultaneously; wherein $R^4$ is selected from hydrogen, methyl, fluoro, chloro, monofluoromethyl, difluoromethyl, trifluoromethyl, formyl, carboxyl and dimethoxymethyl; wherein $R^5$ is selected from methyl, ethyl, n-propyl, isopropyl, n-butyl, sec-butyl, isobutyl, tert-butyl, n-pentyl, isopentyl, neopentyl, phenyl, benzyl, phenethyl, cyclopropyl, cyclobutyl, cyclopentyl, cyclohexyl, cyclopropylmethyl, cyclopropylethyl, cyclobutylmethyl, cyclobutylethyl, cyclopentylmethyl, cyclopentylethyl, cyclohexylmethyl and cyclohexylethyl; wherein $R^6$ is an acidic group selected from COOH and

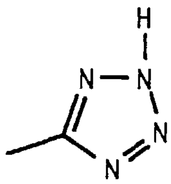 ;

or a stereoisomer or a tautomer thereof or a pharmaceutically-acceptable salt thereof.

**4.** Compound of Claim 3 which is 1-(2-ethylphenyl)-4-butyl-1,3-dihydro-3-[[6-[2-(1H-tetrazol-5-yl)phenyl]-3-pyridinyl]methyl]-2H-imidazol-2-one or a pharmaceutically-acceptable salt thereof.

**5.** Compound of Claim 3 which is 1-(2-tertbutylphenyl)-4-butyl-1,3-dihydro-3-[[6-[2-(1H-tetrazol-5-yl)phenyl]-3-pyridinyl]methyl]-2H-imidazol-2-one or a pharmaceutically-acceptable salt thereof.

**6.** Compound of Claim 3 which is 1-(2,6-dimethoxyphenyl)-4-butyl-1,3-dihydro-3-[[6-[2-(1H-tetrazol-5-yl)phenyl]-3-pyridinyl]methyl]-2H-imidazol-2-one or a pharmaceutically-acceptable salt thereof.

**7.** Compound of Claim 3 which is 1-(2-chloro-6-methylyphenyl)-4-butyl-1,3-dihydro-3-[[6-[2-(1H-tetrazol-5-yl)phenyl]-3-pyridinyl]methyl]-2H-imidazol-2-one or a pharmaceutically-acceptable salt thereof.

**8.** Compound of Claim 3 which is 1-(2,4,6-trimethylphenyl)-4-butyl-1,3-dihydro-3-[[6-[2-(1H-tetrazol-5-yl)phenyl]-3-pyridinyl]methyl]-2H-imidazol-2-one or a pharmaceutically-acceptable salt thereof.

**9.** Compound of Claim 3 which is 1-(2,6-dimethyl-4-tertbutylphenyl)-4-butyl-1,3-dihydro-3-[[6-[2-(1H-tetrazol-5-yl)phenyl]-3-pyridinyl]methyl]-2H-imidazol-2-one or a pharmaceutically-acceptable salt thereof.

**10.** Compound of Claim 3 which is 1-(2,6-diethylphenyl)-4-butyl-1,3-dihydro-3-[[6-[2-(1H-tetrazol-5-yl)phenyl]-3-pyridinyl]methyl]-2H-imidazol-2-one or a pharmaceutically-acceptable salt thereof.

**11.** Compound of Claim 3 which is 1-(2,6-diisopropylphenyl)-4-butyl-1,3-dihydro-3-[[6-[2-(1H-tetrazol-5-yl)phenyl]-3-pyridinyl]methyl]-2H-imidazol-2-one or a pharmaceutically-acceptable salt thereof.

**12.** Compound of Claim 3 of Formula IV

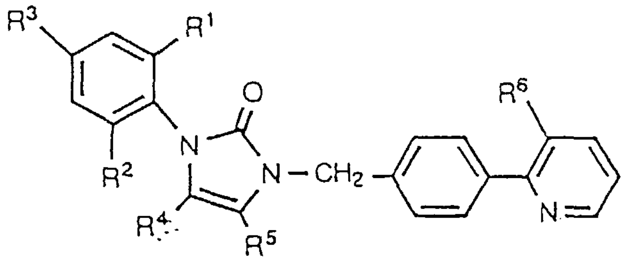

(IV)

wherein each of $R^1$, $R^2$ and $R^3$ is independently selected from methyl, ethyl, n-propyl, isopropyl, tert-butyl, methoxy, ethoxy, propoxy, isopropoxy, tert-butoxy, cyano, fluoro, chloro, iodo, bromo, hydroxy, nitro, amino, N-methylamino, N,N-dimethylamino, N-ethylamino, N,N-diethylamino, carboxyl, methoxycarbonyl, ethoxycarbonyl, formyl, methylcarbonyl, ethylcarbonyl and trifluoromethylcarbonyl; wherein further $R^3$ may be hydrogen; wherein further when $R^1$ is ethyl or tertbutyl, then $R^2$ can be hydrogen; with the proviso that $R^1$ and $R^2$ cannot be fluoro simultaneously; and with the further proviso that $R^1$ and $R^2$ cannot be chloro simultaneously; wherein $R^4$ is selected from hydrogen, methyl, fluoro, chloro, monofluoromethyl, difluoromethyl, trifluoromethyl, formyl, carboxyl and dimethoxymethyl; wherein $R^5$ is selected from methyl, ethyl, n-propyl, isopropyl, n-butyl, sec-butyl, isobutyl, tert-butyl, n-pentyl, isopentyl, neopentyl, phenyl, benzyl, phenethyl, cyclopropyl, cyclobutyl, cyclopentyl, cyclohexyl, cyclopropylmethyl, cyclopropylethyl, cyclobutylmethyl, cyclobutylethyl, cyclopentylmethyl, cyclopentylethyl, cyclohexylmethyl and cyclohexylethyl; wherein $R^6$ is an acidic group selected from COOH and

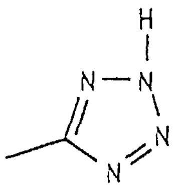

;

or a stereoisomer or a tautomer thereof or a pharmaceutically-acceptable salt thereof.

**13.** Compound of Claim 1 of Formula V

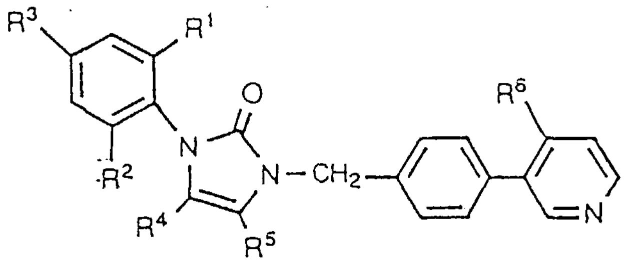

(V)

wherein each of $R^1$, $R^2$ and $R^3$ is independently selected from methyl, ethyl, n-propyl, isopropyl, tert-butyl, methoxy, ethoxy, propoxy, isopropoxy, tert-butoxy, cyano, fluoro, chloro, iodo, bromo, hydroxy, nitro, amino, N-methylamino, N,N-dimethylamino, N-ethylamino, N,N-diethylamino, carboxyl, methoxycarbonyl, ethoxycarbonyl, formyl, methylcarbonyl, ethylcarbonyl and trifluoromethylcarbonyl; wherein further $R^3$ may be hydrogen; wherein further when $R^1$ is ethyl or tertbutyl, then $R^2$ can be hydrogen; with the proviso that $R^1$ and $R^2$ cannot be fluoro simultaneously; and with the further proviso that $R^1$ and $R^2$ cannot be chloro simultaneously; wherein $R^4$ is selected from hydrogen, methyl, fluoro, chloro, monofluoromethyl, difluoromethyl, trifluoromethyl, formyl, carboxyl and dimethoxymethyl; wherein $R^5$ is selected from methyl, ethyl, n-propyl, isopropyl, n-butyl, sec-butyl, isobutyl, tert-butyl, n-pentyl, isopentyl, neopentyl, phenyl, benzyl, phenethyl, cyclopropyl, cyclobutyl, cyclopentyl, cyclohexyl, cyclopropylmethyl, cyclopropylethyl, cyclobutylmethyl, cyclobutylethyl, cyclopentylmethyl, cyclopentylethyl, cyclohexylmethyl and

cyclohexylethyl; wherein $R^6$ is an acidic group selected from COOH and

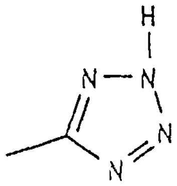

;

or a stereoisomer or a tautomer thereof or a pharmaceutically-acceptable salt thereof.

**14.** Compound of Claim 1 of Formula VI

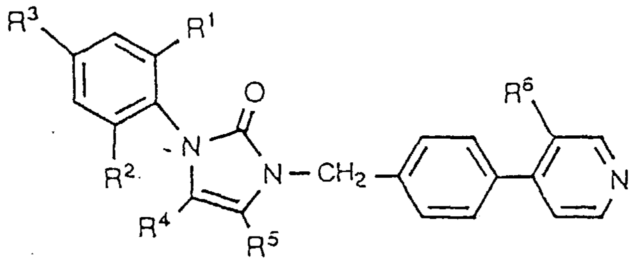

(VI)

wherein each of $R^1$, $R^2$ and $R^3$ is independently selected from methyl, ethyl, n-propyl, isopropyl, tert-butyl, methoxy, ethoxy, propoxy, isopropoxy, tert-butoxy, cyano, fluoro, chloro, iodo, bromo, hydroxy, nitro, amino, N-methylamino, N,N-dimethylamino, N-ethylamino, N,N-diethylamino, carboxyl, methoxycarbonyl, ethoxycarbonyl, formyl, methylcarbonyl, ethylcarbonyl and trifluoromethylcarbonyl; wherein further $R^3$ may be hydrogen; wherein further when $R^1$ is ethyl or tertbutyl, then $R^2$ can be hydrogen; with the proviso that $R^1$ and $R^2$ cannot be fluoro simultaneously; and with the further proviso that $R^1$ and $R^2$ cannot be chloro simultaneously; wherein $R^4$ is selected from hydrogen, methyl, fluoro, chloro, monofluoromethyl, difluoromethyl, trifluoromethyl, formyl, carboxyl and dimethoxymethyl; wherein $R^5$ is selected from methyl, ethyl, n-propyl, isopropyl, n-butyl, sec-butyl, isobutyl, tert-butyl, n-pentyl, isopentyl, neopentyl, phenyl, benzyl, phenethyl, cyclopropyl, cyclobutyl, cyclopentyl, cyclohexyl, cyclopropylmethyl, cyclopropylethyl, cyclobutylmethyl, cyclobutylethyl, cyclopentylmethyl, cyclopentylethyl, cyclohexylmethyl and cyclohexylethyl; wherein $R^6$ is an acidic group selected from COOH and

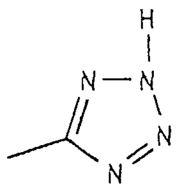

;

or a stereoisomer or a tautomer thereof or a pharmaceutically-acceptable salt thereof.

**15.** Compound of Claim 1 of Formula VII

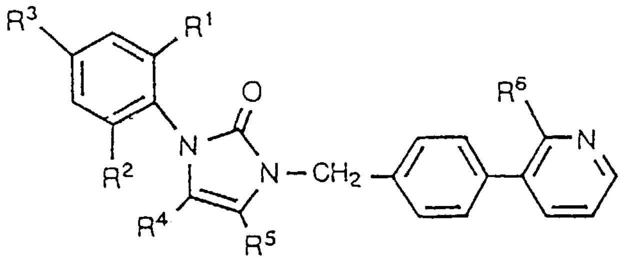 (VII)

wherein each of $R^1$, $R^2$ and $R^3$ is independently selected from methyl, ethyl, n-propyl, isopropyl, tert-butyl, methoxy, ethoxy, propoxy, isopropoxy, tert-butoxy, cyano, fluoro, chloro, iodo, bromo, hydroxy, nitro, amino, N-methylamino, N,N-dimethylamino, N-ethylamino, N,N-diethylamino, carboxyl, methoxycarbonyl, ethoxycarbonyl, formyl, methylcarbonyl, ethylcarbonyl and trifluoromethylcarbonyl; wherein further $R^3$ may be hydrogen; wherein further when $R^1$ is ethyl or tertbutyl, then $R^2$ can be hydrogen; with the proviso that $R^1$ and $R^2$ cannot be fluoro simultaneously; and with the further proviso that $R^1$ and $R^2$ cannot be chloro simultaneously; wherein $R^4$ is selected from hydrogen, methyl, fluoro, chloro, monofluoromethyl, difluoromethyl, trifluoromethyl, formyl, carboxyl and dimethoxymethyl; wherein $R^5$ is selected from methyl, ethyl, n-propyl, isopropyl, n-butyl, sec-butyl, isobutyl, tert-butyl, n-pentyl, isopentyl, neopentyl, phenyl, benzyl, phenethyl, cyclopropyl, cyclobutyl, cyclopentyl, cyclohexyl, cyclopropylmethyl, cyclopropylethyl, cyclobutylmethyl, cyclobutylethyl, cyclopentylmethyl, cyclopentylethyl, cyclohexylmethyl and cyclohexylethyl; wherein $R^6$ is an acidic group selected from COOH and

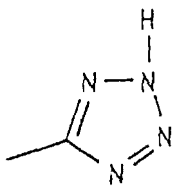 ;

or a stereoisomer or a tautomer thereof or a pharmaceutically-acceptable salt thereof.

**16.** A pharmaceutical composition comprising a therapeutically-effective, amount of an angiotensin II-antagonist compound and a pharmaceutically-acceptable carrier or diluent, said antagonist compound selected from a family of compounds according to any of claims 1 to 15.

**17.** Use of a compound according to any of claims 1 to 15 for preparing a medicament for treating a circulatory disorder or a circulatory-related disorder.

**18.** Use according to claim 17 wherein said circulatory disorder is an cardiovascular disorder.

**19.** Use according to claim 18 wherein said cardio-vascular disorder is hypertension.

**20.** Use according to claim 18 wherein said cardiovascular disorder is congestive heart failure.

**21.** Use according to claim 17 wherein said circulatory-related disorder is glaucoma.

**Patentansprüche**

**1.** Eine Verbindung der Formel I

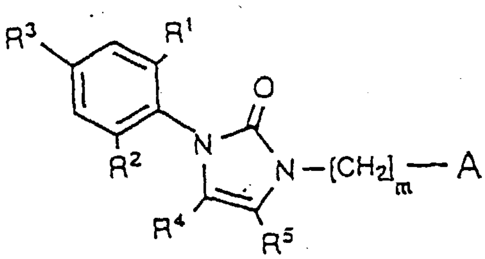

worin A ausgewählt ist aus

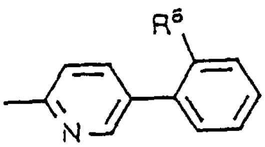

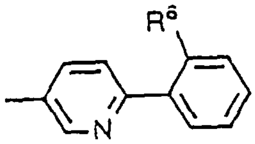

,

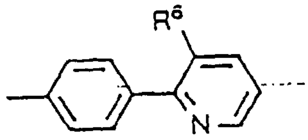

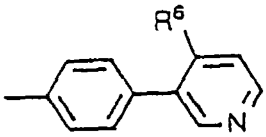

,

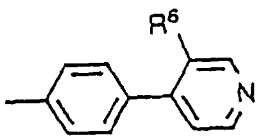

und

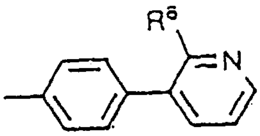

worin m eine Zahl ist ausgewählt aus eins bis einschließlich vier;

worin jeweils $R^1$, $R^2$ und $R^3$ unabhängig voneinander ausgewählt sind aus Alkyl, Alkoxy, Cyano, Halo, Hydroxy, Nitro, Amino, Carboxyl, Alkoxycarbonyl, Formyl, Alkylcarbonyl und Haloalkylcarbonyl; worin weiterhin $R^3$ Wasserstoff sein kann; worin weiterhin wenn $R^1$ Ethyl oder Tertbutyl ist $R^2$ Wasserstoff sein kann; mit der Maßgabe, daß $R^1$ und $R^2$ nicht gleichzeitig Fluor sein können und mit der weiteren Maßgabe, daß $R^1$ und $R^2$ nicht gleichzeitig Chlor sein können; worin $R^4$ ausgewählt ist aus Wasserstoff, Alkyl, Halo, Haloalkyl, Formyl, Carboxyl und Alkoxyalkyl; worin $R^5$ ausgewählt ist aus Alkylphenyl, Phenylalkyl, Cycloalkyl und Cycloalkylalkyl; worin $R^6$ eine Säuregruppe ist ausgewählt aus COOH und

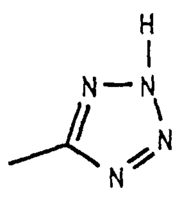

oder ein Stereoisomer oder ein Tautomer davon oder ein pharmazeutisch verträgliches Salz davon.

**2.** Verbindung nach Anspruch 1 der Formel II

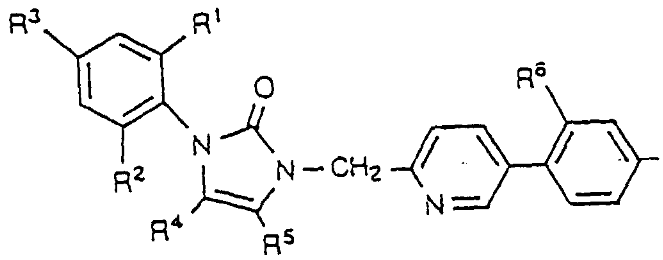

(II)

worin jeweils $R^1$, $R^2$ und $R^3$ unabhängig voneinander ausgewählt sind aus Methyl, Ethyl, n-Propyl, Isopropyl, tert-Butyl, Methoxy, Ethoxy, Propoxy, Isopropoxy, tert-Butoxy, Cyano, Fluor, Chlor, Iod, Brom, Hydroxy, Nitro, Amino, N-Methylamino, N,N-Dimethylamino, N-Ethylamino, N,N-Diethylamino, Carboxyl, Methoxycarbonyl, Ethoxycarbonyl, Formyl, Methylcarbonyl, Ethylcarbonyl und Trifluormethylcarbonyl; worin weiterhin $R^3$ Wasserstoff sein kann; worin weiterhin wenn $R^1$ Ethyl oder tert-Butyl ist, $R^2$ Wasserstoff sein kann; mit der Maßgabe, daß $R^1$ und $R^2$ nicht gleichzeitig Fluor sein können und mit der weiteren Maßgabe, daß $R^1$ und $R^2$ nicht gleichzeitig Chlor sein können; worin $R^4$ ausgewählt ist aus Wasserstoff, Methyl, Fluor, Chlor, Monofluormethyl, Difluormethyl, Trifluormethyl, Formyl, Carboxyl und Dimethoxymethyl; worin $R^5$ ausgewählt ist aus Methyl, Ethyl, n-Propyl, Isopropyl, n-Butyl, sec-Butyl, Isobutyl, tert-Butyl, n-Pentyl, Isopentyl, Neopentyl, Phenyl, Benzyl, Phenethyl, Cyclopropyl, Cyclobutyl, Cyclopentyl, Cyclohexyl, Cyclopropylmethyl, Cyclopropylethyl, Cyclobutylmethyl, Cyclobutylethyl, Cyclopentylmethyl, Cyclopentylethyl, Cyclohexylmethyl und Cyclohexylethyl; worin $R^6$ eine Säuregruppe ist ausgewählt aus COOH und

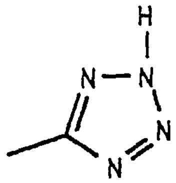

oder ein Stereoisomer oder ein Tautomer davon oder ein pharmazeutisch verträgliches Salz davon.

3. Verbindung nach Anspruch 1 der Formel III

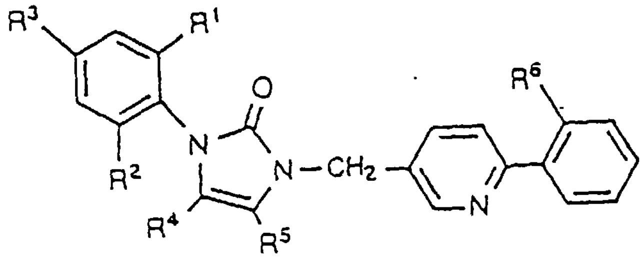

(III)

worin jeweils $R^1$, $R^2$ und $R^3$ unabhängig voneinander ausgewählt sind aus Methyl, Ethyl, n-Propyl, Isopropyl, tert-Butyl, Methoxy, Ethoxy, Propoxy, Isopropoxy, tert-Butoxy, Cyano, Fluor, Chlor, Iod, Brom, Hydroxy, Nitro, Amino, N-Methylamino, N,N-Dimethylamino, N-Ethylamino, N,N-Diethylamino, Carboxyl, Methoxycarbonyl, Ethoxycarbonyl, Formyl, Methylcarbonyl, Ethylcarbonyl und Trifluormethylcarbonyl; worin weiterhin $R^3$ Wasserstoff sein kann; worin weiterhin wenn $R^1$ Ethyl oder tert-Butyl ist, $R^2$ Wasserstoff sein kann; mit der Maßgabe, daß $R^1$ und $R^2$ nicht gleichzeitig Fluor sein können und mit der weiteren Maßgabe, daß $R^1$ und $R^2$ nicht gleichzeitig Chlor sein können; worin $R^4$ ausgewählt ist aus Wasserstoff, Methyl, Fluor, Chlor, Monofluormethyl, Difluormethyl, Trifluormethyl, Formyl, Carboxyl und Dimethoxymethyl; worin $R^5$ ausgewählt ist aus Methyl, Ethyl, n-Propyl, Isopropyl, n-Butyl, sec-Butyl, Isobutyl, tert-Butyl, n-Pentyl, Isopentyl, Neopentyl, Phenyl, Benzyl, Phenethyl, Cyclopropyl, Cyclobutyl, Cyclopentyl, Cyclohexyl, Cyclopropylmethyl, Cyclopropylethyl, Cyclobutylmethyl, Cyclobutylethyl, Cyclopentylmethyl, Cyclopentylethyl, Cyclohexylmethyl und Cyclohexylethyl; worin $R^6$ eine Säuregruppe ist ausgewählt aus COOH und

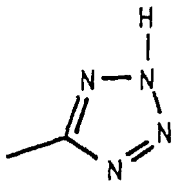

oder ein Stereoisomer oder ein Tautomer davon oder ein pharmazeutisch vertragliches Salz davon.

**4.** Verbindung nach Anspruch 3, nämlich 1-(2-Ethylphenyl)-4-butyl-1,3-dihydro-3-[[6-[2-(1H-tetrazol-5-yl)phenyl]-3-pyridinyl]methyl]-2H-imidazol-2-on oder ein pharmazeutisch verträgliches Salz davon.

**5.** Verbindung nach Anspruch 3, nämlich 1-(2-tertButylphenyl)-4-butyl-1,3-dihydro-3-[[6-[2-(1H-tetrazol-5-yl)phenyl]-3-pyridinyl]methyl]-2H-imidazol-2-on oder ein pharmazeutisch verträgliches Salz davon.

**6.** Verbindung nach Anspruch 3, nämlich 1-(2,6-Dimethoxyphenyl)-4-butyl-1,3-dihydro-3-[[6-[2-(1H-tetrazol-5-yl)phenyl]-3-pyridinyl]methyl]-2H-imidazol-2-on oder ein pharmazeutisch verträgliches Salz davon.

**7.** Verbindung nach Anspruch 3, nämlich 1-(2-Chlor-6-methylphenyl)-4-butyl-1,3-dihydro-3-[[6-[2-(1H-tetrazol-5-yl)phenyl]-3-pyridinyl]methyl]-2H-imidazol-2-on oder ein pharmazeutisch verträgliches Salz davon.

**8.** Verbindung nach Anspruch 3, nämlich 1-(2,4,6-Trimethylphenyl)-4-butyl-1,3-dihydro-3-[[6-[2-(1H-tetrazol-5-yl)phenyl]-3-pyridinyl]methyl]-2H-imidazol-2-on oder ein pharmazeutisch verträgliches Salz davon.

**9.** Verbindung nach Ansruch 3, nämlich 1-(2,6-Dimethyl-4-tertbutylphenyl)-4-butyl-1,3-dihydro-3-[[6-[2-(1H-tetrazol-5-yl)phenyl]-3-pyridinyl]methyl]-2H-imidazol-2-on oder ein pharmazeutisch verträgliches Salz davon.

**10.** Verbindung nach Anspruch 3, nämlich 1-(2,6-Diethylphenyl)-4-butyl-1,3-dihydro-3-[[6-[2-(1H-tetrazol-5-yl)phenyl]-3-pyridinyl]methyl]-2H-imidazol-2-on oder ein pharmazeutisch vertragliches Salz davon.

**11.** Verbindung nach Anspruch 3, nämlich 1-(2,6-Diisopropylphenyl)-4-butyl-1,3-dihydro-3-[[6-[2-(1H-tetrazol-5-yl)phenyl]-3-pyridinyl]methyl]-2H-imidazol-2-on oder ein pharmazeutisch vertragliches Salz davon.

**12.** Verbindung nach Anspruch 3 der Formel IV

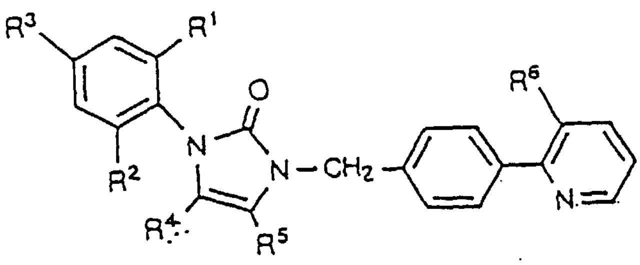

(IV)

worin jeweils $R^1$, $R^2$ und $R^3$ unabhängig voneinander ausgewählt sind aus Methyl, Ethyl, n-Propyl, Isopropyl, tert-Butyl, Methoxy, Ethoxy, Propoxy, Isopropoxy, tert-Butoxy, Cyano, Fluor, Chlor, Iod, Brom, Hydroxy, Nitro, Amino, N-Methylamino, N,N-Dimethylamino, N-Ethylamino, N,N-Diethylamino, Carboxyl, Methoxycarbonyl, Ethoxycarbonyl, Formyl, Methylcarbonyl, Ethylcarbonyl und Trifluormethylcarbonyl; worin weiterhin $R^3$ Wasserstoff sein kann; worin weiterhin wenn $R^1$ Ethyl oder tert-Butyl ist, $R^2$ Wasserstoff sein kann; mit der Maßgabe, daß $R^1$ und $R^2$ nicht gleichzeitig Fluor sein können und mit der weiteren Maßgabe, daß $R^1$ und $R^2$ nicht gleichzeitig Chlor sein können; worin $R^4$ ausgewählt ist aus Wasserstoff, Methyl, Fluor, Chlor, Monofluormethyl, Difluormethyl, Trifluormethyl, Formyl, Carboxyl und Dimethoxymethyl; worin $R^5$ ausgewählt ist aus Methyl, Ethyl, n-Propyl, Isopropyl, n-Butyl, sec-Butyl, Isobutyl, tert-Butyl, n-Pentyl, Isopentyl, Neopentyl, Phenyl, Benzyl, Phenethyl, Cyclopropyl, Cyclobutyl, Cyclopentyl, Cyclohexyl, Cyclopropylmethyl, Cyclopropylethyl, Cyclobutylmethyl, Cyclobutylethyl, Cyclopentylmethyl, Cyclopentylethyl, Cyclohexylmethyl und Cyclohexylethyl; worin $R^6$ eine Säuregruppe ist ausgewählt aus COOH und

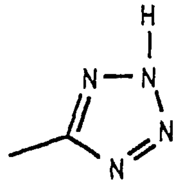

oder ein Stereoisomer oder ein Tautomer davon oder ein pharmazeutisch verträgliches Salz davon.

**13.** Verbindung nach Anspruch 1 der Formel V

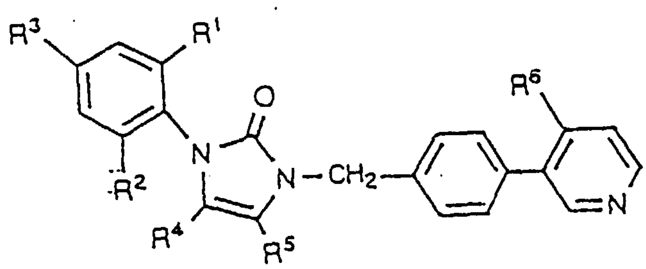

(V)

worin jeweils $R^1$, $R^2$ und $R^3$ unabhängig voneinander ausgewählt sind aus Methyl, Ethyl, n-Propyl, Isopropyl, tert-Butyl, Methoxy, Ethoxy, Propoxy, Isopropoxy, tert-Butoxy, Cyano, Fluor, Chlor, Iod, Brom, Hydroxy, Nitro, Amino, N-Methylamino, N,N-Dimethylamino, N-Ethylamino, N,N-Diethylamino, Carboxyl, Methoxycarbonyl, Ethoxycarbonyl, Formyl, Methylcarbonyl, Ethylcarbonyl und Trifluormethylcarbonyl; worin weiterhin $R^3$ Wasserstoff sein kann; worin weiterhin wenn $R^1$ Ethyl oder tert-Butyl ist, $R^2$ Wasserstoff sein kann; mit der Maßgabe, daß $R^1$ und $R^2$ nicht gleichzeitig Fluor sein können und mit der weiteren Maßgabe, daß $R^1$ und $R^2$ nicht gleichzeitig Chlor sein können; worin $R^4$ ausgewählt ist aus Wasserstoff, Methyl, Fluor, Chlor, Monofluormethyl, Difluormethyl, Trifluormethyl, Formyl, Carboxyl und Dimethoxymethyl; worin $R^5$ ausgewählt ist aus Methyl, Ethyl, n-Propyl, Isopropyl, n-Butyl, sec-Butyl, Isobutyl, tert-Butyl, n-Pentyl, Isopentyl, Neopentyl, Phenyl, Benzyl, Phenethyl, Cyclopropyl, Cyclobutyl, Cyclopentyl, Cyclohexyl, Cyclopropylmethyl, Cyclopropylethyl, Cyclobutylmethyl, Cyclobutylethyl, Cyclopentylmethyl, Cyclopentylethyl, Cyclohexylmethyl und Cyclohexylethyl; worin $R^6$ eine Säuregruppe ist ausgewählt aus COOH und

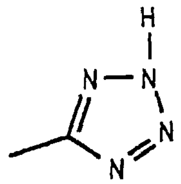

oder ein Stereoisomer oder ein Tautomer davon oder ein pharmazeutisch vertragliches Salz davon.

**14.** Verbindung nach Anspruch 1 der Formel VI

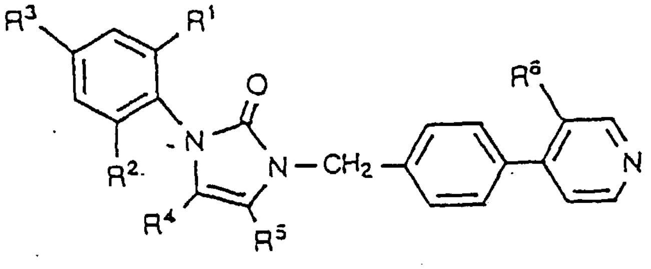

(VI)

worin jeweils $R^1$, $R^2$ und $R^3$ unabhängig voneinander ausgewählt ist aus Methyl, Ethyl, n-Propyl, Isopropyl, tert-Butyl, Methoxy, Ethoxy, Propoxy, Isopropoxy, tert-Butoxy, Cyano, Fluor, Chlor, Iod, Brom, Hydroxy, Nitro, Amino, N-Methylamino, N,N-Dimethylamino, N-Ethylamino, N,N-Diethylamino, Carboxyl, Methoxycarbonyl, Ethoxycarbonyl, Formyl, Methylcarbonyl, Ethylcarbonyl und Trifluormethylcarbonyl; worin weiterhin $R^3$ Wasserstoff sein kann; worin weiterhin wenn $R^1$ Ethyl oder tert-Butyl ist, $R^2$ Wasserstoff sein kann; mit der Maßgabe, daß $R^1$ und $R^2$ nicht gleichzeitig Fluor sein können und mit der weiteren Maßgabe, daß $R^1$ und $R^2$ nicht gleichzeitig Chlor sein können; worin $R^4$ ausgewählt ist aus Wasserstoff, Methyl, Fluor, Chlor, Monofluormethyl, Difluormethyl, Trifluormethyl, Formyl, Carboxyl und Dimethoxymethyl; worin $R^5$ ausgewählt ist aus Methyl, Ethyl, n-Propyl, Isopropyl, n-Butyl, sec-Butyl, Isobutyl, tert-Butyl, n-Pentyl, Isopentyl, Neopentyl, Phenyl, Benzyl, Phenethyl, Cyclopropyl, Cyclobutyl, Cyclopentyl, Cyclohexyl, Cyclopropylmethyl, Cyclopropylethyl, Cyclobutylmethyl, Cyclobutylethyl, Cyclopentylmethyl, Cyclopentylethyl, Cyclohexylmethyl und Cyclohexylethyl; worin $R^6$ eine Säuregruppe ist ausgewählt aus COOH und

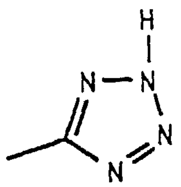

oder ein Stereoisomer oder ein Tautomer davon oder ein pharmazeutisch verträgliches Salz davon.

**15.** Verbindung nach Anspruch 1 der Forml VII

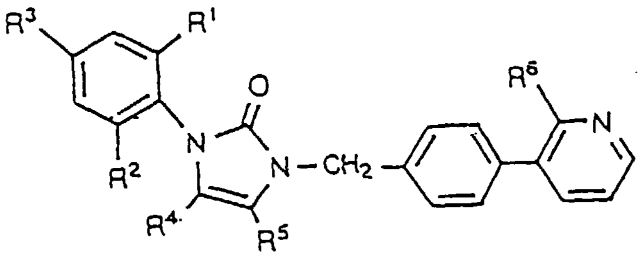

(VII)

worin jeweils $R^1$, $R^2$ und $R^3$ unabhängig voneinander ausgewählt ist aus Methyl, Ethyl, n-Propyl, Isopropyl, tert-Butyl, Methoxy, Ethoxy, Propoxy, Isopropoxy, tert-Butoxy, Cyano, Fluor, Chlor, Iod, Brom, Hydroxy, Nitro, Amino, N-Methylamino, N,N-Dimethylamino, N-Ethylamino, N,N-Diethylamino, Carboxyl, Methoxycarbonyl, Ethoxycarbonyl, Formyl, Methylcarbonyl, Ethylcarbonyl und Trifluormethylcarbonyl; worin weiterhin $R^3$ Wasserstoff sein kann; worin weiterhin wenn $R^1$ Ethyl oder tert-Butyl ist, $R^2$ Wasserstoff sein kann; mit der Maßgabe, daß $R^1$ und $R^2$ nicht gleichzeitig Fluor sein können und mit der weiteren Maßgabe, daß $R^1$ und $R^2$ nicht gleichzeitig Chlor sein können; worin $R^4$ ausgewählt ist aus Wasserstoff, Methyl, Fluor, Chlor, Monofluormethyl, Difluormethyl, Trifluormethyl, Formyl, Carboxyl und Dimethoxymethyl; worin $R^5$ ausgewählt ist aus Methyl, Ethyl, n-Propyl, Isopropyl, n-Butyl, sec-Butyl, Isobutyl, tert-Butyl, n-Pentyl, Isopentyl, Neopentyl, Phenyl, Benzyl, Phenethyl, Cyclopropyl, Cyclobutyl, Cyclopentyl, Cyclohexyl, Cyclopropylmethyl, Cyclopropylethyl, Cyclobutylmethyl, Cyclobutylethyl, Cyclopentylmethyl, Cyclopentylethyl, Cyclohexylmethyl und Cyclohexylethyl; worin $R^6$ eine Säuregruppe ist ausgewählt aus COOH und

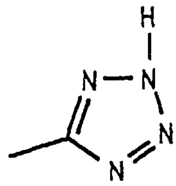

oder ein Stereoisomer oder ein Tautomer davon oder ein pharmazeutisch verträgliches Salz davon.

**16.** Eine pharmazeutische Zusammensetzung, enthaltend eine therapeutisch wirksame Menge einer Angiotensin-II-Antagonist Verbindung und einen pharmazeutisch verträglichen Träger oder eines Verdünnungsmittels, wobei diese Antagonist Verbindung ausgewählt ist aus einer Familie der Verbindungen gemäß einem der Ansprüche 1 bis 15.

**17.** Verwendung einer Verbindung gemäß einem der Ansprüche 1 bis 15 zur Herstellung eines Medikamentes zur Behandlung einer Kreislaufstörung oder einer kreislaufbedingten Störung.

**18.** Verwendung gemäß Anspruch 17, worin diese Kreislaufstörung eine cardiovasculäre Störung ist.

**19.** Verwendung gemäß Anspruch 18, worin diese cardiovasculäre Störung Bluthochdruck ist.

**20.** Verwendung gemäß Anspruch 18, worin diese cardiovasculäre Störung congestives Herzversagen ist.

**21.** Verwendung gemäß Anspruch 17, worin diese kreislaufbedingte Störung Glaucom ist.

**Revendications**

**1.** Composé de formule I

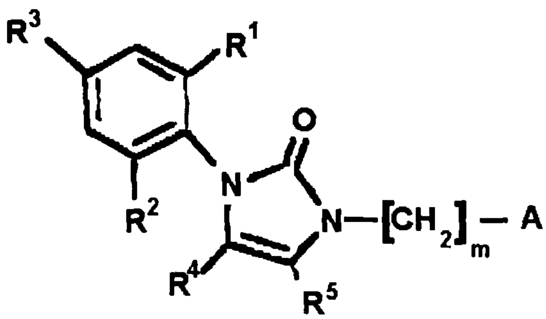

où A est choisi parmi

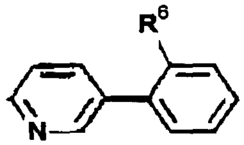
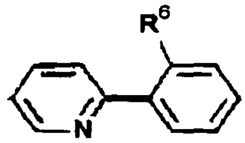
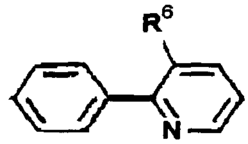

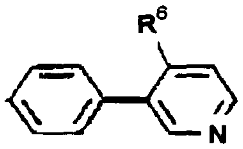
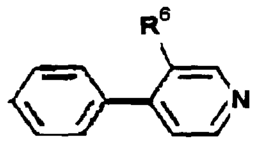
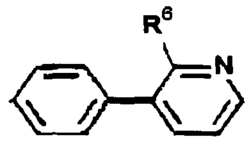

où m est un nombre choisi de un à quatre inclus ; où chacun de $R^1$, $R^2$ et $R^3$ est choisi indépendamment parmi les groupes alkyle, alcoxy, cyano, halogéno, hydroxy, nitro, amino, carboxyle, alcoxycarbonyle, formyle, alkylcarbonyle et halogénocarbonyle ; où de plus $R^3$ peut être un atome d'hydrogène ; où de plus lorsque $R^1$ est un groupe tert-butyle, alors $R^2$ peut être un atome d'hydrogène ; à la condition que $R^1$ et $R^2$ ne puissent être simultanément fluoro ; et à la condition de plus que $R^1$ et $R^2$ ne puissent être simultanément chloro ; où $R^4$ est choisi parmi l'atome d'hydrogène, les groupes alkyle, halogéno, halogénoalkyle, formyle, carboxyle et alcoxyalkyle ; où $R^5$ est choisi parmi les groupes alkylphényle, phénylalkyle, cycloalkyle et cycloalkylalkyle ; où $R^6$ est un groupe acide choisi parmi COOH et

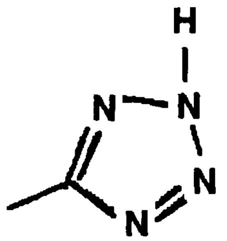 ;

ou bien un de ses stéréoisomères ou tautomères ou un de ses sels acceptables sur le plan pharmaceutique.

**2.** Composé selon la revendication 1, de formule II

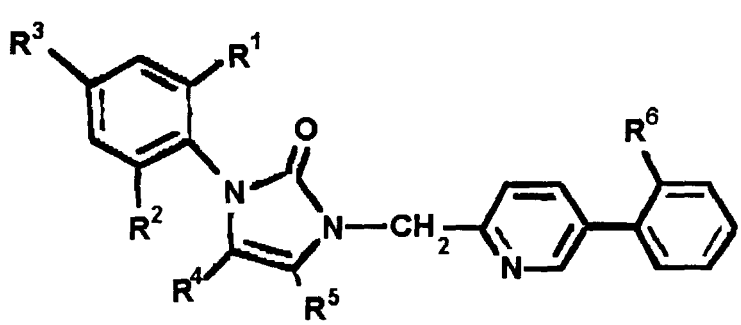 (II)

où chacun de $R^1$, $R^2$ et $R^3$ est choisi indépendamment parmi les groupes méthyle, éthyle, n-propyle, isopropyle, tert-butyle, méthoxy, éthoxy, propoxy, isopropoxy, tert-butoxy, cyano, fluoro, chloro, iodo, bromo, hydroxy,

nitro, amino, N-méthylamino, N,N-diméthylamino, N-éthylamino, N,N-diéthylamino, carboxyle, méthoxycarbonyle, éthoxycarbonyle, formyle, méthylcarbonyle, éthylcarbonyle, et trifluorométhyl-carbonyle ; où de plus $R^3$ peut être un atome d'hydrogène ; où de plus lorsque $R^1$ est un groupe éthyle ou tert-butyle, alors $R^2$ peut être un atome d'hydrogène ; à la condition que $R^1$ et $R^2$ ne puissent être simultanément fluoro ; et à la condition de plus que $R^1$ et $R^2$ ne puissent être simultanément chloro ; ou $R^4$ est choisi parmi l'atome d'hydrogène, les groupes méthyle, fluoro, chloro, monofluorométhyle, difluorométhyle, trifluorométhyle, formyle, carboxyle et diméthoxyméthyle ; où $R^5$ est choisi parmi les groupes méthyle, éthyle, n-propyle, isopropyle, n-butyle, sec-butyle, isobutyle, tert-butyle, n-pentyle, isopentyle, néopentyle, phényle, benzyle, phénéthyle, cyclopropyle, cyclobutyle, cyclopentyle, cyclohexyle, cyclopropylméthyle, cyclopropyléthyle, cyclobutylméthyle, cyclobutyléthyle, cyclopentylméthyle, cyclopentyléthyle, cyclohexylméthyle et cyclohexyléthyle ; où $R^6$ est un groupe acide choisi parmi COOH et

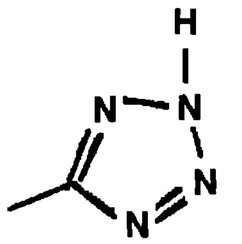 ;

ou bien un de ses stéréoisomères ou tautomères ou un de ses sels acceptables sur le plan pharmaceutique.

3. Composé selon la revendication 1, de formule III

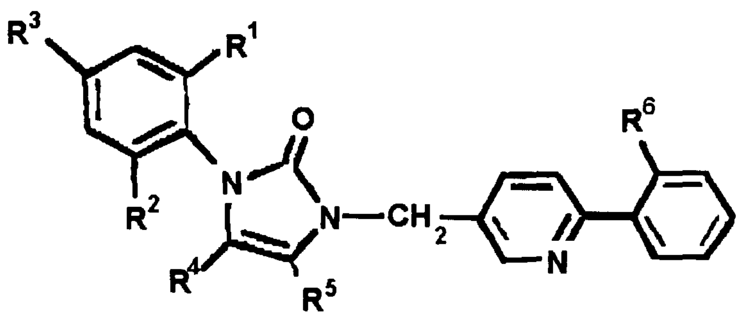

(III)

où chacun de $R^1$, $R^2$ et $R^3$ est choisi indépendamment parmi les groupes méthyle, éthyle, n-propyle, isopropyle, tert-butyle, méthoxy, éthoxy, propoxy, isopropoxy, tert-butoxy, cyano, fluoro, chloro, iodo, bromo, hydroxy, nitro, amino, N-méthylamino, N,N-diméthylamino, N-éthylamino, N,N-diéthylamino, carboxyle, méthoxycarbonyle, éthoxycarbonyle, formyle, méthylcarbonyle, éthylcarbonyle, et trifluorométhyl-carbonyle ; où de plus $R^3$ peut être un atome d'hydrogène ; où de plus lorsque $R^1$ est un groupe éthyle ou tert-butyle, alors $R^2$ peut être un atome d'hydrogène ; à la condition que $R^1$ et $R^2$ ne puissent être simultanément fluoro ; et à la condition de plus que $R^1$ et $R^2$ ne puissent être simultanément chloro ; où $R^4$ est choisi parmi l'atome d'hydrogène, les groupes méthyle, fluoro, chloro, monofluorométhyle, dufluorométhyle, trifluorométhyle, formyle, carboxyle et diméthoxyméthyle ; où $R^5$ est choisi parmi les groupes méthyle, éthyle, n-propyle, isopropyle, n-butyle, sec-butyle, isobutyle, tert-butyle, n-pentyle, isopentyle, néopentyle, phényle, benzyle, phénéthyle, cyclopropyle, cyclobutyle, cyclopentyle, cyclohexyle, cyclopropylméthyle, cyclopropyléthyle, cyclobutylméthyle, cyclobutyléthyle, cyclopentylméthyle, cyclopentyléthyle, cyclohexylméthyle et cyclohexyléthyle ; où $R^6$ est un groupe acide choisi parmi COOH et

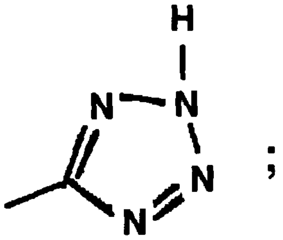

ou bien un de ses stéréoisomères ou tautomères ou un de ses sels acceptables sur le plan pharmaceutique.

4. Composé selon la revendication 3, qui est la 1-(2-éthylphényl)-4-butyl-1,3-dihydro-3-[[6-[2-(1H-tétrazol-5-yl)phényl]-3-pyridinyl]méthyl]-2H-imidazol-2-one ou un de ses sels acceptables sur le plan pharmaceutique.

5. Composé selon la revendication 3, qui est la 1-(2-tert-butylphényl)-4-butyl-1,3-dihydro-3-[[6-[2-(1H- tétrazol-5-yl)phényl]-3-pyridinyl]méthyl]-2H-imidazol-2-one ou un de ses sels acceptables sur le plan pharmaceutique.

6. Composé selon la revendication 3, qui est la 1-(2,6-diméthoxyphényl)-4-butyl-1,3-dihydro-3-[[6-[2-(1H-tétrazol-5-yl)phényl]-3-pyridinyl]méthyl]-2H-imidazol-2-one ou un de ses sels acceptables sur le plan pharmaceutique

7. Composé selon la revendication 3, qui est la 1-(2-chloro-6-méthylphényl)-4-butyl-1,3-dihydro-3-[[6-[2-(1H-tétrazol-5-yl)phényl]-3-pyridinyl]méthyl]-2H-imidazol-2-one ou un de ses sels acceptables sur le plan pharmaceutique.

8. Composé selon la revendication 3, qui est la 1-(2,4,6-triméthylphényl)-4-butyl-1,3-dihydro-3-[[6-[2-(1H-tétrazol-5-yl)phényl]-3-pyridinyl]méthyl]-2H-imidazol-2-one ou un de ses sels acceptables sur le plan pharmaceutique.

9. Composé selon la revendication 3, qui est la 1-(2,6-diméthyl-4-tert-butyl-phényl)-4-butyl-1,3-dihydro-3-[[6-[2-(1H-tétrazol-5-yl)phényl]-3-pyridinyl]méthyl]-2H-imidazol-2-one ou un de ses sels acceptables sur le plan pharmaceutique.

10. Composé selon la revendication 3, qui est la 1-(2,6-diéthylphényl)-4-butyl-1,3-dihydro-3-[[6-[2-(1H-tétrazol-5-yl)phényl]-3-pyridinyl]méthyl]-2H-imidazol-2-one ou un de ses sels acceptables sur le plan pharmaceutique.

11. Composé selon la revendication 3, qui est la 1-(2,6-diisopropylphényl)-4-butyl-1,3-dihydro-3-[[6-[2-(1H-tétrazol-5-yl)phényl]-3-pyridinyl]méthyl]-2H-imidazol-2-one ou un de ses sels acceptables sur le plan pharmaceutique.

12. Composé selon la revendication 3, de formule IV

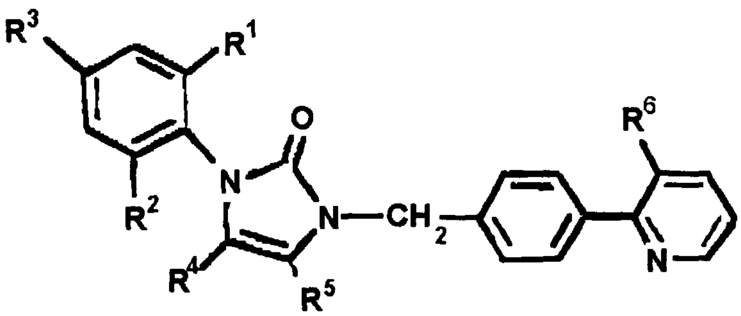

(IV)

où chacun de $R^1$, $R^2$ et $R^3$ est choisi indépendamment parmi les groupes méthyle, éthyle, n-propyle, isopropyle, tert-butyle, méthoxy, éthoxy, propoxy, isopropoxy, tert-butoxy, cyano, fluoro, chloro, iodo, bromo, hydroxy,

nitro, amino, N-méthylamino, N,N-diméthylamino, N-éthylamino, N,N-diéthylamino, carboxyle, méthoxycarbonyle, éthoxycarbonyle, formyle, méthylcarbonyle, éthylcarbonyle, et trifluorométhyl-carbonyle ; où de plus $R^3$ peut être un atome d'hydrogène ; où de plus lorsque $R^1$ est un groupe éthyle ou tert-butyle, alors $R^2$ peut être un atome d'hydrogène ; à la condition que $R^1$ et $R^2$ ne puissent être simultanément fluoro ; et à la condition de plus que $R^1$ et $R^2$ ne puissent être simultanément chloro ; où $R^4$ est choisi parmi l'atome d'hydrogène, les groupes méthyle, fluoro, chloro, monofluorométhyle, difluorométhyle, trifluorométhyle, formyle, carboxyle et diméthoxyméthyle ; où $R^5$ est choisi parmi les groupes méthyle, éthyle, n-propyle, isopropyle, n-butyle, sec-butyle, isobutyle, tert-butyle, n-pentyle, isopentyle, néopentyle, phényle, benzyle, phénéthyle, cyclopropyle, cyclobutyle, cyclopentyle, cyclohexyle, cyclopropylméthyle, cyclopropyléthyle, cyclobutylméthyle, cyclobutyléthyle, cyclopentylméthyle, cyclopentyléthyle, cyclohexylméthyle et cyclohexyléthyle ; où $R^6$ est un groupe acide choisi parmi COOH et

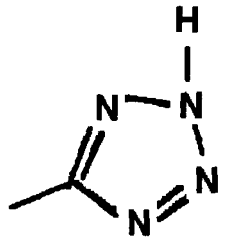 ;

ou bien un de ses stéréoisomères ou tautomères ou un de ses sels acceptables sur le plan pharmaceutique.

**13.** Composé selon la revendication 1, de formule V

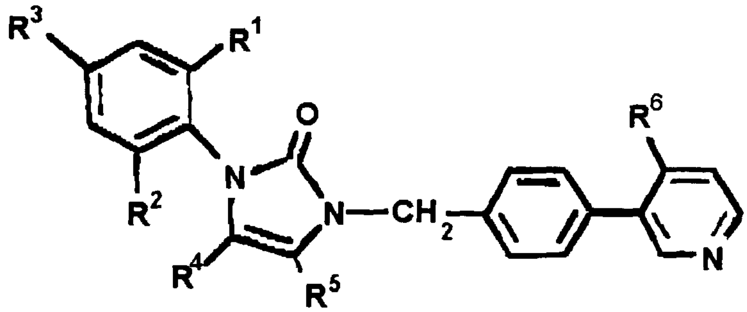 (V)

où chacun de $R^1$, $R^2$ et $R^3$ est choisi indépendamment parmi les groupes méthyle, éthyle, n-propyle, isopropyle, tert-butyle, méthoxy, éthoxy, propoxy, isopropoxy, tert-butoxy, cyano, fluoro, chloro, iodo, bromo, hydroxy, nitro, amino, N-méthylamino, N,N-diméthylamino, N-éthylamino, N,N-diéthylamino, carboxyle, méthoxycarbonyle, éthoxycarbonyle, formyle, méthylcarbonyle, éthylcarbonyle, et trifluorométhyl-carbonyle ; où de plus $R^3$ peut être un atome d'hydrogène ; où de plus lorsque $R^1$ est un groupe éthyle ou tert-butyle, alors $R^2$ peut être un atome d'hydrogène ; à la condition que $R^1$ et $R^2$ ne puissent être simultanément fluoro ; et à la condition de plus que $R^1$ et $R^2$ ne puissent être simultanément chloro ; où $R^4$ est choisi parmi l'atome d'hydrogène, les groupes méthyle, fluoro, chloro, monofluorométhyle, difluorométhyle, trifluorométhyle, formyle, carboxyle et diméthoxyméthyle ; ou $R^5$ est choisi parmi les groupes méthyle, éthyle, n-propyle, isopropyle, n-butyle, sec-butyle, isobutyle, tert-butyle, n-pentyle, isopentyle, néopentyle, phényle, benzyle, phénéthyle, cyclopropyle, cyclobutyle, cyclopentyle, cyclohexyle, cyclopropylméthyle, cyclopropyléthyle, cyclobutylméthyle, cyclobutyléthyle, cyclopentylméthyle, cyclopentyléthyle, cyclohexylméthyle et cyclohexyléthyle ; où $R^6$ est un groupe acide choisi parmi COOH et

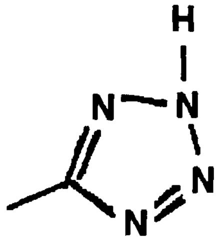 ;

ou bien un de ses stéréoisomères ou tautomères ou un de ses sels acceptables sur le plan pharmaceutique.

**14.** Composé selon la revendication 1, de formule VI

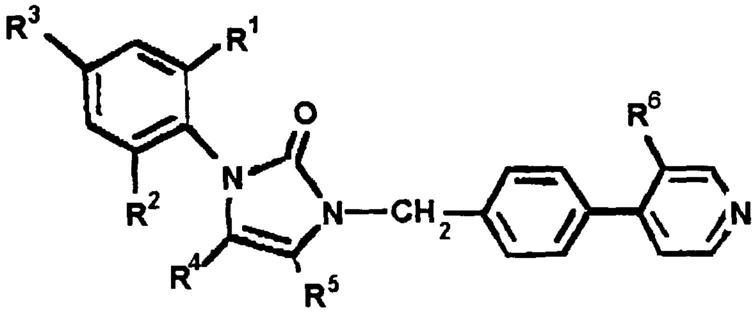 (VI)

où chacun de $R^1$, $R^2$ et $R^3$ est choisi indépendamment parmi les groupes méthyle, éthyle, n-propyle, isopropyle, tert-butyle, méthoxy, éthoxy, propoxy, isopropoxy, tert-butoxy, cyano, fluoro, chloro, iodo, bromo, hydroxy, nitro, amino, N-méthylamino, N,N-diméthylamino, N-éthylamino, N,N-diéthylamino, carboxyle, méthoxycarbonyle, éthoxycarbonyle, formyle, méthylcarbonyle, éthylcarbonyle, et trifluorométhyl-carbonyle ; où de plus $R^3$ peut être un atome d'hydrogène ; où de plus lorsque $R^1$ est un groupe éthyle ou tert-butyle, alors $R^2$ peut être un atome d'hydrogène ; à la condition que $R^1$ et $R^2$ ne puissent être simultanément fluoro ; et à la condition de plus que $R^1$ et $R^2$ ne puissent être simultanément chloro ; ou $R^4$ est choisi parmi l'atome d'hydrogène, les groupes méthyle, fluoro, chloro, monofluorométhyle, difluorométhyle, trifluorométhyle, formyle, carboxyle et diméthoxyméthyle ; où $R^5$ est choisi parmi les groupes méthyle, éthyle, n-propyle, isopropyle, n-butyle, sec-butyle, isobutyle, tert-butyle, n-pentyle, isopentyle, néopentyle, phényle, benzyle, phénéthyle, cyclopropyle, cyclobutyle, cyclopentyle, cyclohexyle, cyclopropylméthyle, cyclopropyléthyle, cyclobutylméthyle, cyclobutyléthyle, cyclopentylméthyle, cyclopentyléthyle, cyclohexylméthyle et cyclohexyléthyle ; où $R^6$ est un groupe acide choisi parmi COOH et

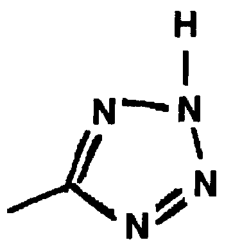 ;

ou bien un de ses stéréoisomères ou tautomères ou un de ses sels acceptables sur le plan pharmaceutique.

**15.** Composé selon la revendication 1, de formule VII

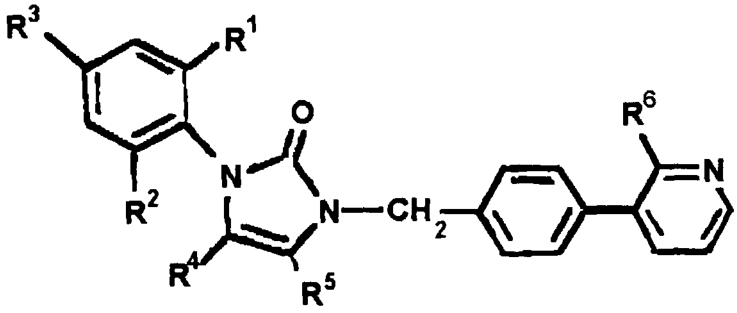 (VII)

où chacun de $R^1$, $R^2$ et $R^3$ est choisi indépendamment parmi les groupes méthyle, éthyle, n-propyle, isopropyle, tert-butyle, méthoxy, éthoxy, propoxy, isopropoxy, tert-butoxy, cyano, fluoro, chloro, iodo, bromo, hydroxy, nitro, amino, N-méthylamino, N,N-diméthylamino, N-éthylamino, N,N-diéthylamino, carboxyle, méthoxycarbonyle, éthoxycarbonyle, formyle, méthylcarbonyle, éthylcarbonyle, et trifluorométhyl-carbonyle ; où de plus $R^3$ peut être un atome d'hydrogène ; où de plus lorsque $R^1$ est un groupe éthyle ou tert-butyle, alors $R^2$ peut être un atome d'hydrogène ; à la condition que $R^1$ et $R^2$ ne puissent être simultanément fluoro ; et à la condition de plus que $R^1$ et $R^2$ ne puissent être simultanément chloro ; où $R^4$ est choisi parmi l'atome d'hydrogène, les groupes méthyle, fluoro, chloro, monofluorométhyle, difluorométhyle, trifluorométhyle, formyle, carboxyle et diméthoxyméthyle ; où $R^5$ est choisi parmi les groupes méthyle, éthyle, n-propyle, isopropyle, n-butyle, sec-butyle, isobutyle, tert-butyle, n-pentyle, isopentyle, néopentyle, phényle, benzyle, phénéthyle, cyclopropyle, cyclobutyle, cyclopentyle, cyclohexyle, cyclopropylméthyle, cyclopropyléthyle, cyclobutylméthyle, cyclobutyléthyle, cyclopentylméthyle, cyclopentyléthyle, cyclohexylméthyle et cyclohexyléthyle ; où $R^6$ est un groupe acide choisi parmi COOH et

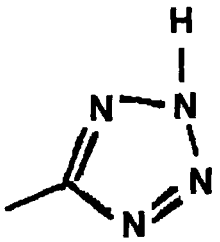 ;

ou bien un de ses stéréoisomères ou tautomères ou un de ses sels acceptables sur le plan pharmaceutique.

**16.** Composition pharmaceutique comprenant une quantité efficace sur le plan thérapeutique d'un composé antagoniste envers l'angiotensine II et d'un véhicule ou diluant acceptable sur le plan pharmaceutique, ledit composé antagoniste étant choisi dans une famille de composés selon l'une quelconque des revendications 1 à 15.

**17.** Utilisation d'un composé selon l'une quelconque des revendications 1 à 15, pour préparer un médicament destiné au traitement d'un trouble circulatoire or un trouble lié à la circulation.

**18.** Utilisation selon la revendication 17, dans laquelle le trouble circulatoire est un trouble cardio-vasculaire.

**19.** Utilisation selon la revendication 18, dans laquelle ledit trouble cardio-vasculaire est l'hypertension.

**20.** Utilisation selon la revendication 18, dans laquelle ledit trouble cardio-vasculaire est l'insuffisance cardiaque congestive.

**21.** Utilisation selon la revendication 17, dans laquelle ledit trouble lié à la circulation est un glaucome.